(19) Europäisches Patentamt / European Patent Office / Office européen des brevets

(11) EP 4 118 696 B1

(12) # EUROPÄISCHE PATENTSCHRIFT

(45) Veröffentlichungstag und Bekanntmachung des Hinweises auf die Patenterteilung:
**20.12.2023 Patentblatt 2023/51**

(21) Anmeldenummer: **21709416.8**

(22) Anmeldetag: **08.03.2021**

(51) Internationale Patentklassifikation (IPC):
***C07D 209/56*** *(2006.01)* ***H10K 71/12*** *(2023.01)*
***H10K 85/30*** *(2023.01)* ***H10K 85/60*** *(2023.01)*
*H10K 50/11* *(2023.01)*

(52) Gemeinsame Patentklassifikation (CPC):
**C07D 405/04; C07D 405/10; C07D 405/14; C07D 409/04; C07D 409/14; C07D 487/04; C07D 487/06; C07D 495/04; H10K 71/12; H10K 85/342; H10K 85/654; H10K 85/6572; H10K 85/6574; H10K 85/6576;** H10K 50/11;

(Forts.)

(86) Internationale Anmeldenummer:
**PCT/EP2021/055717**

(87) Internationale Veröffentlichungsnummer:
**WO 2021/180614 (16.09.2021 Gazette 2021/37)**

(54) **ORGANISCHE ELEKTROLUMINESZIERENDE VORRICHTUNG**

ORGANIC ELECTROLUMINESCENT APPARATUS

APPAREIL ÉLECTROLUMINESCENT ORGANIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.03.2020 EP 20162382**

(43) Veröffentlichungstag der Anmeldung:
**18.01.2023 Patentblatt 2023/03**

(73) Patentinhaber: **Merck Patent GmbH**
**64293 Darmstadt (DE)**

(72) Erfinder:
- **PARHAM, Amir Hossain**
  **64293 DARMSTADT (DE)**
- **KROEBER, Jonas Valentin**
  **64293 DARMSTADT (DE)**
- **ENGELHART, Jens**
  **64293 DARMSTADT (DE)**
- **EHRENREICH, Christian**
  **64293 DARMSTADT (DE)**
- **EICKHOFF, Christian**
  **64293 DARMSTADT (DE)**

(56) Entgegenhaltungen:
**KR-A- 20170 113 320 US-A1- 2015 333 273**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
H10K 2101/10; H10K 2101/90; Y02E 10/549

## Beschreibung

Die vorliegende Erfindung betrifft eine organische elektrolumineszierende Vorrichtung enthaltend eine Mischung, die ein elektronentransportierendes Hostmaterial und ein lochtransportierendes Hostmaterial umfasst, sowie eine Formulierung enthaltend eine Mischung der Hostmaterialien und eine Mischung enthaltend die Hostmaterialien. Das elektronentransportierende Hostmaterial entspricht einer Verbindung der Formel (1) aus der Klasse von Verbindungen enthaltend eine durch ein Dibenzofuran oder Dibenzothiophen substituierte Pyridin,- Pyrimidin- oder Triazin-Einheit.

Der Aufbau organischer Elektrolumineszenzvorrichtungen (z.B. OLEDs - organic light emitting diodes oder OLECs - organic light emitting electrochemical cells), in denen organische Halbleiter als funktionelle Materialien eingesetzt werden, ist seit Langem bekannt. Als emittierende Materialien werden hierbei neben fluoreszierenden Emittern zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfach gesteigerte Energie- und Leistungseffizienz möglich. Generell gibt es bei OLEDs, insbesondere auch bei OLEDs die Triplettemission (Phosphoreszenz) zeigen, jedoch immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung, und davon insbesondere die Host- bzw. Matrixmaterialien. Verbesserungen dieser Materialien können zu deutlichen Verbesserungen elektrolumineszierender Vorrichtungen führen.

Hostmaterialien zur Verwendung in organischen elektronischen Vorrichtungen sind dem Fachmann gut bekannt. Im Stand der Technik wird häufig auch der Begriff Matrixmaterial verwendet, wenn ein Hostmaterial für phosphoreszierende Emitter gemeint ist. Diese Verwendung des Begriffs gilt auch für die vorliegende Erfindung. Mittlerweile wurde eine Vielzahl von Hostmaterialien sowohl für fluoreszierende als auch für phosphoreszierende elektronische Vorrichtungen entwickelt.

Eine weitere Möglichkeit, die Leistungsdaten elektronischer Vorrichtungen, insbesondere von organischen Elektrolumineszenzvorrichtungen, zu verbessern, besteht darin, Kombinationen aus zwei oder mehr Materialien, insbesondere Hostmaterialien bzw. Matrixmaterialien, zu verwenden.

In US 6,392,250 B1 wird die Verwendung einer Mischung bestehend aus einem Elektronentransportmaterial, einem Lochtransportmaterial und einem fluoreszierenden Emitter in der Emissionsschicht einer OLED offenbart. Mit Hilfe dieser Mischung konnte die Lebensdauer der OLED gegenüber dem Stand der Technik verbessert werden.

In US 6,803,720 B1 wird die Verwendung einer Mischung enthaltend einen phosphoreszierenden Emitter sowie ein Loch- und ein Elektronentransportmaterial in der Emissionsschicht einer OLED offenbart. Dabei sind sowohl das Loch- und das Elektrontransportmaterial kleine organische Moleküle.

In WO2011088877 werden spezielle heterocyclische Verbindungen beschrieben, die in einer organischen lichtemittierenden Vorrichtung als lichtemittierende Verbindung, als Hostmaterial oder lochtransportierendem Material eingesetzt werden können.

Gemäß WO2015156587 können spezielle Carbazol-Derivate in einer Mischung mit Biscarbazolen als Hostmaterialien verwendet werden.

Gemäß WO2015169412 können beispielsweise Triazin-Dibenzofuran-Carbazolderivate und Triazin-Dibenzothiophen-Carbazolderivate in einer lichtemittierenden Schicht als Hostmaterial verwendet werden.

In US9771373 werden spezielle Carbazolderivate als Hostmaterial für eine lichtemittierende Schicht einer elektrolumineszierenden Vorrichtung beschrieben, die zusammen mit einem weiteren Hostmaterial eingesetzt werden können.

In KR20160046077 werden spezielle Triazin-Dibenzofuran-Carbazol- und Triazin-Dibenzothiophen-Carbazol-Derivate in einer lichtemittierenden Schicht zusammen mit einem weiteren Hostmaterial und einem speziellen Emitter beschrieben. Das Carbazol ist hierbei über das N-Atom an die Dibenzofuran bzw. Dibenzothiophen-Einheit gebunden.

In WO2016015810 werden Triazin-Dibenzofuran-Carbazol- und Triazin-Dibenzothiophen-Carbazol-Verbindungen beschrieben, wobei der Triazinsubstituent direkt oder über einen Linker in 1-Position des Dibenzofurans/Dibenzothiophens gebunden ist, und wobei der Carbazolsubstituent über sein N-Atom in 8-Position des Dibenzofurans/Dibenzothiophens gebunden ist. Die beschriebenen Verbindungen können in einer Mischung mit einem weiteren Matrixmaterial verwendet werden. In KR2018010149 werden ähnliche Verbindungen beschrieben. In WO2018174678 und WO2018174679 werden Vorrichtungen offenbart, enthaltend in einer organischen Schicht eine Mischung von Carbazol-Dibenzofuran-Derivaten mit Biscarbazolen, wobei die Verknüpfung der Carbazol-Einheit über das N-Atom an das Dibenzofurangerüst an jeder Position des Dibenzofurans möglich ist.

In KR20170113318 werden spezielle heterocyclische Verbindungen beschrieben, die als Hostmaterial in einer lichtemittierenden Schicht einer organischen lichtemittierenden Vorrichtung eingesetzt werden können.

In KR20170113320 werden spezielle Dibenzofuranderivate beschrieben, die als Hostmaterial in einer lichtemittierenden Schicht einer organischen lichtemittierenden Vorrichtung eingesetzt werden können.

In CN107973786 werden Triazin-Dibenzofuran-Carbazol- und Triazin-Dibenzothiophen-Carbazol-Verbindungen beschrieben. Der Triazinsubstituent ist direkt oder über einen Linker in 1-Position des Dibenzofurans/Dibenzothiophens gebunden. Das Carbazolderivat ist über das N-Atom direkt oder über einen Linker in 6-Position des Dibenzofurans/Dibenzothiophens gebunden. Es wird weiter berichtet, dass diese Materialien mit einem Biscarbazol H2 in einem Verhältnis 10:90 bis 90:10 gemischt werden können.

In US20190006590 wird eine elektronische Vorrichtung beschrieben, enthaltend eine spezielle Abfolge von zwei emittierenden Schichten, wobei jede emittierende Schicht zwei Hostmaterialien enthält. Die erste emittierende Schicht enthält Host 1-1 und Host 1-2. Die zweite emittierende Schicht enthält Host 2-1 und Host 2-2, wobei Host 1-2 und Host 2-1 die gleichen Materialien sind. Ansprüche 7 und 8 beschreiben spezielle Biscarbazole als Hostmaterial 1-2. Die Ansprüche 9 und 10 beschreiben spezielle Triazinderivate als Hostmaterial 2-2.

In US2019047991 werden doppelt substituierte Triazin-Dibenzofuranderivate beschrieben und deren Verwendung als organisches Material in einer organischen lichtemittierenden Vorrichtung.

In WO19031679 werden organische lichtemittierende Vorrichtungen beschrieben, enthaltend in der emittierenden Schicht ein erstes Hostmaterial enthaltend doppelt substituiertes Triazin-Dibenzofuranderivat und ein zweites Hostmaterial.

In WO19007866 werden Zusammensetzungen beschrieben, die einen elektronentransportierenden Host und einen lochtransportierenden Host umfasst, wobei der lochtransportierende Host ein Biscarbazol bedeutet.

In WO2020022860 werden organische lichtemittierende Vorrichtungen beschrieben, enthaltend in der emittierenden Schicht ein deuteriertes Triazinderivat und ein Biscarbazolderivat.

In US2015/333273 (A) und KR20170113320 (A) werden organische lichtemittierende Vorrichtungen beschrieben, enthaltend in der emittierenden Schicht ein erstes Hostmaterial und ein zweites Hostmaterial.

Allerdings besteht bei Verwendung dieser Materialien oder bei der Verwendung von Mischungen der Materialien noch Verbesserungsbedarf, insbesondere in Bezug auf Effizienz, Betriebsspannung und/oder Lebensdauer der organischen elektrolumineszierenden Vorrichtung. Aufgabe der vorliegenden Erfindung ist daher die Bereitstellung einer Kombination von Hostmaterialien, welche sich für den Einsatz in einer organischen elektrolumineszierenden Vorrichtung, insbesondere in einer fluoreszierenden oder phosphoreszierenden OLED eignen und zu guten Device-Eigenschaften insbesondere im Hinblick auf eine verbesserte Lebensdauer, führen, sowie die Bereitstellung der entsprechenden elektrolumineszierenden Vorrichtung.

Es wurde nun gefunden, dass die Kombination mindestens einer Verbindung der Formel (1) als erstes Hostmaterial und mindestens einer lochtransportierenden Verbindung der Formel (2) als zweites Hostmaterial in einer Licht emittierenden Schicht einer organischen elektrolumineszierenden Vorrichtung, diese Aufgabe lösen und die Nachteile aus dem Stand der Technik beseitigen. Die Verwendung einer derartigen Materialkombination zur Herstellung der lichtemittierenden Schicht in einer organischen elektrolumineszierenden Vorrichtung führt zu sehr guten Eigenschaften dieser Vorrichtungen, insbesondere hinsichtlich der Lebensdauer, insbesondere bei gleicher oder verbesserter Effizienz und/oder Betriebsspannung. Die Vorteile zeigen sich insbesondere auch bei Anwesenheit einer lichtemittierenden Komponente in der Emissionsschicht, insbesondere bei Kombination mit Emittern der Formel (III), bei Konzentrationen zwischen 2 und 15 Gew.-%.

Ein erster Gegenstand der vorliegenden Erfindung ist daher eine organische elektrolumineszierende Vorrichtung umfassend eine Anode, eine Kathode und mindestens eine organische Schicht, enthaltend mindestens eine lichtemittierende Schicht, wobei die mindestens eine lichtemittierende Schicht mindestens eine Verbindung der Formel (1) als Hostmaterial 1 und mindestens eine Verbindung der Formel (2) als Hostmaterial 2 enthält,

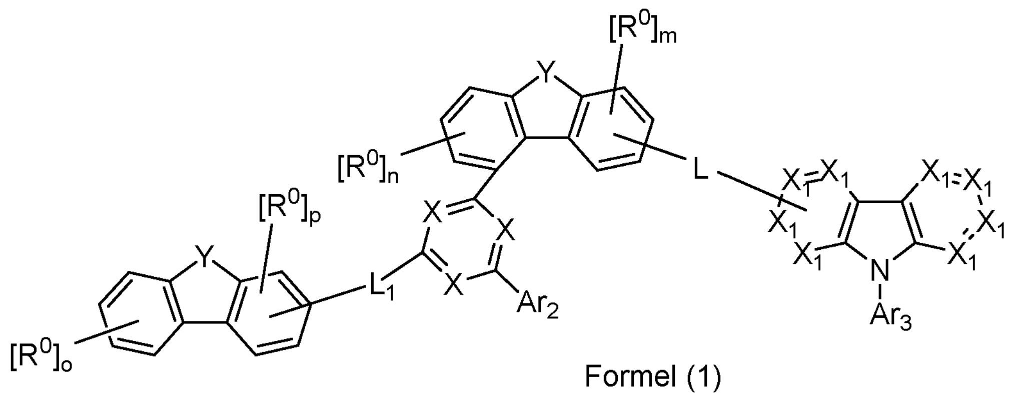

Formel (1)

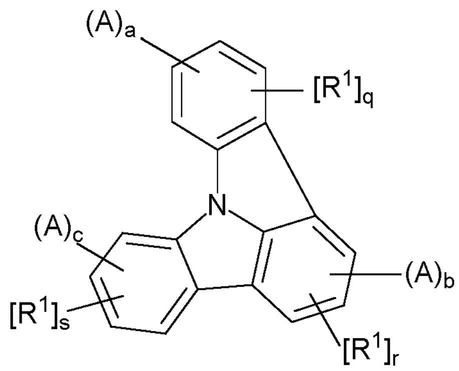

Formel (2) ,

wobei für die verwendeten Symbole und Indizes gilt:

X ist bei jedem Auftreten gleich oder verschieden $CR^0$ oder N, wobei mindestens ein Symbol X für N steht;

$X_1$ ist bei jedem Auftreten gleich oder verschieden CH, CR oder N, wobei maximal 3 Symbole $X_1$ N bedeuten können;

$X_2$ ist bei jedem Auftreten gleich oder verschieden CH, $CR^1$ oder N, wobei maximal 2 Symbole $X_2$ N bedeuten können;

Y ist bei jedem Auftreten gleich oder verschieden ausgewählt aus O oder S;

L und $L_1$ sind bei jedem Auftreten gleich oder verschieden eine Einfachbindung oder ein aromatisches oder heteroaromatisches Ringsystem mit 5 bis 30 aromatischen Ringatomen;

$R^0$ ist bei jedem Auftreten unabhängig voneinander H, D oder ein unsubstituiertes oder teilweise oder vollständig deuteriertes aromatisches Ringsystem mit 6 bis 18 C-Atomen;

R und R# sind bei jedem Auftreten gleich oder verschieden ausgewählt aus der Gruppe bestehend aus D, F, Cl, Br, I, CN, $NO_2$, $C(=O)R^2$, $P(=O)(Ar_1)_2$, $P(Ar_1)_2$, $B(Ar_1)_2$, $Si(Ar_1)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C=CR^2$, $Si(R^2)_2$, C=O, C=S, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 aromatischen Ringatomen, das jeweils mit einem oder mehreren Resten $R^2$ substituiert sein kann, einer Aryloxy- oder Heteroaryloxygruppe mit 5 bis 40 aromatischen Ringatomen, die mit einem oder mehreren Resten $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann; dabei können zwei Substituenten R, die an dasselbe Kohlenstoffatom oder an benachbarte Kohlenstoffatome gebunden sind oder ein Substituent R zusammen mit $Ar_3$ ein monocyclisches oder polycyclisches, aliphatisches, aromatisches oder heteroaromatisches Ringsystem bilden, das mit einem oder mehreren Resten $R^2$ substituiert sein kann;

$R^1$ ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen; dabei können zwei Substituenten $R^1$, die an dasselbe Kohlenstoffatom oder an benachbarte Kohlenstoffatome gebunden sind ein monocyclisches oder polycyclisches, aliphatisches, aromatisches oder heteroaromatisches Ringsystem bilden, das mit einem oder mehreren Resten $R^2$ substituiert sein kann;

$R^2$ ist bei jedem Auftreten gleich oder verschieden ausgewählt aus der Gruppe bestehend aus H, D, F, Cl, Br, I, CN, $NO_2$, $N(Ar_1)_2$, $NH_2$, $N(R^3)_2$, $C(=O)Ar_1$, C(=O)H, $C(=O)R^3$, $P(=O)(Ar_1)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, wobei eine oder mehrere nichtbenachbarte $CH_2$-Gruppen durch HC=CH, $R^3C=CR^3$, C=C, $Si(R^3)_2$, $Ge(R^3)_2$, $Sn(R^3)_2$, C=O, C=S, C=Se,

$C=NR^3$, $P(=O)(R^3)$, SO, $SO_2$, NH, $NR^3$, O, S, CONH oder $CONR^3$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 aromatischen Ringatomen, das jeweils mit einem oder mehreren Resten $R^3$ substituiert sein kann, einer Aryloxy- oder Heteroaryloxygruppe mit 5 bis 60 aromatischen Ringatomen, die mit einem oder mehreren Resten $R^3$ substituiert sein kann, oder einer Kombination dieser Systeme, wobei optional zwei oder mehr benachbarte Substituenten $R^2$ ein monocyclisches oder polycyclisches, aliphatisches, aromatisches oder heteroaromatisches Ringsystem bilden können, das mit einem oder mehreren Resten $R^3$ substituiert sein kann;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^3$ miteinander ein mono- oder polycyclisches, aliphatisches Ringsystem bilden;

$Ar_1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^3$ substituiert sein kann; dabei können zwei Reste $Ar_1$, welche an dasselbe N-Atom, P-Atom oder B-Atom binden, auch durch eine Einfachbindung oder eine Brücke, ausgewählt aus $N(R^3)$, $C(R^3)_2$, O oder S, miteinander verbrückt sein;

$Ar_2$ ist jeweils unabhängig voneinander bei jedem Auftreten eine Aryl- oder Heteroarylgruppe mit 5 bis 40 aromatischen Ringatomen, die mit einem oder mehreren Resten $R^2$ substituiert sein kann;

$Ar_3$ ist jeweils unabhängig voneinander bei jedem Auftreten ein aromatisches Ringsystem mit 6 bis 40 aromatischen Ringatomen oder ein heteroaromatisches Ringsystem mit 5 bis 40 aromatischen Ringatomen, das mit einem oder mehreren Resten $R^2$ substituiert sein kann;

A ist bei jedem Auftreten unabhängig voneinander eine Gruppe der Formel (3) oder (4),

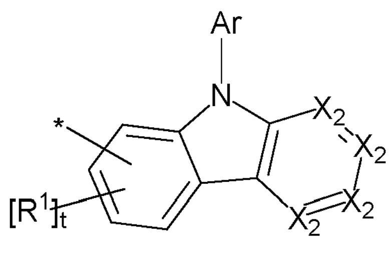

Formel (3)

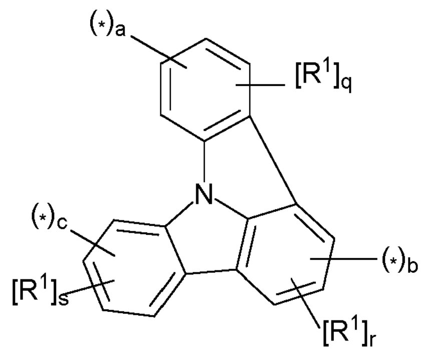

Formel (4) .

Ar ist jeweils unabhängig voneinander bei jedem Auftreten eine Arylgruppe mit 6 bis 40 aromatischen Ringatomen, die mit einem oder mehreren Resten R# substituiert sein kann, oder eine Heteroarylgruppe mit 5 bis 40 aromatischen Ringatomen, die mit einem oder mehreren Resten R# substituiert sein kann;

* kennzeichnet die Bindungsstelle an die Formel (2);

a, b, c bedeuten jeweils unabhängig voneinander bei jedem Auftreten 0 oder 1, wobei die Summe der Indizes bei jedem Auftreten a+b+c 1 bedeutet;

o bedeutet 1, 2, 3 oder 4;

n, m und p bedeuten unabhängig voneinander bei jedem Auftreten 1, 2 oder 3; und

q, r, s, t bedeuten jeweils unabhängig bei jedem Auftreten 0 oder 1.

Weitere Gegenstände der Erfindung umfassen ein Verfahren zur Herstellung der organischen elektrolumineszierenden Vorrichtungen sowie Mischungen enthaltend mindestens eine Verbindung der Formel (1) und mindestens eine Verbindung der Formel (2), spezielle Materialkombinationen und Formulierungen, die derartige Mischungen bzw. Materialkombinationen enthalten. Die entsprechenden bevorzugten Ausführungsformen, wie nachfolgend beschrieben, sind ebenfalls Gegenstand der vorliegenden Erfindung. Die überraschenden und vorteilhaften Effekte werden durch spezifische Selektion der Verbindungen der Formel (1) und der Verbindungen der Formel (2) erreicht.

Die erfindungsgemäße organische elektrolumineszierende Vorrichtung ist beispielsweise ein organischer lichtemittierender Transistor (OLET), ein organisches Feld-Quench-Device (OFQD), eine organische lichtemittierende elektrochemische Zelle (OLEC, LEC, LEEC), eine organische Laserdiode (O-Laser) oder eine organische lichtemittierende Diode (OLED). Die erfindungsgemäße organische elektrolumineszierende Vorrichtung ist insbesondere eine organische lichtemittierende Diode oder eine organische lichtemittierende elektrochemische Zelle. Besonders bevorzugt ist die erfindungsgemäße Vorrichtung eine OLED.

Die organische Schicht der erfindungsgemäßen Vorrichtung, die die lichtemittierende Schicht enthaltend die Materialkombination aus mindestens einer Verbindung der Formel (1) und mindestens einer Verbindung der Formel (2) enthält, wie zuvor beschrieben oder nachfolgend beschrieben, enthält bevorzugt neben dieser lichtemittierenden Schicht (EML), eine Lochinjektionsschicht (HIL), eine Lochtransportschicht (HTL), eine Elektronentransportschicht (ETL), eine Elektroneninjektionsschicht (EIL) und/oder eine Lochblockierschicht (HBL). Es können in der erfindungsgemäßen Vorrichtung auch mehrere Schichten dieser Gruppe ausgewählt aus EML, HIL, HTL, ETL, EIL und HBL enthalten sein. Die Vorrichtung kann aber auch anorganische Materialien enthalten oder auch Schichten, welche vollständig aus anorganischen Materialien aufgebaut sind.

Es ist bevorzugt, dass die lichtemittierende Schicht enthaltend mindestens eine Verbindung der Formel (1) und mindestens eine Verbindung der Formel (2) eine phosphoreszierende Schicht ist, die dadurch gekennzeichnet ist, dass sie zusätzlich zu der Hostmaterialienkombination der Verbindungen der Formel (1) und Formel (2), wie zuvor beschrieben, mindestens einen phosphoreszierenden Emitter enthält. Eine geeignete Auswahl an Emittern und bevorzugte Emitter werden nachfolgend beschrieben.

Eine Arylgruppe im Sinne dieser Erfindung enthält 6 bis 40 aromatische Ringatome, bevorzugt C-Atome. Eine Heteroarylgruppe im Sinne dieser Erfindung enthält 5 bis 40 aromatische Ringatome, wobei die Ringatome C-Atome und mindestens ein Heteroatom umfassen, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Phenyl, abgeleitet von Benzol, bzw. ein einfacher heteroaromatischer Cyclus, beispielsweise abgeleitet von Pyridin, Pyrimidin oder Thiophen, oder eine kondensierte Aryl- oder Heteroarylgruppe, beispielsweise abgeleitet von Naphthalin, Anthracen, Phenanthren, Chinolin oder Isochinolin, verstanden. Eine Arylgruppe mit 6 bis 18 C-Atomen ist daher vorzugsweise Phenyl, Naphthyl, Phenanthryl oder Triphenylenyl, wobei die Anbindung der Arylgruppe als Substituent dabei nicht eingeschränkt ist. Die Aryl- oder Heteroarylgruppe im Sinne dieser Erfindung kann einen oder mehrere Reste R tragen, wobei der Substituent R nachfolgend beschrieben wird.

Ein aromatisches Ringsystem im Sinne dieser Erfindung enthält 6 bis 40 C-Atome im Ringsystem. Das aromatisches Ringsystem umfasst auch Arylgruppen, wie zuvor beschrieben.

Ein aromatisches Ringsystem mit 6 bis 18 C-Atomen wird vorzugsweise aus Phenyl, vollständig deuteriertes Phenyl, Biphenyl, Naphthyl, Phenanthryl und Triphenylenyl ausgewählt.

Ein heteroaromatisches Ringsystem im Sinne dieser Erfindung enthält 5 bis 40 Ringatome und mindestens ein Heteroatom. Ein bevorzugtes heteroaromatisches Ringsystem hat 10 bis 40 Ringatome und mindestens ein Heteroatom. Das heteroaromatische Ringsystem umfasst auch Heteroarylgruppen, wie zuvor beschrieben. Die Heteroatome im heteroaromatischen Ringsystem sind bevorzugt ausgewählt aus N, O und/oder S.

Unter einem aromatischen oder heteroaromatischen Ringsystem im Sinne dieser Erfindung wird ein System verstanden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische bzw. heteroaromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sind Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls von der Definition des aromatischen bzw. heteroaromatischen Ringsystems umfasst.

Unter einem aromatischen oder heteroaromatischen Ringsystem mit 5 - 40 aromatischen Ringatomen, welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren,

1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die Abkürzung $Ar_1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^3$ substituiert sein kann; dabei können zwei Reste $Ar_1$, welche an dasselbe N-Atom, P-Atom oder B-Atom binden, auch durch eine Einfachbindung oder eine Brücke, ausgewählt aus $N(R^3)$, $C(R^3)_2$, O oder S, miteinander verbrückt sein, wobei der Rest $R^3$ oder die Substituenten $R^3$ eine Bedeutung hat/haben, wie zuvor oder nachfolgend beschrieben. Bevorzugt ist $Ar_1$ eine Arylgruppe mit 6 bis 40 aromatischen Ringatomen, wie zuvor beschrieben. Besonders bevorzugt ist $Ar_1$ Phenyl, das mit einem oder mehreren nicht aromatischen Resten $R^3$ substituiert sein kann. $Ar_1$ ist vorzugsweise unsubstituiert.

Die Abkürzung $Ar_2$ ist bei jedem Auftreten jeweils unabhängig voneinander eine Aryl- oder Heteroarylgruppe mit 5 bis 40 aromatischen Ringatomen, die mit einem oder mehreren Resten $R^2$ substituiert sein kann, wobei der Rest $R^2$ oder die Substituenten $R^2$ eine Bedeutung hat/haben, wie zuvor oder nachfolgend beschrieben. Die genannten Ausführungen für die Aryl-und Heteroarylgruppen mit 5 bis 40 aromatischen Ringatomen gelten hier entsprechend.

Die Abkürzung $Ar_3$ ist jeweils unabhängig voneinander bei jedem Auftreten ein aromatisches Ringsystem mit 6 bis 40 aromatischen Ringatomen oder ein heteroaromatisches Ringsystem mit 5 bis 40 aromatischen Ringatomen, das mit einem oder mehreren Resten $R^2$ substituiert sein kann, wobei der Rest $R^2$ oder die Substituenten $R^2$ eine Bedeutung hat/haben, wie zuvor oder nachfolgend beschrieben. Die genannten Ausführungen für die Aryl-und Heteroarylgruppen mit 5 bis 40 aromatischen Ringatomen gelten hier entsprechend.

Die Abkürzung Ar ist jeweils unabhängig voneinander bei jedem Auftreten eine Arylgruppe mit 6 bis 40 aromatischen Ringatomen, die mit einem oder mehreren Resten R# substituiert sein kann, oder eine Heteroarylgruppe mit 5 bis 40 aromatischen Ringatomen, die mit einem oder mehreren Resten R# substituiert sein kann, wobei die Ausführungen für die Arylgruppe bzw. Heteroarylgruppe entsprechend gelten, wie zuvor beschrieben. Der Rest R# oder die Reste R# haben eine Bedeutung, wie zuvor beschrieben oder nachfolgend beschrieben. Die Abkürzung Ar ist jeweils unabhängig voneinander bevorzugt bei jedem Auftreten eine Arylgruppe mit 6 bis 40 aromatischen Ringatomen, die mit einem oder mehreren Resten R# substituiert sein kann, oder eine Heteroarylgruppe mit 5 bis 40 aromatischen Ringatomen, enthaltend O oder S als Heteroatom, die mit einem oder mehreren Resten R# substituiert sein kann, wobei die Ausführungen für die Arylgruppe, Heteroarylgruppe und R# entsprechend gelten, wie zuvor beschrieben oder nachfolgend beschrieben.

Unter einer cyclischen Alkyl-, Alkoxy- oder Thioalkylgruppe im Sinne dieser Erfindung wird eine monocyclische, eine bicyclische oder eine polycyclische Gruppe verstanden.

Im Rahmen der vorliegenden Erfindung werden unter einer geradkettigen, verzweigten oder cyclischen $C_1$- bis $C_{20}$-Alkylgruppe beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1 -Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden.

Unter einer geradkettigen oder verzweigten $C_1$- bis $C_{20}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einer geradkettigen $C_1$- bis $C_{20}$-Thioalkylgruppe werden beispielsweise S-Alkylgruppen verstanden, beispielsweise Thiomethyl, 1-Thioethyl, 1-Thio-i-propyl, 1-Thio-n-propoyl, 1-Thio-i-butyl, 1-Thio-n-butyl oder 1-Thio-t-butyl.

Eine Aryloxy- oder Heteroaryloxygruppe mit 5 bis 40 aromatischen Ringatomen bedeutet O-Aryl oder O-Heteroaryl und bedeutet, dass die Aryl- bzw. Heteroarylgruppe über ein Sauerstoffatom gebunden wird, wobei die Aryl- bzw. Heteroarylgruppe eine Bedeutung hat, wie zuvor beschrieben.

Eine Aralkyl- oder Heteroaralkylgruppe mit 5 bis 40 aromatischen Ringatomen bedeutet, dass eine Alkylgruppe, wie zuvor beschrieben, mit einer Arylgruppe bzw. Heteroarylgruppe substituiert ist, wobei die Aryl- bzw. Heteroarylgruppe eine Bedeutung hat, wie zuvor beschrieben.

Ein phosphoreszierender Emitter im Sinne der vorliegenden Erfindung ist eine Verbindung, die Lumineszenz aus einem angeregten Zustand mit höherer Spinmultiplizität zeigt, also einem Spinzustand > 1, insbesondere aus einem angeregten Triplettzustand. Im Sinne dieser Anmeldung sollen alle lumineszierenden Komplexe mit Übergangsmetallen oder Lanthaniden als phosphoreszierende Emitter angesehen werden. Eine genauere Definition erfolgt nachfolgend.

Wenn die Hostmaterialien der lichtemittierenden Schicht umfassend mindestens eine Verbindung der Formel (1), wie zuvor beschrieben oder nachfolgend bevorzugt beschrieben, und mindestens eine Verbindung der Formel (2), wie zuvor beschrieben oder nachfolgend beschrieben, für einen phosphoreszierenden Emitter eingesetzt wird, ist es bevorzugt, wenn deren Triplettenergie nicht wesentlich kleiner als die Triplettenergie des phosphoreszierenden Emitters ist. Dabei gilt bevorzugt für das Triplettniveau $T_1$(Emitter) - $T_1$(Matrix) $\leq$ 0.2 eV, besonders bevorzugt $\leq$ 0.15 eV, ganz besonders bevorzugt $\leq$ 0.1 eV. Dabei ist $T_1$(Matrix) das Triplettniveau des Matrixmaterials in der Emissionsschicht, wobei diese Bedingung für jedes der beiden Matrixmaterialien gilt, und $T_1$(Emitter) ist das Triplettniveau des phosphoreszierenden Emitters. Enthält die Emissionsschicht mehr als zwei Matrixmaterialien, so gilt die oben genannte Beziehung bevorzugt auch für jedes weitere Matrixmaterial.

Im Folgenden wird das Hostmaterial 1 und dessen bevorzugte Ausführungsformen beschrieben, welches/welche in der erfindungsgemäßen Vorrichtung enthalten ist/sind. Die bevorzugten Ausführungsformen des Hostmaterials 1 der Formel (1) gelten auch für die erfindungsgemäße Mischung und/oder Formulierung.

In Verbindungen der Formel (1) steht das Symbol L als Linker für eine Einfachbindung oder ein aromatisches oder heteroaromatisches Ringsystem mit 5 bis 30 aromatischen Ringatomen.

In Verbindungen der Formel (1) steht das Symbol L bevorzugt für eine Bindung oder einen Linker ausgewählt aus der Gruppe L-1 bis L-33,

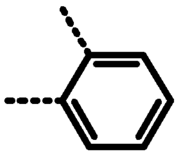
L-1

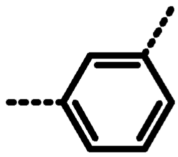
L-2

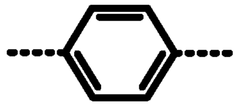
L-3

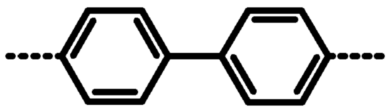
L-4

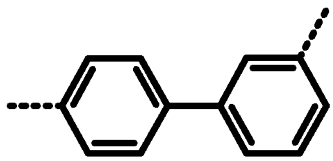
L-5

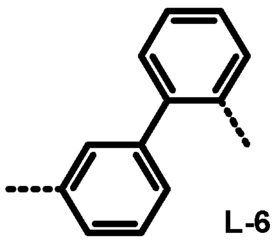
L-6

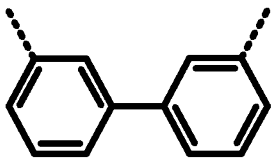
L-7

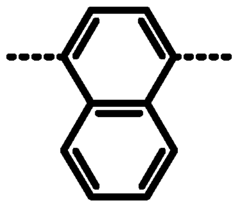
L-8

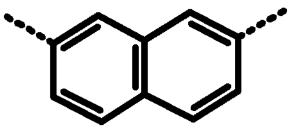
L-9

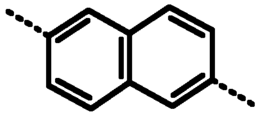
L-10

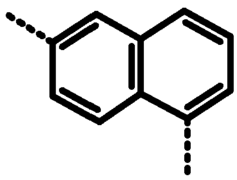
L-11

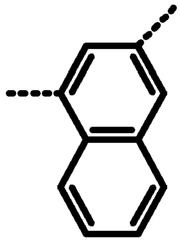
L-12

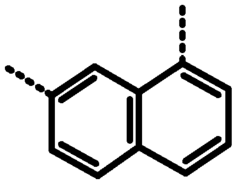
L-13

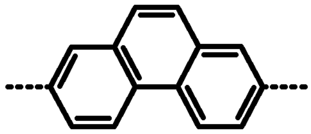
L-14

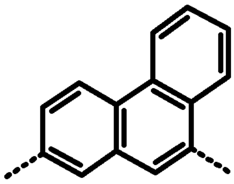
L-15

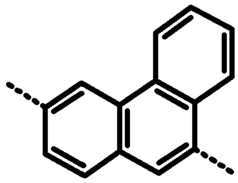
L-16

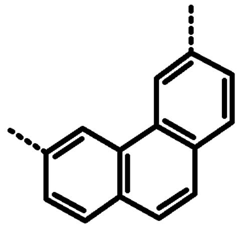
L-17

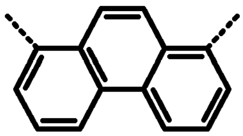
L-18

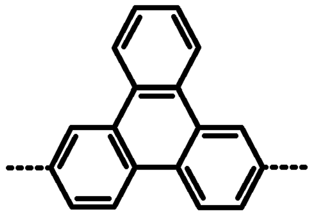
L-19

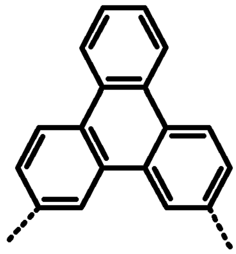
L-20

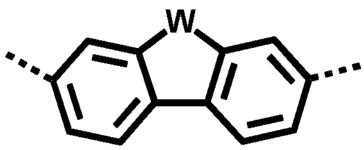
L-21

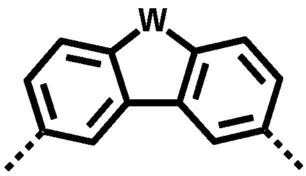
L-22

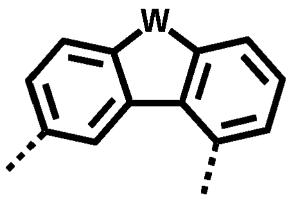
L-23

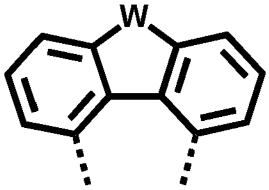
L-24

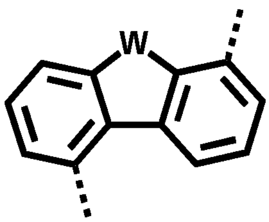
L-25

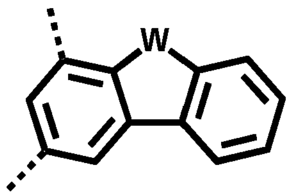
L-26

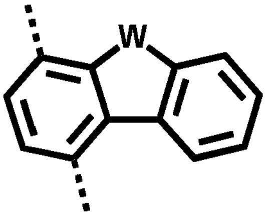
L-27

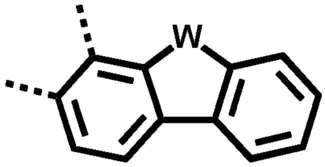
L-28

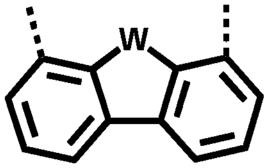
L-29

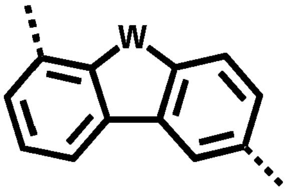
L-30

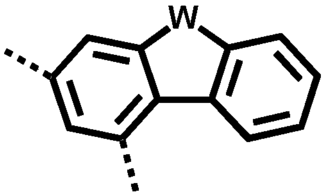
L-31

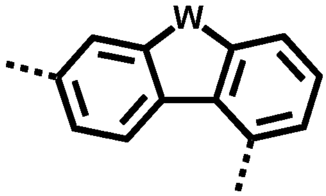
L-32

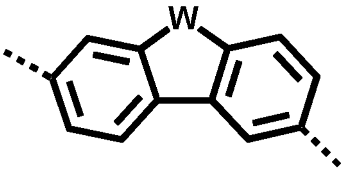
L-33

,

wobei W jeweils unabhängig voneinander O oder S bedeutet.

Ein weiterer Gegenstand der Erfindung ist daher die elektrolumineszierende Vorrichtung, wie zuvor beschrieben, wobei der Linker L im Hostmaterial 1 ausgewählt wird aus einer Bindung oder den Linkern der Gruppe L-1 bis L-33, wie zuvor beschrieben.

In Verbindungen der Formel (1) steht das Symbol L in einer Ausführungsform besonders bevorzugt für eine Bindung oder einen Linker ausgewählt aus L-1 bis L-3, L-7, L-21, L-22, L-23, L-25 bis L-27 und L-30 bis L-33,

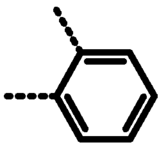
L-1

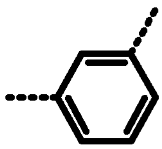
L-2

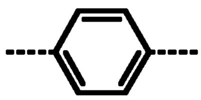
L-3

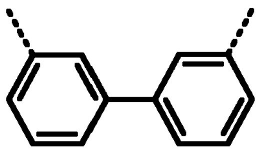
L-7

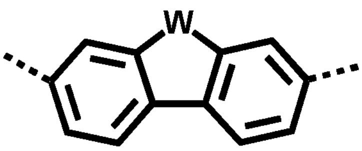
L-21

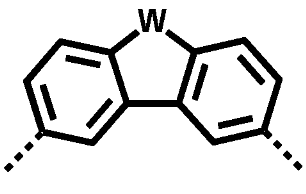
L-22

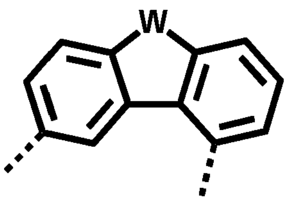
L-23

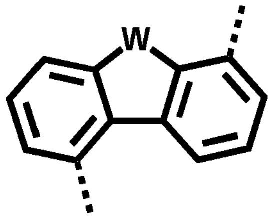
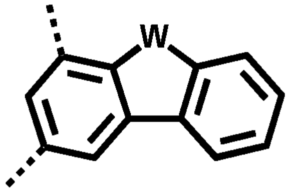
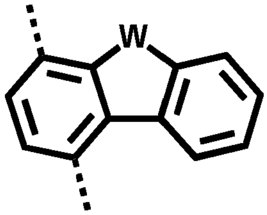

L-25 L-26 L-27

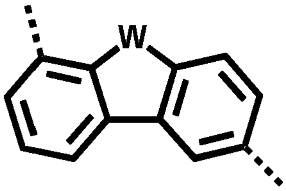
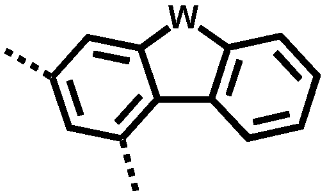
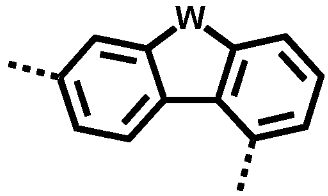

L-30 L-31 L-32

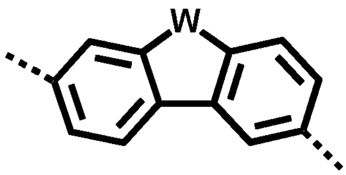

L-33

,

wobei W jeweils unabhängig voneinander O oder S bedeutet. W ist besonders bevorzugt O.

Verbindungen der Formel (1), in denen L bevorzugt eine Einfachbindung bedeutet, können durch die Formel (1a) beschrieben werden,

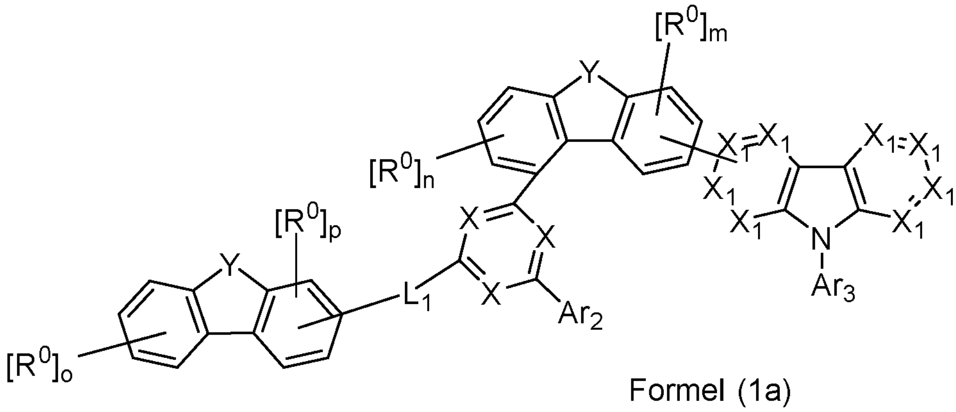

Formel (1a)

,

wobei Y, $L_1$, X, $X_1$, $Ar_2$, $R^0$, $Ar_3$, n, m, o und p eine zuvor genannte oder nachfolgend bevorzugt genannte Bedeutung haben.

In Verbindungen der Formel (1) steht das Symbol $L_1$ als Linker für eine Einfachbindung oder ein aromatisches oder heteroaromatisches Ringsystem mit 5 bis 30 aromatischen Ringatomen.

In Verbindungen der Formel (1) steht das Symbol $L_1$ bevorzugt für eine Einfachbindung oder einen Linker ausgewählt aus der Gruppe L-1 bis L-33, wie zuvor beschrieben. In Verbindungen der Formel (1) oder (1a) steht das Symbol $L_1$ bevorzugt für eine Einfachbindung oder einen Linker L-2 oder L-3, wie zuvor beschrieben, oder besonders bevorzugt für eine Einfachbindung.

Verbindungen der Formel (1), in denen $L_1$ bevorzugt eine Einfachbindung bedeutet, können durch die Formel (1b) beschrieben werden,

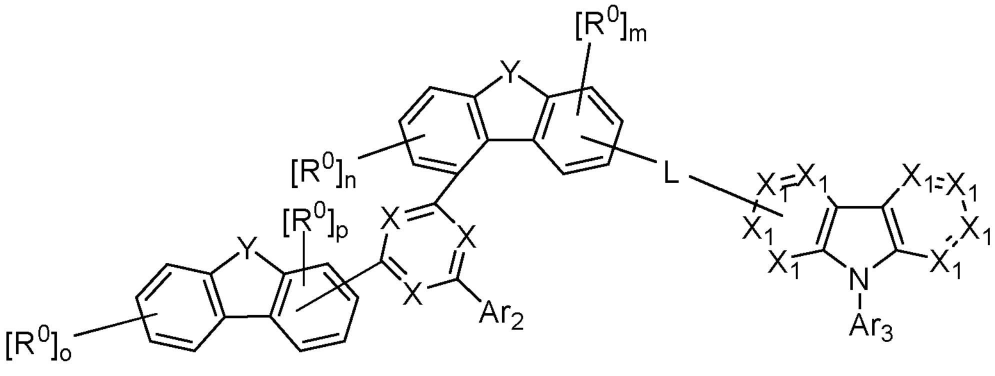

Formel (1b)

.

wobei Y, L, X, $X_1$, $Ar_2$, $R^0$, $Ar_3$, n, m, o und p eine zuvor genannte oder nachfolgend bevorzugt genannte Bedeutung haben.

Ein weiterer Gegenstand der Erfindung ist daher eine elektrolumineszierende Vorrichtung, wie zuvor beschrieben, wobei $L_1$ im Hostmaterial 1 eine Einfachbindung bedeutet.

In Verbindungen der Formeln (1), (1a) und (1b) oder bevorzugten Ausführungsformen des Hostmaterials der Formeln (1), (1a) und (1b) steht das Symbol X für $CR^0$ oder N, wobei mindestens eine Gruppe X N bedeutet.

Der Substituent

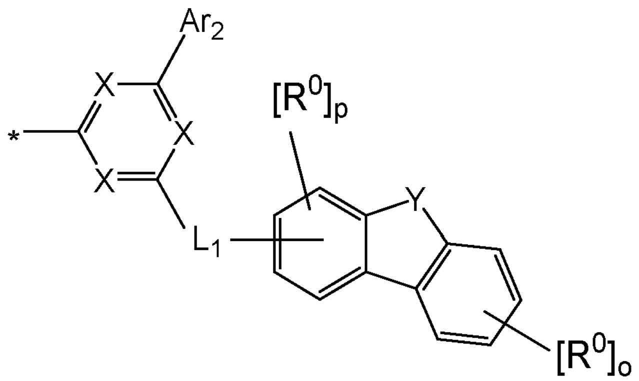

hat daher die folgenden Bedeutungen, wobei * die Verknüpfungsstelle mit dem Dibenzofuran bzw. Dibenzothiophen kennzeichnet und $L_1$, $R^0$, o, p, Y und $Ar_2$ eine zuvor angegebene Bedeutung oder eine bevorzugt angegebene Bedeutung haben:

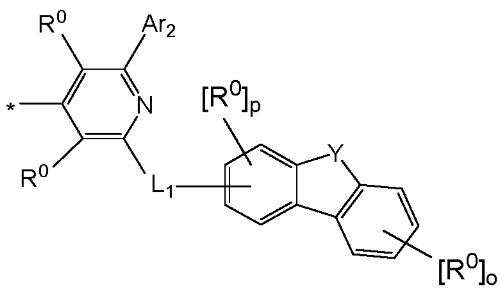

,

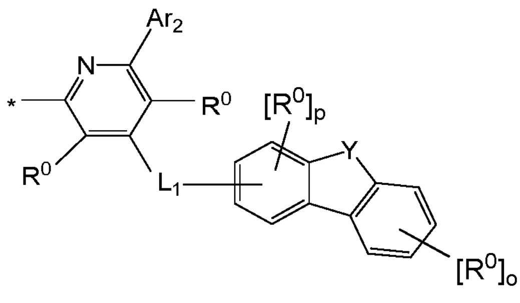

,

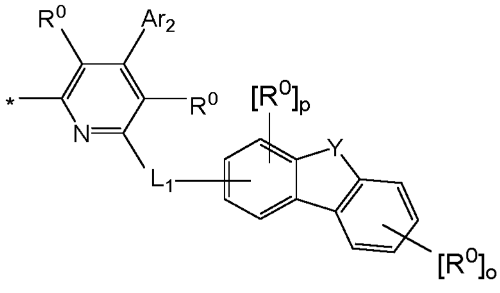
,
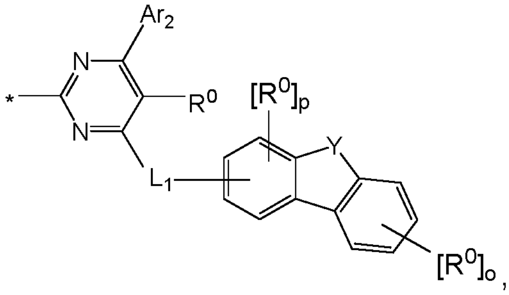
,

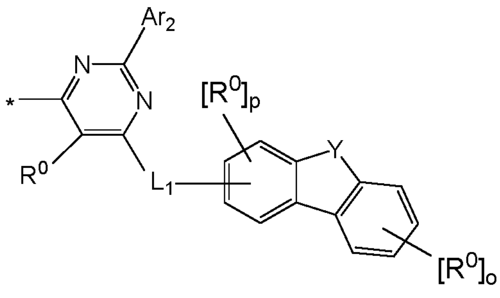
,
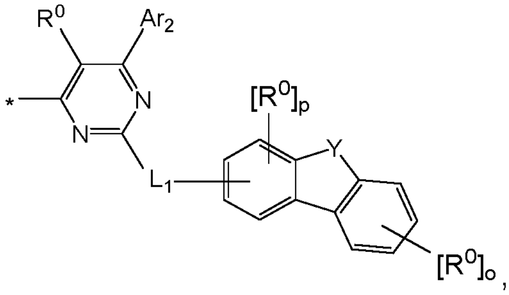
,

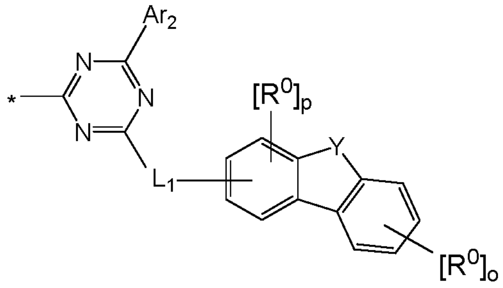
.

In diesen vorstehend genannten Substituenten ist Y bevorzugt O.

In Hostmaterial 1 steht X bevorzugt zwei Mal für N und ein X steht für $CR^0$ oder alle X sind N. In Hostmaterial 1 stehen besonders bevorzugt alle X für N, wobei $R^0$ eine zuvor genannte oder nachfolgend genannte Bedeutung hat.

Bevorzugte Hostmaterialien 1 entsprechen demzufolge den Verbindungen der Formeln (1), (1a), (1b), (1c) oder (1d),

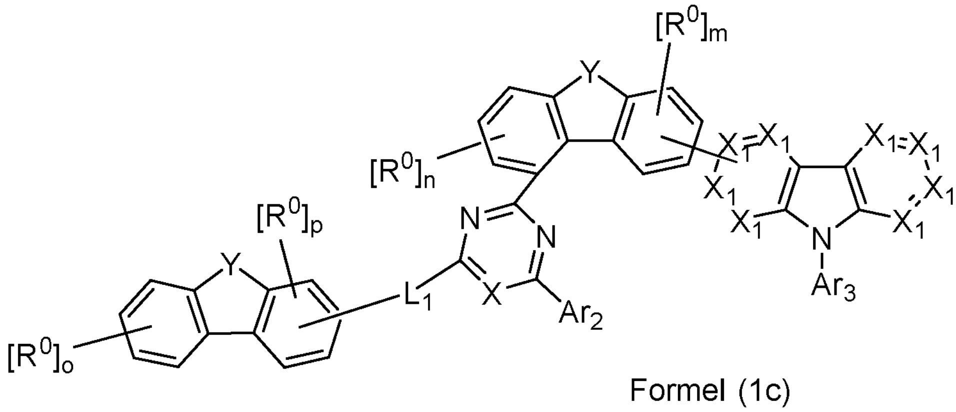

Formel (1c)

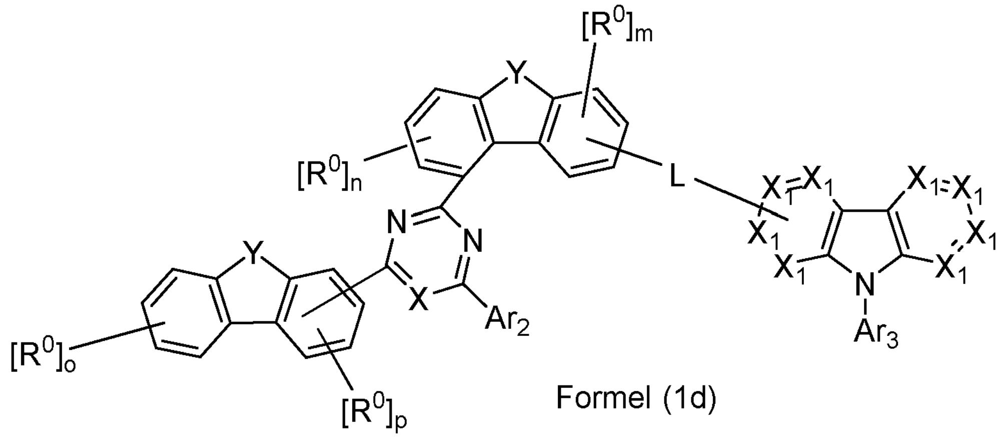

Formel (1d)

,

wobei Y, L, $L_1$, X, $X_1$, $Ar_2$, $R^0$, $Ar_3$, o, p, n und m eine zuvor genannte oder zuvor oder nachfolgend bevorzugt genannte Bedeutung haben.

$R^0$ ist bei jedem Auftreten gleich oder verschieden bevorzugt ausgewählt aus der Gruppe H, D oder ein unsubstituiertes oder teilweise oder vollständig deuteriertes aromatisches Ringsystem mit 6 bis 18 C-Atomen. $R^0$ ist bei jedem Auftreten bevorzugt H, D oder ein unsubstituiertes aromatisches Ringsystem mit 6 bis 18 C-Atomen. $R^0$ ist bei jedem Auftreten besonders bevorzugt H oder D. $R^0$ ist bei jedem Auftreten ganz besonders bevorzugt H.

In Verbindungen der Formeln (1), (1a), (1b), (1c) und (1d) ist o bevorzugt 4, wenn $R^0$ H oder D bedeutet. Wenn $R^0$ nicht H oder D bedeutet, dann ist o bevorzugt 1 oder 2, besonders bevorzugt 1.

In Verbindungen der Formeln (1), (1a), (1b), (1c) und (1d) ist p bevorzugt 3, wenn $R^0$ H oder D bedeutet. Wenn $R^0$ nicht H oder D bedeutet, dann ist p bevorzugt 1.

In Verbindungen der Formeln (1), (1a), (1b), (1c) und (1d) ist m bevorzugt 3, wenn $R^0$ H oder D bedeutet. Wenn $R^0$ nicht H oder D bedeutet, dann ist m bevorzugt 1.

In Verbindungen der Formeln (1), (1a), (1b), (1c) und (1d) ist n bevorzugt 3, wenn $R^0$ H oder D bedeutet. Wenn $R^0$ nicht H oder D bedeutet, dann ist n bevorzugt 1.

Verbindungen der Formel (1), in denen X bei jedem Auftreten gleich N bedeutet, L eine Einfachbindung bedeutet, R° H ist, o 4 ist und m, n und p 3 sind, werden durch die Formel (1e) dargestellt,

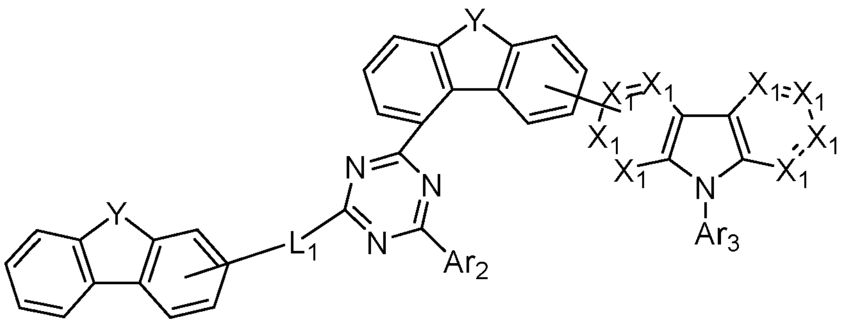

Formel (1e)

,

wobei Y, $L_1$, $Ar_2$, $Ar_3$ und $X_1$ eine zuvor angegebene Bedeutung haben oder eine nachfolgend oder zuvor bevorzugt angegebene Bedeutung haben.

Verbindungen der Formel (1), in denen X bei jedem Auftreten gleich N bedeutet, $L_1$ eine Einfachbindung bedeutet, R° H ist, o 4 ist und m, n und p 3 sind und m, n, o und p 0 sind, werden durch die Formel (1f) dargestellt,

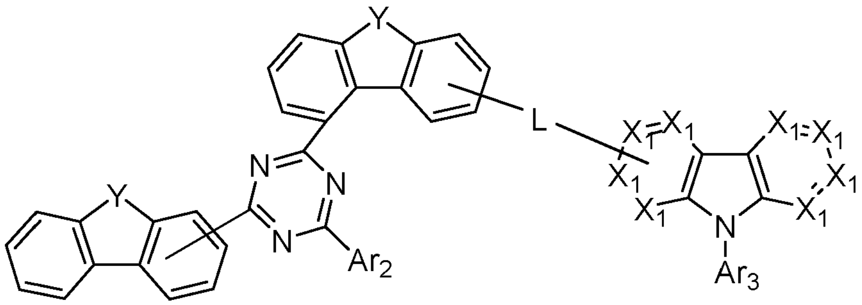

Formel (1f)

,

wobei Y, L, $Ar_2$, $Ar_3$ und $X_1$ eine zuvor angegebene Bedeutung haben oder eine nachfolgend oder zuvor bevorzugt angegebene Bedeutung haben.

Verbindungen der Formel (1e) sind bevorzugte Ausführungsformen der Verbindungen der Formel (1) und des Hostmaterials 1. In Verbindungen der Formel (1e) steht bevorzugt ein Y für O im Substituenten, der direkt an Triazin gebunden ist und das zweite Symbol Y steht für O oder S. In bevorzugten Ausführungsformen der Verbindungen der Formel (1e) bedeuten beide Symbole Y O.

Verbindungen der Formel (1f) sind bevorzugte Ausführungsformen der Verbindungen der Formel (1) und des Hostmaterials 1. In Verbindungen der Formel (1f) steht bevorzugt ein Y für O im Substituenten, der direkt an Triazin gebunden ist und das zweite Symbol Y steht für O oder S. In bevorzugten Ausführungsformen der Verbindungen der Formel (1f) bedeuten beide Symbole Y O.

In Verbindungen der Formeln (1), (1a), (1b), (1c), (1d), (1e) und (1f) oder bevorzugt beschriebenen Verbindungen der Formeln (1), (1a), (1b), (1c), (1d), (1e) und (1f), steht $Ar_2$ jeweils unabhängig voneinander bevorzugt für eine Arylgruppe mit 6 bis 40 C-Atomen, wie zuvor beschrieben oder bevorzugt beschrieben, die mit einem oder mehreren Resten $R^2$ substituiert sein kann oder für eine Heteroarylgruppe mit 10 bis 40 C-Atomen, wie zuvor beschrieben, die mit einem oder mehreren Resten $R^2$ substituiert sein kann. Dabei können mehrere Reste $R^2$, die an dasselbe Kohlenstoffatom oder an benachbarte Kohlenstoffatome gebunden sind, ein mono-cyclisches oder polycyclisches, aliphatisches, aromatisches oder heteroaromatisches Ringsystem bilden, das mit einem oder mehreren Resten $R^3$ substituiert sein kann.

Die Verknüpfung der Arylgruppe oder der Heteroarylgruppe ist dabei nicht eingeschränkt und kann über ein C-Atom oder über ein Heteroatom, beispielsweise ein N-Atom, erfolgen.

$Ar_2$ kann bevorzugt aus folgenden Gruppen Ar-1 bis Ar-17 ausgewählt werden, wobei $R^2$ und $Ar_1$ eine zuvor angegebene oder bevorzugt angegebene Bedeutung haben und wobei ausgeschlossen ist, dass durch $R^2$ oder $Ar_1$ zwei Heteroatome direkt miteinander verbunden sind:

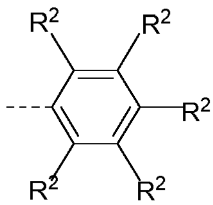

Ar-1

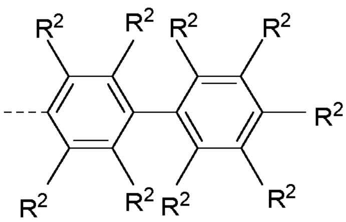

Ar-2

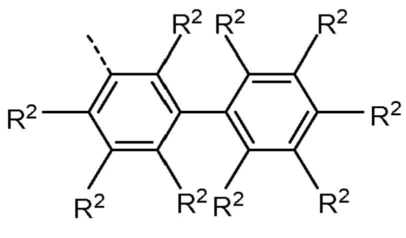

Ar-3

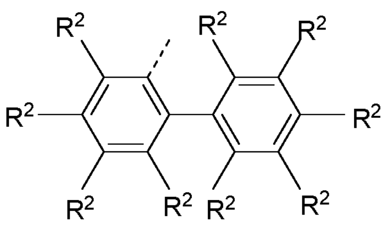

Ar-4

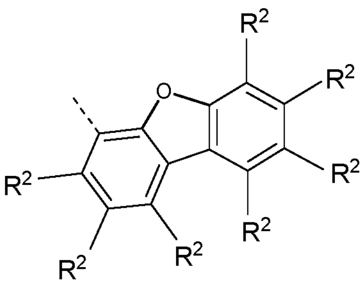

Ar-5

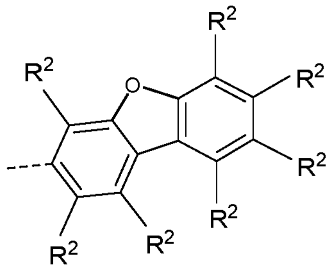

Ar-6

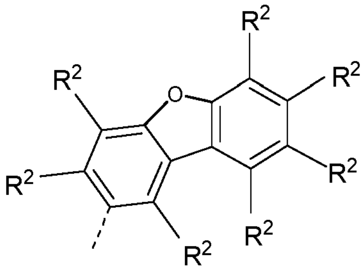

Ar-7

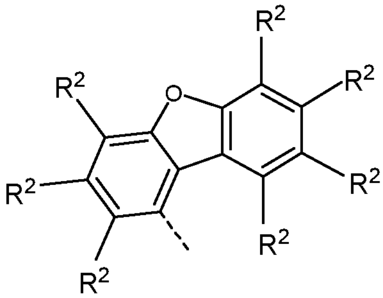

Ar-8

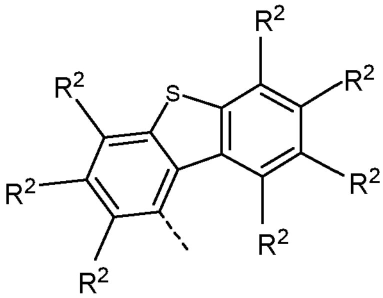

Ar-9

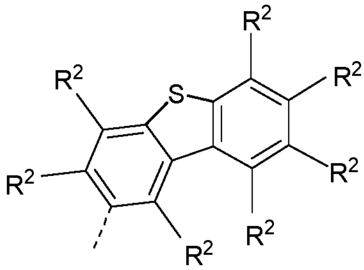

Ar-10

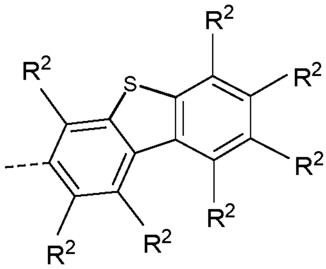

Ar-11

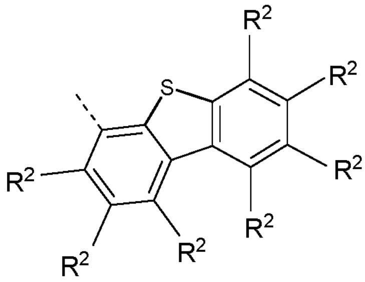

Ar-12

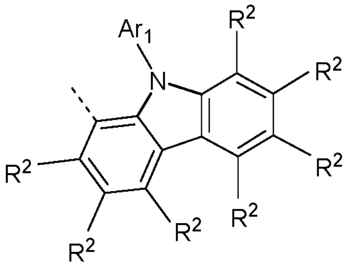

Ar-13

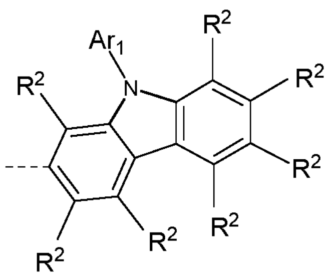

Ar-14

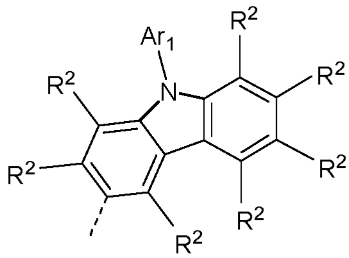

Ar-15

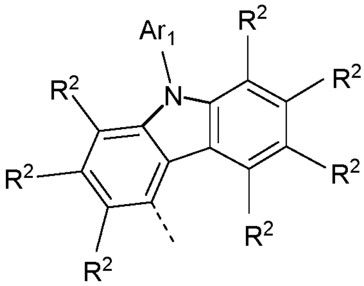

Ar-16

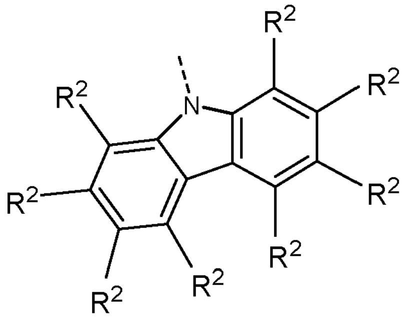

Ar-17

.

Die gestrichelte Linie kennzeichnet die Bindungsstelle an den Rest der Formeln (1), (1a), (1b), (1c), (1d), (1e) oder (1f).

Besonders bevorzugt steht $Ar_2$ für Ar-1, Ar-5, Ar-6, Ar-9, Ar-17, besonders bevorzugt für Ar-1, wobei $R^2$ eine zuvor angegebene oder nachfolgend bevorzugt angegebene Bedeutung hat.

$R^2$ in Substituenten der Formeln Ar-1 bis Ar-17, wie zuvor beschrieben, wird bevorzugt ausgewählt aus der Gruppe H, D, CN, einem aromatischen oder heteroaromatischen Ringsystem mit 5 bis 40 aromatischen Ringatomen, das jeweils mit einem oder mehreren Resten $R^3$ substituiert sein kann, wobei zwei Substituenten $R^2$, die an dasselbe Kohlenstoffatom oder an benachbarte Kohlenstoffatome gebunden sind zusammen ein monocyclisches oder polycyclisches, aliphatisches, aromatisches oder heteroaromatisches Ringsystem bilden können, das mit einem oder mehreren Resten $R^3$ substituiert sein kann.

Wenn mehrere Reste $R^2$ bzw. zwei Reste $R^2$ an benachbarte Kohlenstoffatome gebunden sind, so wird das mono-cyclische oder polycyclische, aliphatische, aromatische oder heteroaromatische Ringsystem bevorzugt aus der Gruppe (S1) bis (S4) ausgewählt,

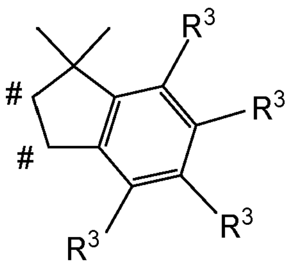

(S1)

,

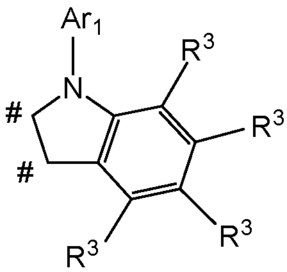

(S2)

,

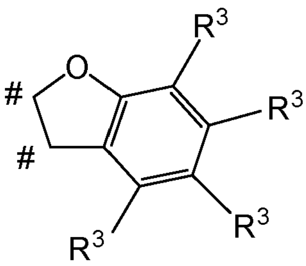

(S3)

,

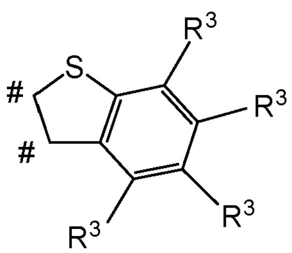

(S4)

,

wobei $Ar_1$ und $R^3$ eine zuvor oder nachfolgend genannte Bedeutung haben und # die Anknüpfungsstellen an den Rest der jeweiligen Struktur kennzeichnet, beispielsweise an Ar-1 bis Ar-17 oder $Ar_2$. Besonders bevorzugt wird hierbei (S1) oder (S2) ausgewählt.

$Ar_1$ in Substituenten der Formeln Ar-13 bis Ar-16 und (S2), wie zuvor beschrieben, ist bevorzugt Phenyl.

Die Verknüpfung der über den Linker $L_1$ gebundenen Dibenzofuran- oder Dibenzothiophengruppe in den Verbindungen der Formeln (1), (1a), (1b), (1c), (1d), (1e) und (1f) oder bevorzugten Verbindungen der Formeln (1), (1a), (1b), (1c), (1d), (1e) und (1f) ist dabei nicht eingeschränkt und kann über jedes beliebige C-Atom erfolgen. Besonders bevorzugt ist die Dibenzofuran- oder Dibenzothiophengruppe in Position 2, 3 oder 4 über den Linker $L_1$ and den Rest der Formeln (1), (1a), (1b), (1c), (1d), (1e) und (1f) gebunden. Ganz besonders bevorzugt ist die Dibenzofuran- oder Dibenzothiophengruppe in Position 3 über den Linker $L_1$ and den Rest der Formeln (1), (1a), (1b), (1c), (1d), (1e) und (1f) gebunden.

In Verbindungen der Formeln (1), (1a), (1b), (1c), (1d), (1e) und (1f) oder bevorzugten Verbindungen der Formeln (1), (1a), (1b), (1c), (1d), (1e) und (1f) ist $X_1$ bei jedem Auftreten gleich oder verschieden CH, CR oder N, wobei maximal 3 Symbole $X_1$ N bedeuten können.

In Verbindungen der Formeln (1), (1a), (1b), (1c), (1d), (1e) und (1f) oder bevorzugten Verbindungen der Formeln (1), (1a), (1b), (1c), (1d), (1e) und (1f) ist $X_1$ bei jedem Auftreten gleich oder verschieden bevorzugt CH, CR oder N, wobei maximal 1 Symbol $X_1$ N bedeutet.

In Verbindungen der Formeln (1), (1a), (1b), (1c), (1d), (1e) und (1f) oder bevorzugten Verbindungen der Formeln (1), (1a), (1b), (1c), (1d), (1e) und (1f) ist $X_1$ bei jedem Auftreten gleich oder verschieden besonders bevorzugt CH oder CR.

In Verbindungen der Formeln (1), (1a), (1b), (1c), (1d), (1e) und (1f) oder bevorzugten Verbindungen der Formeln (1), (1a), (1b), (1c), (1d), (1e) und (1f) ist $X_1$ bei jedem Auftreten gleich oder verschieden besonders bevorzugt CH oder CR, wobei maximal 3 Symbole $X_1$ CR bedeuten können.

In einer Ausführungsform von Verbindungen der Formeln (1), (1a), (1b), (1c), (1d), (1e) und (1f) oder bevorzugten Verbindungen der Formeln (1), (1a), (1b), (1c), (1d), (1e) und (1f) ist $X_1$ bei jedem Auftreten gleich CH.

In einer Ausführungsform von Verbindungen der Formeln (1), (1a), (1b), (1c), (1d), (1e) und (1f) oder bevorzugten Verbindungen der Formeln (1), (1a), (1b), (1c), (1d), (1e) und (1f) ist $X_1$ bei Auftreten gleich CH oder zwei Mal CR, wobei zwei benachbarte Substituenten R zusammen ein mono-cyclisches oder polycyclisches, aliphatisches, aromatisches oder heteroaromatisches Ringsystem bilden können, das mit einem oder mehreren Resten $R^2$ substituiert sein kann.

Wenn mehrere Reste R an benachbarte Kohlenstoffatome gebunden sind, so wird das mono-cyclische oder polycyclische, aliphatische, aromatische oder heteroaromatische Ringsystem bevorzugt aus der Gruppe (S-1) bis (S-4) ausgewählt,

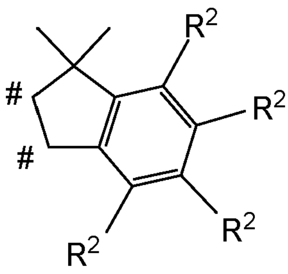
(S-1) ,

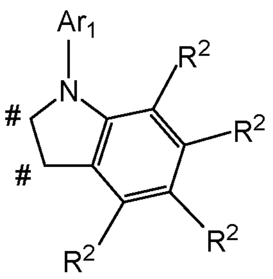
(S-2) ,

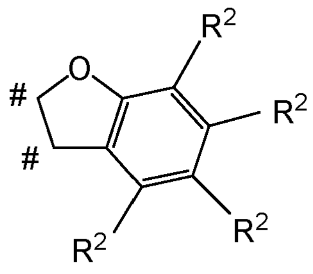
(S-3) ,

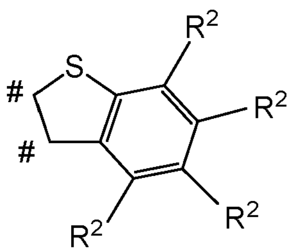
(S-4) ,

wobei $Ar_1$ und $R^2$ eine zuvor oder nachfolgend genannte Bedeutung haben und # die Anknüpfungsstellen an den Rest der jeweiligen Struktur kennzeichnet, beispielsweise an benachbarte Positionen, gekennzeichnet durch $X_1$ in Verbindungen der Formeln (1), (1a), (1b), (1c), (1d), (1e) bis (1f). Besonders bevorzugt wird hierbei (S-1) oder (S-2) ausgewählt.

In einer Ausführungsform von Verbindungen der Formeln (1), (1a), (1b), (1c), (1d), (1e) und (1f) oder bevorzugten Verbindungen der Formeln (1), (1a), (1b), (1c), (1d), (1e) und (1f) ist $X_1$ bei Auftreten gleich CH oder ein Mal CR, wobei

der Substituent R zusammen mit $Ar_3$ ein monocyclisches oder polycyclisches, aliphatisches, aromatisches oder heteroaromatisches Ringsystem bilden kann, das mit einem oder mehreren Resten $R^2$ substituiert sein kann.

In Verbindungen der Formeln (1), (1a), (1b), (1c), (1d), (1e) und (1f) oder bevorzugt beschriebenen Verbindungen der Formeln (1), (1a), (1b), (1c), (1d), (1e) und (1f), steht $Ar_3$ jeweils unabhängig voneinander bevorzugt für eine Arylgruppe mit 6 bis 40 C-Atomen, wie zuvor beschrieben oder bevorzugt beschrieben, die mit einem oder mehreren Resten $R^2$ substituiert sein kann oder für eine Heteroarylgruppe mit 10 bis 40 C-Atomen, wie zuvor beschrieben, die mit einem oder mehreren Resten $R^2$ substituiert sein kann. Dabei können mehrere Reste $R^2$, die an dasselbe Kohlenstoffatom oder an benachbarte Kohlenstoffatome gebunden sind, ein mono-cyclisches oder polycyclisches, aliphatisches, aromatisches oder heteroaromatisches Ringsystem bilden, das mit einem oder mehreren Resten $R^2$ substituiert sein kann oder $Ar_3$ kann mit einem Substituenten R einer Position $X_1$ ein mono-cyclisches oder polycyclisches, aliphatisches, aromatisches oder heteroaromatisches Ringsystem bilden, das mit einem oder mehreren Resten $R^2$ substituiert sein kann.

In Verbindungen der Formeln (1), (1a), (1b), (1c), (1d), (1e) und (1f) oder bevorzugt beschriebenen Verbindungen der Formeln (1), (1a), (1b), (1c), (1d), (1e) und (1f), steht $Ar_3$ jeweils unabhängig voneinander bevorzugt für eine Arylgruppe mit 6 bis 40 C-Atomen, wie zuvor beschrieben oder bevorzugt beschrieben, die mit einem oder mehreren Resten $R^2$ substituiert sein kann oder für eine Heteroarylgruppe mit 10 bis 40 C-Atomen enthaltend ein O-Atom oder ein S-Atom, wie zuvor beschrieben, die mit einem oder mehreren Resten $R^2$ substituiert sein kann. Dabei können mehrere Reste $R^2$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^2$ substituiert sein kann oder $Ar_3$ kann mit einem Substituenten R einer Position $X_1$ ein monocyclisches oder polycyclisches, aliphatisches, aromatisches oder heteroaromatisches Ringsystem bilden, das mit einem oder mehreren Resten $R^2$ substituiert sein kann.

Die Verknüpfung der Arylgruppe oder der Heteroarylgruppe ist dabei nicht eingeschränkt, erfolgt jedoch jeweils über ein C-Atom.

$Ar_3$ kann bevorzugt aus den Gruppen Ar-1 bis Ar-12 ausgewählt werden, wie zuvor beschrieben, wobei $R^2$ eine zuvor angegebene oder bevorzugt angegebene Bedeutung hat.

Besonders bevorzugt ist $Ar_3$ unsubstituiert, d.h. in den bevorzugten Gruppen Ar-1 bis Ar-12 für $Ar_3$ ist $R_2$ bevorzugt H.

Besonders bevorzugt steht $Ar_3$ für Ar-1 bis Ar-4, wobei $R^2$ eine zuvor angegebene oder nachfolgend bevorzugt angegebene Bedeutung hat.

$R^2$ in Substituenten der Formeln Ar-1 bis Ar-12, wie zuvor beschrieben, wird bevorzugt ausgewählt aus der Gruppe H, CN, einem aromatischen oder heteroaromatischen Ringsystem mit 5 bis 40 aromatischen Ringatomen, das jeweils mit einem oder mehreren Resten $R^3$ substituiert sein kann.

In Verbindungen der Formeln (1), (1a), (1b), (1c), (1d), (1e) und (1f) oder bevorzugt beschriebenen Verbindungen der Formeln (1), (1a), (1b), (1c), (1d), (1e) und (1f), steht die Gruppe

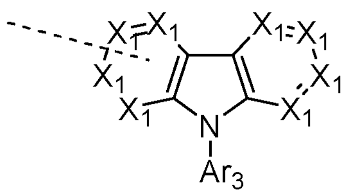

auch bevorzugt für eine Gruppe der Formel (5) oder (6)

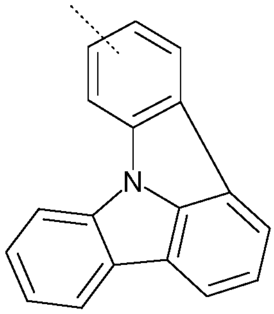

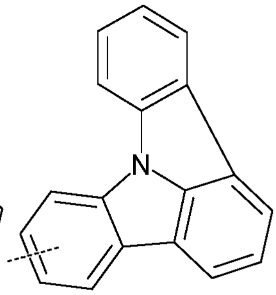

Formel (5) Formel (6) ,

wobei die gestrichelte Linie die Anbindung an den Rest der Formeln (1), (1a), (1b), (1c), (1d), (1e) und (1f) kennzeichnet.

$R^3$ in Verbindungen der Formeln (1), (1a), (1b), (1c), (1d), (1e) und (1f), wie zuvor beschrieben oder bevorzugt beschrieben, wird bei jedem Auftreten unabhängig voneinander bevorzugt ausgewählt aus der Gruppe H, CN, einem aromatischen oder heteroaromatischen Ringsystem mit 5 bis 40 aromatischen Ringatomen, in dem ein oder mehrere H-Atome durch D oder CN ersetzt sein können. $R^3$ in Verbindungen der Formeln (1), (1a), (1b), (1c), (1d), (1e) und (1f), wie zuvor beschrieben oder bevorzugt beschrieben, wird bei jedem Auftreten unabhängig voneinander besonders bevorzugt ausgewählt aus H, Phenyl oder deuteriertem Phenyl.

Beispiele für geeignete Hostmaterialien der Formeln (1), (1a), (1b), (1c), (1d), (1e) und (1f), die erfindungsgemäß ausgewählt werden, und bevorzugt in Kombination mit mindestens einer Verbindung der Formel (2) in der erfindungsgemäßen elektrolumineszierenden Vorrichtung verwendet werden, sind die nachstehend genannten Strukturen der Tabelle 1.

Tabelle 1:

| | | |
|---|---|---|
| 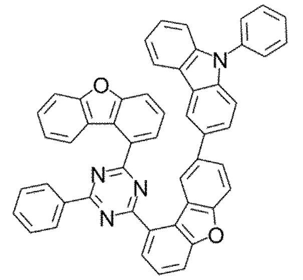 | 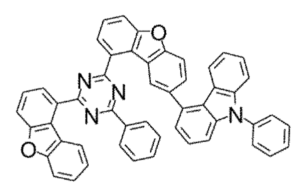 | 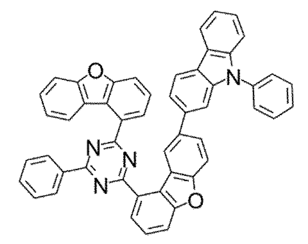 |
| 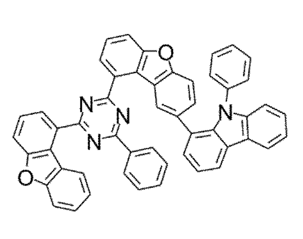 | 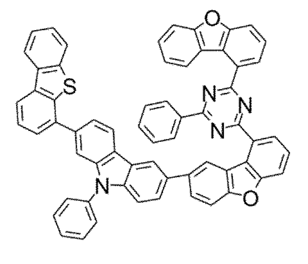 | 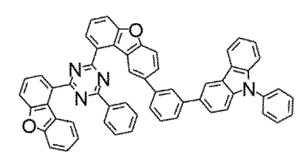 |
| 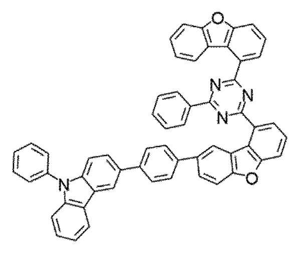 | 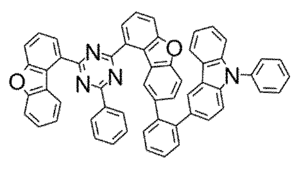 | 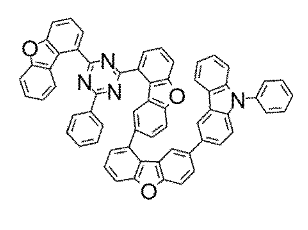 |
| 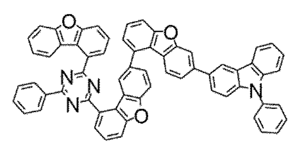 | 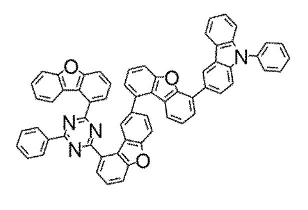 | 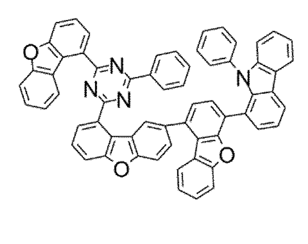 |
| 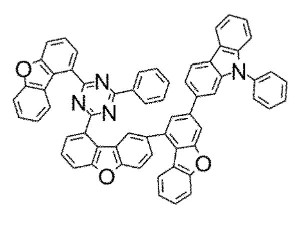 | 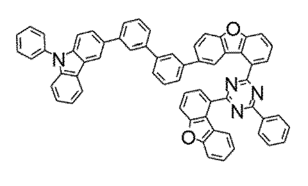 | 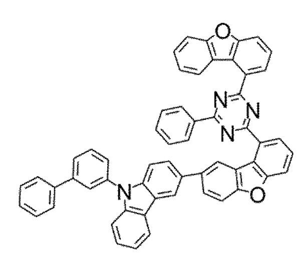 |

| | | |
|---|---|---|
| 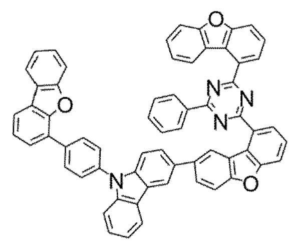 | 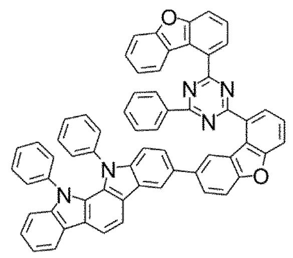 | 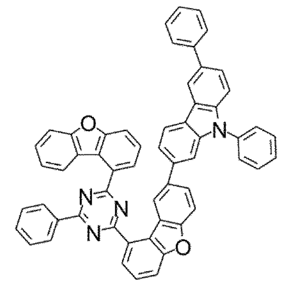 |
| 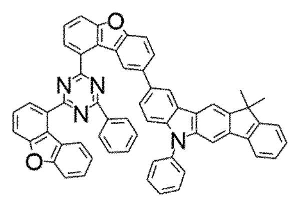 | 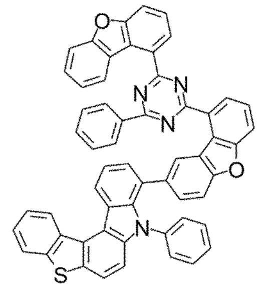 | 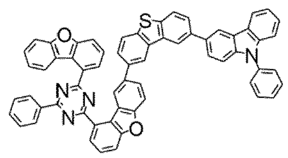 |
| 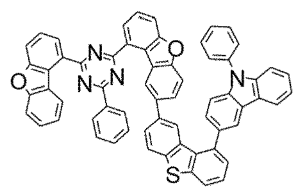 | 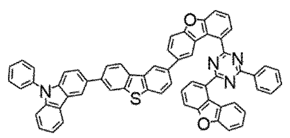 | 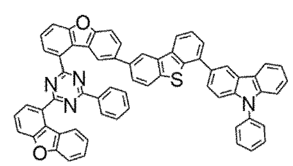 |
| 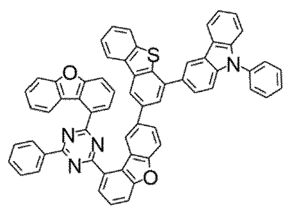 | 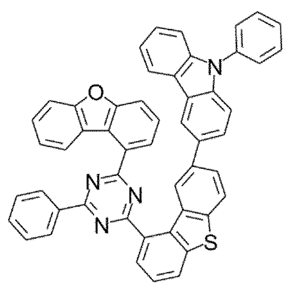 | 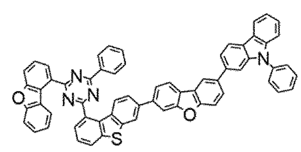 |
| 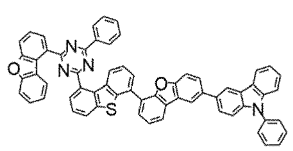 | 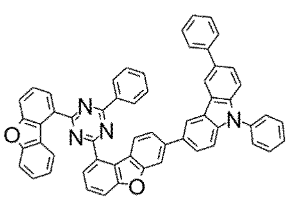 | 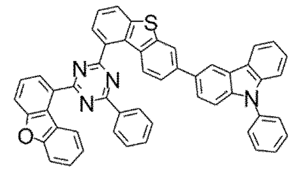 |

| | | |
|---|---|---|
| 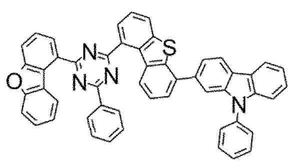 | 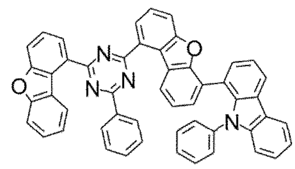 | 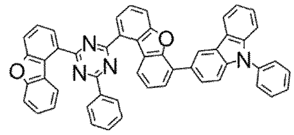 |
| 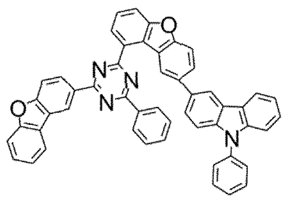 | 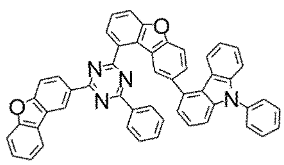 | 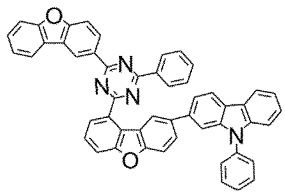 |
| 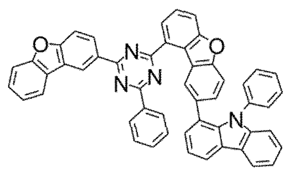 | 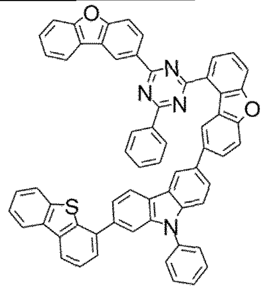 | 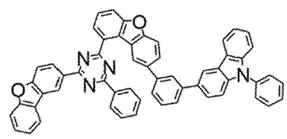 |
| 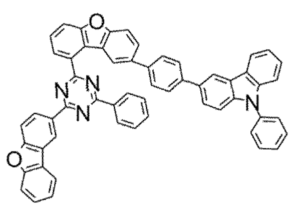 | 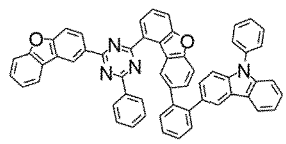 | 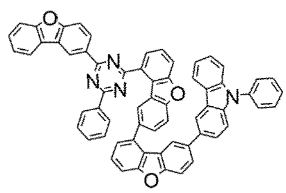 |
| 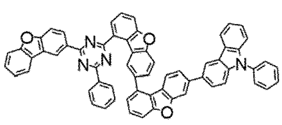 | 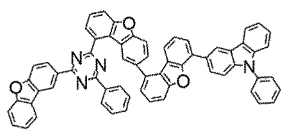 | 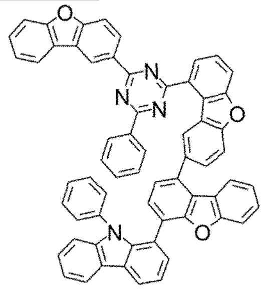 |

| | | |
|---|---|---|
| 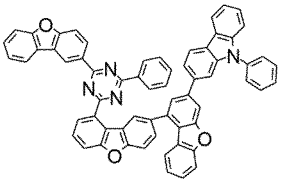 | 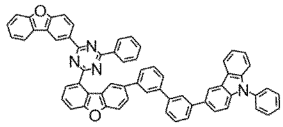 | 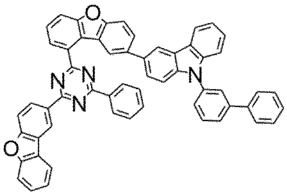 |
| 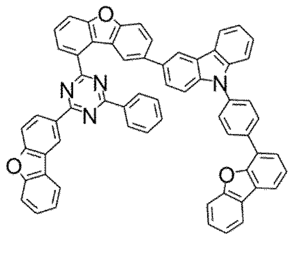 | 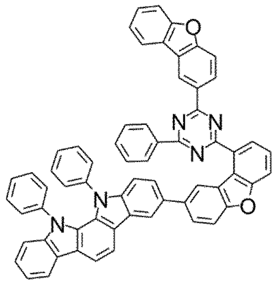 | 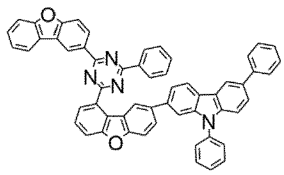 |
| 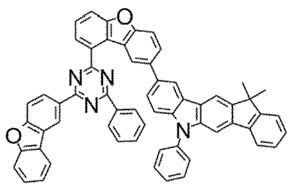 | 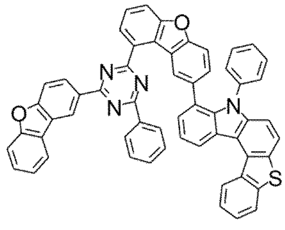 | 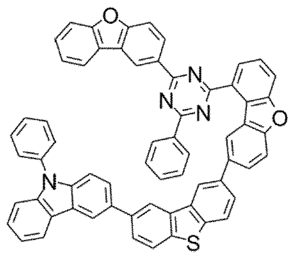 |
| 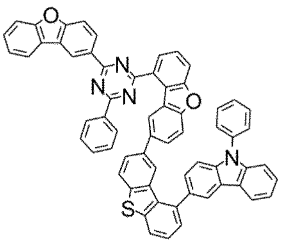 | 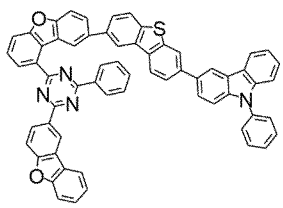 | 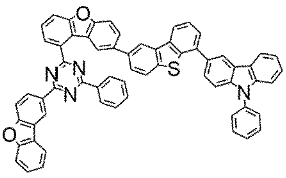 |
| 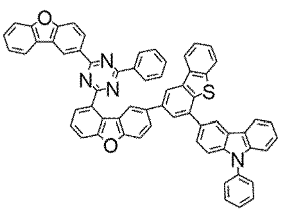 | 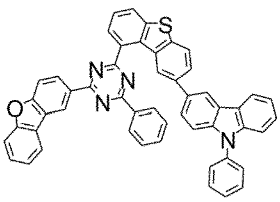 | 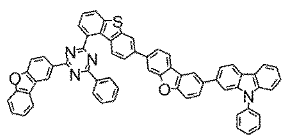 |

| | | |
|---|---|---|
| 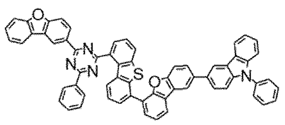 | 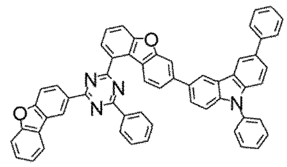 | 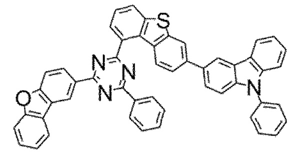 |
| 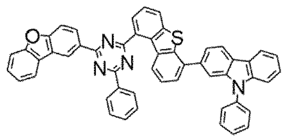 | 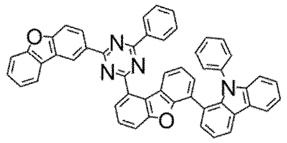 | 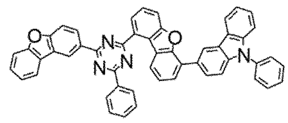 |
| 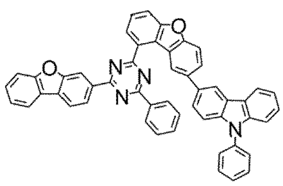 | 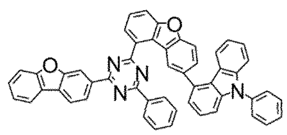 | 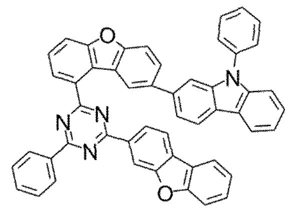 |
| 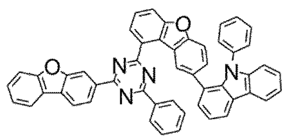 | 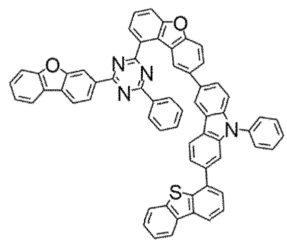 | 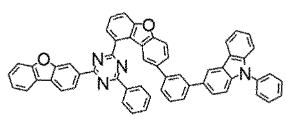 |
| 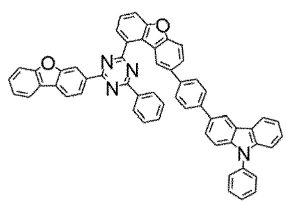 | 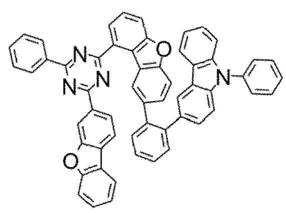 | 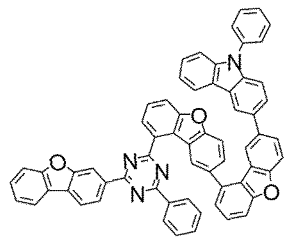 |

| | | |
|---|---|---|
| 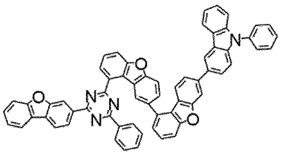 | 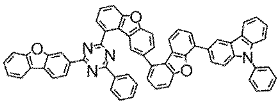 | 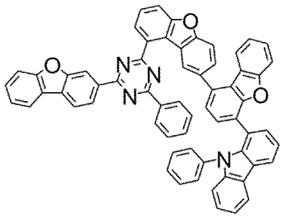 |
| 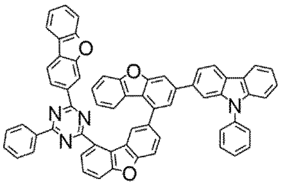 | 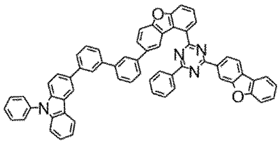 | 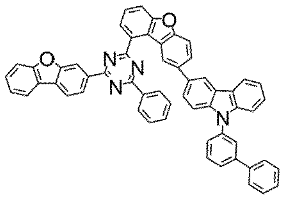 |
| 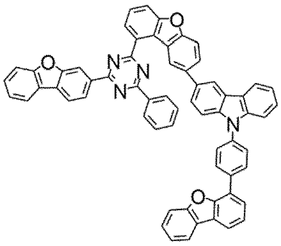 | 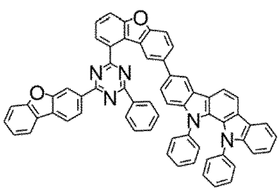 | 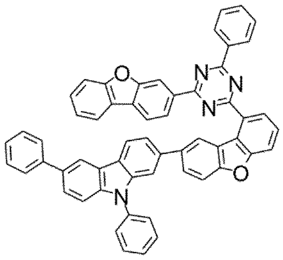 |
| 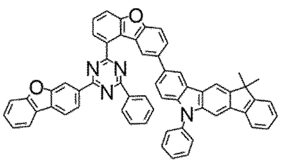 | 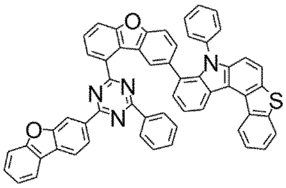 | 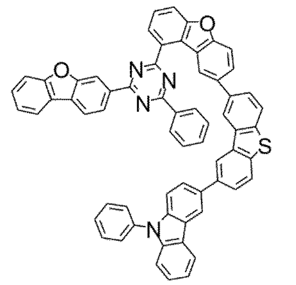 |
| 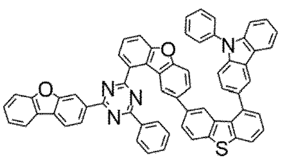 | 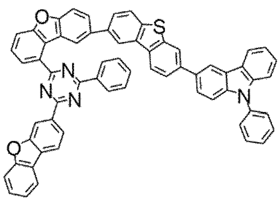 | 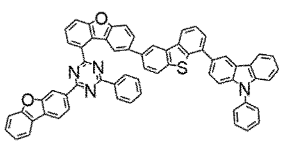 |

| | | |
|---|---|---|
| 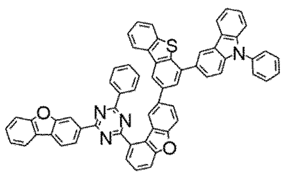 | 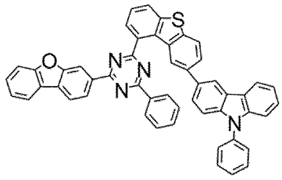 | 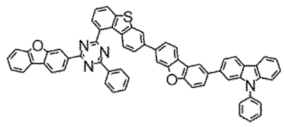 |
| 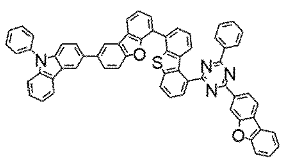 | 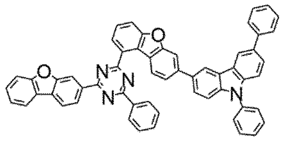 | 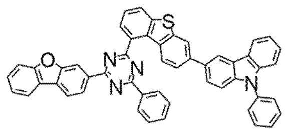 |
| 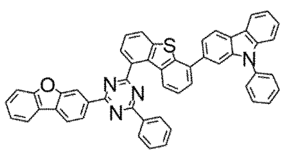 | 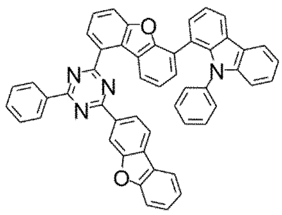 | 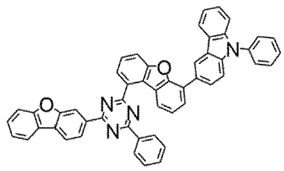 |
| 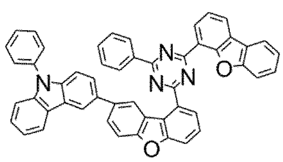 | 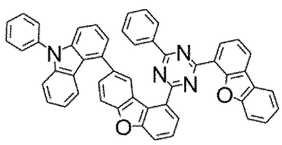 | 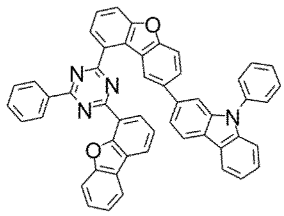 |
| 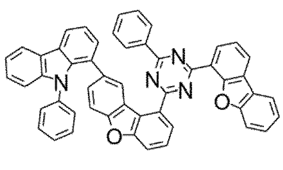 | 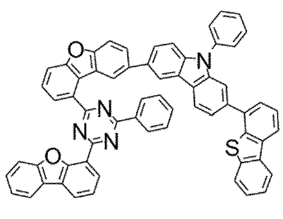 | 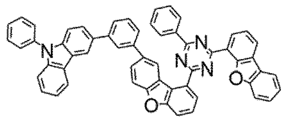 |

| | | |
|---|---|---|
| 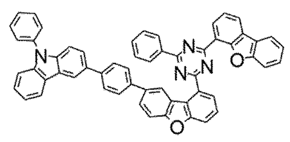 | 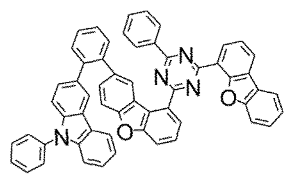 | 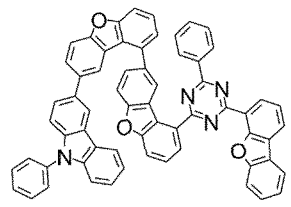 |
| 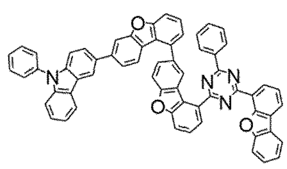 | 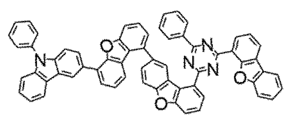 | 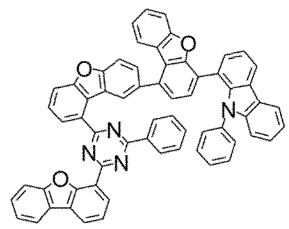 |
| 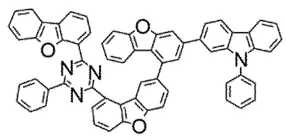 | 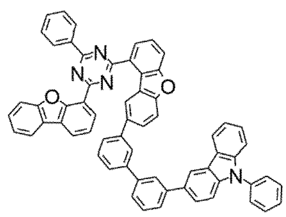 | 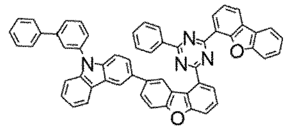 |
| 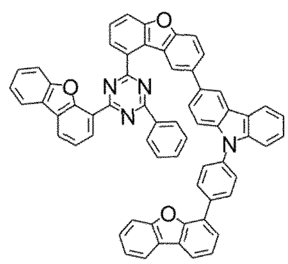 | 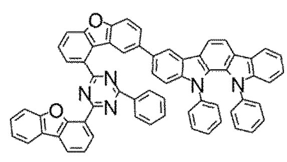 | 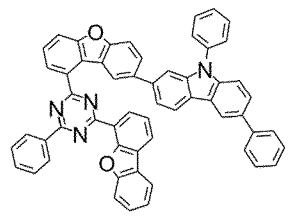 |
| 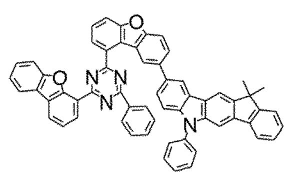 | 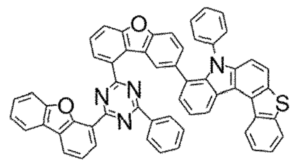 | 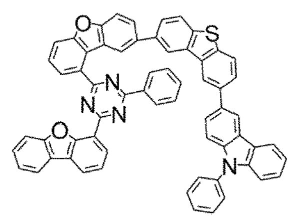 |

| | | |
|---|---|---|
| 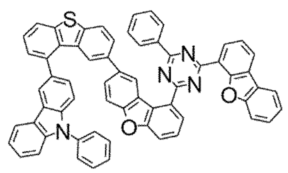 | 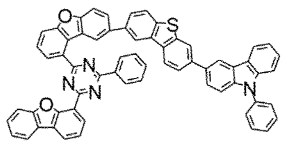 | 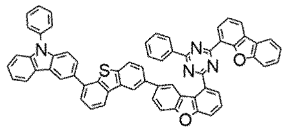 |
| 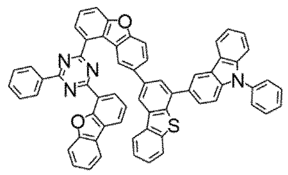 | 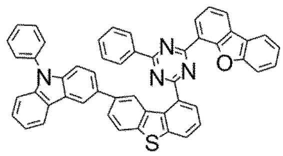 | 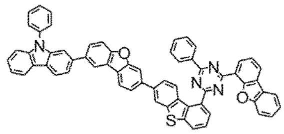 |
| 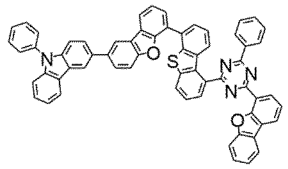 | 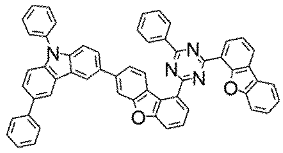 | 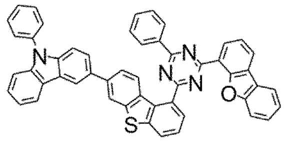 |
| 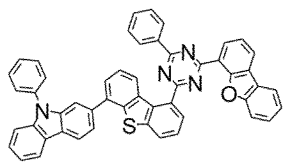 | 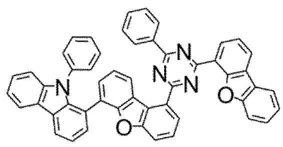 | 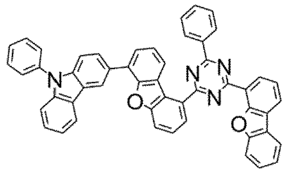 |
| 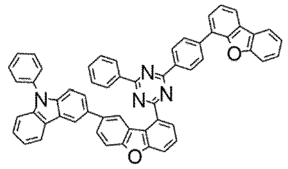 | 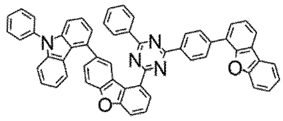 | 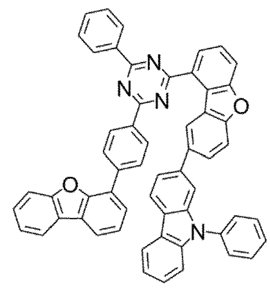 |

| | | |
|---|---|---|
| 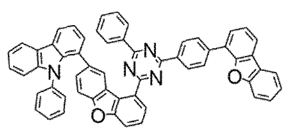 | 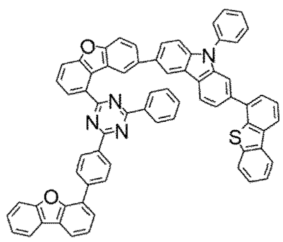 | 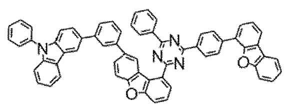 |
| 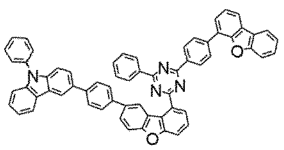 | 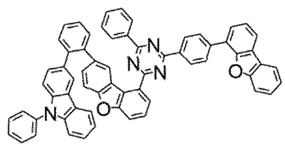 | 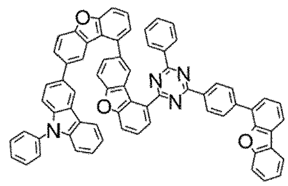 |
| 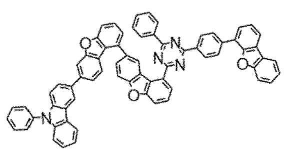 | 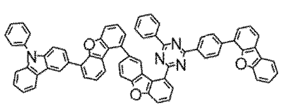 | 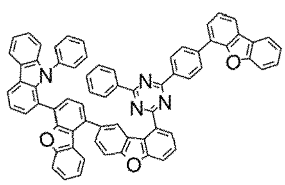 |
| 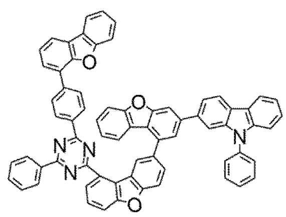 | 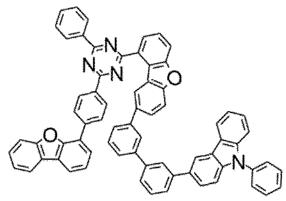 | 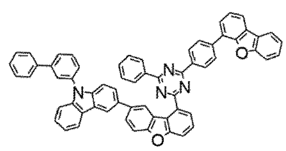 |
| 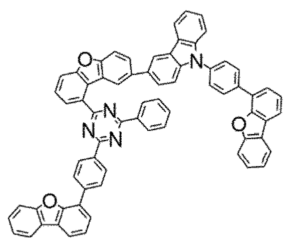 | 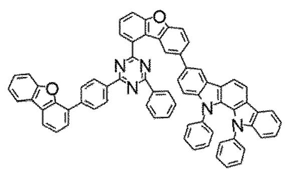 | 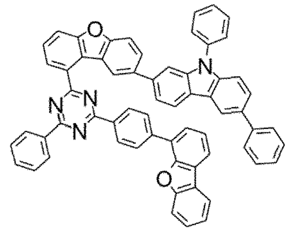 |

| | | |
|---|---|---|
| 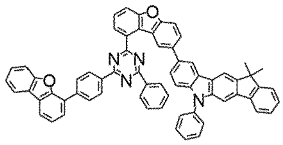 | 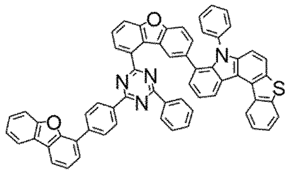 | 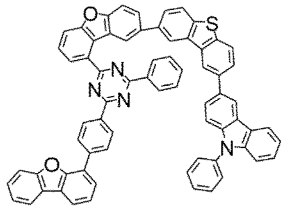 |
| 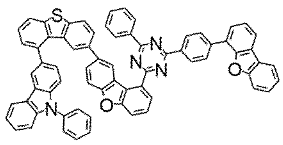 | 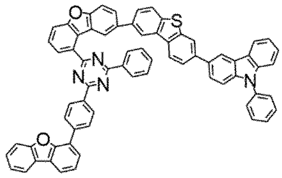 | 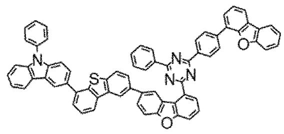 |
| 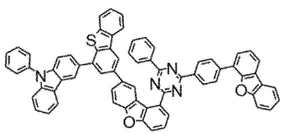 | 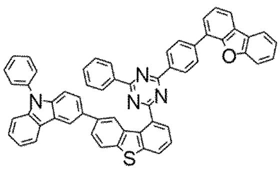 | 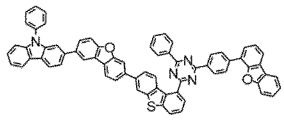 |
| 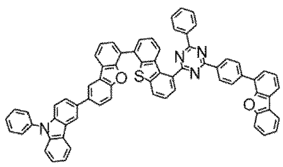 | 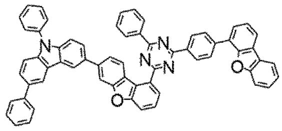 | 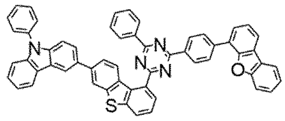 |
| 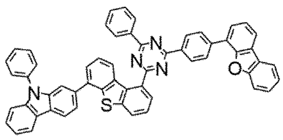 | 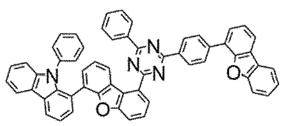 | 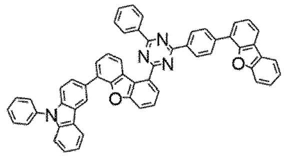 |

| | | |
|---|---|---|
| 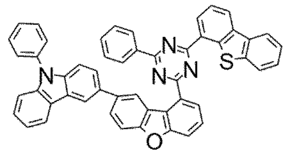 | 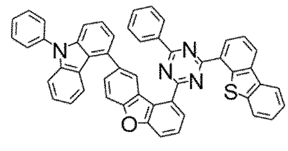 | 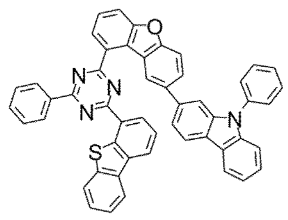 |
| 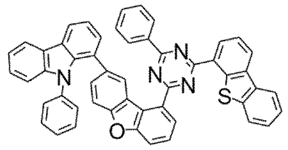 | 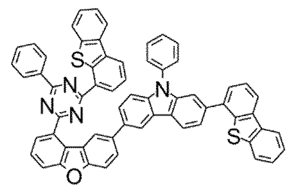 | 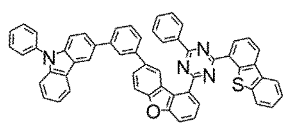 |
| 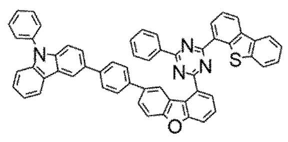 | 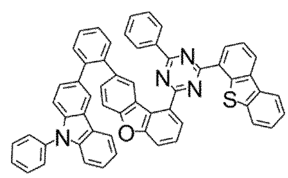 | 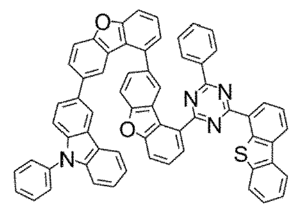 |
| 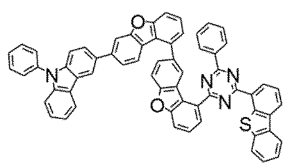 | 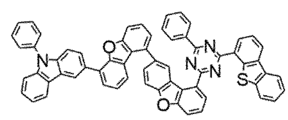 | 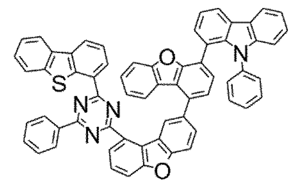 |
| 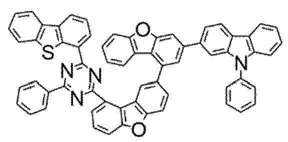 | 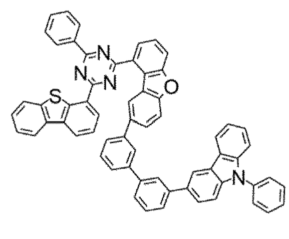 | 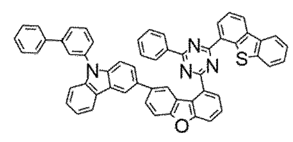 |

| | | |
|---|---|---|
| 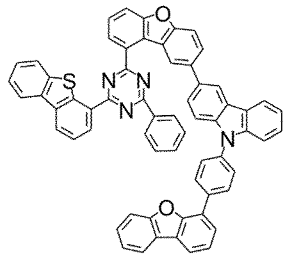 | 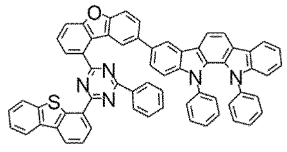 | 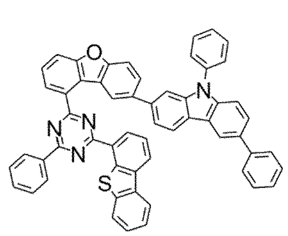 |
| 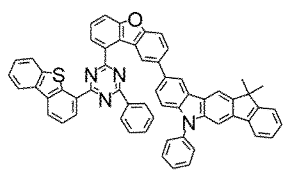 | 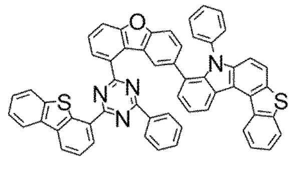 | 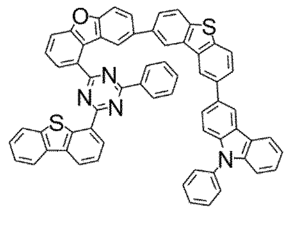 |
| 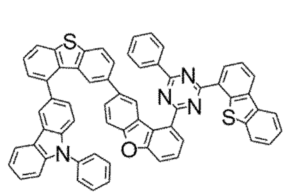 | 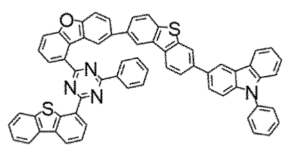 | 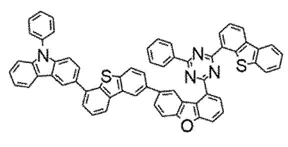 |
| 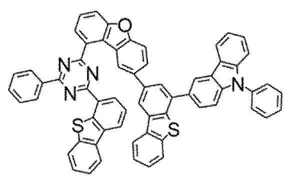 | 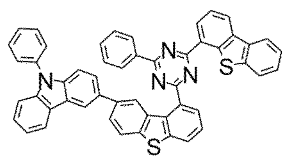 | 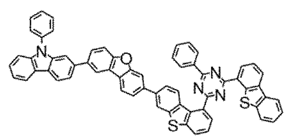 |
| 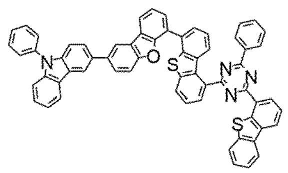 | 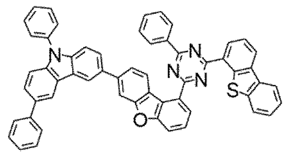 | 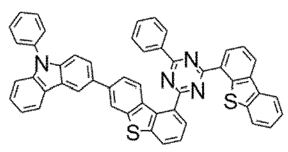 |

| | | |
|---|---|---|
| 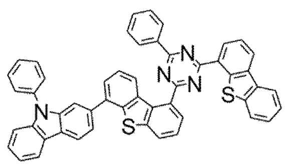 | 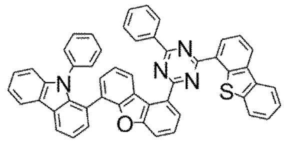 | 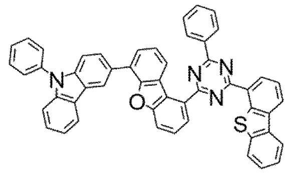 |
| 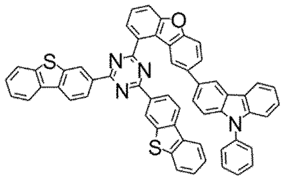 | 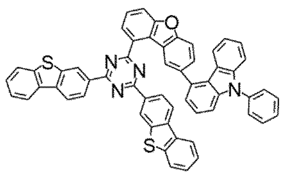 | 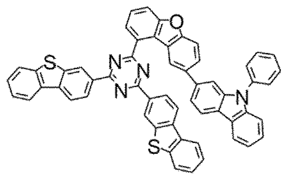 |
| 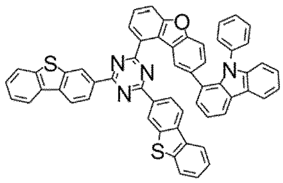 | 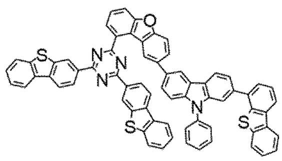 | 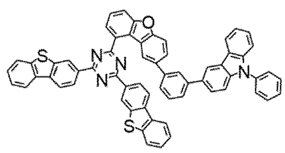 |
| 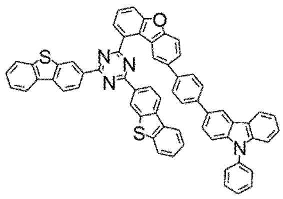 | 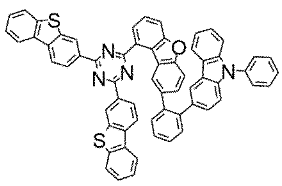 | 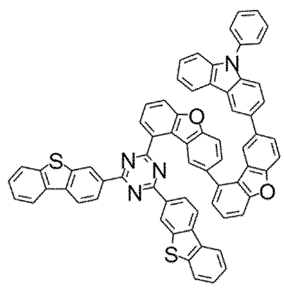 |
| 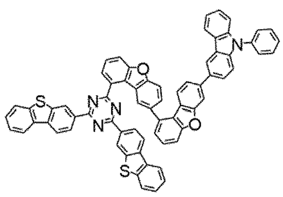 | 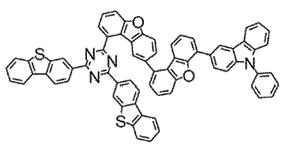 | 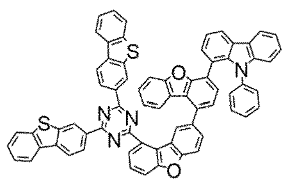 |

| | | |
|---|---|---|
| 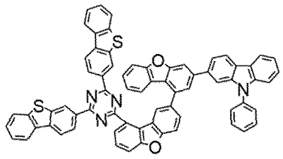 | 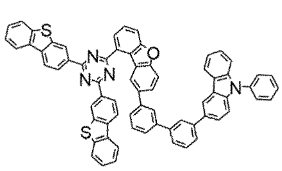 | 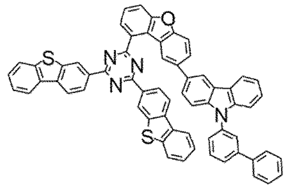 |
| 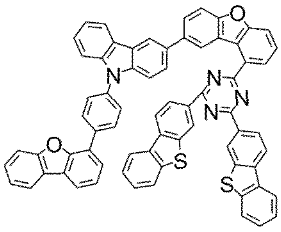 | 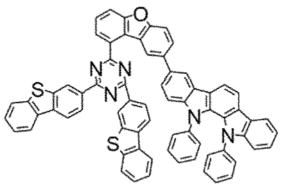 | 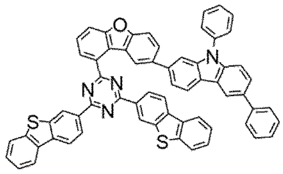 |
| 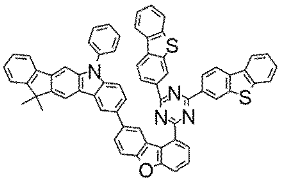 | 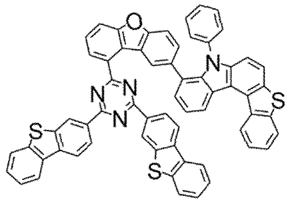 | 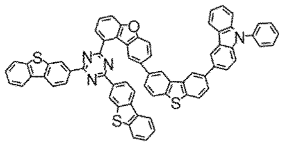 |
| 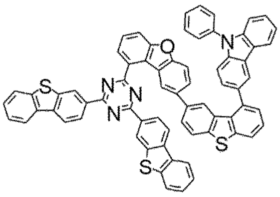 | 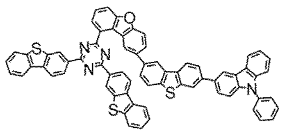 | 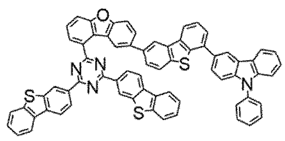 |
| 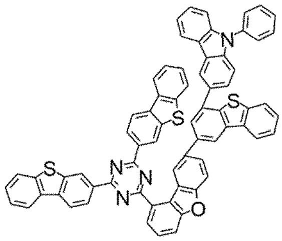 | 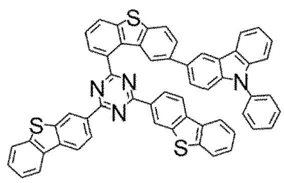 | 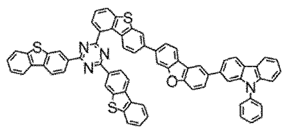 |

| | | |
|---|---|---|
| 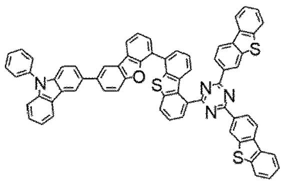 | 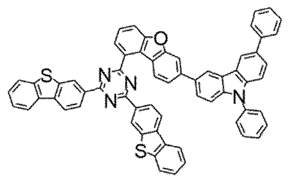 | 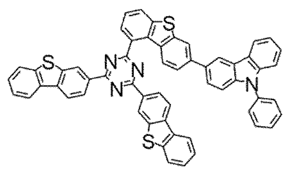 |
| 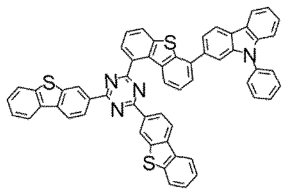 | 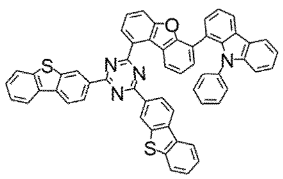 | 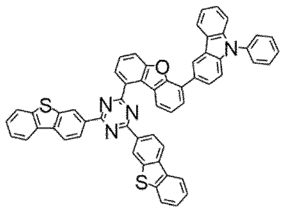 |
| 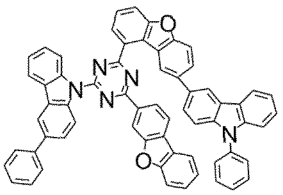 | 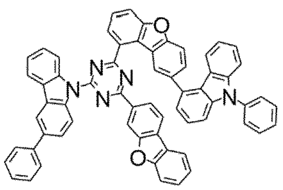 | 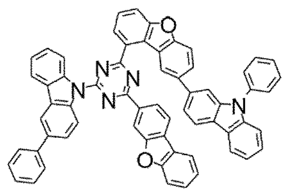 |
| 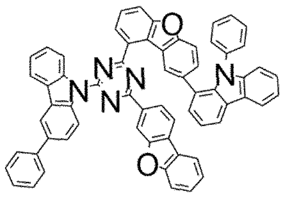 | 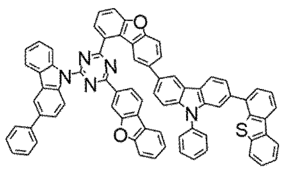 | 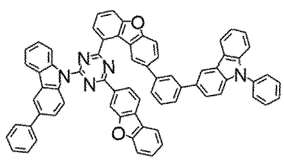 |
| 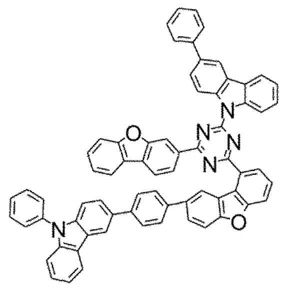 | 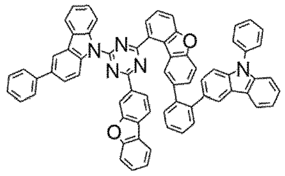 | 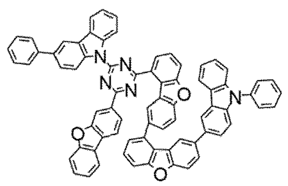 |

| | | |
|---|---|---|
| 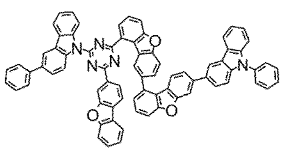 | 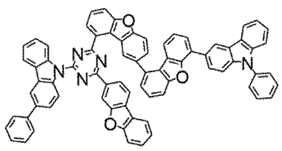 | 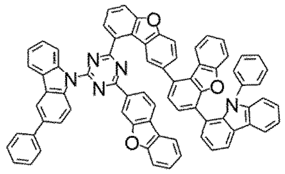 |
| 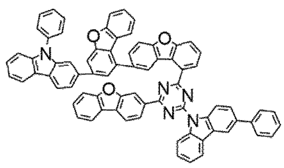 | 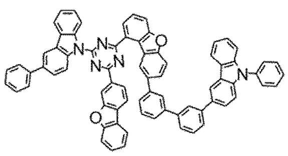 | 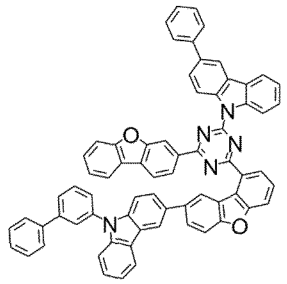 |
| 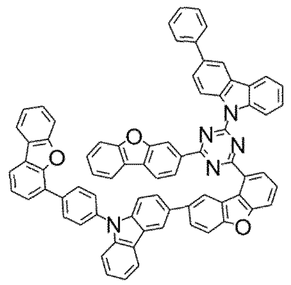 | 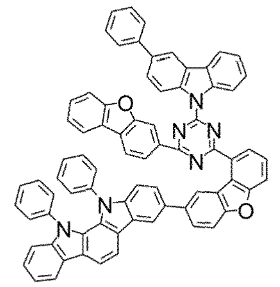 | 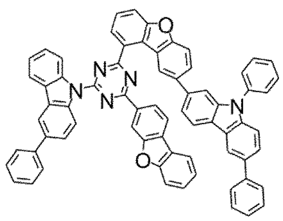 |
| 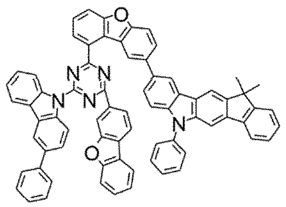 | 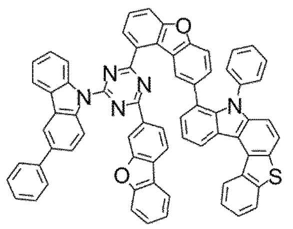 | 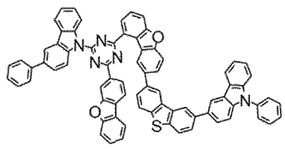 |
| 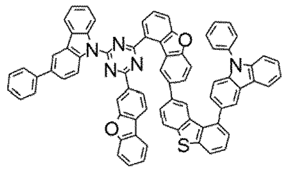 | 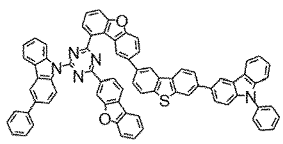 | 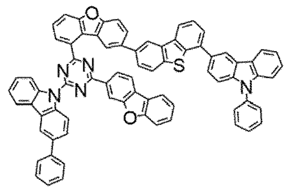 |

| | | |
|---|---|---|
| 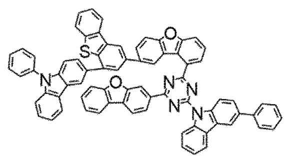 | 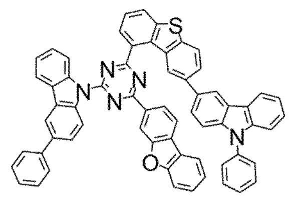 | 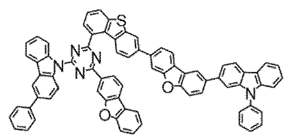 |
| 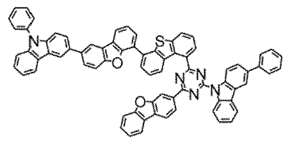 | 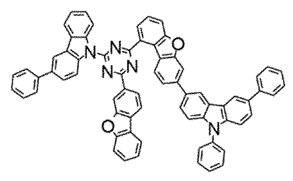 | 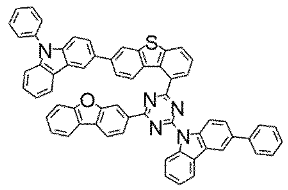 |
| 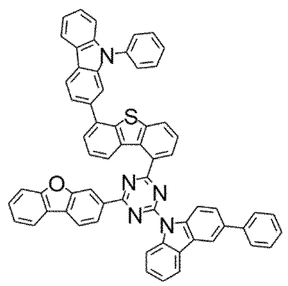 | 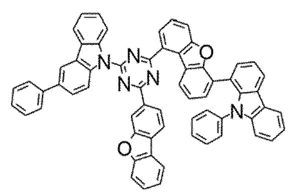 | 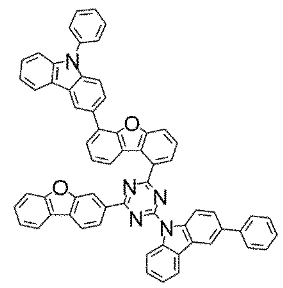 |
| 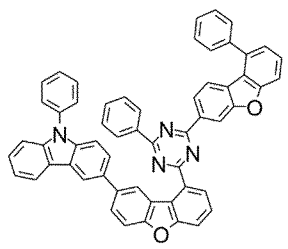 | 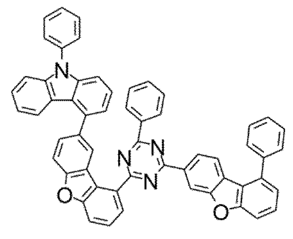 | 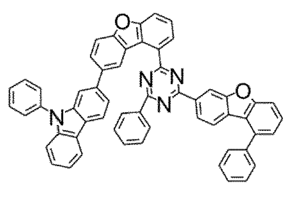 |
| 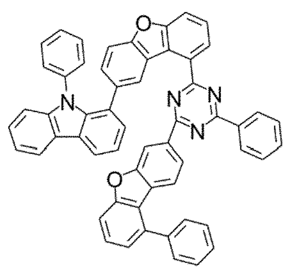 | 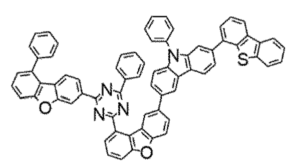 | 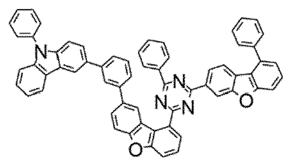 |

| | | |
|---|---|---|
| 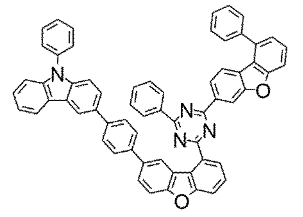 | 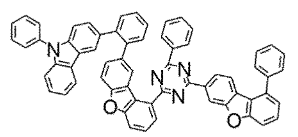 | 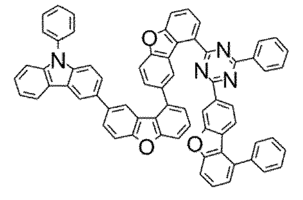 |
| 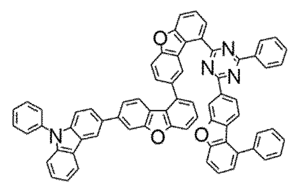 | 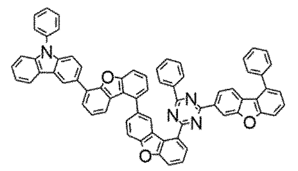 | 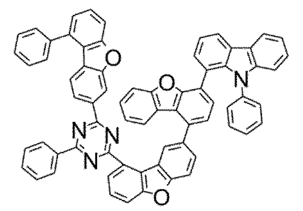 |
| 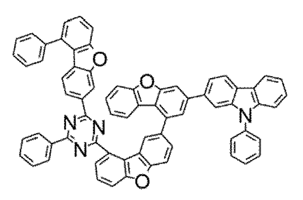 | 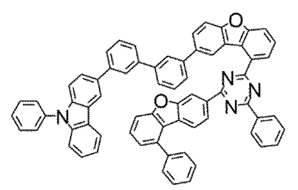 | 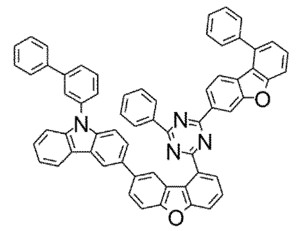 |
| 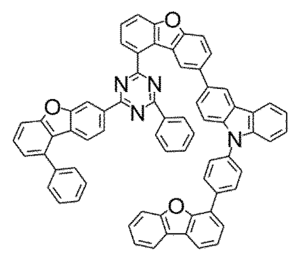 | 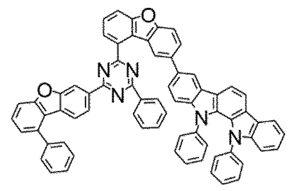 | 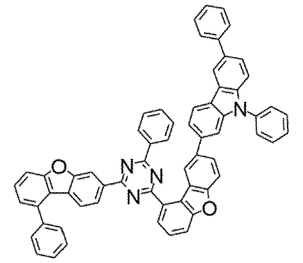 |
| 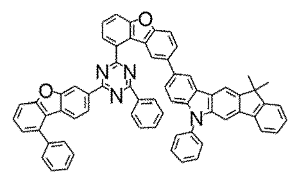 | 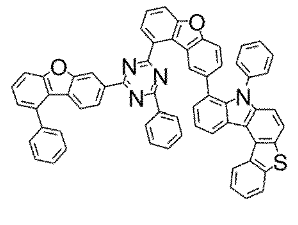 | 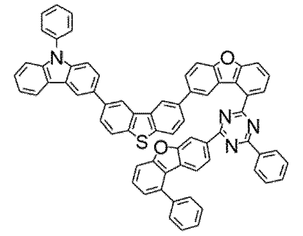 |

| | | |
|---|---|---|
| 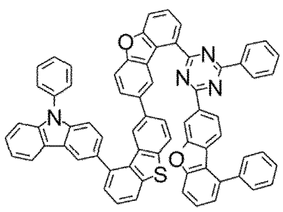 | 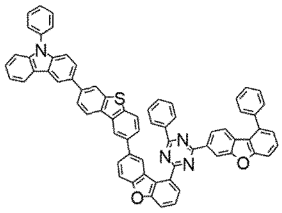 | 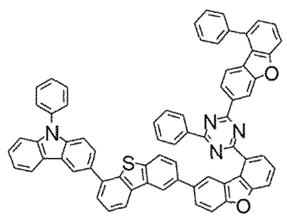 |
| 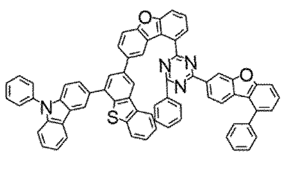 | 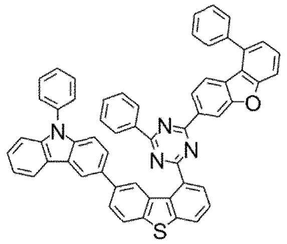 | 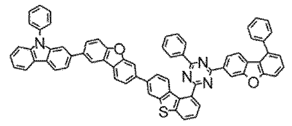 |
| 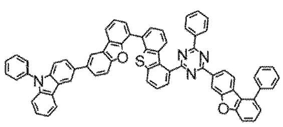 | 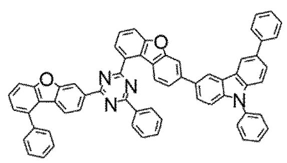 | 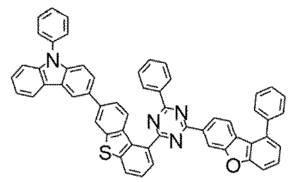 |
| 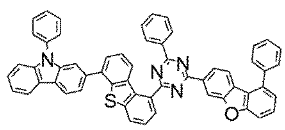 | 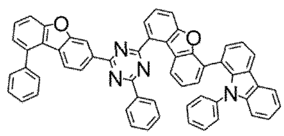 | 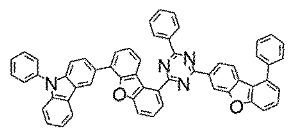 |
| 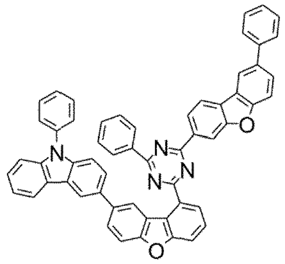 | 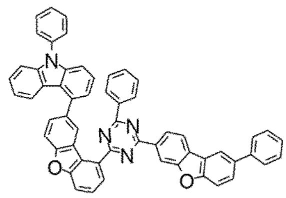 | 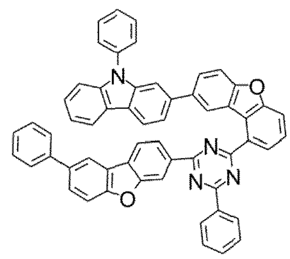 |

| | | |
|---|---|---|
| 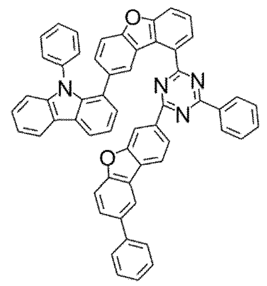 | 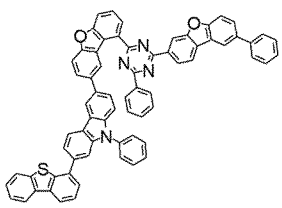 | 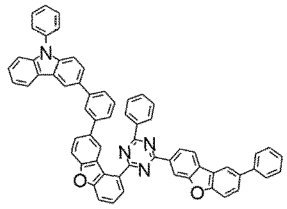 |
| 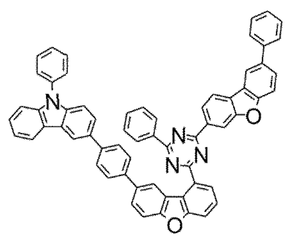 | 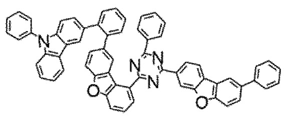 | 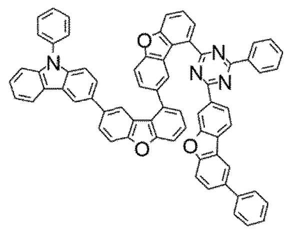 |
| 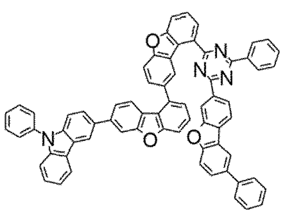 | 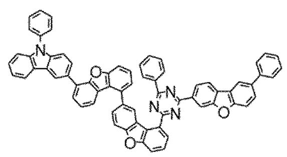 | 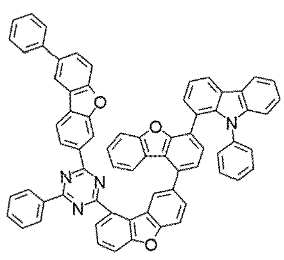 |
| 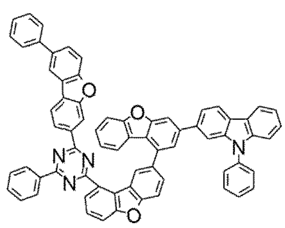 | 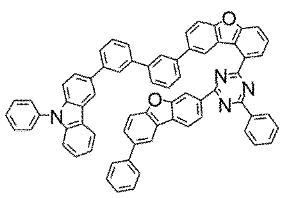 | 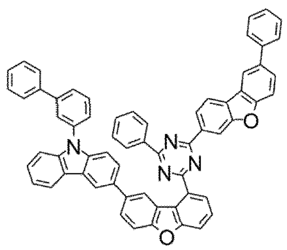 |
| 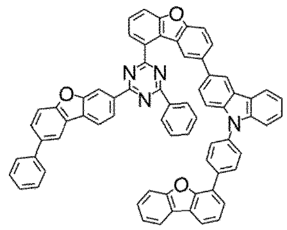 | 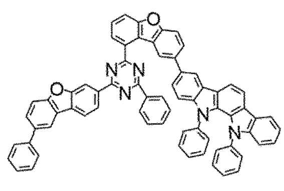 | 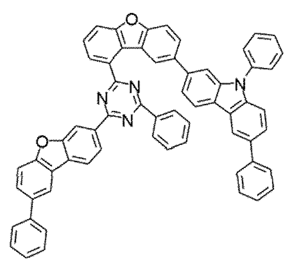 |

| | | |
|---|---|---|
| 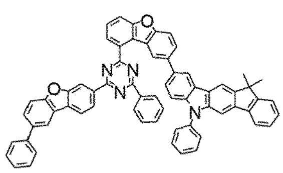 | 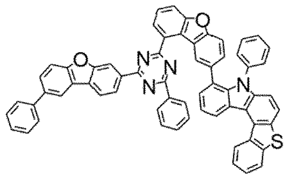 | 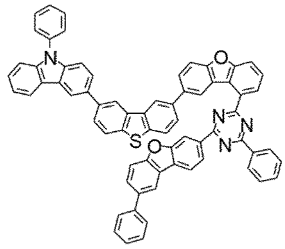 |
| 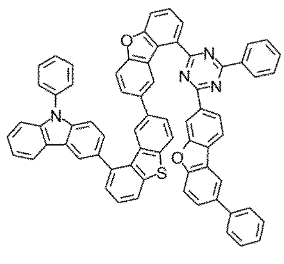 | 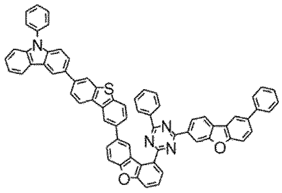 | 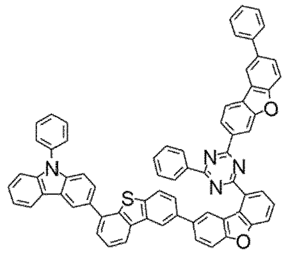 |
| 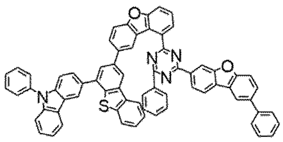 | 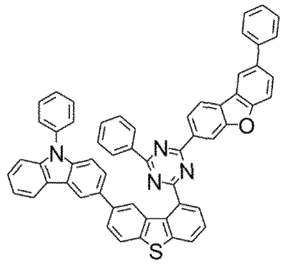 | 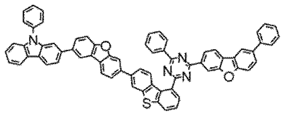 |
| 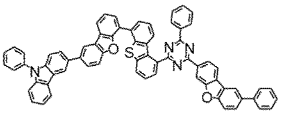 | 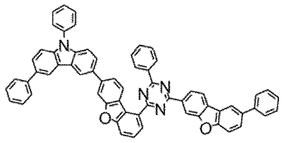 | 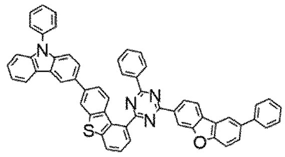 |
| 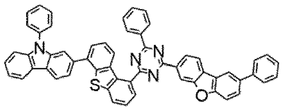 | 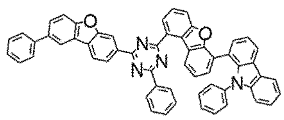 | 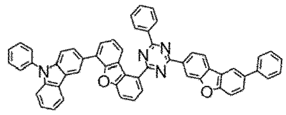 |

| | | |
|---|---|---|
| 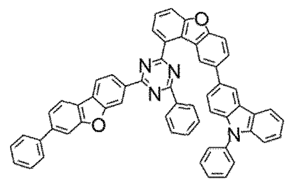 | 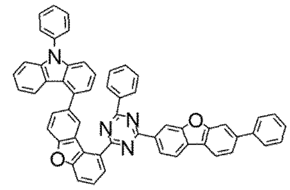 | 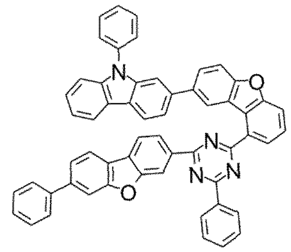 |
| 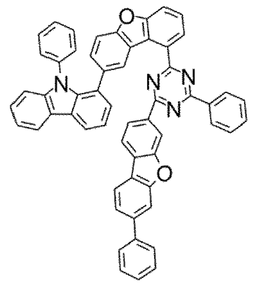 | 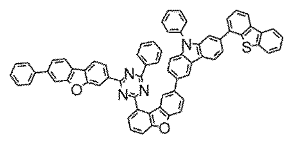 | 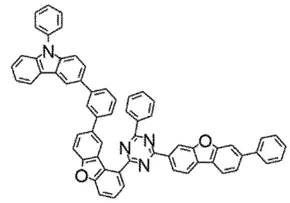 |
| 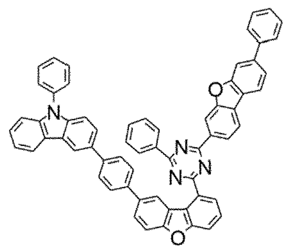 | 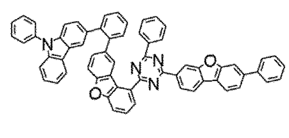 | 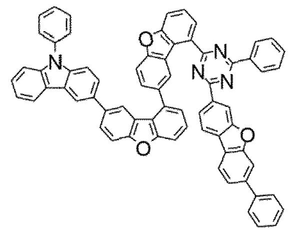 |
| 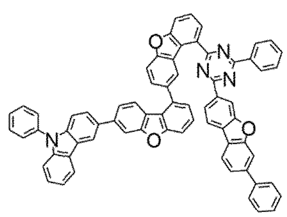 | 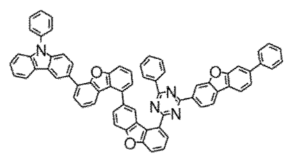 | 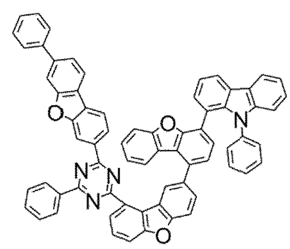 |
| 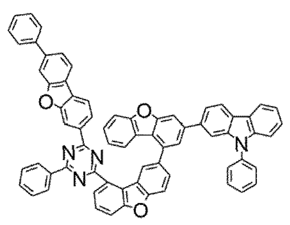 | 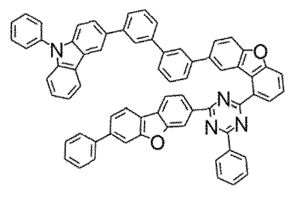 | 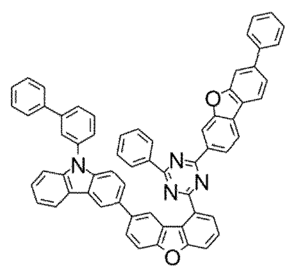 |

| | | |
|---|---|---|
| 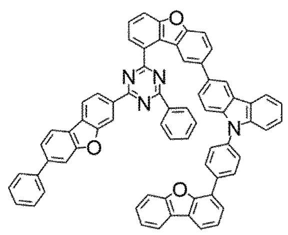 | 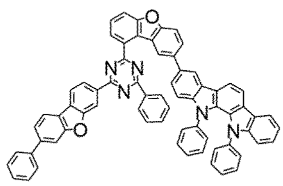 | 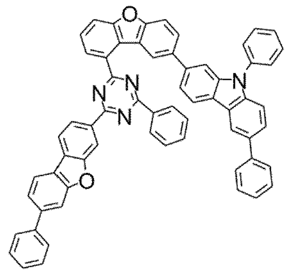 |
| 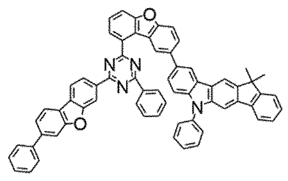 | 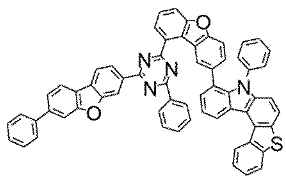 | 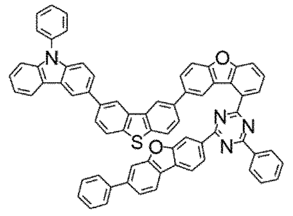 |
| 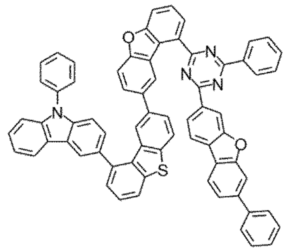 | 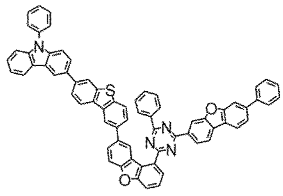 | 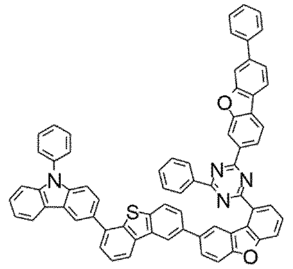 |
| 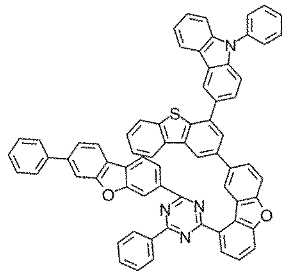 | 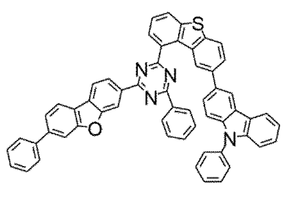 | 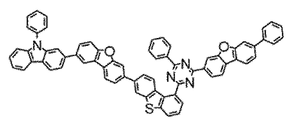 |
| 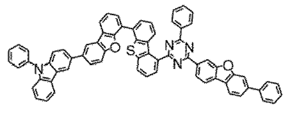 | 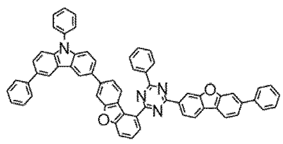 | 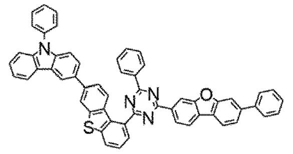 |

| | | |
|---|---|---|
| 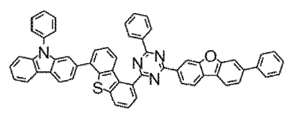 | 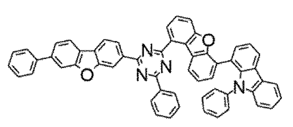 | 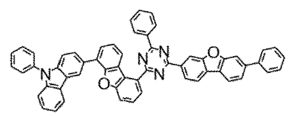 |
| 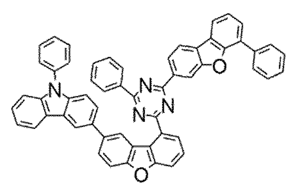 | 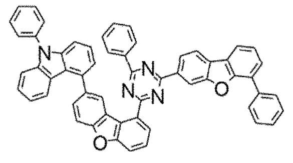 | 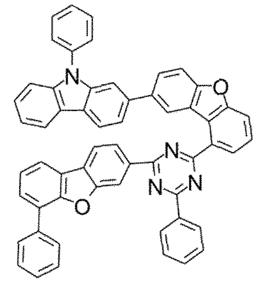 |
| 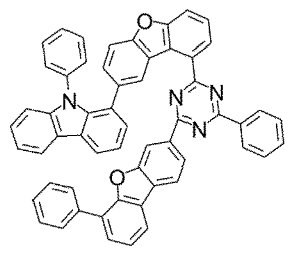 | 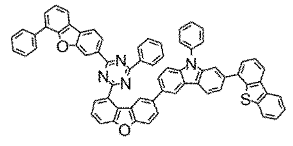 | 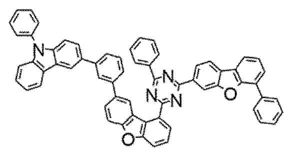 |
| 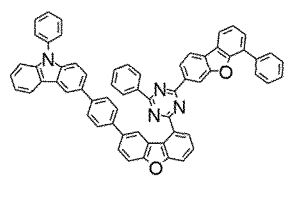 | 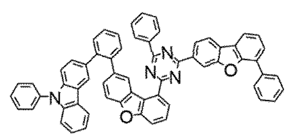 | 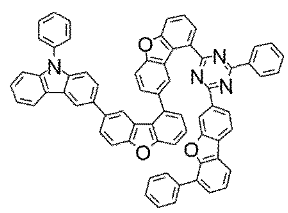 |
| 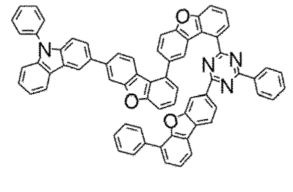 | 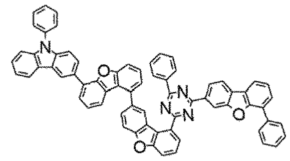 | 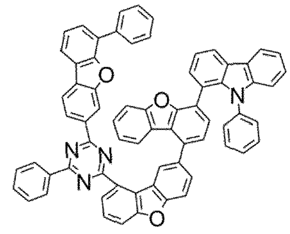 |

| | | |
|---|---|---|
| 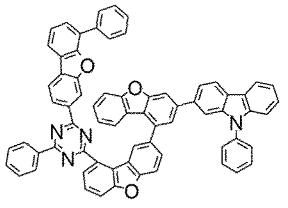 | 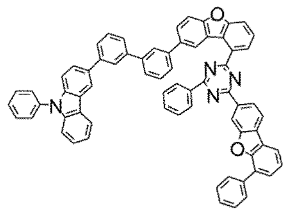 | 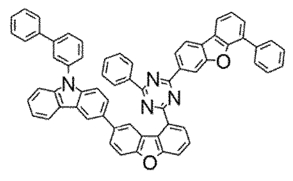 |
| 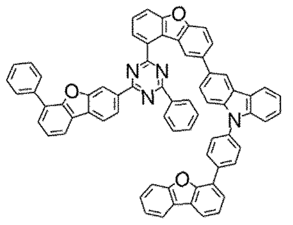 | 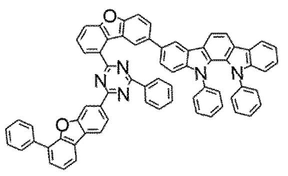 | 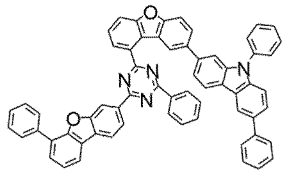 |
| 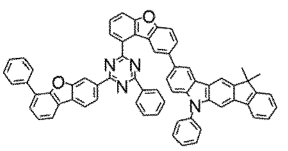 | 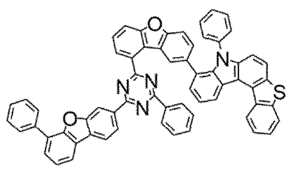 | 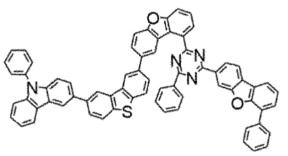 |
| 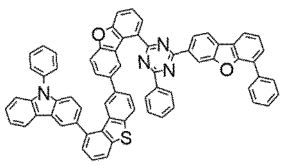 | 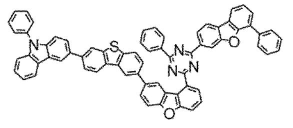 | 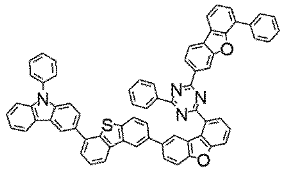 |
| 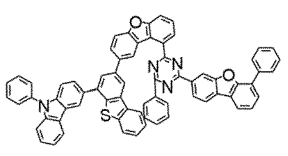 | 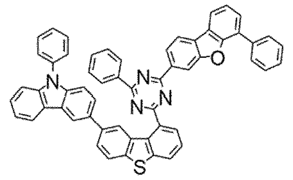 | 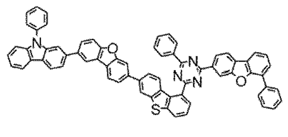 |

| | | |
|---|---|---|
| 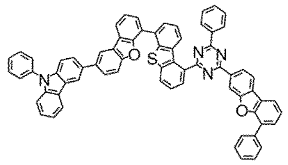 | 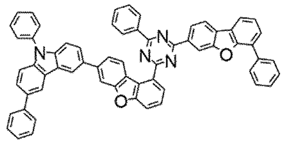 | 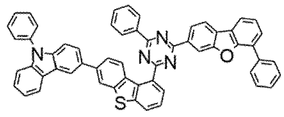 |
| 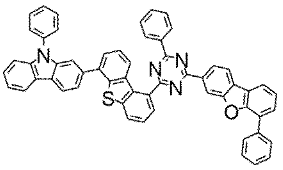 | 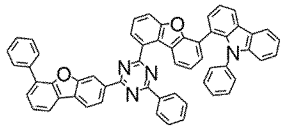 | 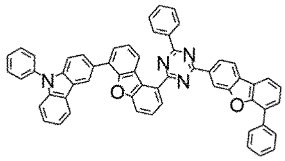 |
| 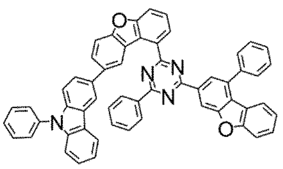 | 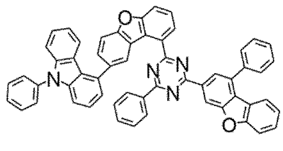 | 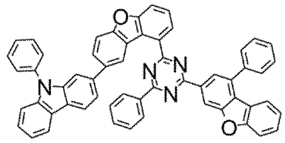 |
| 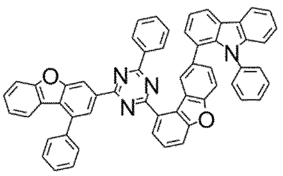 | 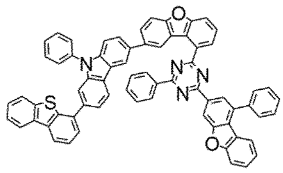 | 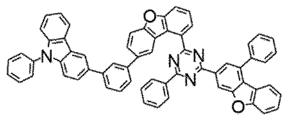 |
| 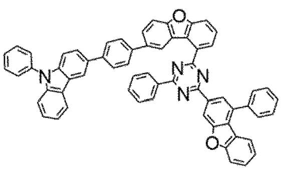 | 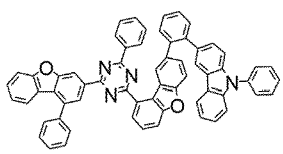 | 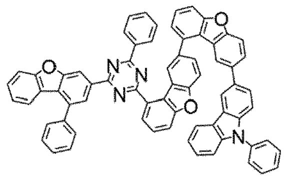 |

| | | |
|---|---|---|
| 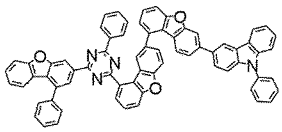 | 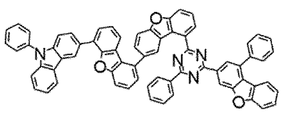 | 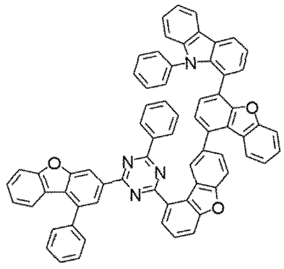 |
| 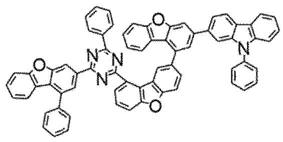 | 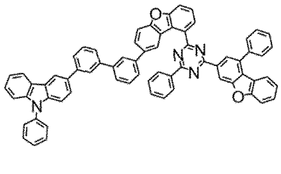 | 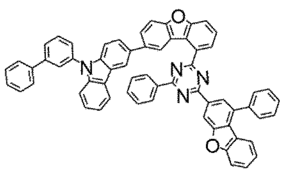 |
| 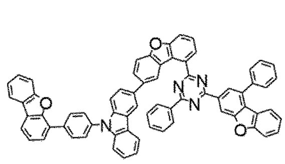 | 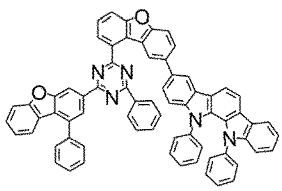 | 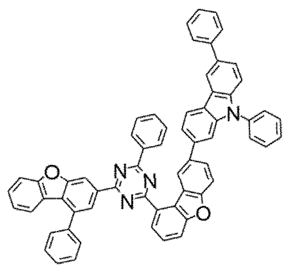 |
| 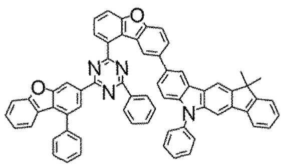 | 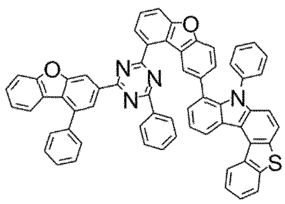 | 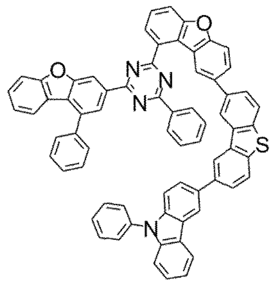 |
| 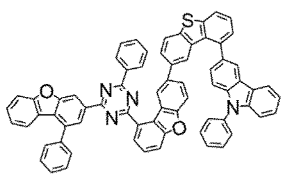 | 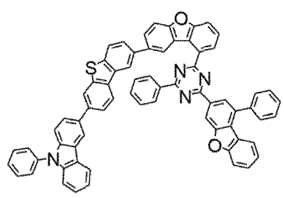 | 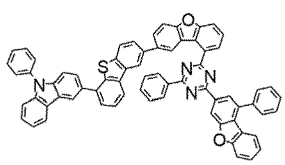 |

| | | |
|---|---|---|
| 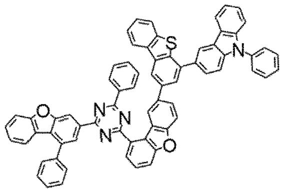 | 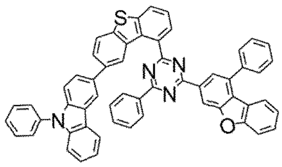 | 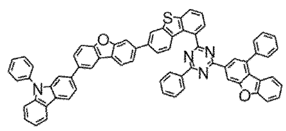 |
| 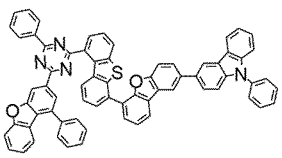 | 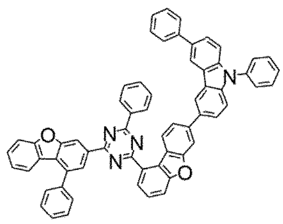 | 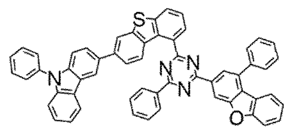 |
| 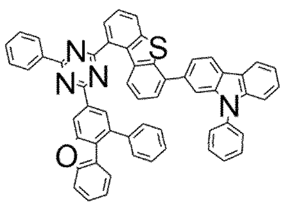 | 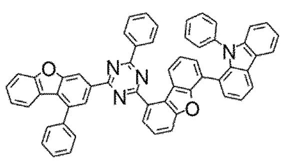 | 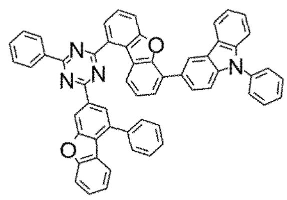 |
| 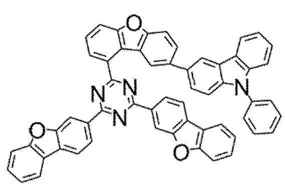 | 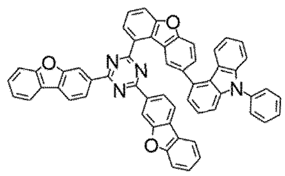 | 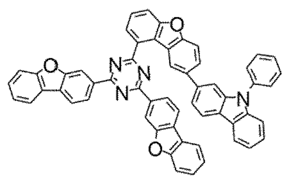 |
| 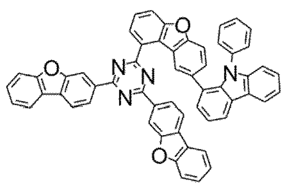 | 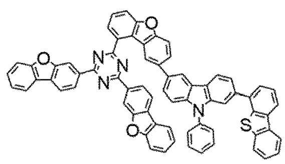 | 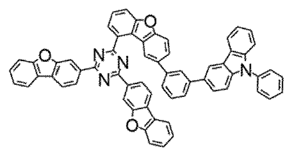 |

| | | |
|---|---|---|
| 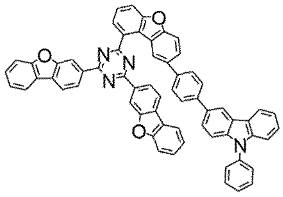 | 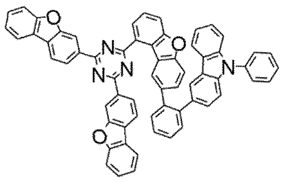 | 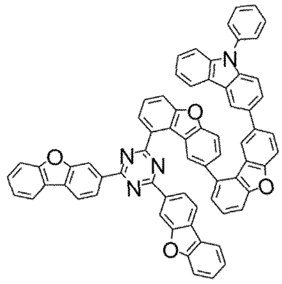 |
| 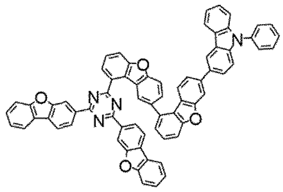 | 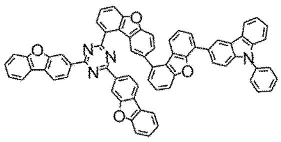 | 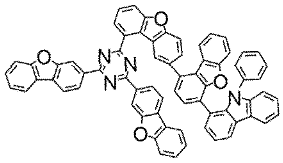 |
| 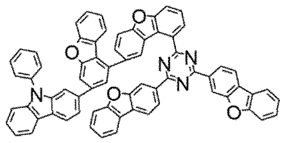 | 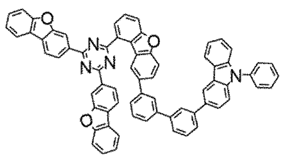 | 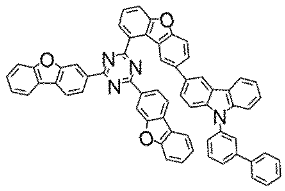 |
| 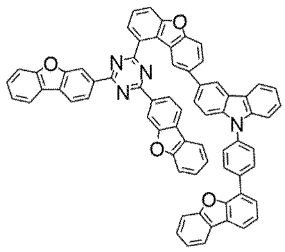 | 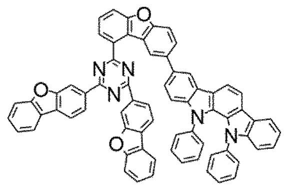 | 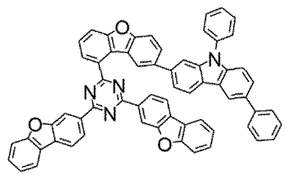 |
| 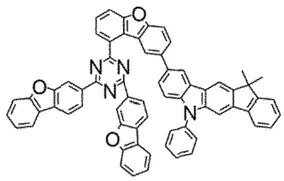 | 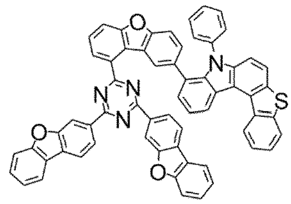 | 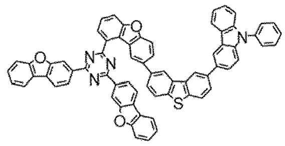 |

| | | |
|---|---|---|
| 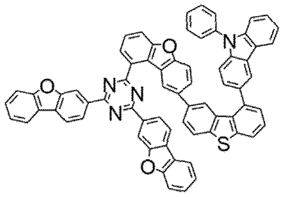 | 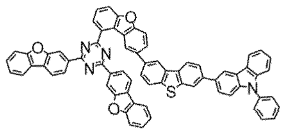 | 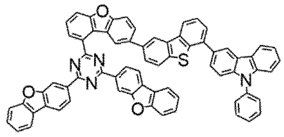 |
| 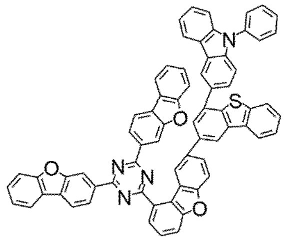 | 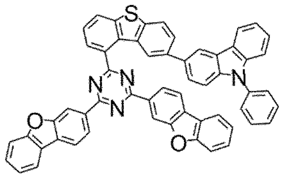 | 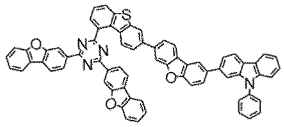 |
| 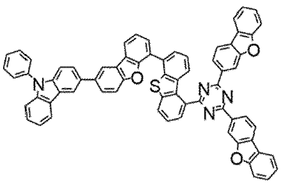 | 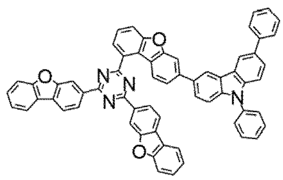 | 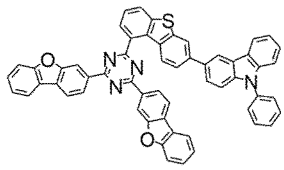 |
| 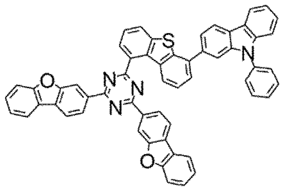 | 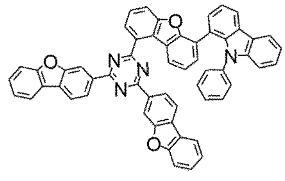 | 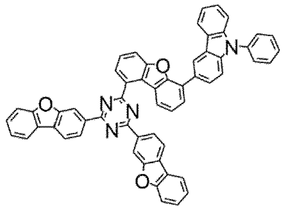 |
| 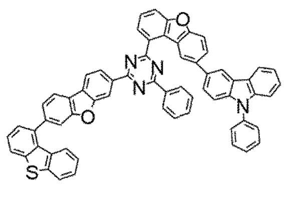 | 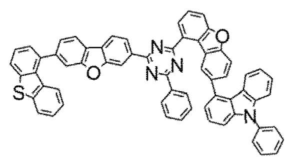 | 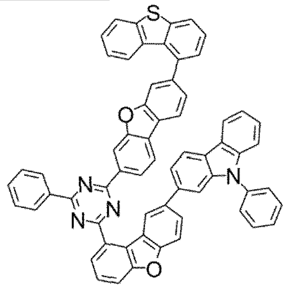 |

| | | |
|---|---|---|
| 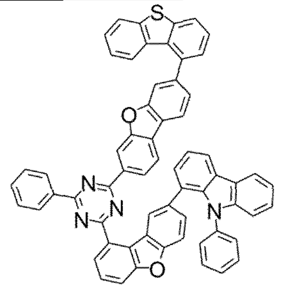 | 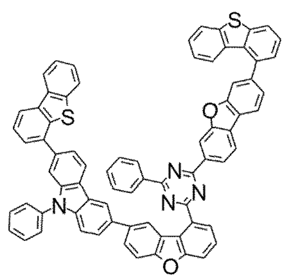 | 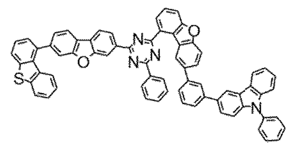 |
| 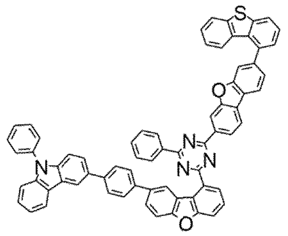 | 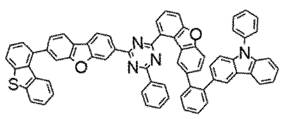 | 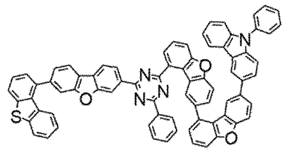 |
| 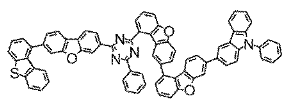 | 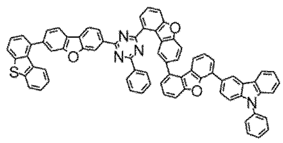 | 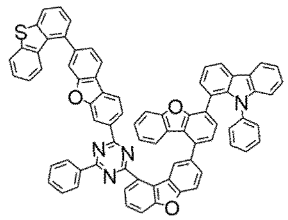 |
| 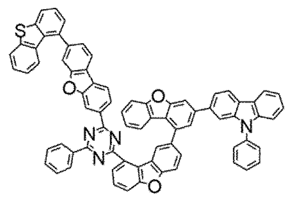 | 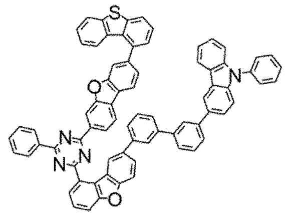 | 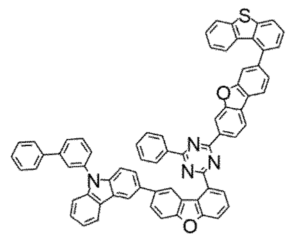 |
| 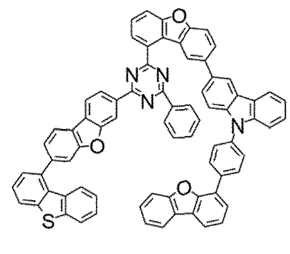 | 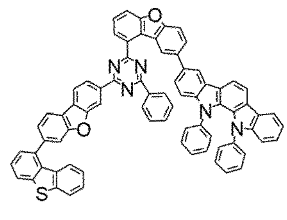 | 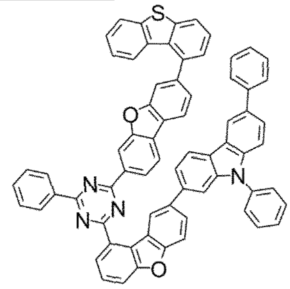 |

| | | |
|---|---|---|
| 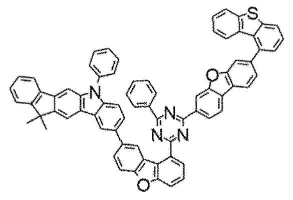 | 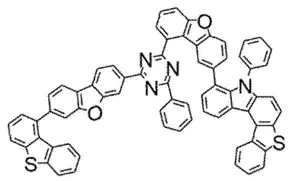 | 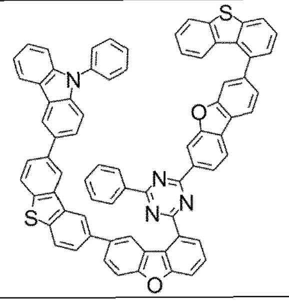 |
| 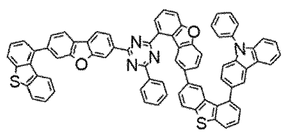 | 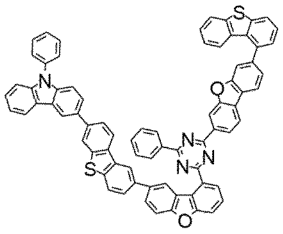 | 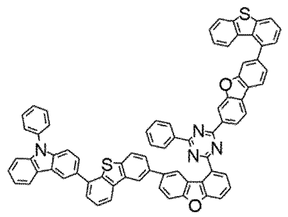 |
| 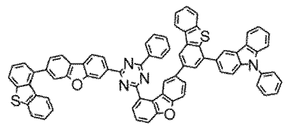 | 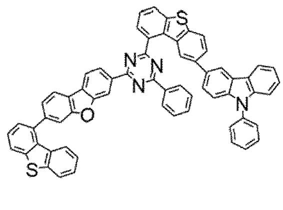 | 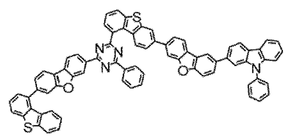 |
| 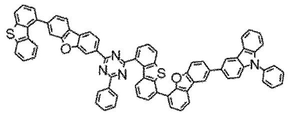 | 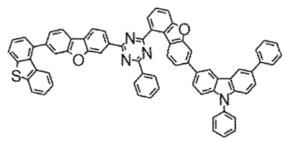 | 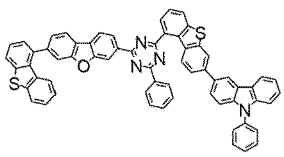 |
| 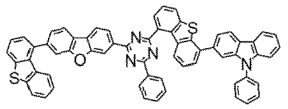 | 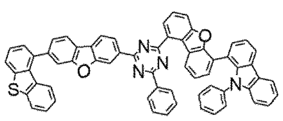 | 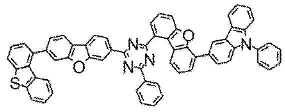 |

| | | |
|---|---|---|
| 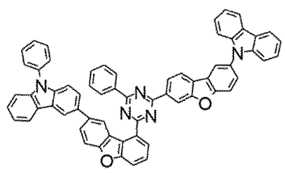 | 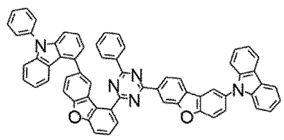 | 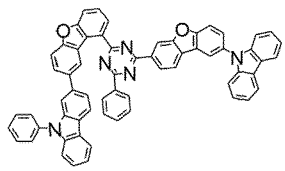 |
| 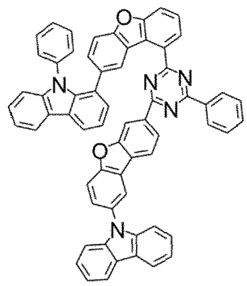 | 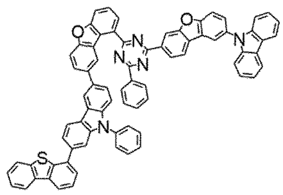 | 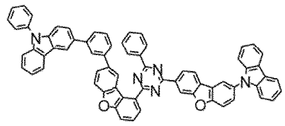 |
| 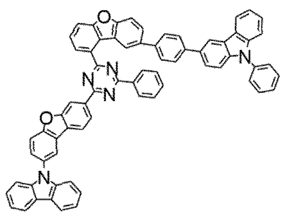 | 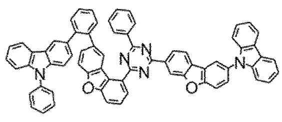 | 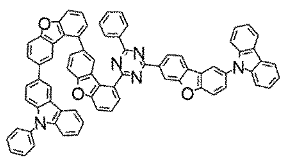 |
| 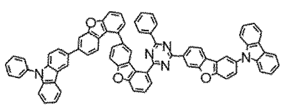 | 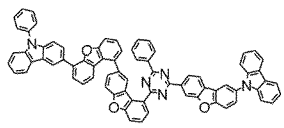 | 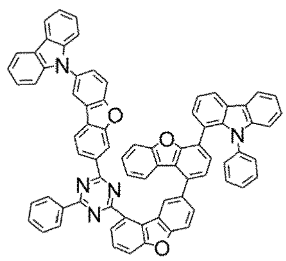 |
| 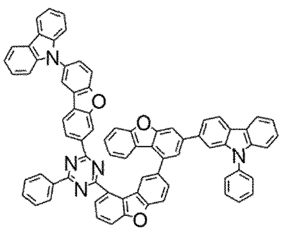 | 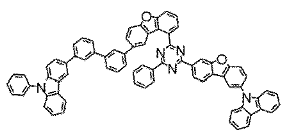 | 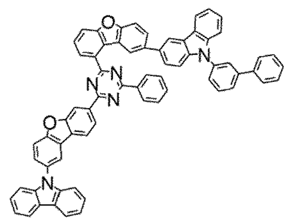 |

| | | |
|---|---|---|
| 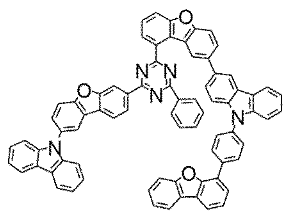 | 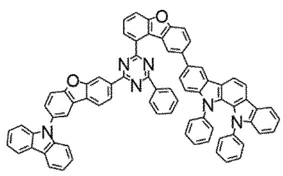 | 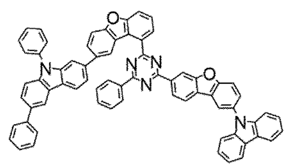 |
| 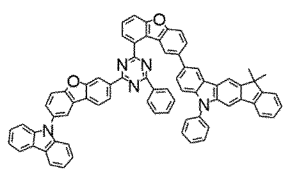 | 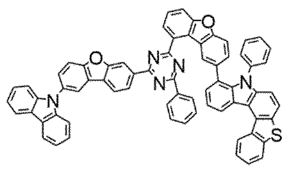 | 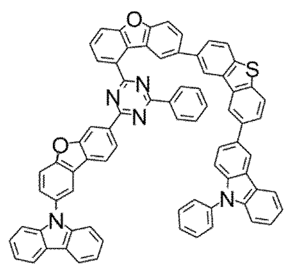 |
| 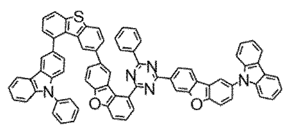 | 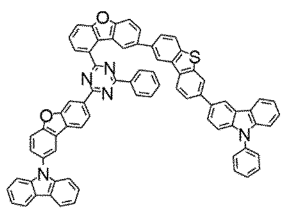 | 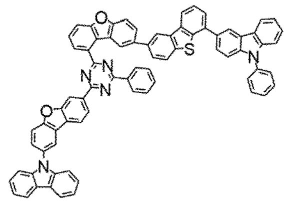 |
| 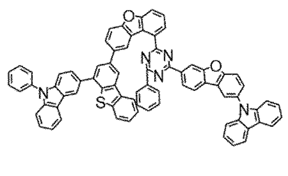 | 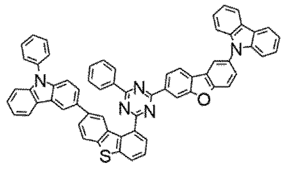 | 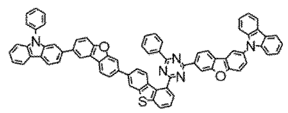 |
| 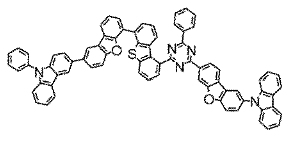 | 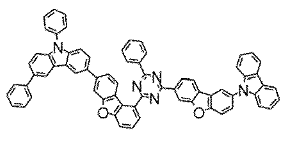 | 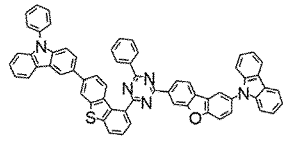 |

| | | |
|---|---|---|
| 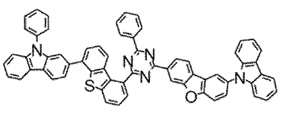 | 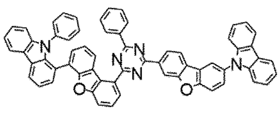 | 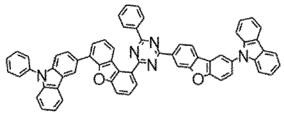 |
| 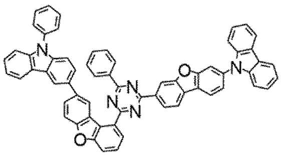 | 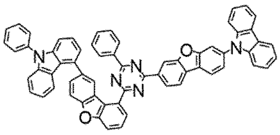 | 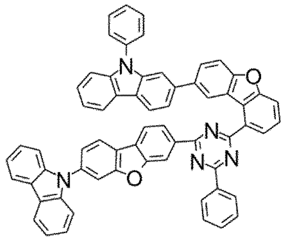 |
| 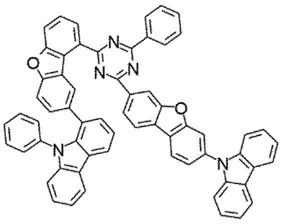 | 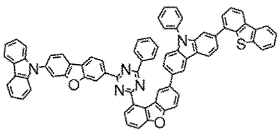 | 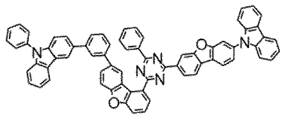 |
| 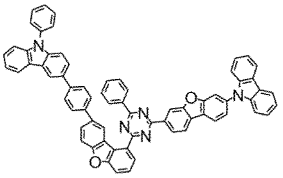 | 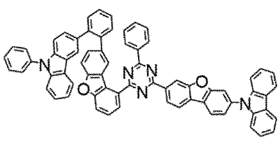 | 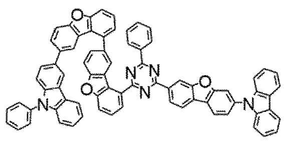 |
| 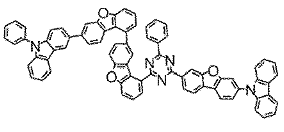 | 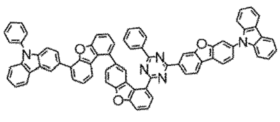 | 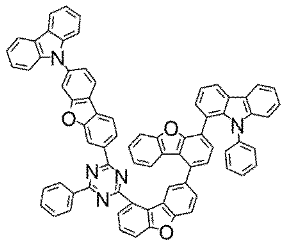 |

| | | |
|---|---|---|
| 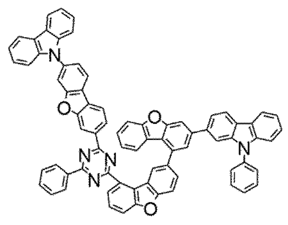 | 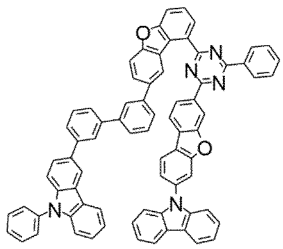 | 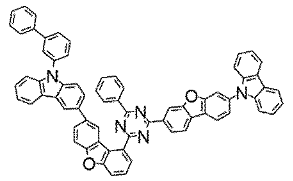 |
| 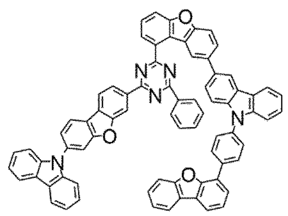 | 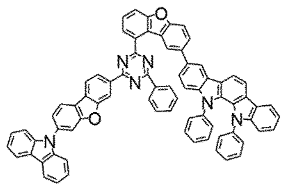 | 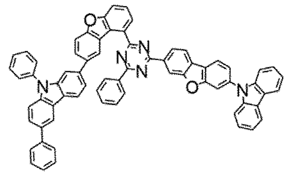 |
| 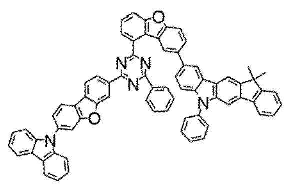 | 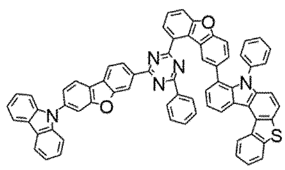 | 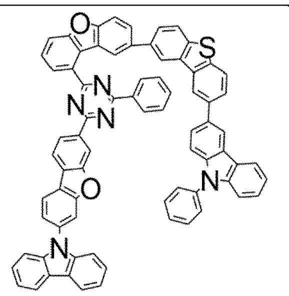 |
| 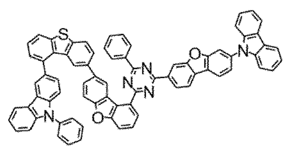 | 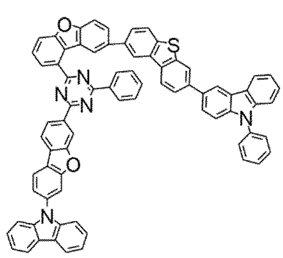 | 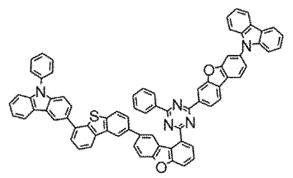 |
| 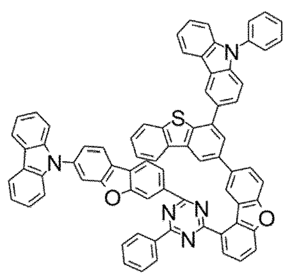 | 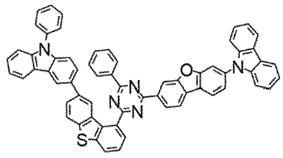 | 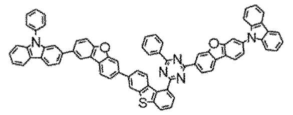 |

| | | |
|---|---|---|
| 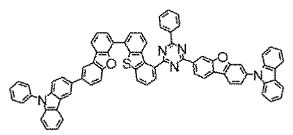 | 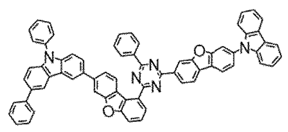 | 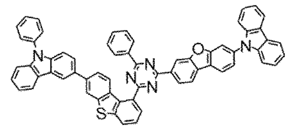 |
| 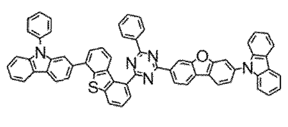 | 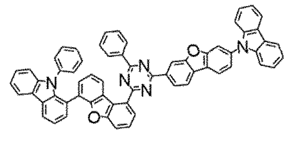 | 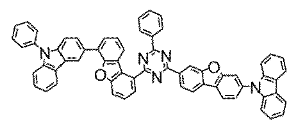 |
| 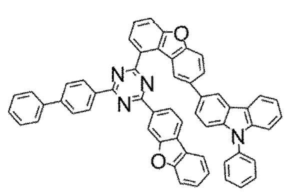 | 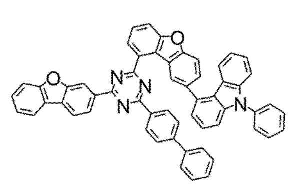 | 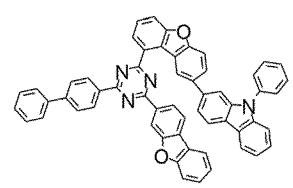 |
| 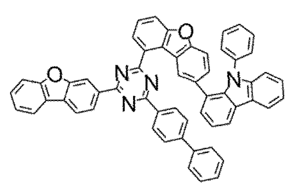 | 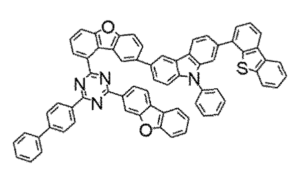 | 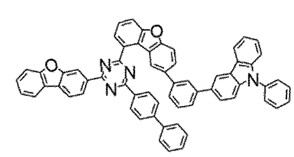 |
| 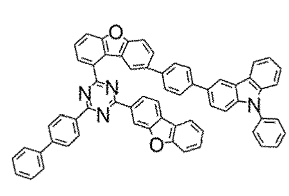 | 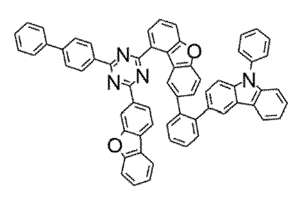 | 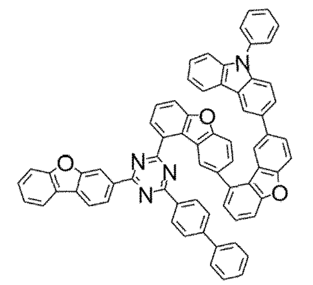 |

| | | |
|---|---|---|
| 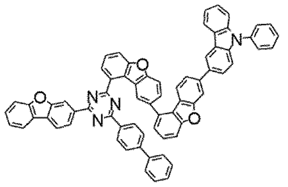 | 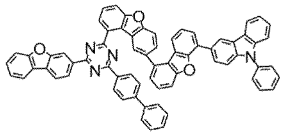 | 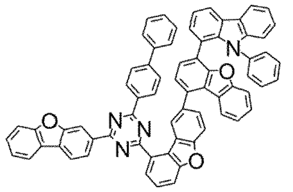 |
| 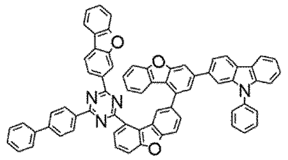 | 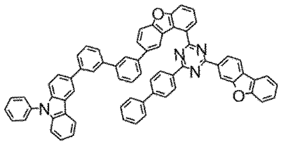 | 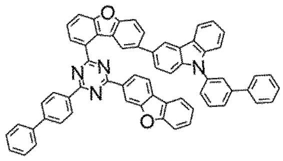 |
| 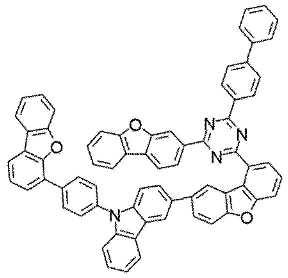 | 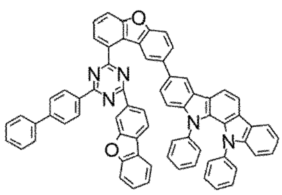 | 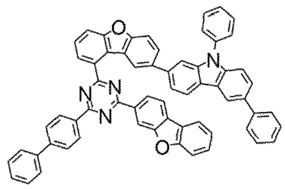 |
| 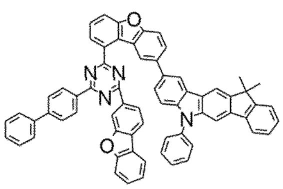 | 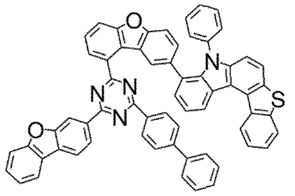 | 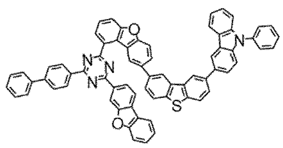 |
| 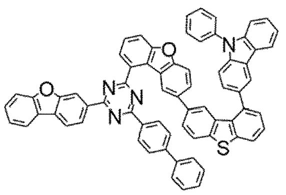 | 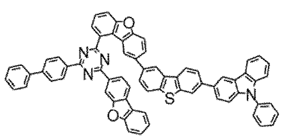 | 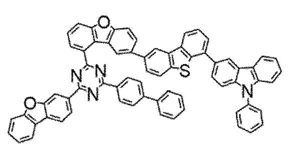 |

| | | |
|---|---|---|
| 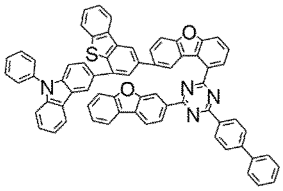 | 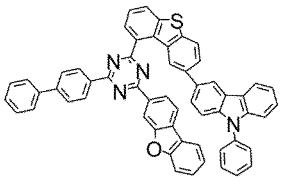 | 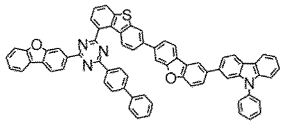 |
| 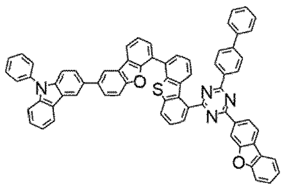 | 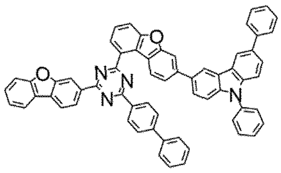 | 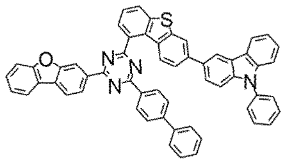 |
| 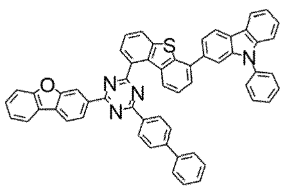 | 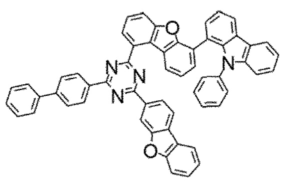 | 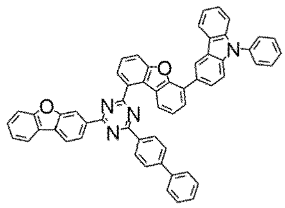 |
| 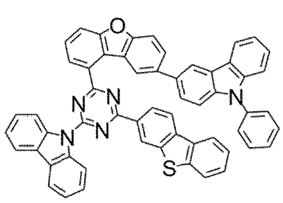 | 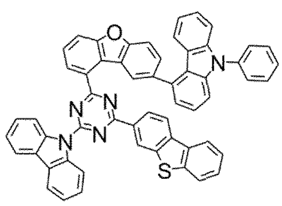 | 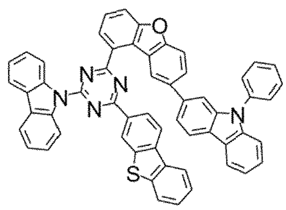 |
| 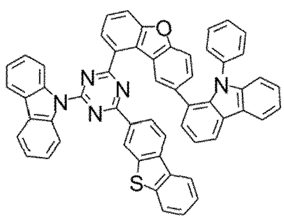 | 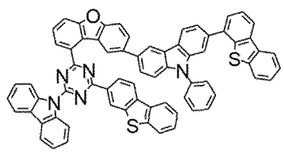 | 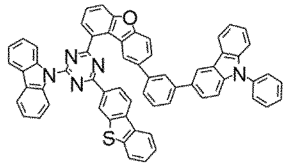 |

| | | |
|---|---|---|
| 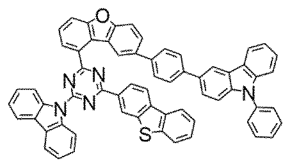 | 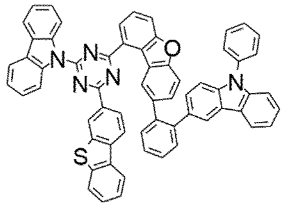 | 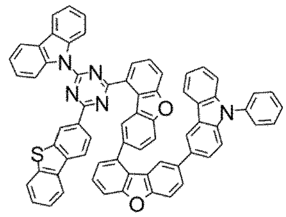 |
| 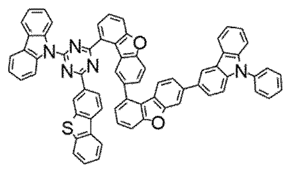 | 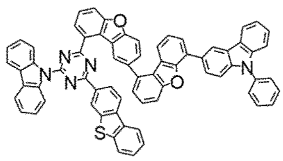 | 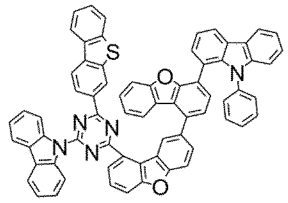 |
| 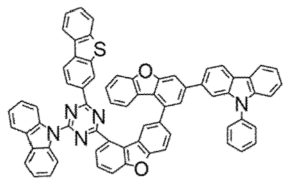 | 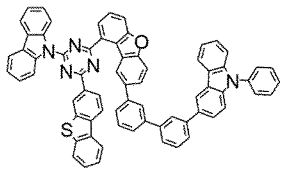 | 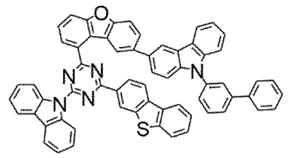 |
| 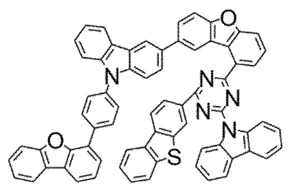 | 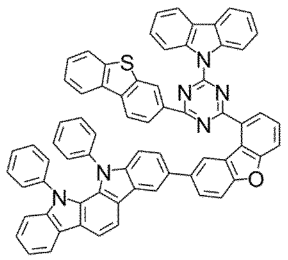 | 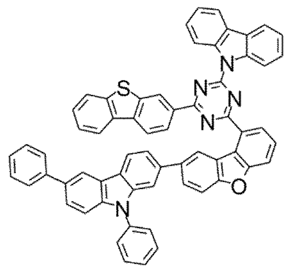 |
| 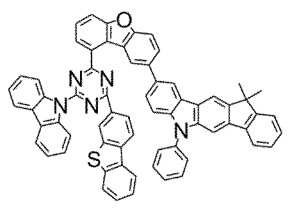 | 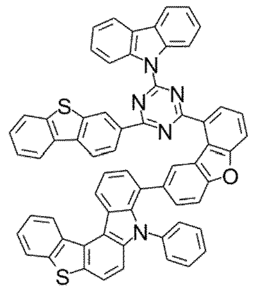 | 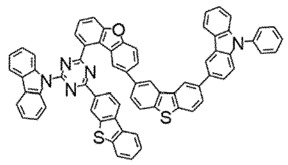 |

| | | |
|---|---|---|
| 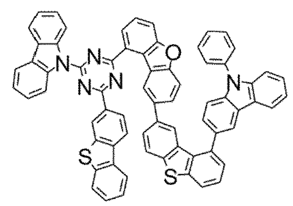 | 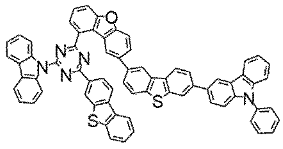 | 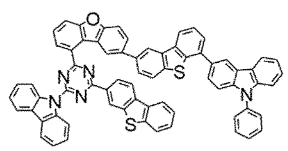 |
| 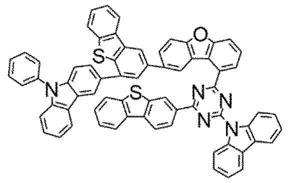 | 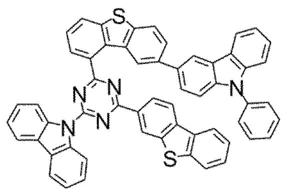 | 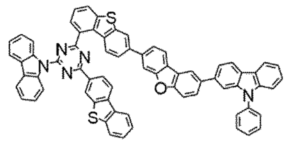 |
| 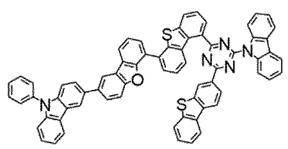 | 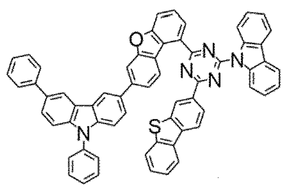 | 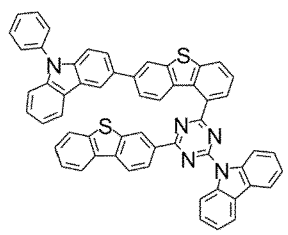 |
| 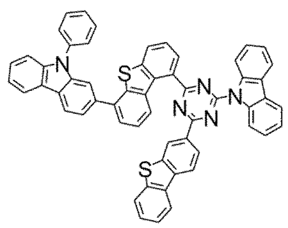 | 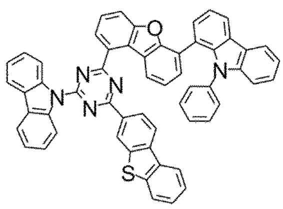 | 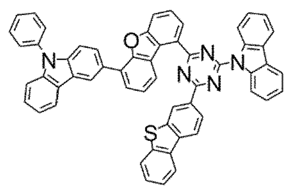 |
| 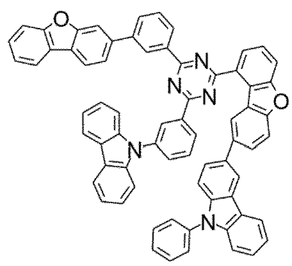 | 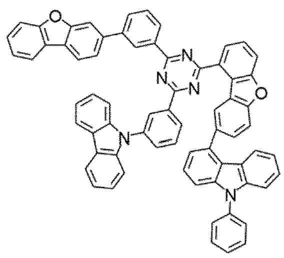 | 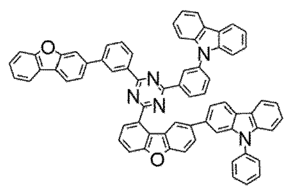 |

| | | |
|---|---|---|
| 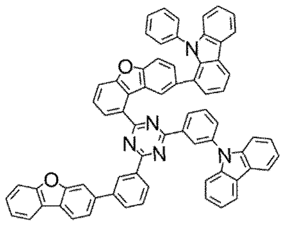 | 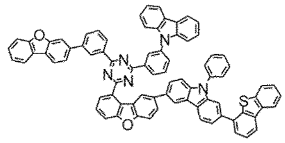 | 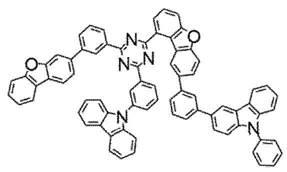 |
| 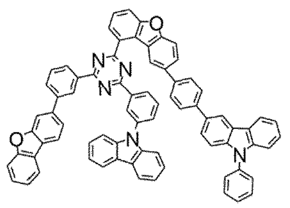 | 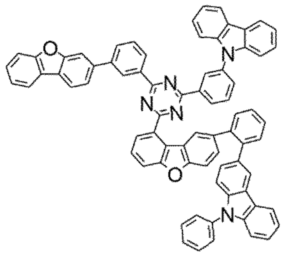 | 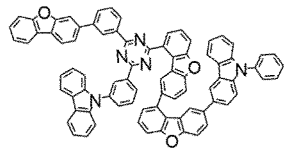 |
| 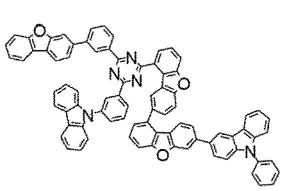 | 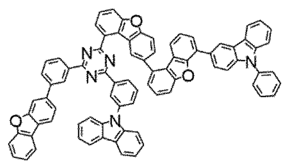 | 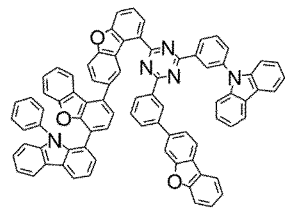 |
| 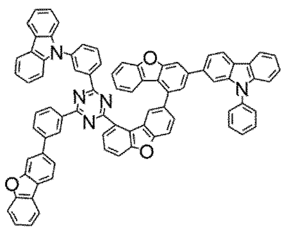 | 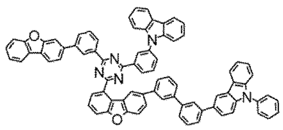 | 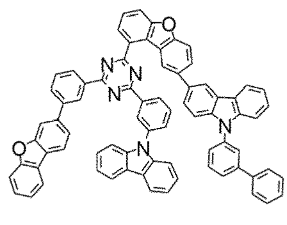 |
| 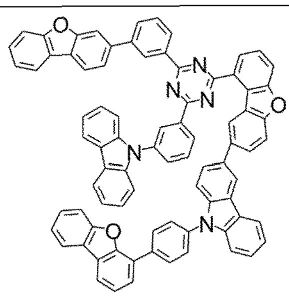 | 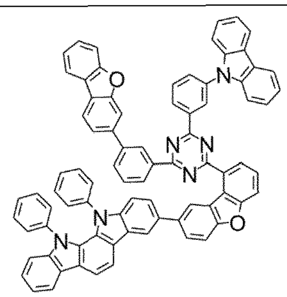 | 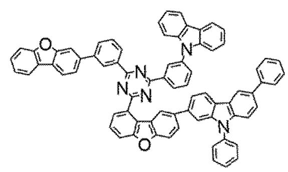 |

| | | |
|---|---|---|
| 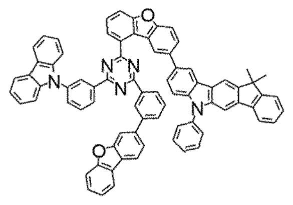 | 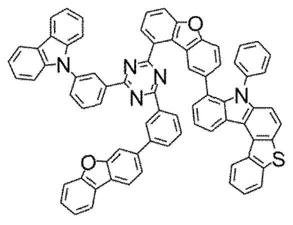 | 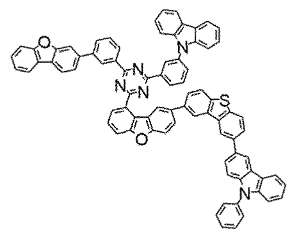 |
| 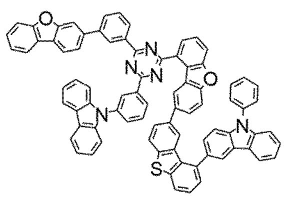 | 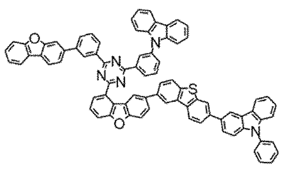 | 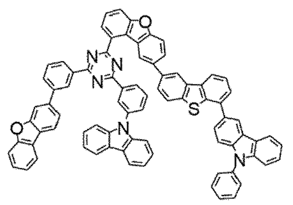 |
| 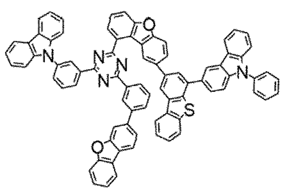 | 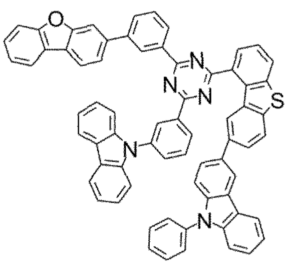 | 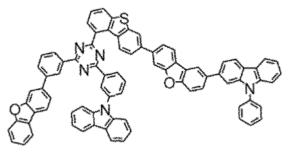 |
| 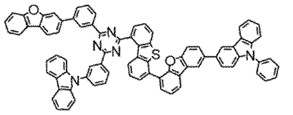 | 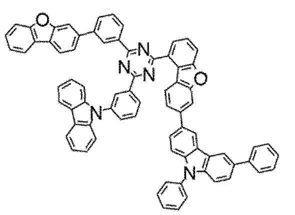 | 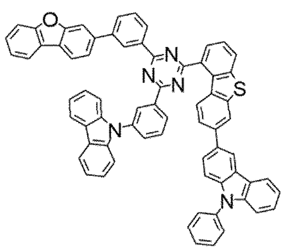 |
| 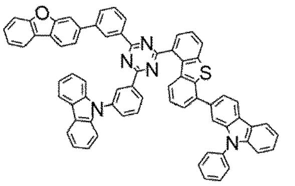 | 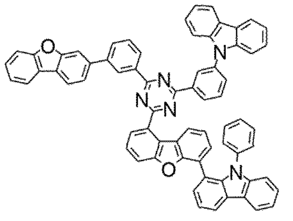 | 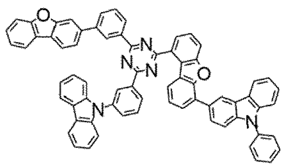 |

| | | |
|---|---|---|
| 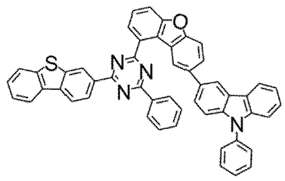 | 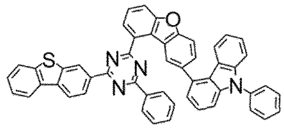 | 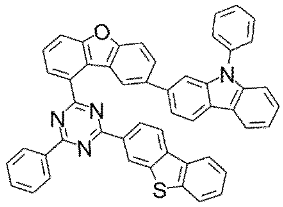 |
| 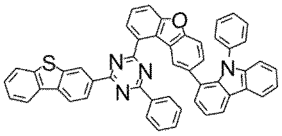 | 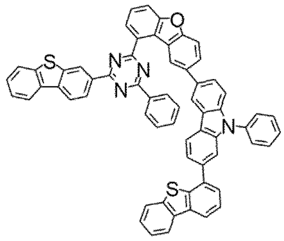 | 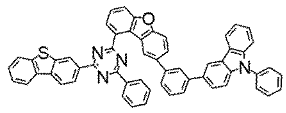 |
| 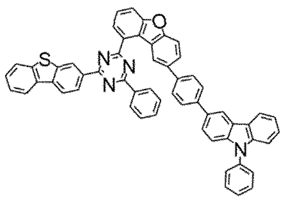 | 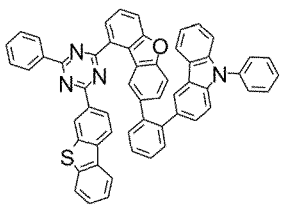 | 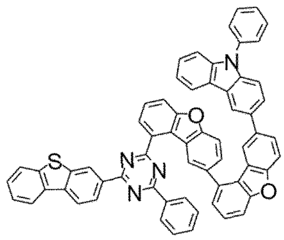 |
| 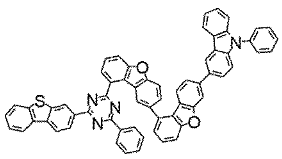 | 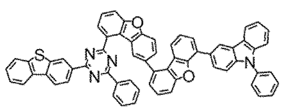 | 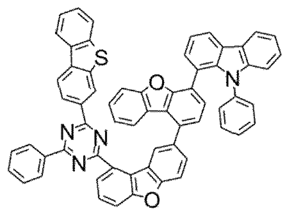 |
| 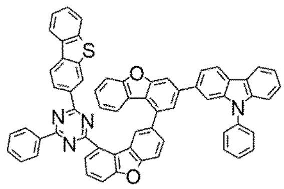 | 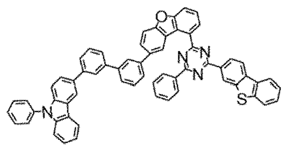 | 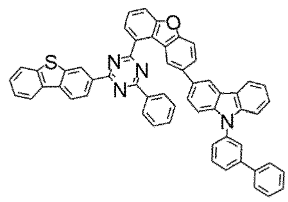 |

| | | |
|---|---|---|
| 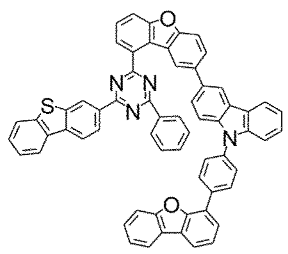 | 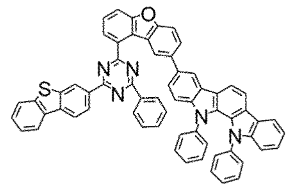 | 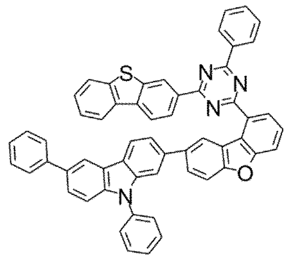 |
| 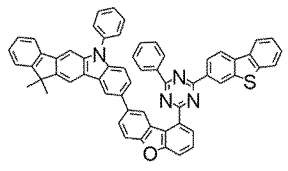 | 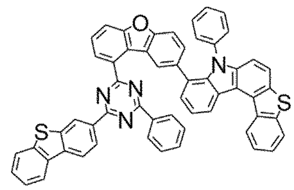 | 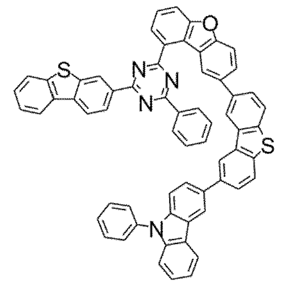 |
| 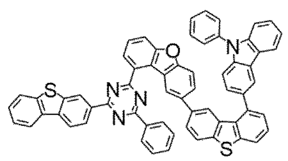 | 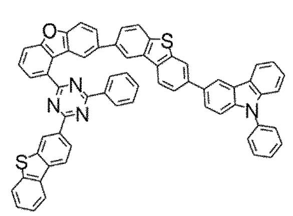 | 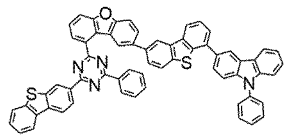 |
| 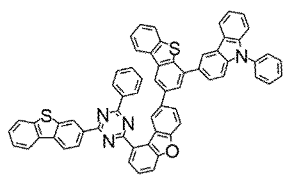 | 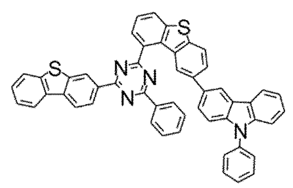 | 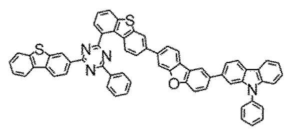 |
| 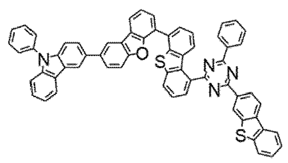 | 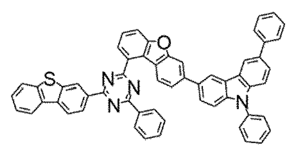 | 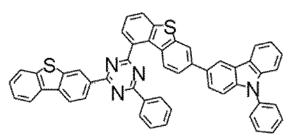 |

| | | |
|---|---|---|
| 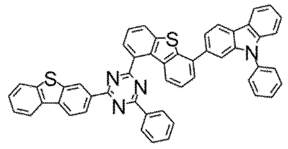 | 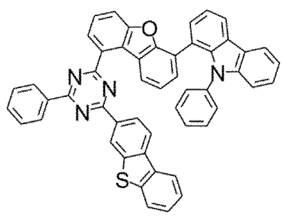 | 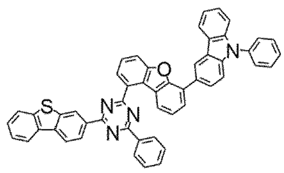 |
| 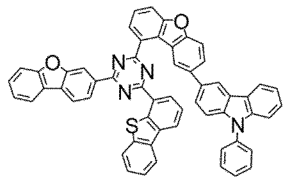 | 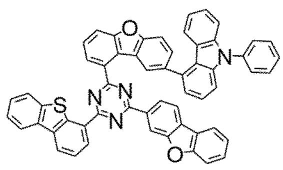 | 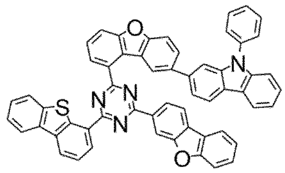 |
| 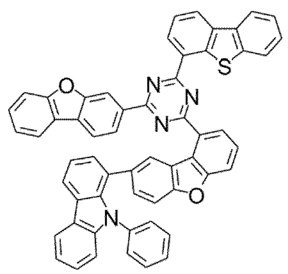 | 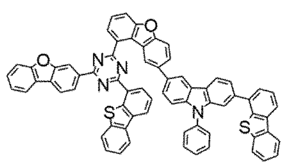 | 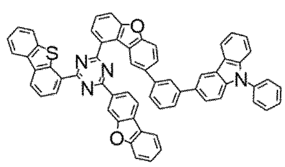 |
| 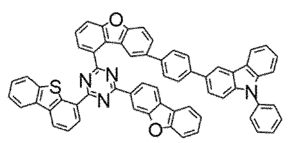 | 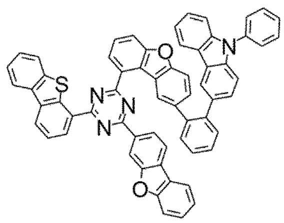 | 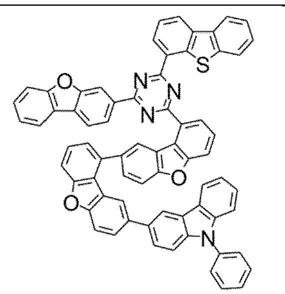 |
| 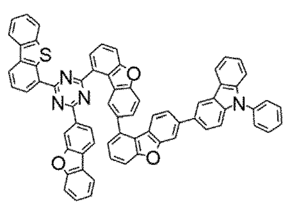 | 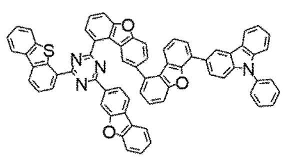 | 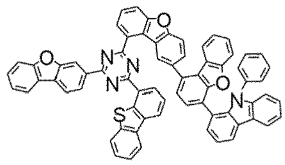 |

| | | |
|---|---|---|
| 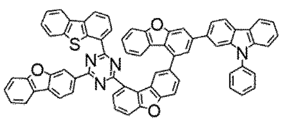 | 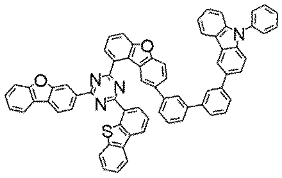 | 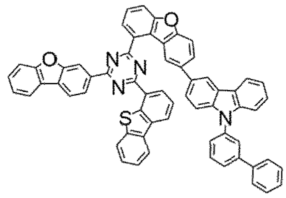 |
| 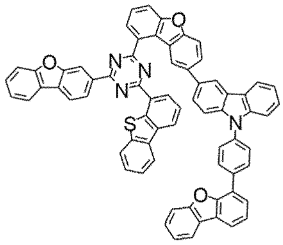 | 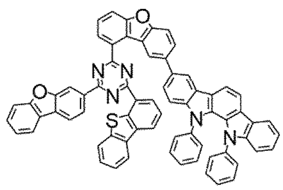 | 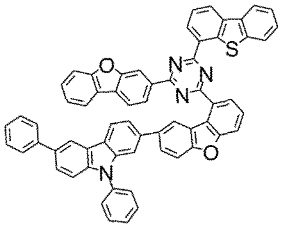 |
| 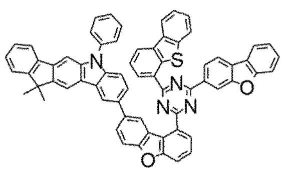 | 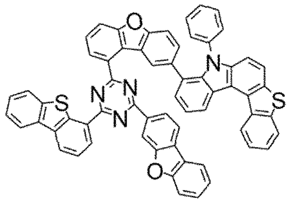 | 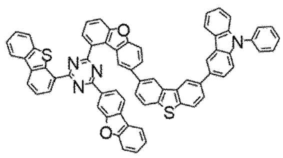 |
| 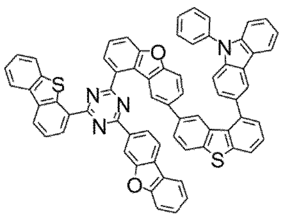 | 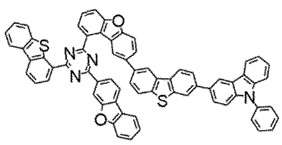 | 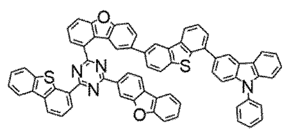 |
| 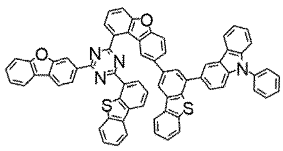 | 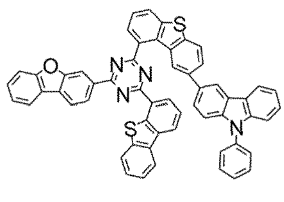 | 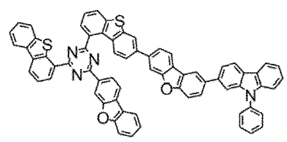 |

| | | |
|---|---|---|
| 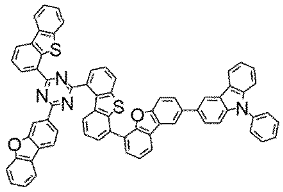 | 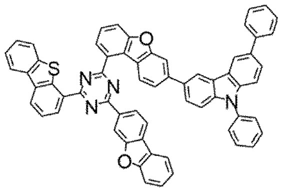 | 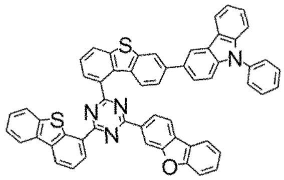 |
| 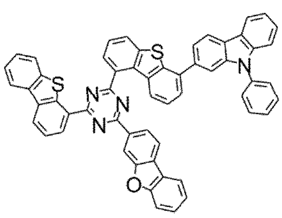 | 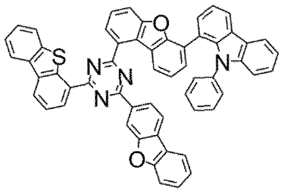 | 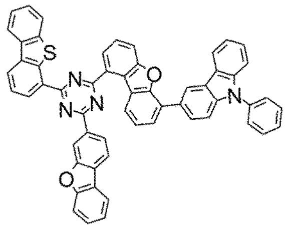 |
| 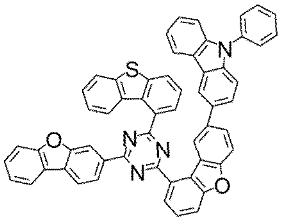 | 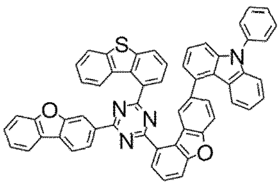 | 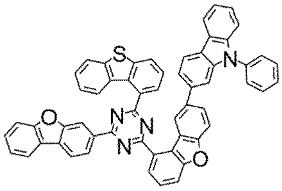 |
| 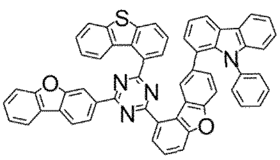 | 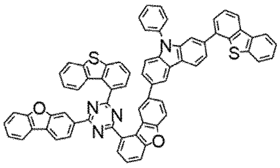 | 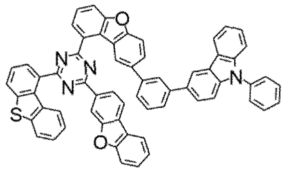 |
| 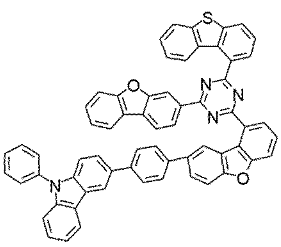 | 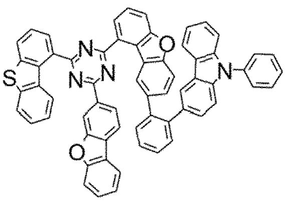 | 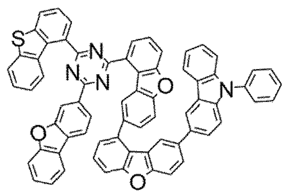 |

| | | |
|---|---|---|
| 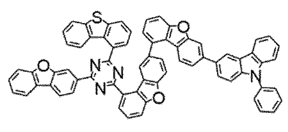 | 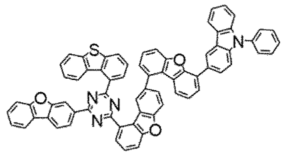 | 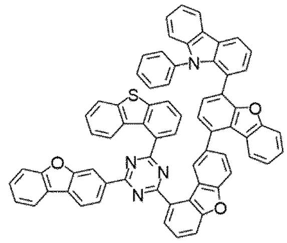 |
| 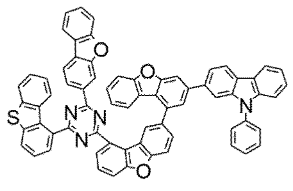 | 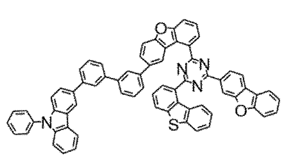 | 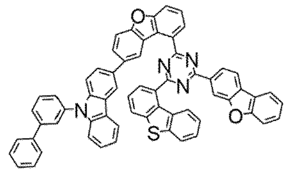 |
| 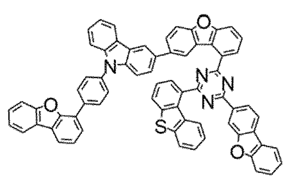 | 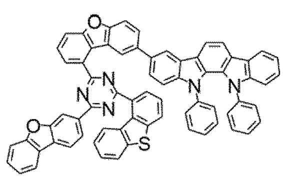 | 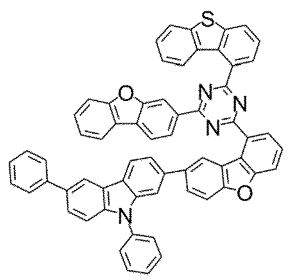 |
| 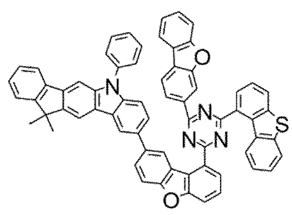 | 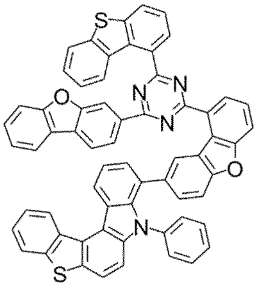 | 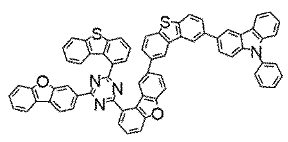 |
| 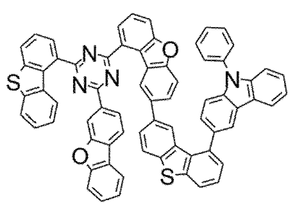 | 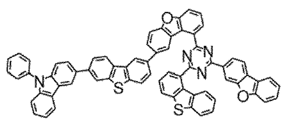 | 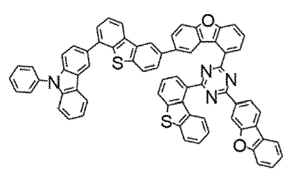 |

| | | |
|---|---|---|
| 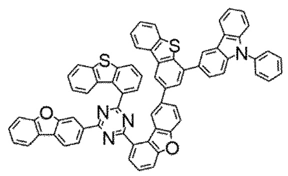 | 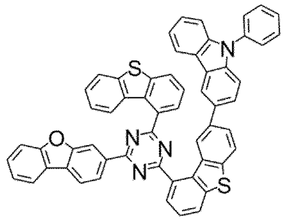 | 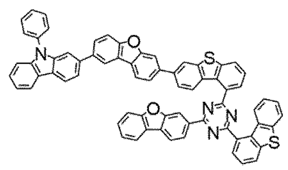 |
| 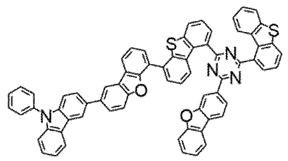 | 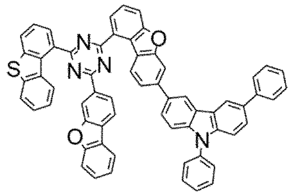 | 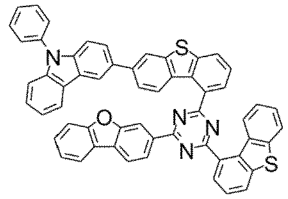 |
| 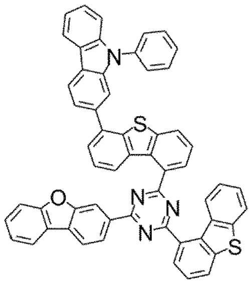 | 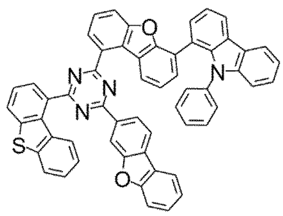 | 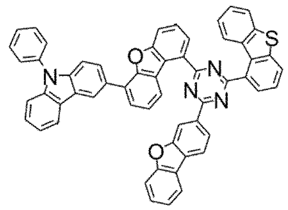 |
| 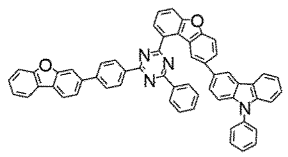 | 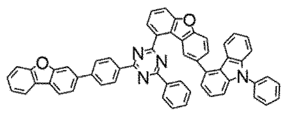 | 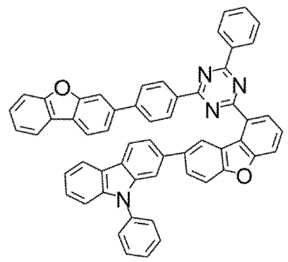 |
| 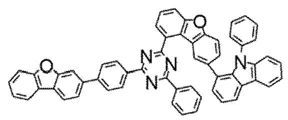 | 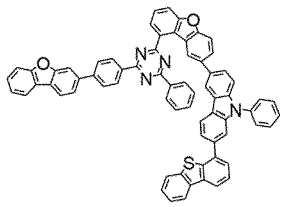 | 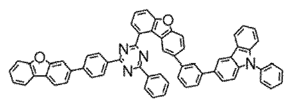 |

| | | |
|---|---|---|
| 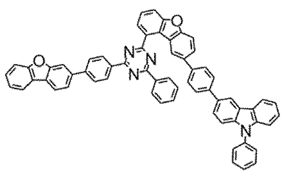 | 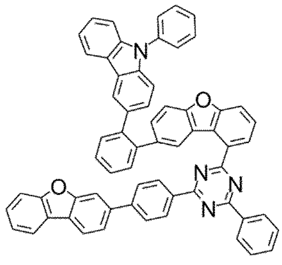 | 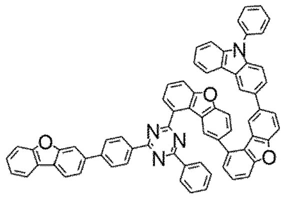 |
| 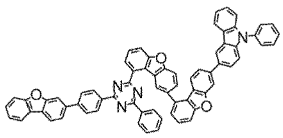 | 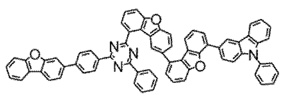 | 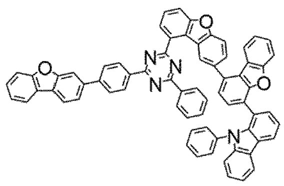 |
| 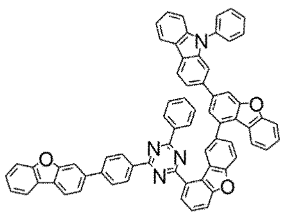 | 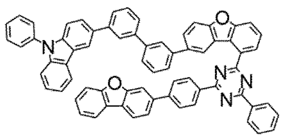 | 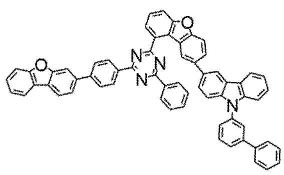 |
| 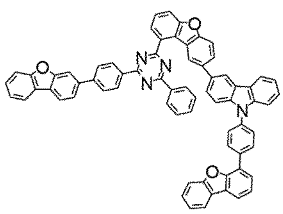 | 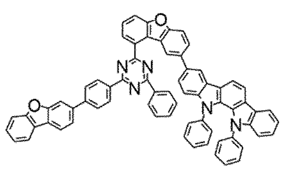 | 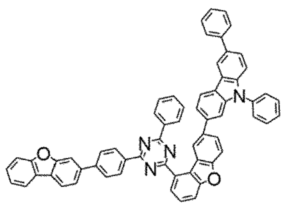 |
| 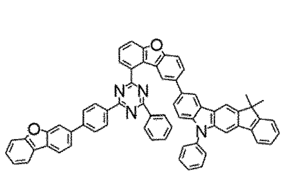 | 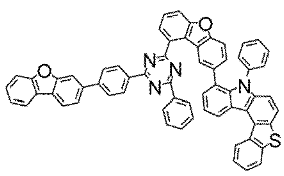 | 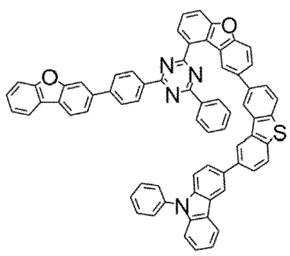 |

| | | |
|---|---|---|
| 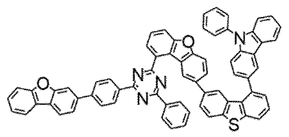 | 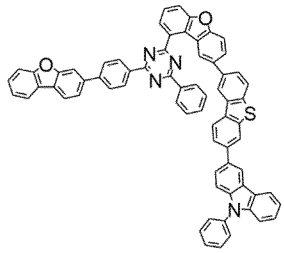 | 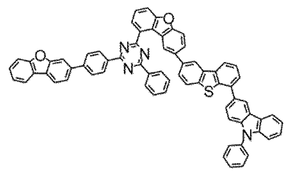 |
| 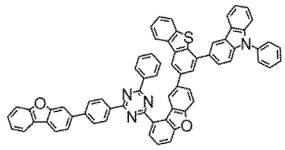 | 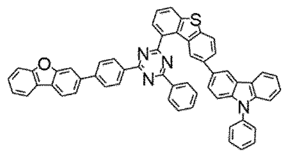 | 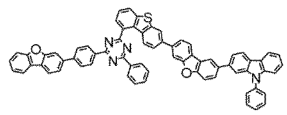 |
| 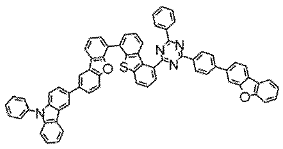 | 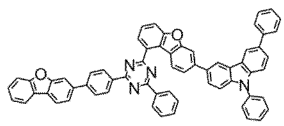 | 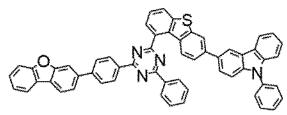 |
| 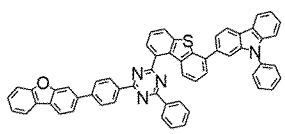 | 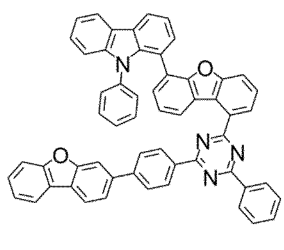 | 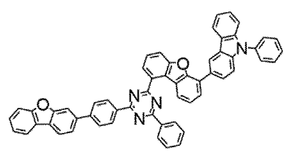 |
| 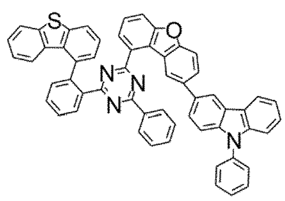 | 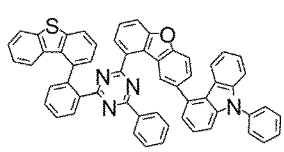 | 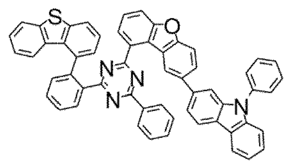 |

| | | |
|---|---|---|
| 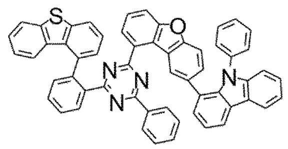 | 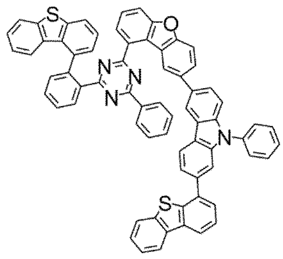 | 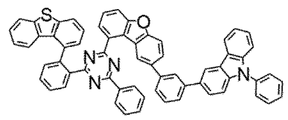 |
| 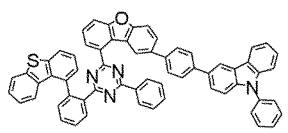 | 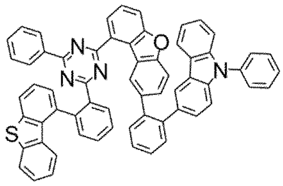 | 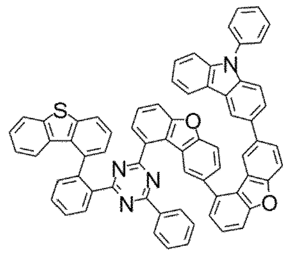 |
| 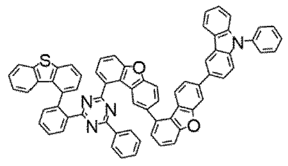 | 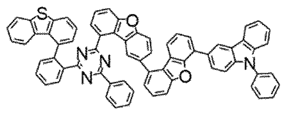 | 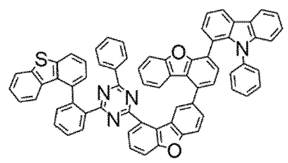 |
| 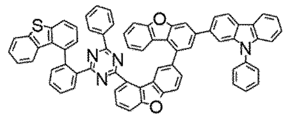 | 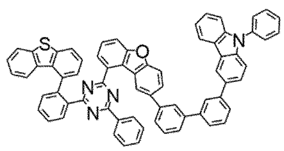 | 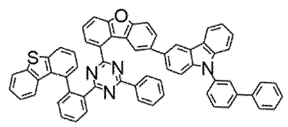 |
| 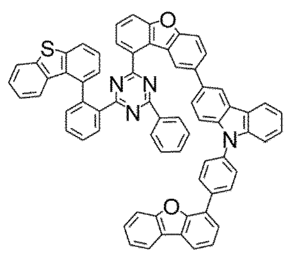 | 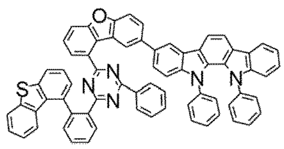 | 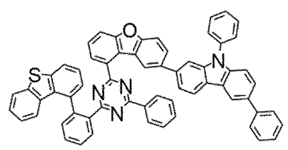 |

| | | |
|---|---|---|
| 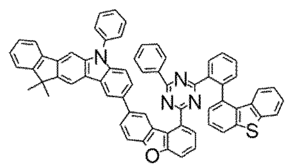 | 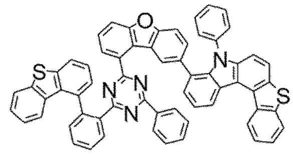 | 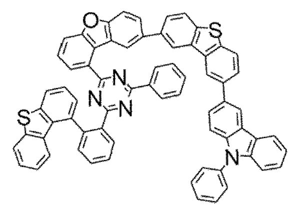 |
| 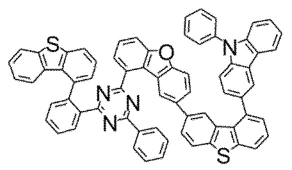 | 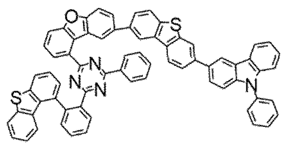 | 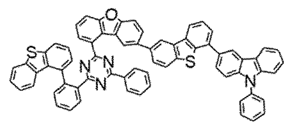 |
| 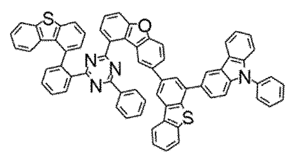 | 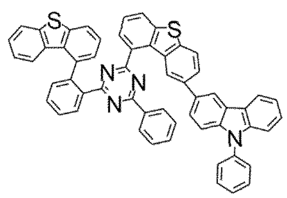 | 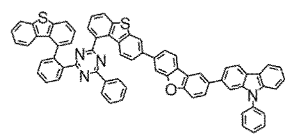 |
| 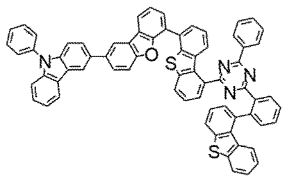 | 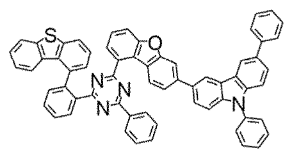 | 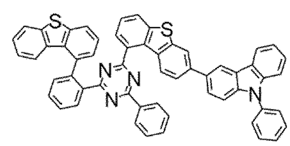 |
| 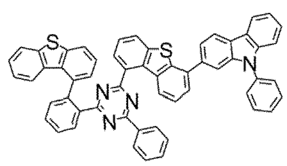 | 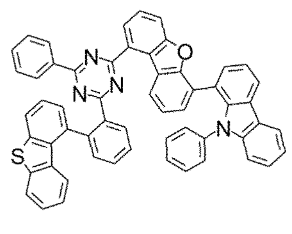 | 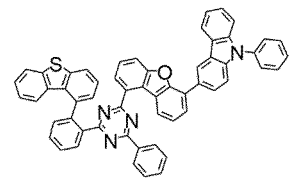 |

| | | |
|---|---|---|
| 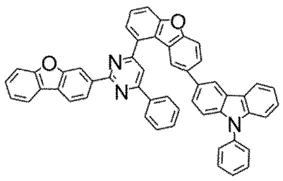 | 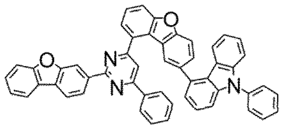 | 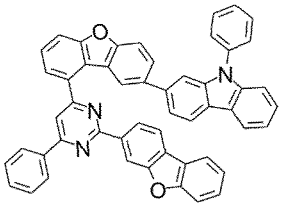 |
| 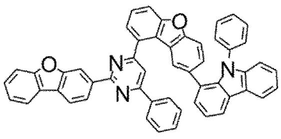 | 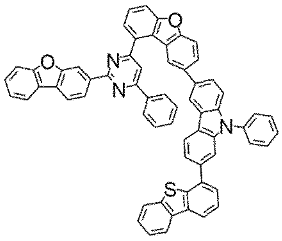 | 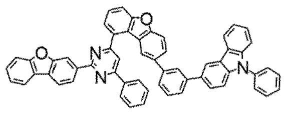 |
| 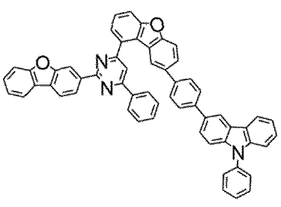 | 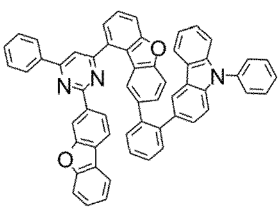 | 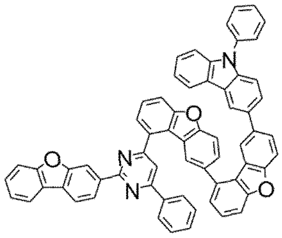 |
| 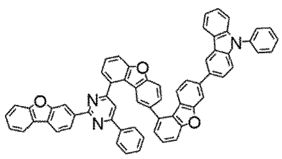 | 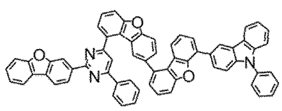 | 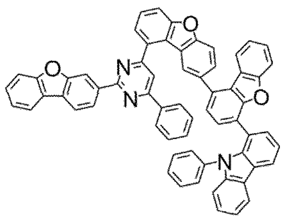 |
| 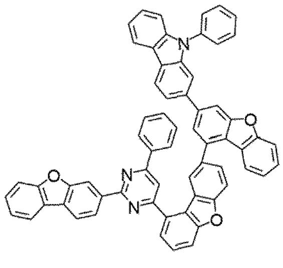 | 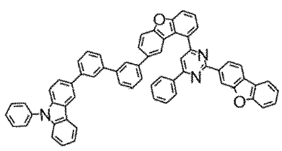 | 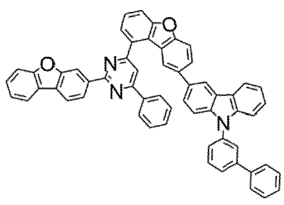 |

| | | |
|---|---|---|
| 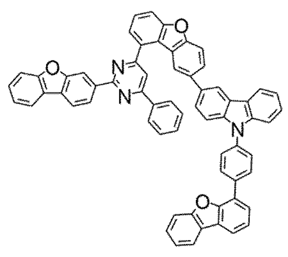 | 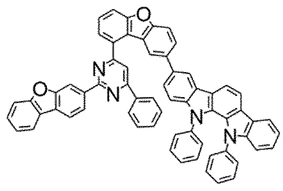 | 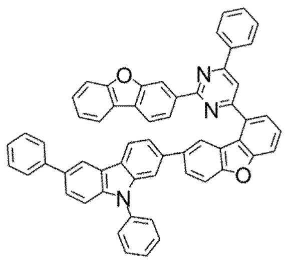 |
| 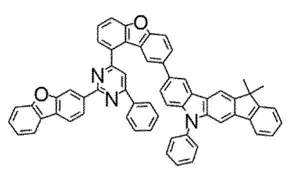 | 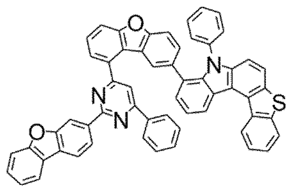 | 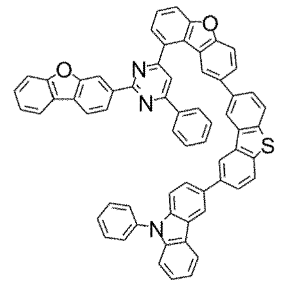 |
| 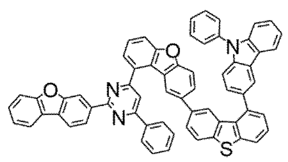 | 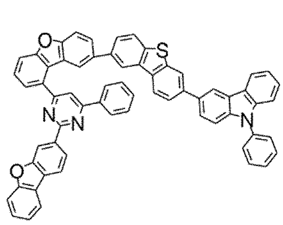 | 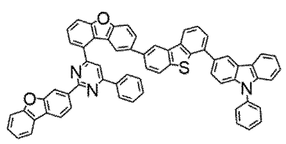 |
| 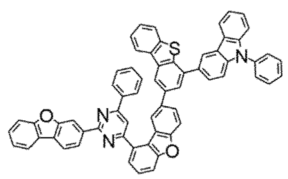 | 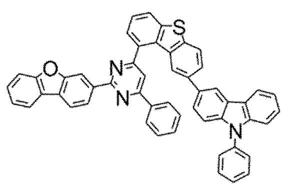 | 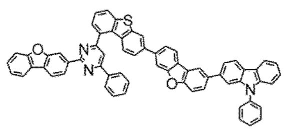 |
| 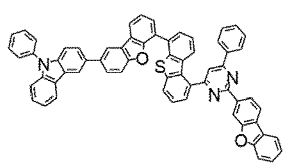 | 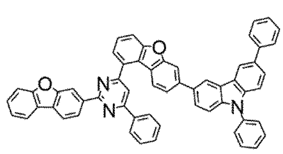 | 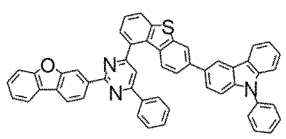 |

| | | |
|---|---|---|
| 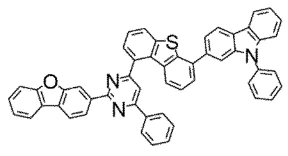 | 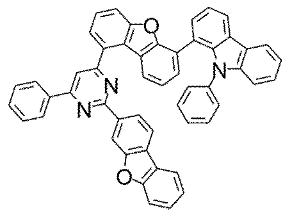 | 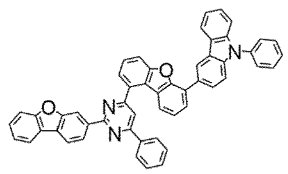 |
| 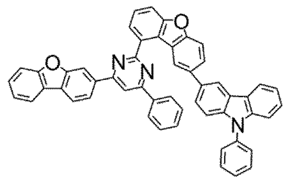 | 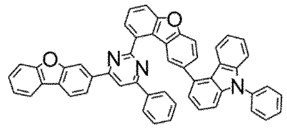 | 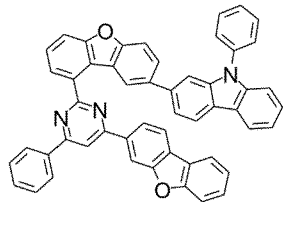 |
| 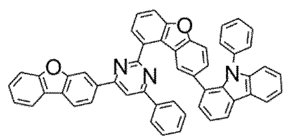 | 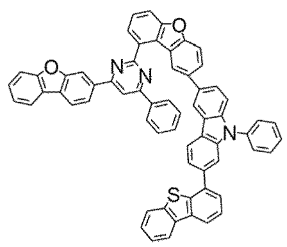 | 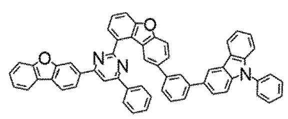 |
| 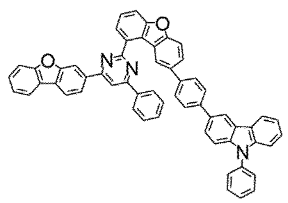 | 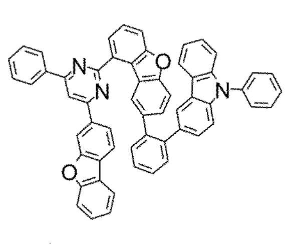 | 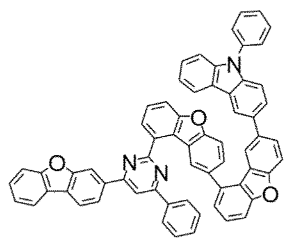 |
| 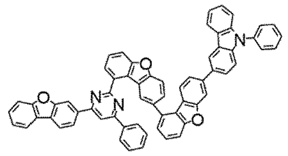 | 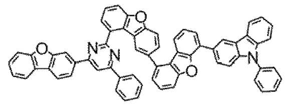 | 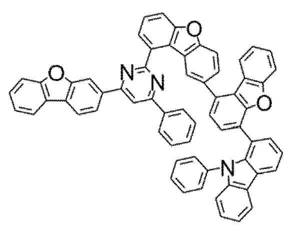 |

| | | |
|---|---|---|
| 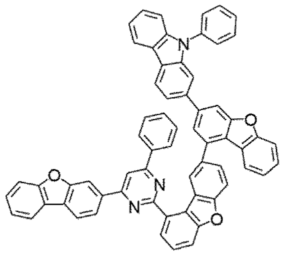 | 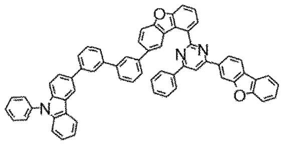 | 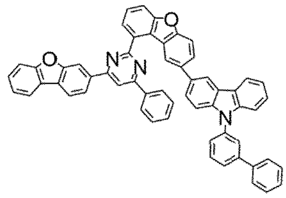 |
| 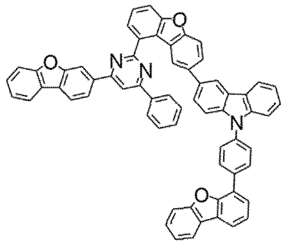 | 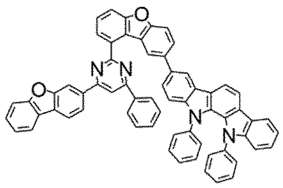 | 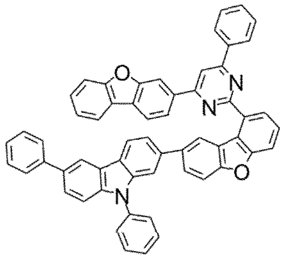 |
| 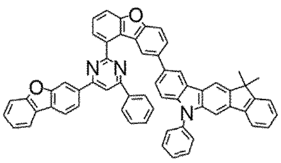 | 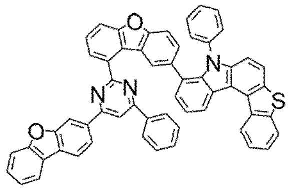 | 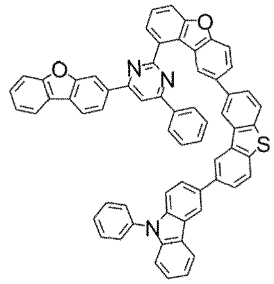 |
| 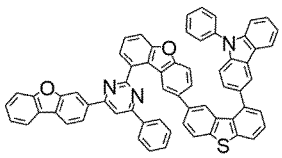 | 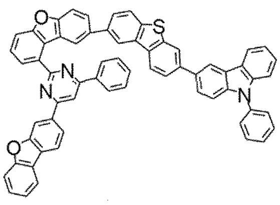 | 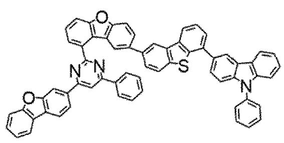 |
| 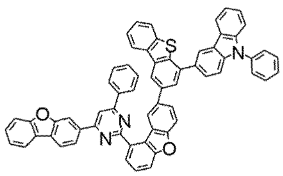 | 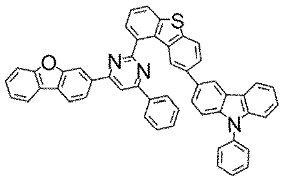 | 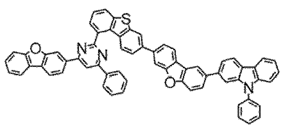 |

| | | |
|---|---|---|
| 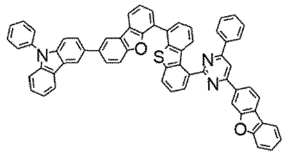 | 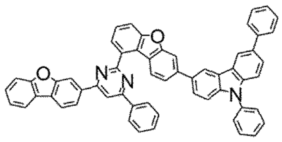 | 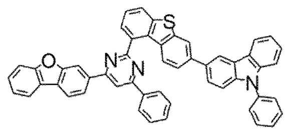 |
| 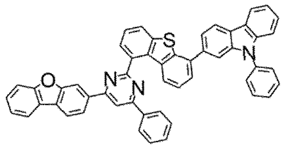 | 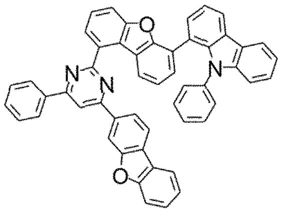 | 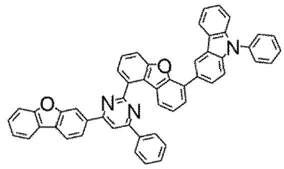 |
| 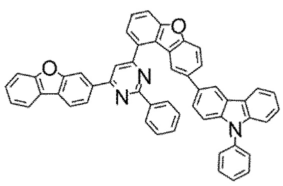 | 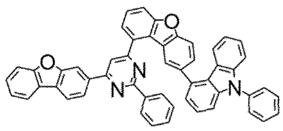 | 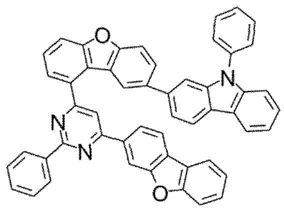 |
| 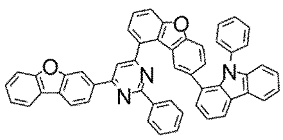 | 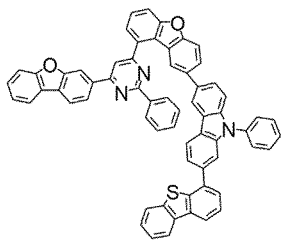 | 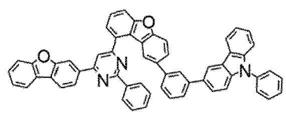 |
| 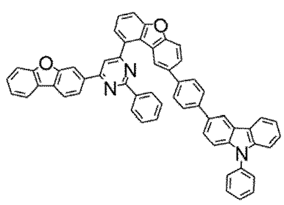 | 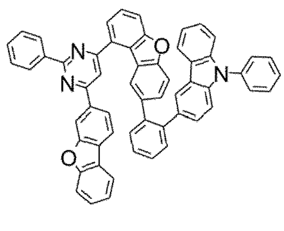 | 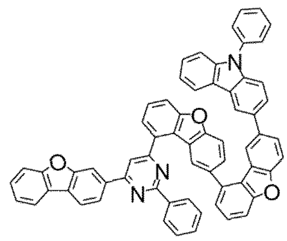 |

| | | |
|---|---|---|
| 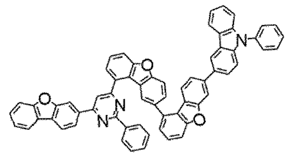 | 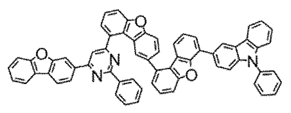 | 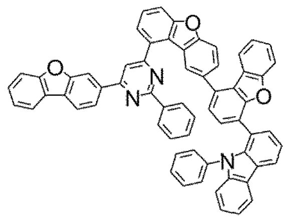 |
| 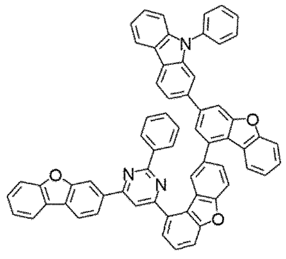 | 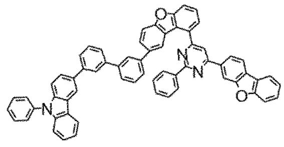 | 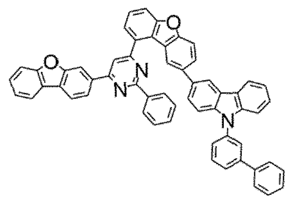 |
| 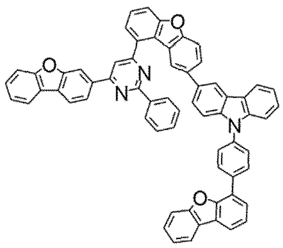 | 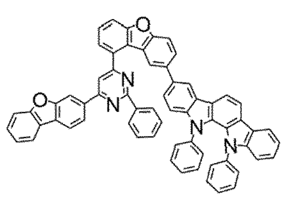 | 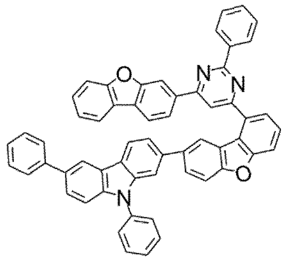 |
| 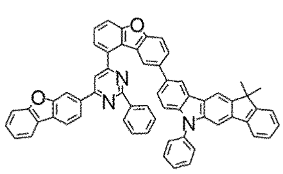 | 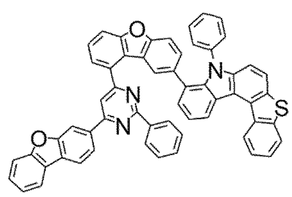 | 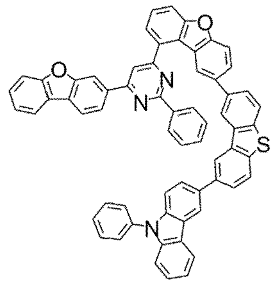 |
| 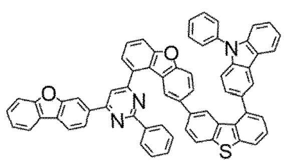 | 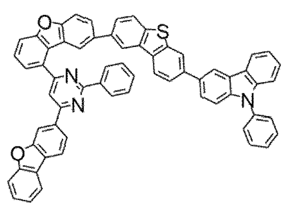 | 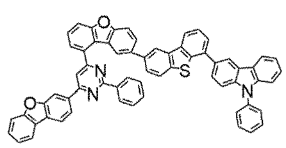 |

| | | |
|---|---|---|
| 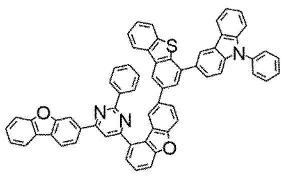 | 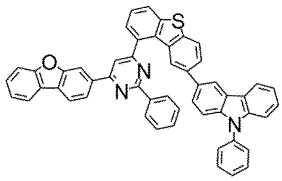 | 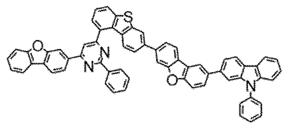 |
| 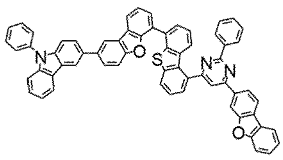 | 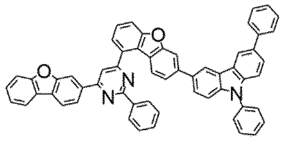 | 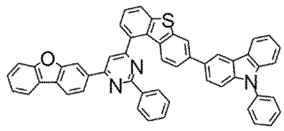 |
| 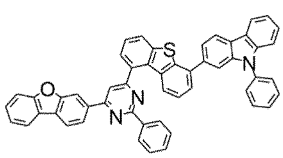 | 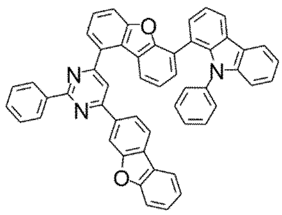 | 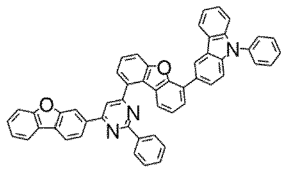 |
| 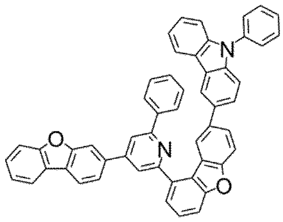 | 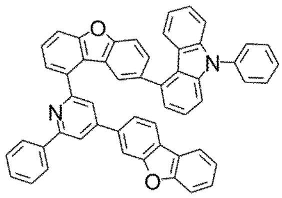 | 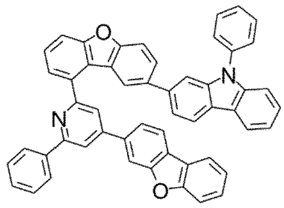 |
| 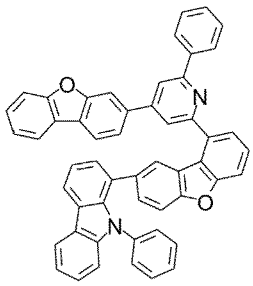 | 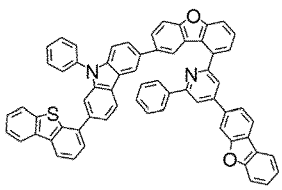 | 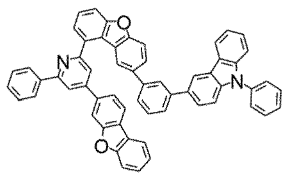 |

| | | |
|---|---|---|
| 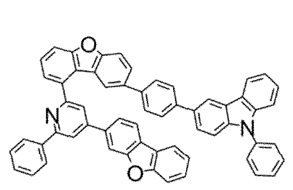 | 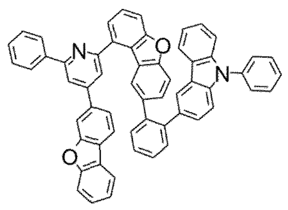 | 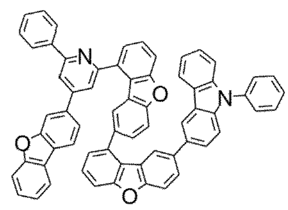 |
| 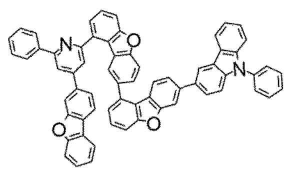 | 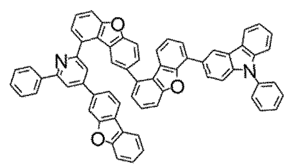 | 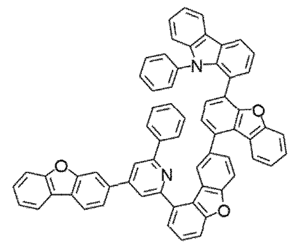 |
| 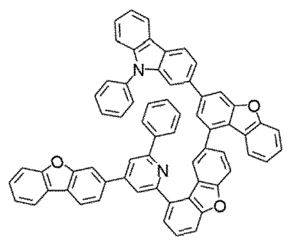 | 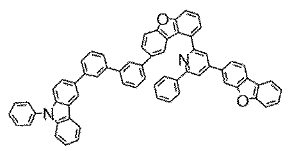 | 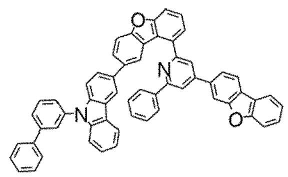 |
| 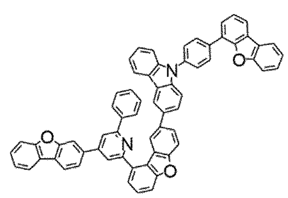 | 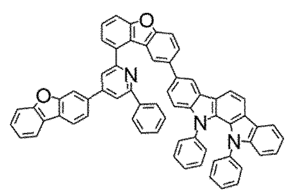 | 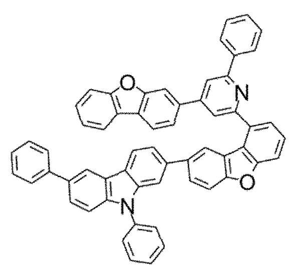 |
| 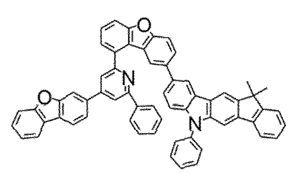 | 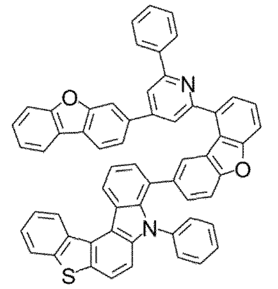 | 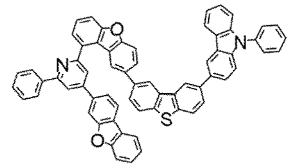 |

| | | |
|---|---|---|
| 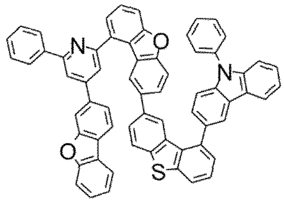 | 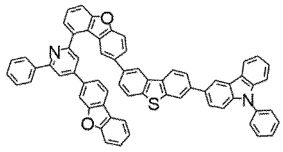 | 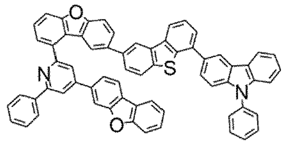 |
| 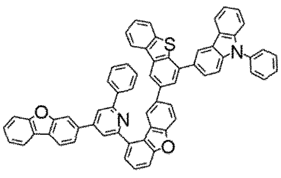 | 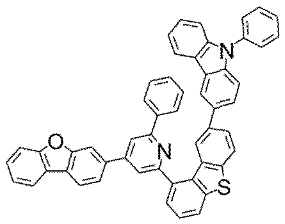 | 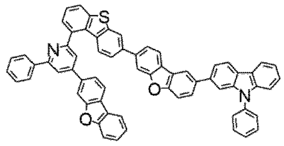 |
| 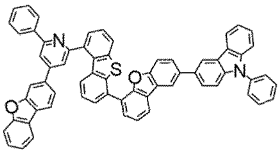 | 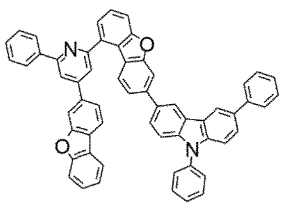 | 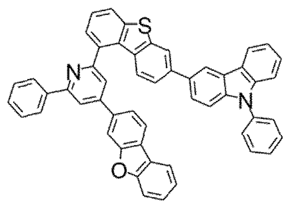 |
| 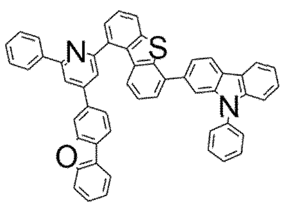 | 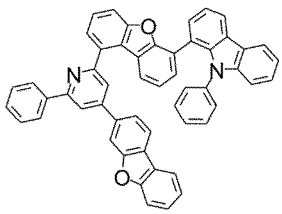 | 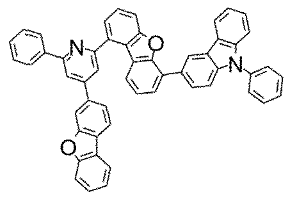 |
| 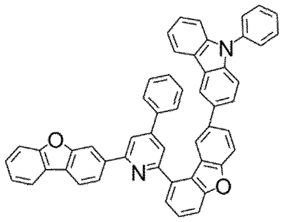 | 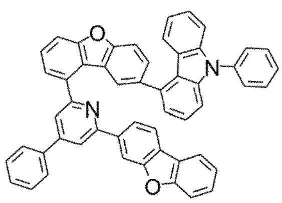 | 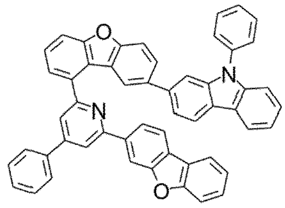 |

| | | |
|---|---|---|
| 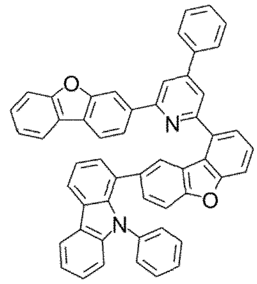 | 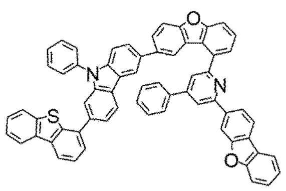 | 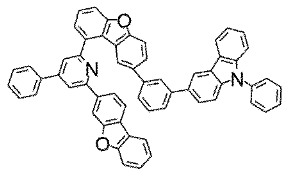 |
| 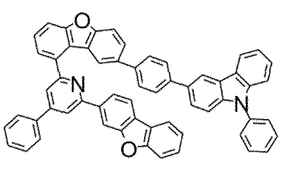 | 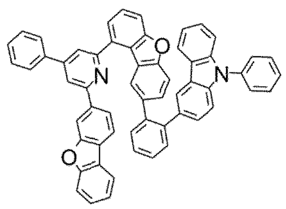 | 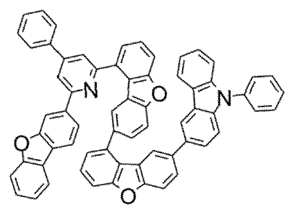 |
| 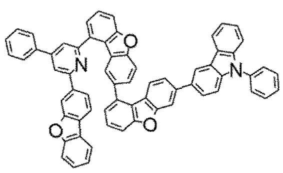 | 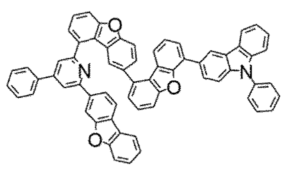 | 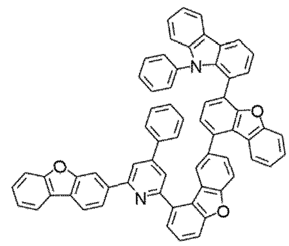 |
| 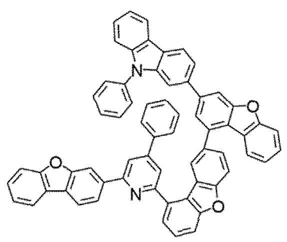 | 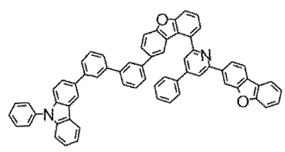 | 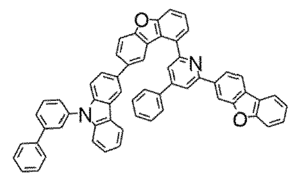 |
| 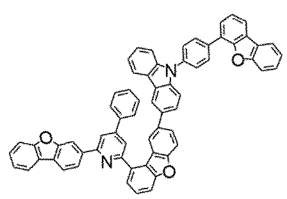 | 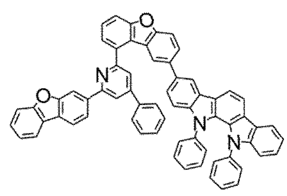 | 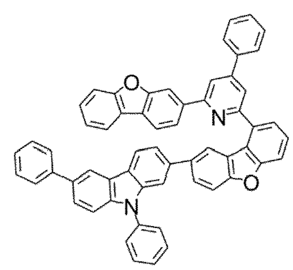 |

| | | |
|---|---|---|
| 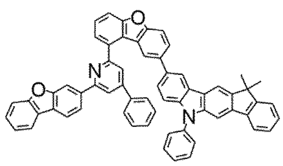 | 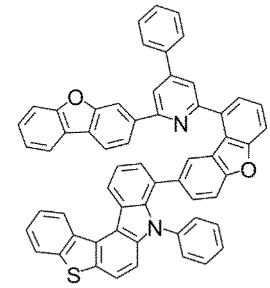 | 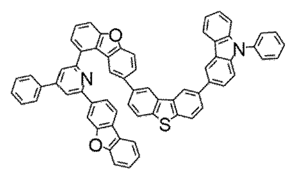 |
| 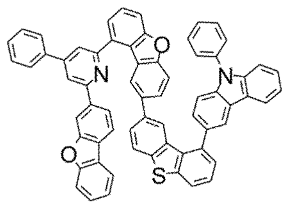 | 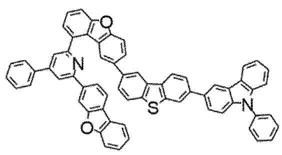 | 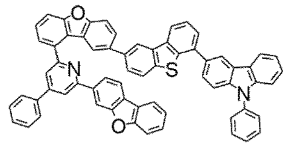 |
| 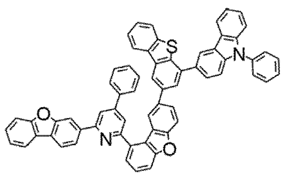 | 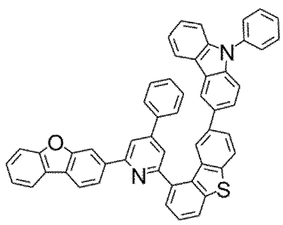 | 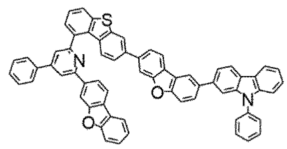 |
| 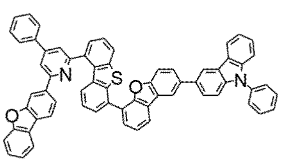 | 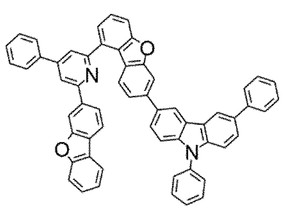 | 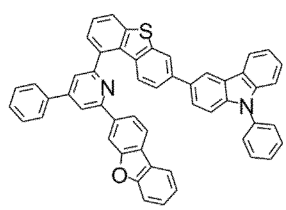 |
| 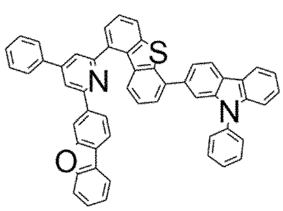 | 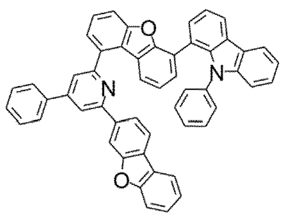 | 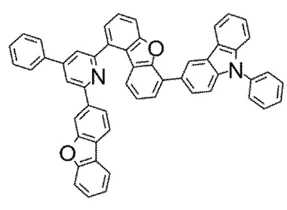 |

| | | |
|---|---|---|
| 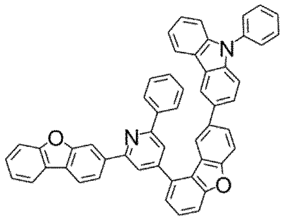 | 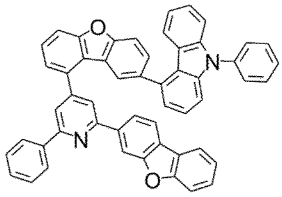 | 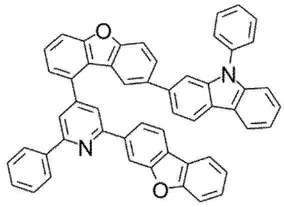 |
| 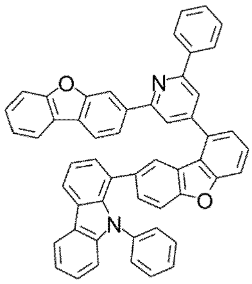 | 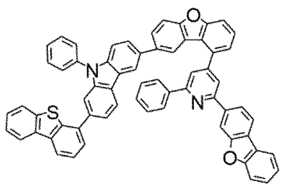 | 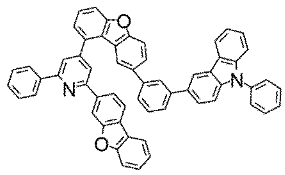 |
| 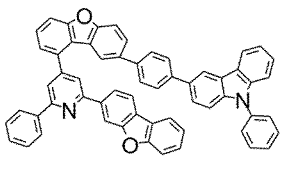 | 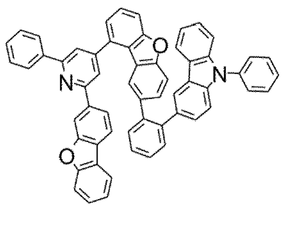 | 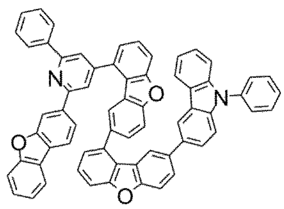 |
| 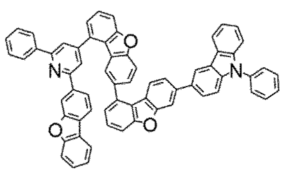 | 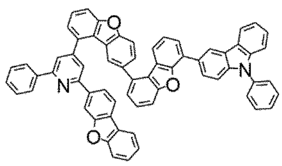 | 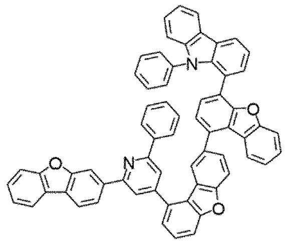 |
| 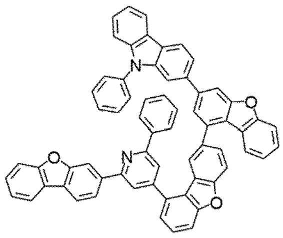 | 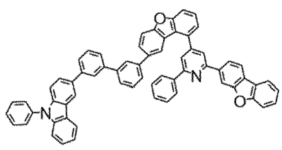 | 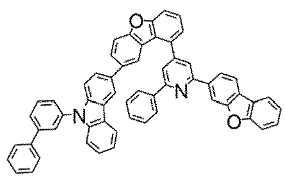 |

| | | |
|---|---|---|
| 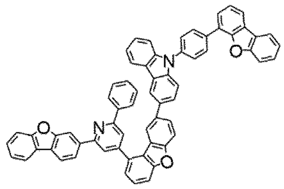 | 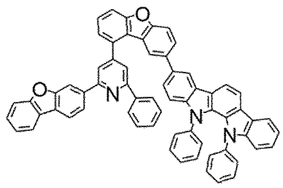 | 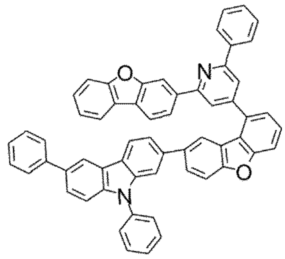 |
| 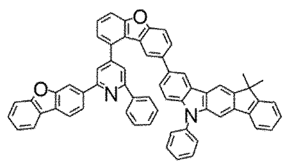 | 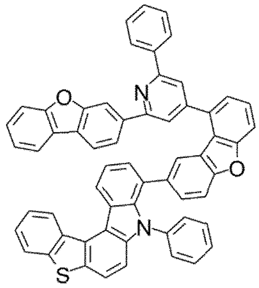 | 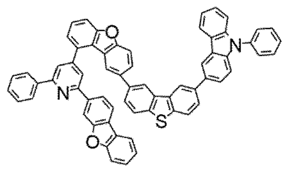 |
| 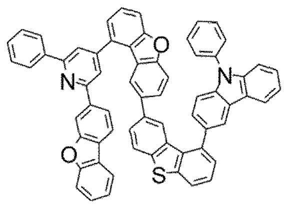 | 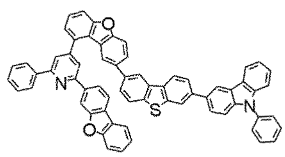 | 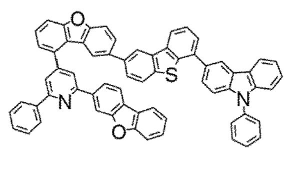 |
| 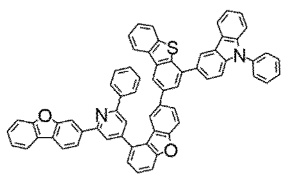 | 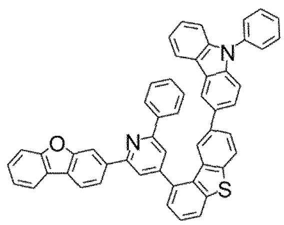 | 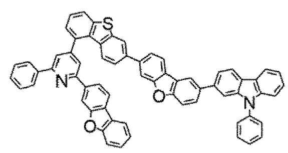 |
| 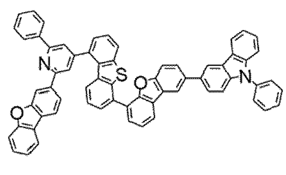 | 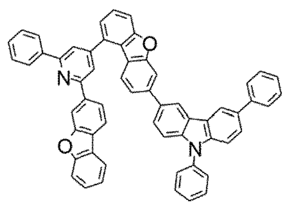 | 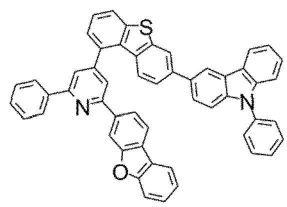 |

| | | |
|---|---|---|
| 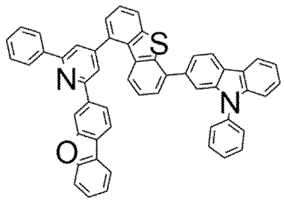 | 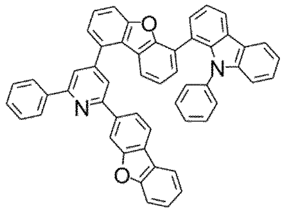 | 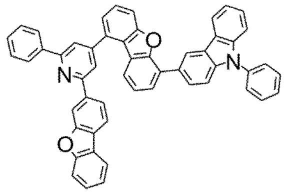 |

Besonders geeignete Verbindungen der Formeln (1), (1a), (1b), (1c), (1d), (1e) und (1f), die bevorzugt in Kombination mit mindestens einer Verbindung der Formel (2) in der erfindungsgemäßen elektrolumineszierenden Vorrichtung verwendet werden, sind die Verbindungen **E1** bis **E48**.

Tabelle 2:

| | | |
|---|---|---|
| 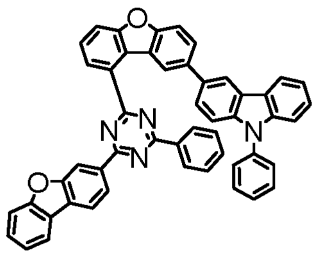 | 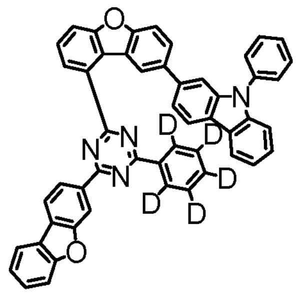 | 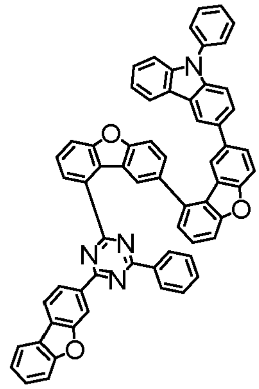 |
| **E1** | **E2** | **E3** |
| 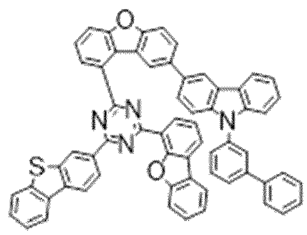 | 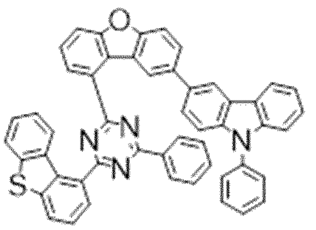 | 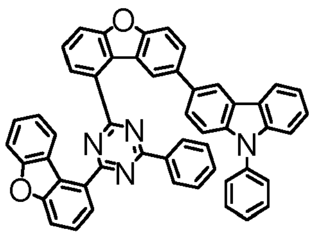 |
| **E4** | **E5** | **E6** |
| 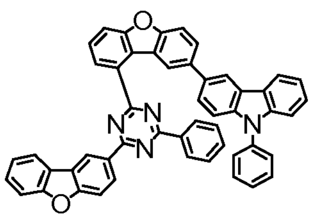 | 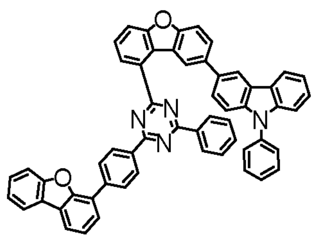 | 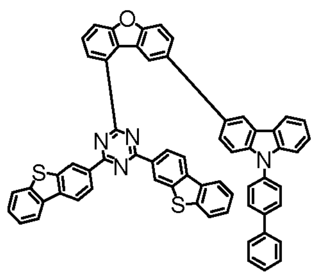 |
| **E7** | **E8** | **E9** |

(fortgesetzt)

| | | |
|---|---|---|
| 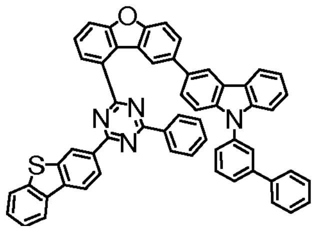 | 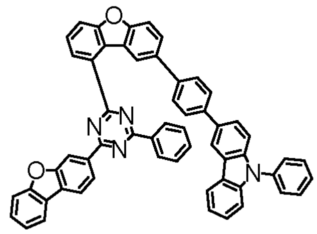 | 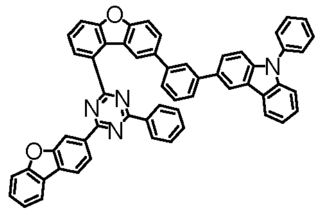 |
| **E10** | **E11** | **E12** |
| 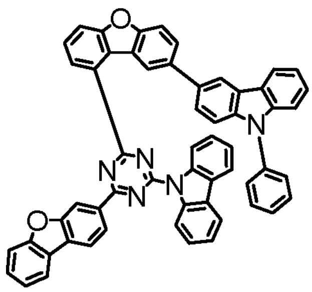 | 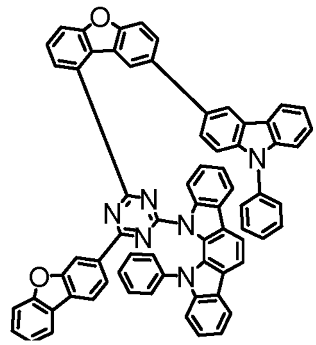 | 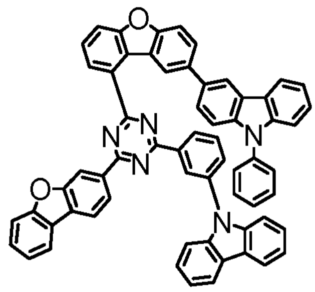 |
| **E13** | **E14** | **E15** |
| 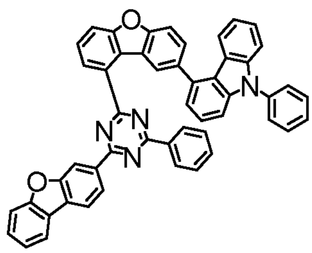 | 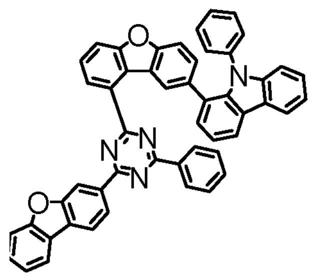 | 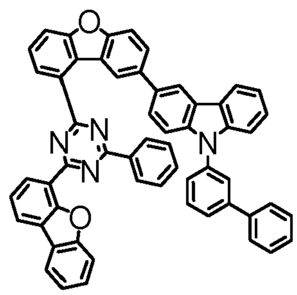 |
| **E16** | **E17** | **E18** |
| 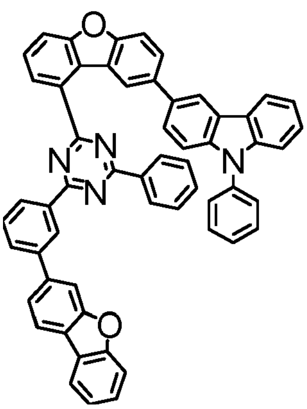 | 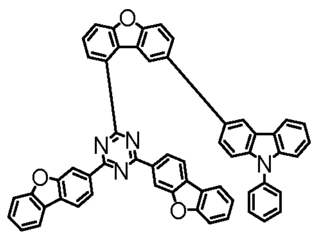 | 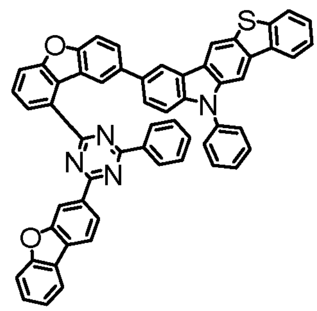 |
| **E19** | **E20** | **E21** |

(fortgesetzt)

| | | |
|---|---|---|
| 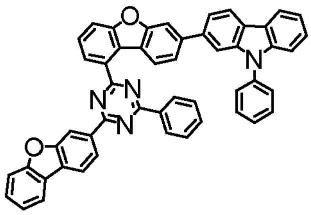 | 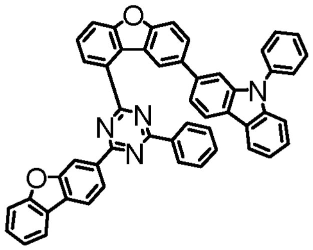 | 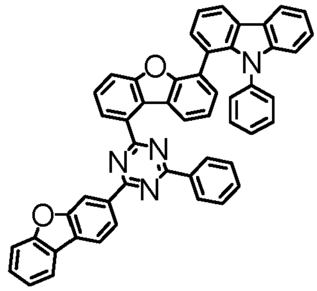 |
| **E22** | **E23** | **E24** |
| 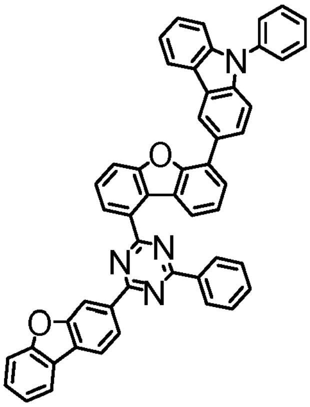 | 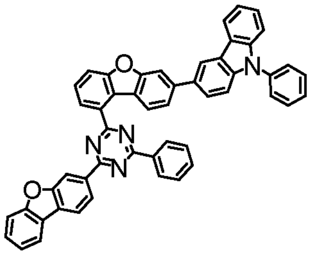 | 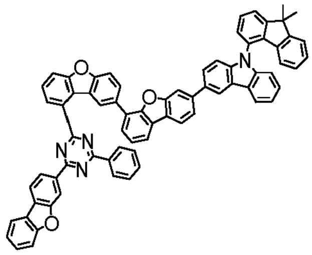 |
| **E25** | **E26** | **E27** |
| 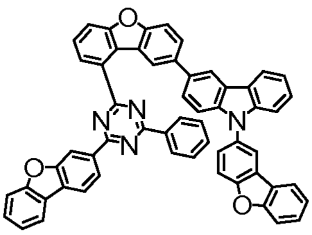 | 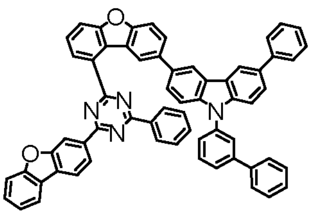 | 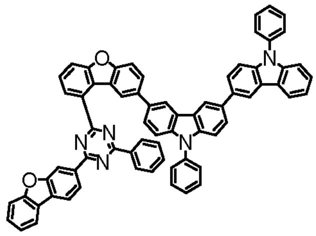 |
| **E28** | **E29** | **E30** |
| 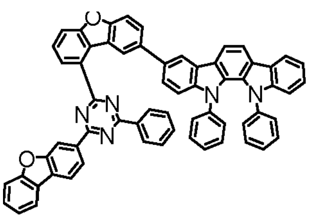 | 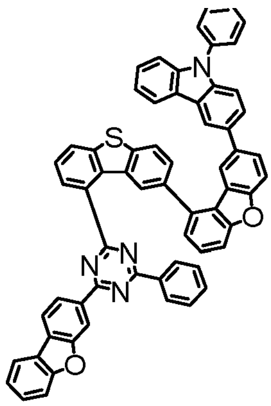 | 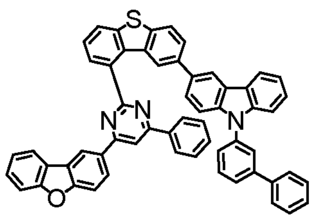 |
| **E31** | **E32** | **E33** |

(fortgesetzt)

| | | |
|---|---|---|
| 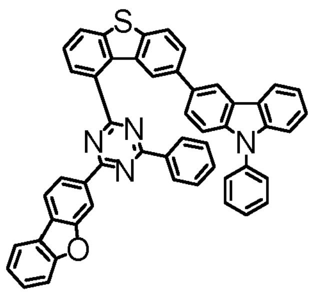 | 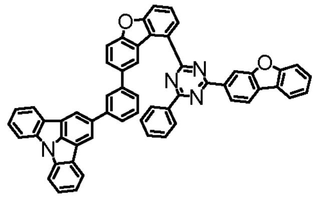 | 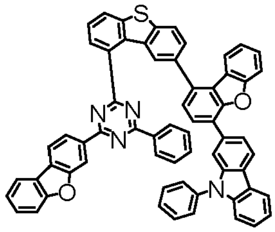 |
| **E34** | **E35** | **E36** |
| 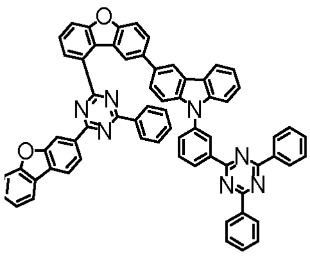 | 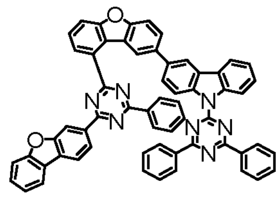 | 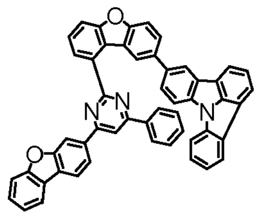 |
| **E37** | **E38** | **E39** |
| 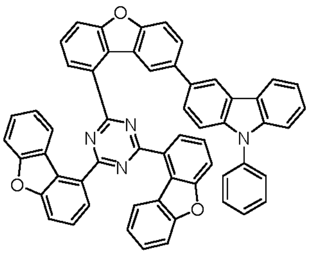 | 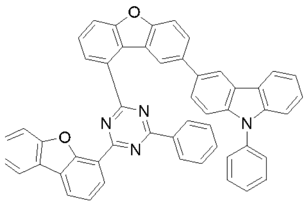 1 | 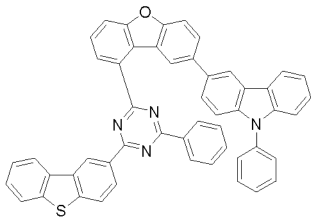 |
| **E40** | **E41** | **E42** |
| 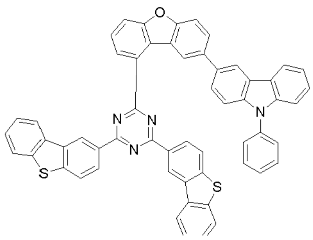 | 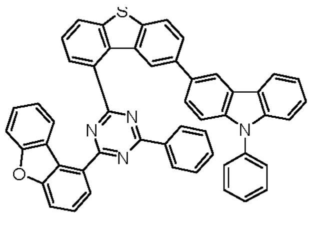 | 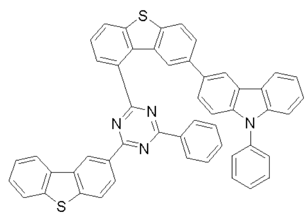 |
| **E43** | **E44** | **E45** |

(fortgesetzt)

| | | |
|---|---|---|
| 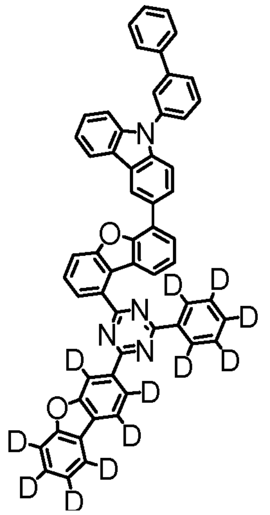 | 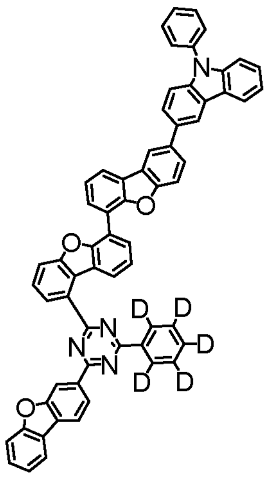 | 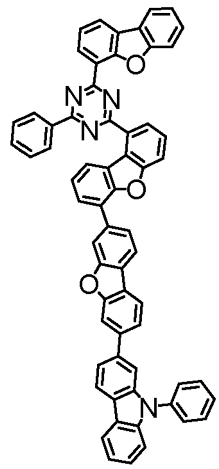 |
| **E46** | **E47** | **E48.** |

Die Herstellung der Verbindungen der Formel (1) oder der bevorzugten Verbindungen der Tabelle 1 sowie der Verbindungen **E1** bis **E48** ist dem Fachmann bekannt. Die Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Ein geeignetes Syntheseverfahren ist allgemein im folgenden Schema 1 dargestellt, wobei die verwendeten Symbole und Indizes die oben genannten Bedeutungen aufweisen.

Schema 1:

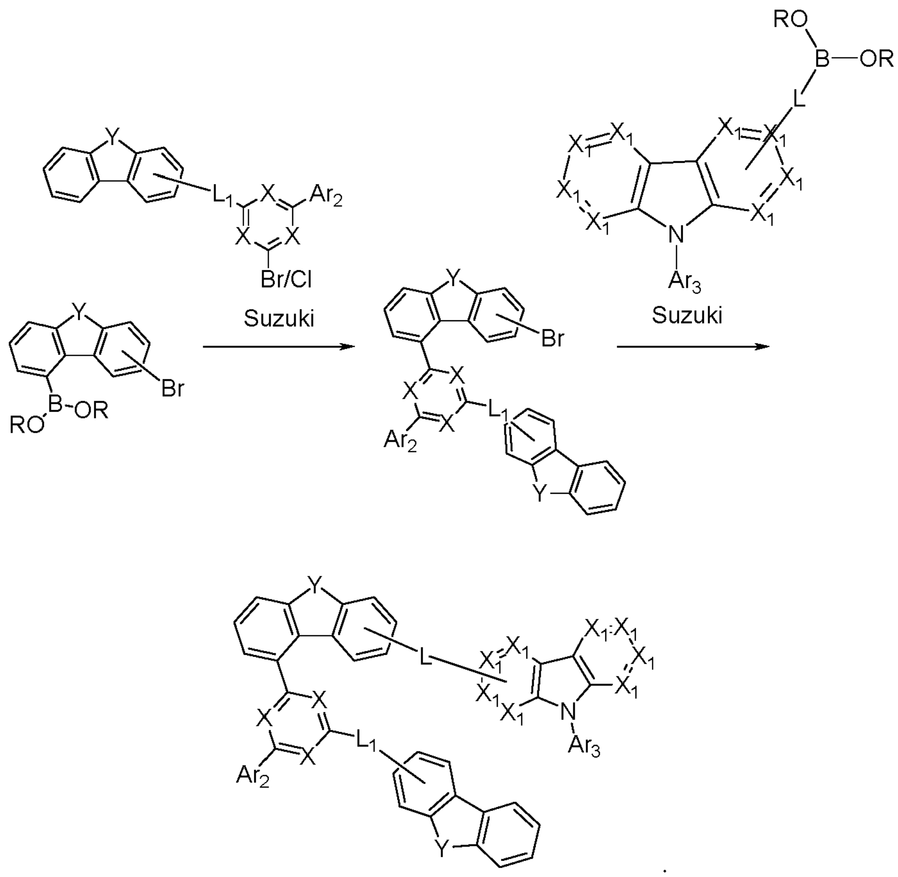

Im Folgenden wird das Hostmaterial 2 und dessen bevorzugte Ausführungsformen beschrieben, welches/welche in der erfindungsgemäßen Vorrichtung enthalten ist/sind. Die bevorzugten Ausführungsformen des Hostmaterials 2 der Formel (2) gelten auch für die erfindungsgemäße Mischung und/oder Formulierung.

Hostmaterial 2 ist mindestens eine Verbindung der Formel (2),

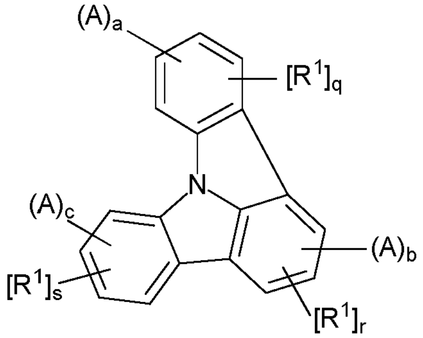

Formel (2) ,

wobei für die verwendeten Symbole und Indizes gilt:

A ist bei jedem Auftreten unabhängig voneinander eine Gruppe der Formel (3) oder (4),

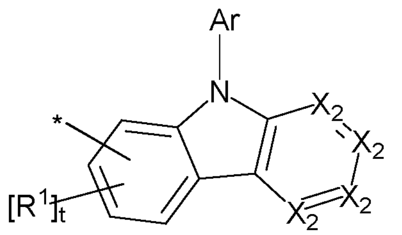

Formel (3) ,

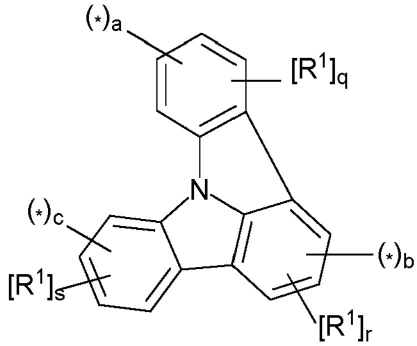

Formel (4) ;

$X_2$ ist bei jedem Auftreten gleich oder verschieden CH, $CR^1$ oder N, wobei maximal 2 Symbole $X_2$ N bedeuten können;

* kennzeichnet die Bindungsstelle an die Formel (2);

$R^1$ ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen; dabei können zwei Substituenten $R^1$, die an dasselbe Kohlenstoffatom oder an benachbarte Kohlenstoffatome gebunden sind ein monocyclisches oder polycyclisches, aliphatisches, aromatisches oder heteroaromatisches Ringsystem bilden, das mit einem oder mehreren Resten $R^2$ substituiert sein kann;

Ar ist jeweils unabhängig voneinander bei jedem Auftreten eine Arylgruppe mit 6 bis 40 aromatischen Ringatomen, die mit einem oder mehreren Resten R# substituiert sein kann, oder eine Heteroarylgruppe mit 5 bis 40 aromatischen Ringatomen, die mit einem oder mehreren Resten R# substituiert sein kann;

R# ist bei jedem Auftreten gleich oder verschieden ausgewählt aus der Gruppe bestehend aus D, F, Cl, Br, I, CN, $NO_2$, $C(=O)R^2$, $P(=O)(Ar_1)_2$, $P(Ar_1)_2$, $B(Ar_1)_2$, $Si(Ar_1)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C=CR^2$, $Si(R^2)_2$, C=O, C=S, $C=NR^2$, $P(=O)(R^2)$, SO, $SO_2$, $NR^2$, O, S oder $CONR^2$ ersetzt sein können

und wobei ein oder mehrere H-Atome durch D, F, Cl, Br, I, CN oder $NO_2$ ersetzt sein können, einem aromatischen oder heteroaromatischen Ringsystem mit 5 bis 40 aromatischen Ringatomen, das jeweils mit einem oder mehreren Resten $R^2$ substituiert sein kann, einer Aryloxy- oder Heteroaryloxygruppe mit 5 bis 40 aromatischen Ringatomen, die mit einem oder mehreren Resten $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann;

$R^2$ ist bei jedem Auftreten gleich oder verschieden ausgewählt aus der Gruppe bestehend aus H, D, F, Cl, Br, I, CN, $NO_2$, $N(Ar_1)_2$, $NH_2$, $N(R^3)_2$, $C(=O)Ar_1$, C(=O)H, $C(=O)R^3$, $P(=O)(Ar_1)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, wobei eine oder mehrere nicht-benachbarte $CH_2$-Gruppen durch HC=CH, $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH oder $CONR^3$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 aromatischen Ringatomen, das jeweils mit einem oder mehreren Resten $R^3$ substituiert sein kann, einer Aryloxy- oder Heteroaryloxygruppe mit 5 bis 60 aromatischen Ringatomen, die mit einem oder mehreren Resten $R^3$ substituiert sein kann, oder einer Kombination dieser Systeme, wobei optional zwei oder mehr benachbarte Substituenten $R^2$ ein monocyclisches oder polycyclisches, aliphatisches, aromatisches oder heteroaromatisches Ringsystem bilden können, das mit einem oder mehreren Resten $R^3$ substituiert sein kann;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^3$ miteinander ein mono- oder polycyclisches, aliphatisches Ringsystem bilden;

$Ar_1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^3$ substituiert sein kann; dabei können zwei Reste $Ar_1$, welche an dasselbe N-Atom, P-Atom oder B-Atom binden, auch durch eine Einfachbindung oder eine Brücke, ausgewählt aus $N(R^3)$, $C(R^3)_2$, O oder S, miteinander verbrückt sein;

a, b, c bedeuten jeweils unabhängig voneinander bei jedem Auftreten 0 oder 1, wobei die Summe der Indizes bei jedem Auftreten a+b+c 1 bedeutet; und

q, r, s, t bedeuten jeweils unabhängig bei jedem Auftreten 0 oder 1.

In einer Ausführungsform der Erfindung werden für die erfindungsgemäße Vorrichtung Verbindungen der Formel (2) ausgewählt, wie zuvor beschrieben, die mit Verbindungen der Formel (1), wie zuvor beschrieben oder bevorzugt beschrieben, oder mit den Verbindungen der Tabelle 1 oder den Verbindungen **E1** bis **E48,** in der lichtemittierenden Schicht verwendet werden.

In Verbindungen der Formel (2) bedeuten a, b, c jeweils unabhängig voneinander bei jedem Auftreten 0 oder 1, wobei die Summe der Indizes bei jedem Auftreten a+b+c 1 bedeutet. Bevorzugt hat c die Bedeutung 1.

Verbindungen der Formel (2) können durch folgende Formeln (2a), (2b) und (2c) dargestellt werden,

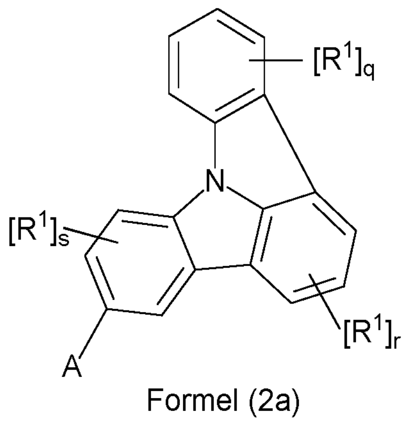

Formel (2a) ,

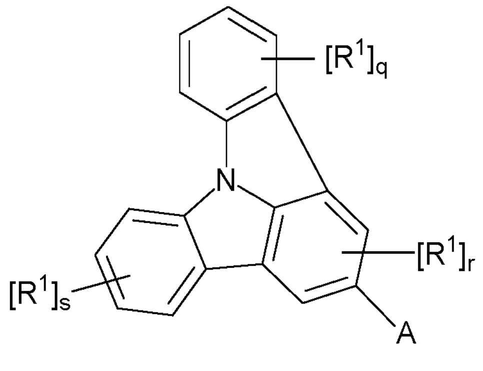

Formel (2b) ,

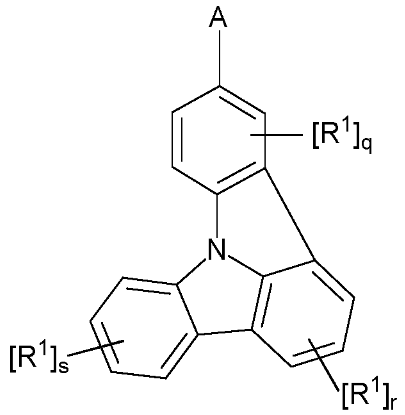

Formel (2c) ,

wobei A, $R^1$, q, r, s und t eine zuvor genannte oder nachfolgend genannte Bedeutung haben. Verbindungen der Formel (2a) sind hierbei bevorzugt.

Ein weiterer Gegenstand der Erfindung ist demzufolge eine organische elektrolumineszierende Vorrichtung, wie zuvor beschrieben oder bevorzugt beschrieben, wobei das Hostmaterial 2 einer Verbindung der Formel (2a), (2b) oder (2c) entspricht.

$R^1$ in Verbindungen der Formel (2) und der Formeln (2a) bis (2c) oder bevorzugten Verbindungen der Formeln (2) und (2a) bis (2c), wie zuvor beschrieben, ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen; dabei können zwei Substituenten $R^1$, die an dasselbe Kohlenstoffatom oder an benachbarte Kohlenstoffatome gebunden sind ein monocyclisches oder polycyclisches, aliphatisches, aromatisches oder heteroaromatisches Ringsystem bilden, das mit einem oder mehreren Resten $R^2$ substituiert sein kann.

Wenn mehrere Reste $R^1$ an benachbarte Kohlenstoffatome gebunden sind, so wird das mono-cyclische oder polycyclische, aliphatische, aromatische oder heteroaromatische Ringsystem bevorzugt aus der Gruppe (S-1) bis (S-4) ausgewählt,

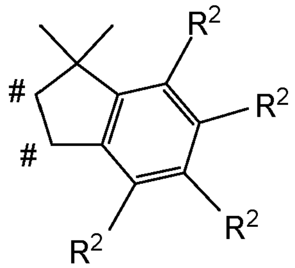

(S-1) ,

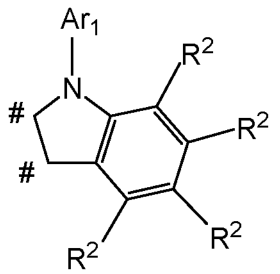

(S-2) ,

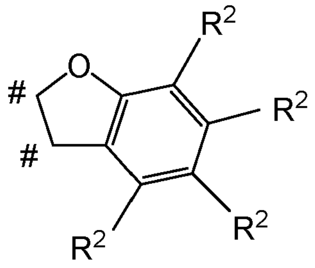

(S-3) ,

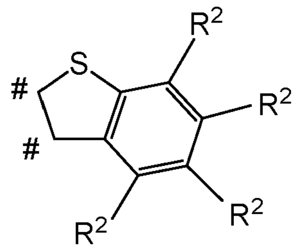

(S-4) ,

wobei $Ar_1$ und $R^2$ eine zuvor genannte Bedeutung oder bevorzugt genannte Bedeutung haben und # die Anknüpfungsstellen an den Rest der jeweiligen Struktur kennzeichnet, beispielsweise an benachbarte Positionen, gekennzeichnet durch $X_2$ in Verbindungen der Formeln (2), (2a), (2b) und (2c). Besonders bevorzugt wird hierbei (S-1) oder (S-2) ausgewählt.

$R^1$ in Verbindungen der Formel (2) und der Formeln (2a) bis (2c) oder bevorzugten Verbindungen der Formeln (2) und (2a) bis (2c), wie zuvor beschrieben, ist bei jedem Auftreten gleich oder verschieden bevorzugt ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen. Der Substituent $R^1$ steht bei jedem Auftreten unabhängig voneinander besonders bevorzugt für CN oder eine Arylgruppe mit 6 bis 40 C-Atomen, wie zuvor beschrieben. $R^1$ ist bei jedem Auftreten unabhängig voneinander besonders bevorzugt Phenyl.

In Verbindungen der Formeln (2), (2a), (2b) oder (2c) ist die Summe der Indizes q+r+s bevorzugt 0, 1 oder 2, wobei $R^1$ eine zuvor angegebene Bedeutung hat. In Verbindungen der Formeln (2), (2a), (2b) oder (2c) ist die Summe der Indizes q+r+s bevorzugt 0 oder 1, wobei $R^1$ eine zuvor angegebene Bedeutung hat.

In Verbindungen der Formeln (2), (2a), (2b) oder (2c) sind q, r und s bevorzugt 0 oder 1. Bevorzugt ist q 1, falls die Summe der Indizies q+r+s 1 ist. Bevorzugt sind q, r und s 0.

In Formel (4)

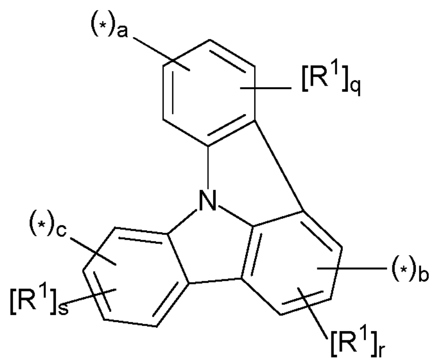

Formel (4)

sind q, r und s 0 oder 1, wobei $R^1$ eine zuvor angegebene Bedeutung hat. Bevorzugt ist die Summe der Indizes q+r+s in Formel (4) 0 oder 1. In Formel (4) sind q, r und s besonders bevorzugt 0.

In Formel (3)

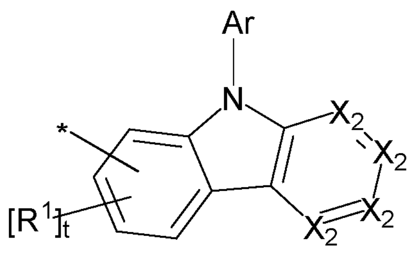

Formel (3)

ist t jeweils unabhängig voneinander bevorzugt 0 oder 1. In Formel (3) ist t bevorzugt gleich und entspricht 0.

In Verbindungen der Formeln (2), (2a), (2b) und (2c) oder bevorzugten Verbindungen der Formeln (2), (2a), (2b) und (2c) ist $X_2$ bei jedem Auftreten gleich oder verschieden CH, $CR^1$ oder N, wobei maximal 2 Symbole $X_2$ N bedeuten können.

In Verbindungen der Formeln (2), (2a), (2b) und (2c) oder bevorzugten Verbindungen der Formeln (2), (2a), (2b) und (2c) ist $X_2$ bei jedem Auftreten gleich oder verschieden bevorzugt CH, $CR^1$ oder N, wobei maximal 1 Symbol $X_2$ N bedeutet.

In Verbindungen der Formeln (2), (2a), (2b) und (2c) oder bevorzugten Verbindungen der Formeln (2), (2a), (2b) und (2c) ist $X_2$ bei jedem Auftreten gleich oder verschieden besonders bevorzugt zwei Mal CH und zwei Mal $CR^1$ oder drei Mal CH und ein Mal $CR^1$, wobei die Substituenten $R^1$ bei jedem Auftreten unabhängig voneinander eine zuvor angegebene Bedeutung haben.

Ar ist jeweils unabhängig voneinander bei jedem Auftreten eine Arylgruppe mit 6 bis 40 aromatischen Ringatomen, die mit einem oder mehreren Resten R# substituiert sein kann, oder eine Heteroarylgruppe mit 5 bis 40 aromatischen Ringatomen, die mit einem oder mehreren Resten R# substituiert sein kann, wobei der Rest R# eine zuvor angegebene oder nachfolgend bevorzugt angegebene Bedeutung hat.

Ar ist jeweils unabhängig voneinander bei jedem Auftreten bevorzugt eine Arylgruppe mit 6 bis 40 aromatischen Ringatomen, die mit einem oder mehreren Resten R# substituiert sein kann, oder eine Heteroarylgruppe mit 5 bis 40 aromatischen Ringatomen, enthaltend O oder S als Heteroatom, die mit einem oder mehreren Resten R# substituiert sein kann, wobei der Rest R# eine zuvor angegebene oder bevorzugt angegebene Bedeutung hat.

Ar ist bevorzugt bei jedem Auftreten eine Arylgruppe mit 6 bis 18 C-Atomen, die mit einem oder mehreren Resten R# substituiert sein kann, oder Dibenzofuranyl oder Dibenzothiophenyl, das mit einem oder mehreren Resten R# substituiert sein kann, wobei der Rest# eine zuvor angegebene oder nachfolgend bevorzugt angegebene Bedeutung hat.

Ar ist besonders bevorzugt Phenyl, mit Dibenzofuran substituiertes Phenyl, mit Dibenzothiophen substituiertes Phenyl, 1,3-Biphenyl, 1-4-Biphenyl, Terphenyl, Quaterphenyl, Naphthyl, Fluorenyl, 9,9-Diphenyl-fluorenyl, Bispirofluorenyl, Triphenylenyl, Dibenzofuranyl, mit Phenyl substituiertes Dibenzofuranyl, Dibenzothiophenyl oder mit Phenyl substituiertes Dibenzothiophenyl. Ar ist ganz besonders bevorzugt Phenyl, 1,3-Biphenyl, 1-4-Biphenyl, Naphth-2-yl oder Triphenyl-2-yl.

In Verbindungen der Formeln (2), (2a), (2b) und (2c) oder bevorzugten Verbindungen der Formeln (2), (2a), (2b) und (2c) ist R# bei jedem Auftreten gleich oder verschieden bevorzugt ausgewählt aus der Gruppe bestehend aus D, CN und einem aromatischen oder heteroaromatischen Ringsystem mit 5 bis 40 aromatischen Ringatomen, das jeweils mit einem oder mehreren Resten $R^2$ substituiert sein kann.

In Verbindungen der Formeln (2), (2a), (2b) und (2c) oder bevorzugten Verbindungen der Formeln (2), (2a), (2b) und (2c) ist R# bei jedem Auftreten gleich oder verschieden besonders bevorzugt ein nicht substituiertes aromatisches Ringsystem mit 5 bis 20 aromatischen Ringatomen, bevorzugt Phenyl.

In einer bevorzugten Ausführungsform der Erfindung entspricht A der Formel (4), wie zuvor beschrieben oder mit Substituenten, wie bevorzugt beschrieben.

In einer bevorzugten Ausführungsform der Erfindung entspricht A der Formel (3), wie zuvor beschrieben oder mit Substituenten, wie bevorzugt beschrieben.

Verbindungen der Formeln (2), (2a), (2b) und (2c), wobei A der Formel (3) entspricht und q, r, s und t 0 bedeuten, können durch die Formeln (2d) und (2e) dargestellt werden,

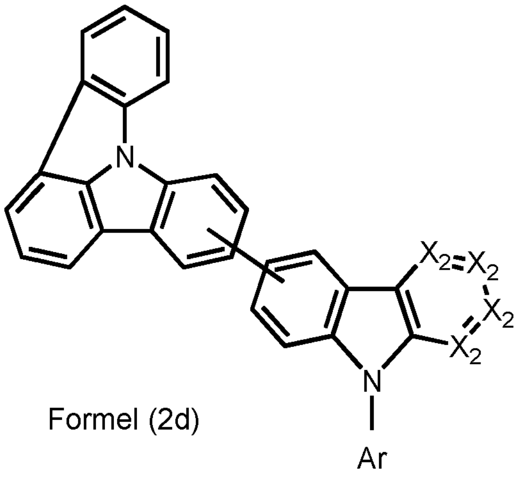

Formel (2d)

,

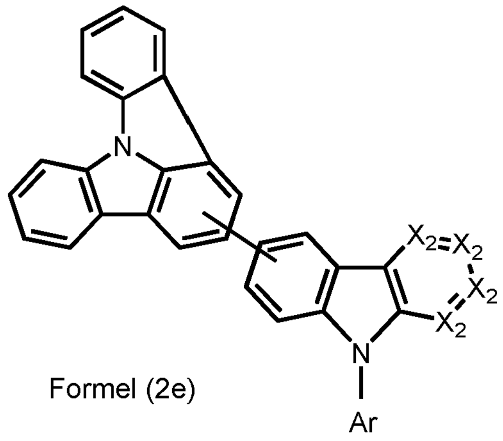

Formel (2e)

,

wobei $X_2$ und Ar eine zuvor genannte oder bevorzugt genannte Bedeutung haben.

Ein weiterer Gegenstand der Erfindung ist demzufolge eine organische elektrolumineszierende Vorrichtung, wie zuvor beschrieben oder bevorzugt beschrieben, wobei die mindestens eine Verbindung der Formel (2) einer Verbindung der Formel (2d) oder der Formel (2e) entspricht.

In einer bevorzugten Ausführungsform der Verbindungen der (2), (2a), (2b), (2c), (2d) oder (2e) sind die Substituenten der Formeln (3) oder (4) jeweils in 2-Position oder 5-Position des Indolo[3,2,1-*jk*]carbazoles miteinander verknüpft, wie nachfolgend schematisch dargestellt, wobei die gestrichelte Linie die Verknüpfung an den Substituenten der Formel (3) bzw. (4) anzeigt:

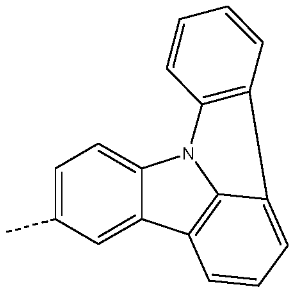

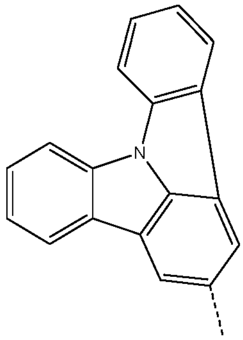

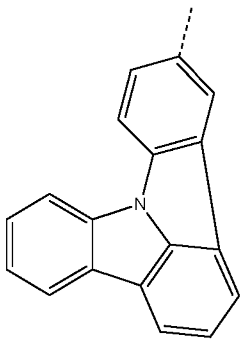

.

Beispiele für geeignete Hostmaterialien der Formel (2), (2a), (2b), (2c), (2d) und (2e), die erfindungsgemäß ausgewählt werden, und bevorzugt in Kombination mit mindestens einer Verbindung der Formel (1) in der erfindungsgemäßen elektrolumineszierenden Vorrichtung verwendet werden, sind die nachstehend genannten Strukturen der Tabelle 3.

Tabelle 3:

| | | |
|---|---|---|
| 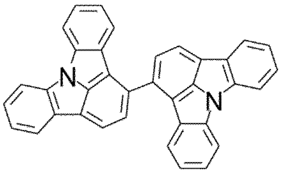 | 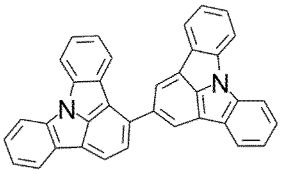 | 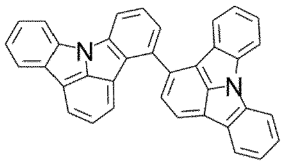 |
| 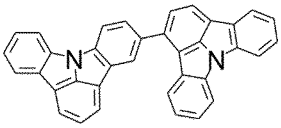 | 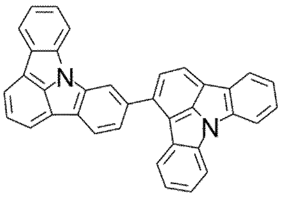 | 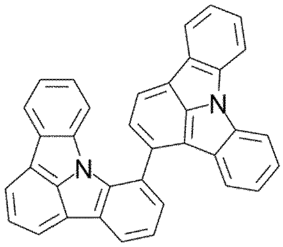 |
| 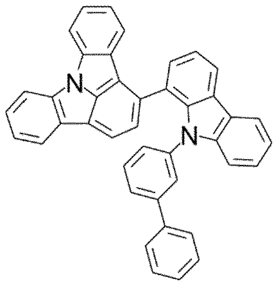 | 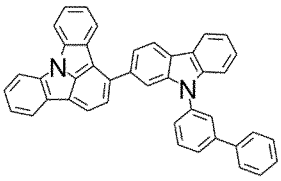 | 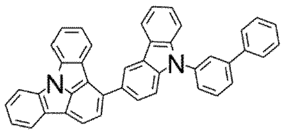 |
| 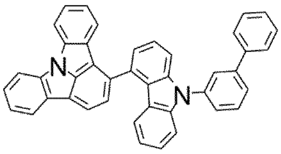 | 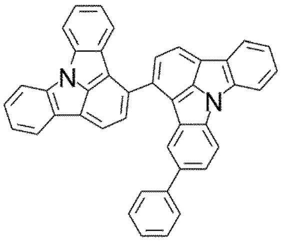 | 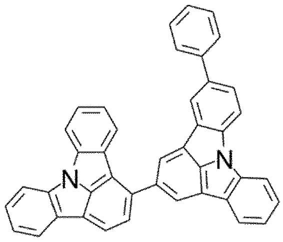 |

| | | |
|---|---|---|
| 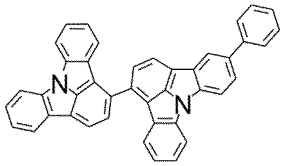 | 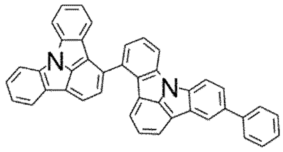 | 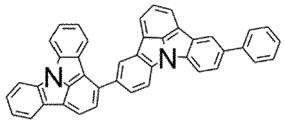 |
| 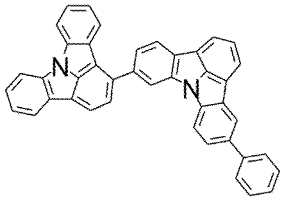 | 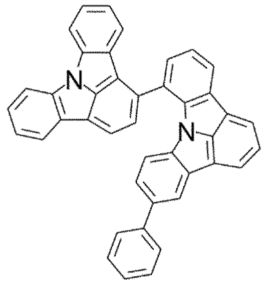 | 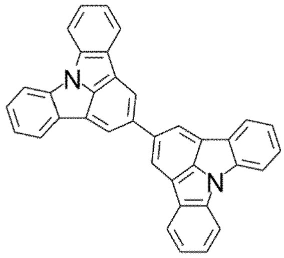 |
| 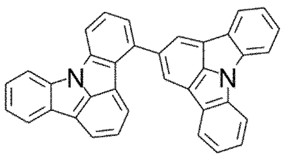 | 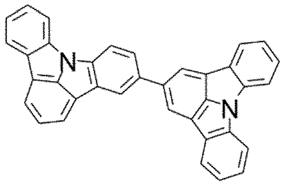 | 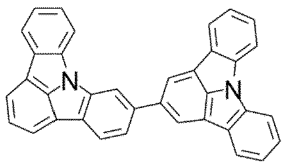 |
| 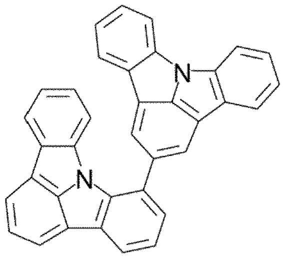 | 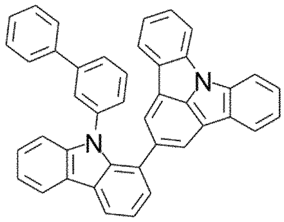 | 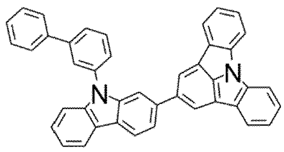 |
| 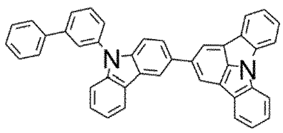 | 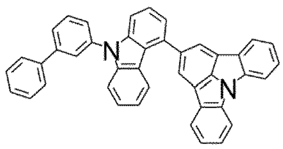 | 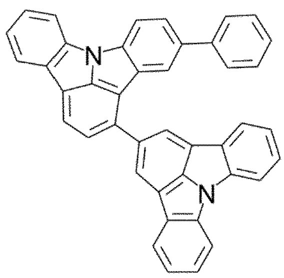 |

| | | |
|---|---|---|
| 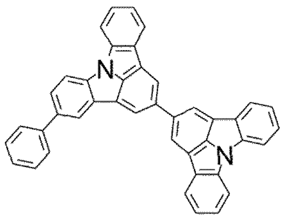 | 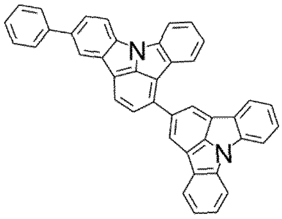 | 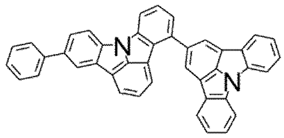 |
| 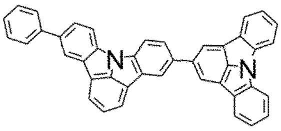 | 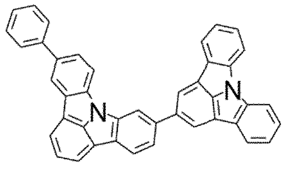 | 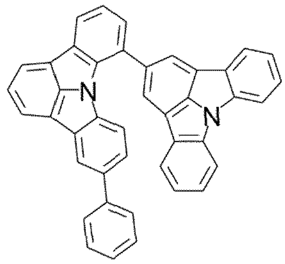 |
| 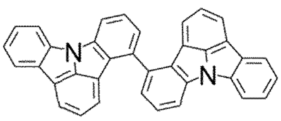 | 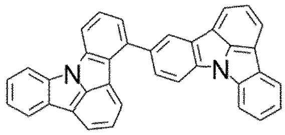 | 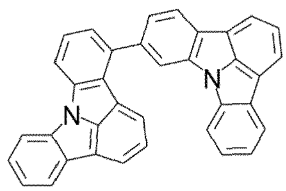 |
| 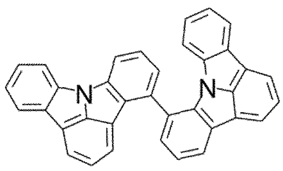 | 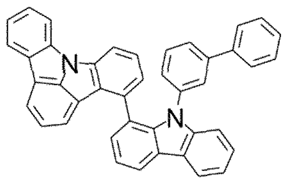 | 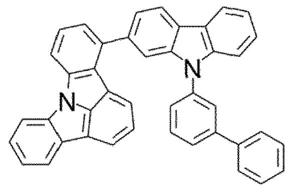 |
| 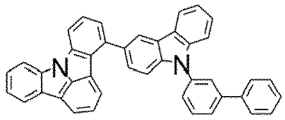 | 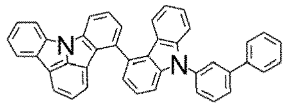 | 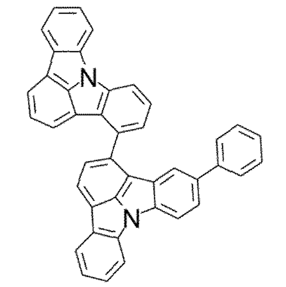 |

| | | |
|---|---|---|
| 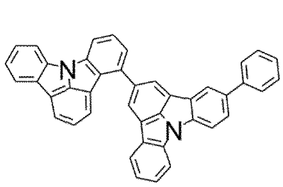 | 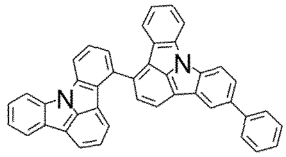 | 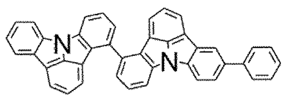 |
| 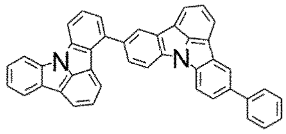 | 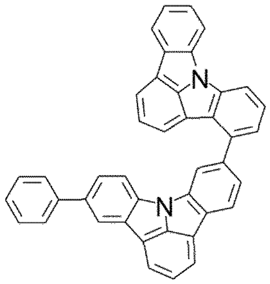 | 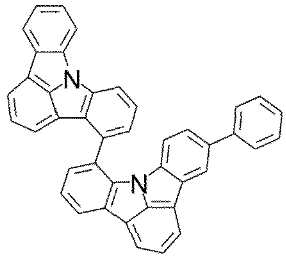 |
| 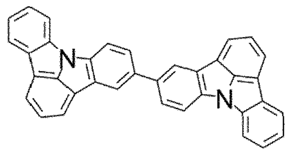 | 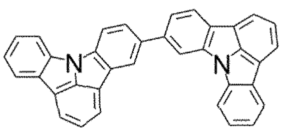 | 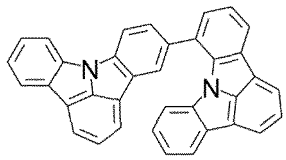 |
| 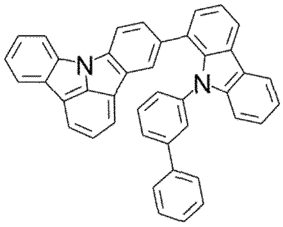 | 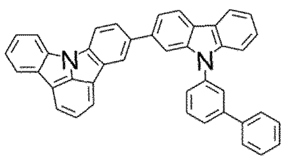 | 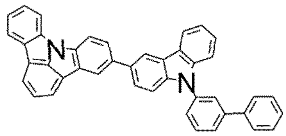 |
| 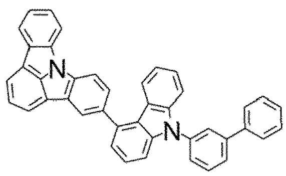 | 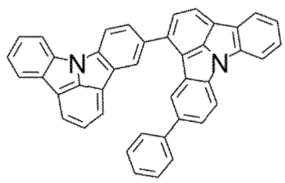 | 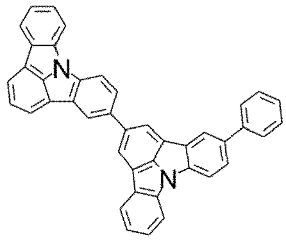 |

| | | |
|---|---|---|
| 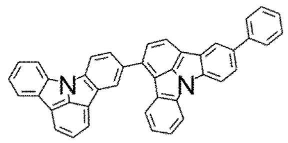 | 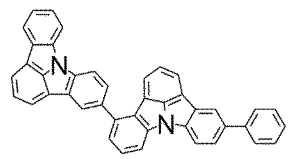 | 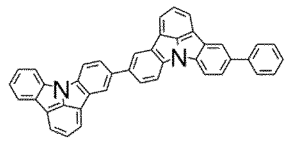 |
| 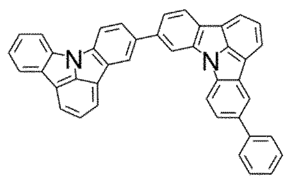 | 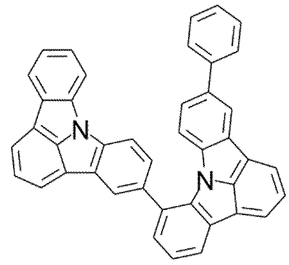 | 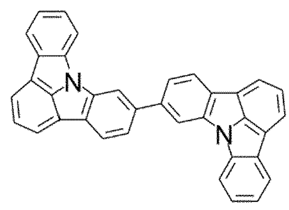 |
| 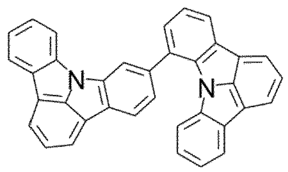 | 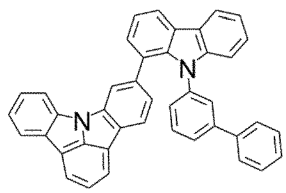 | 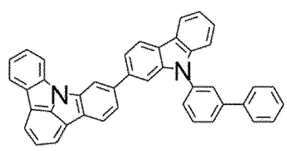 |
| 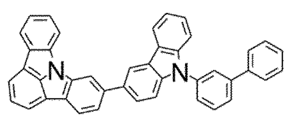 | 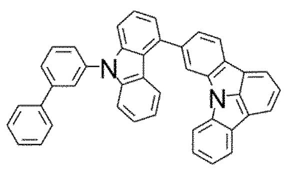 | 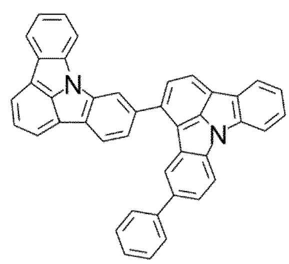 |
| 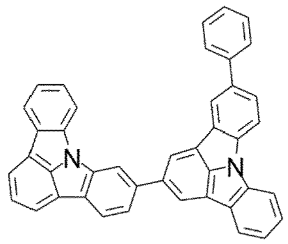 | 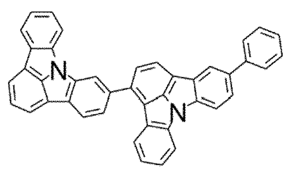 | 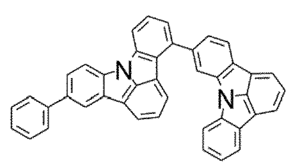 |

| | | |
|---|---|---|
| 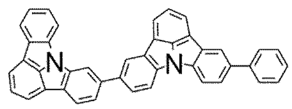 | 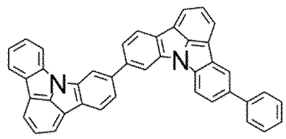 | 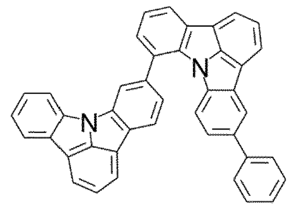 |
| 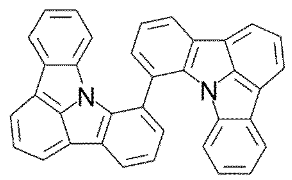 | 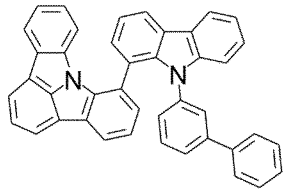 | 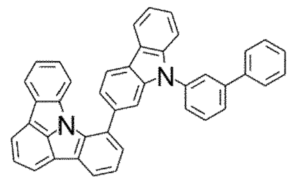 |
| 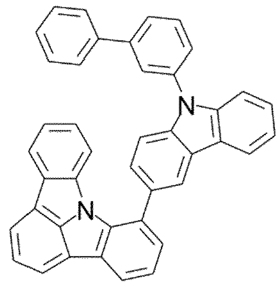 | 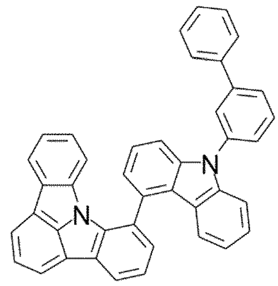 | 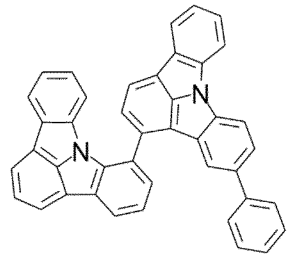 |
| 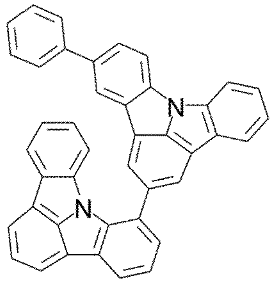 | 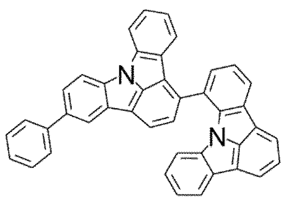 | 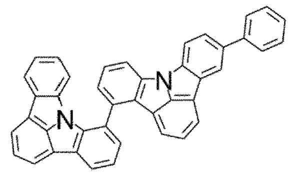 |
| 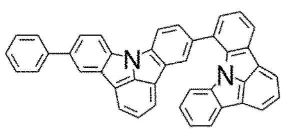 | 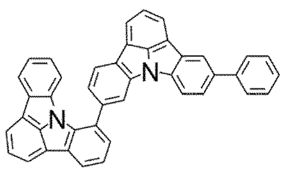 | 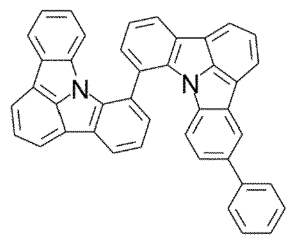 |

Besonders geeignete Verbindungen der Formel (2), die bevorzugt in Kombination mit mindestens einer Verbindung der Formel (1) in der erfindungsgemäßen elektrolumineszierenden Vorrichtung verwendet werden, sind die Verbindungen **H1** bis **H21** der Tabelle 4.

Tabelle 4:

| | | |
|---|---|---|
| 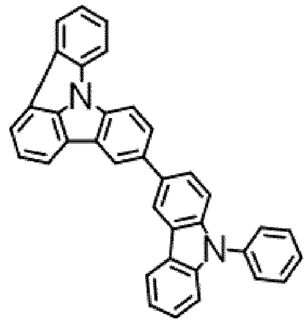 | 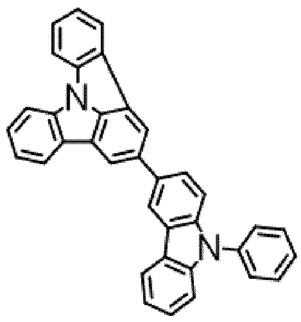 | 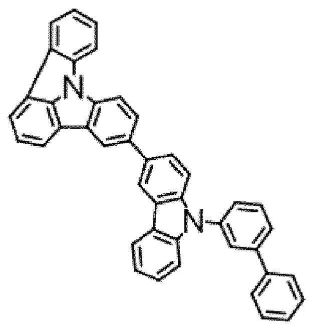 |
| **H1** | **H2** | **H3** |
| 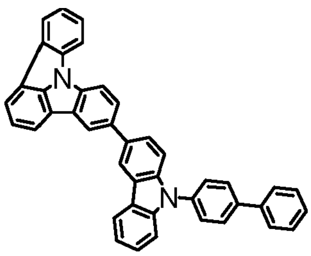 | 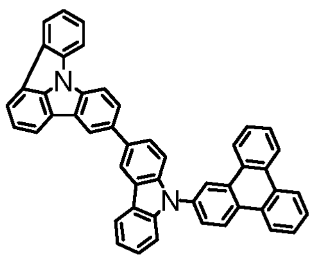 | 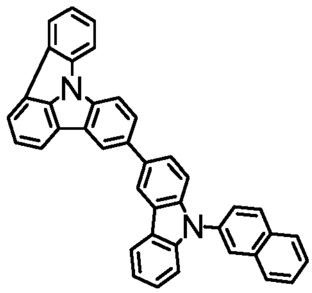 |
| **H4** | **H5** | **H6** |
| 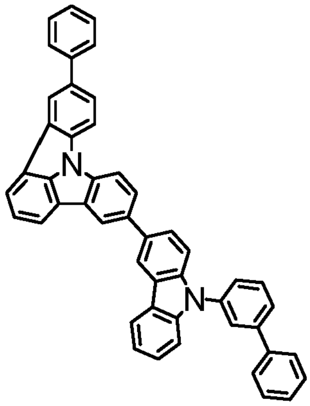 | 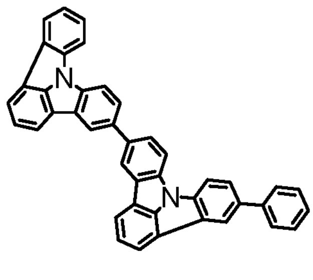 | 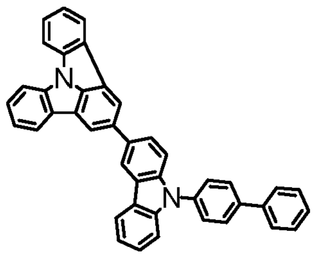 |
| **H7** | **H8** | **H9** |
| 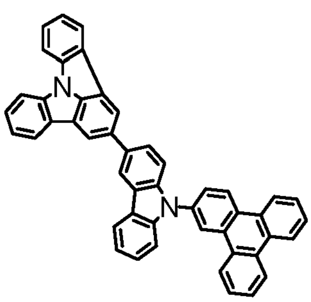 | 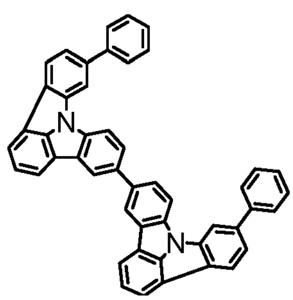 | 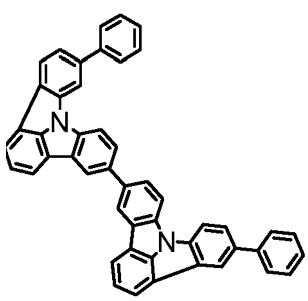 1 |
| **H10** | **H11** | **H12** |

(fortgesetzt)

| | | |
|---|---|---|
| 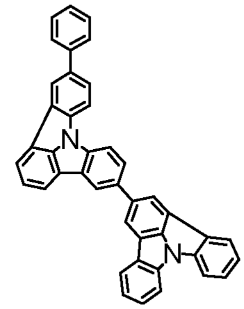 | 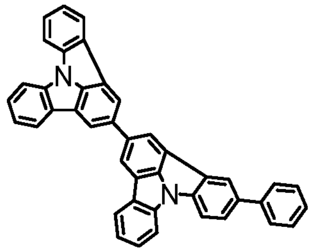 | 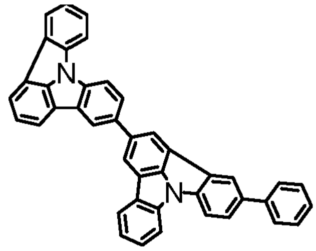 |
| **H13** | **H14** | **H15** |
| 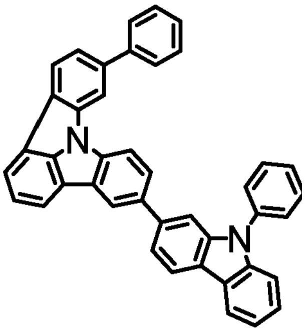 | 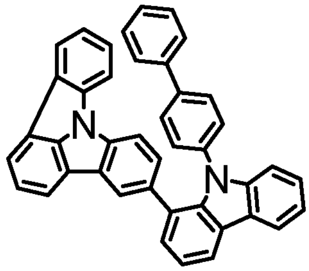 | 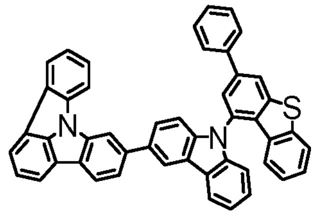 |
| **H16** | **H17** | **H18** |
| 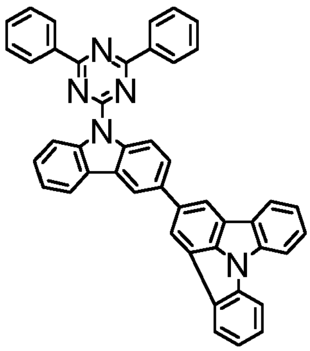 | 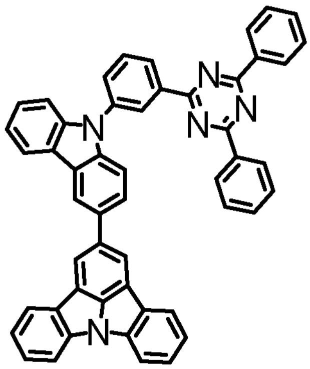 | 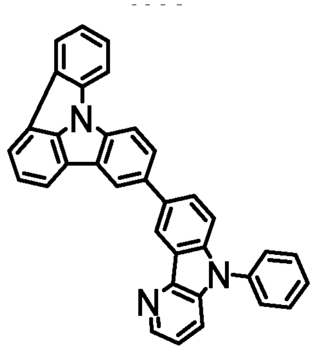 |
| **H19** | **H20** | **H21.** |

Ganz besonders geeignete Verbindungen der Formel (2), die bevorzugt in Kombination mit mindestens einer Verbindung der Formel (1) in der erfindungsgemäßen elektrolumineszierenden Vorrichtung verwendet werden, sind die Verbindungen **H1, H3, H4, H5, H6, H7, H8, H11** und **H12.**

Die Herstellung der Verbindungen der Formel (2) oder der bevorzugten Verbindungen der Formel (2), (2a), (2b), (2c), (2d) und (2e), sowie der Verbindungen der Tabelle 3 und Verbindungen **H1** bis **H21** ist dem Fachmann bekannt. Die Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Ein geeignetes Syntheseverfahren ist allgemein im folgenden Schema 2 dargestellt, wobei die verwendeten Symbole und Indizes die oben genannten Bedeutungen aufweisen.

Schema 2:

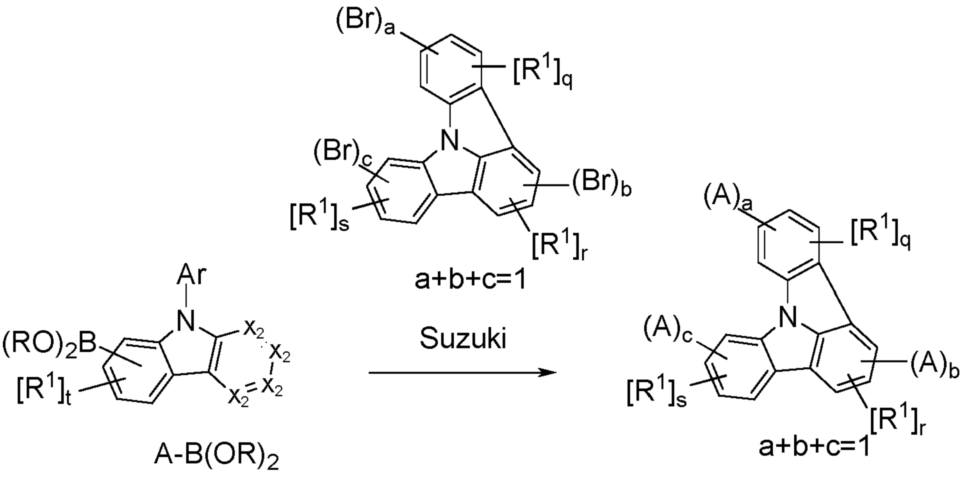


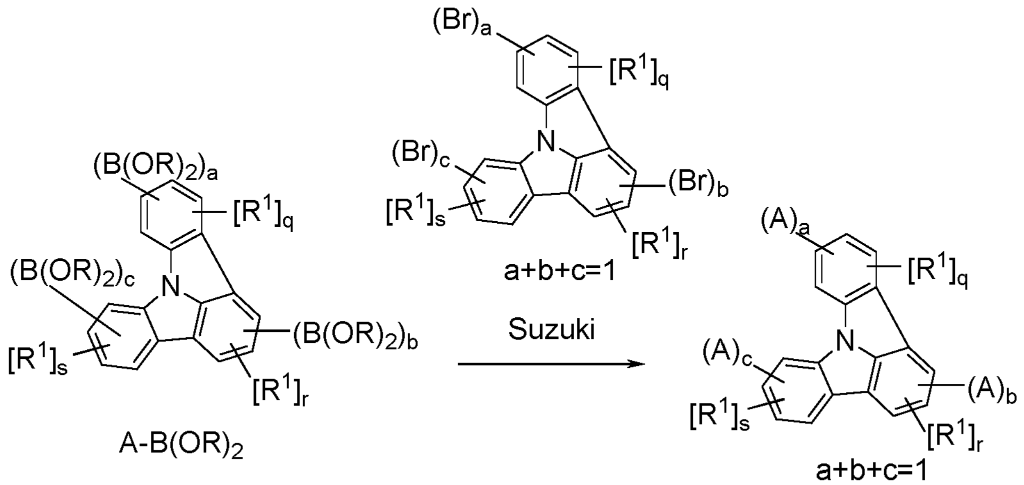


Die vorstehend genannten Hostmaterialien der Formel (1) sowie deren bevorzugt beschriebene Ausführungsformen oder die Verbindungen der Tabelle 1 und der Verbindungen **E1** bis **E48** können in der erfindungsgemäßen Vorrichtung beliebig mit den genannten Hostmaterialien der Formel (2), (2a), (2b), (2c), (2d) und (2e) sowie deren bevorzugt beschriebene Ausführungsformen oder den Verbindungen der Tabelle 3 oder den Verbindungen **H1** bis **H21** kombiniert werden.

Ein weiterer Gegenstand der Erfindung sind ebenfalls Mischungen enthaltend mindestens eine Verbindung der Formel (1) als Hostmaterial 1 und mindestens eine Verbindung der Formel (2) als Hostmaterial 2,

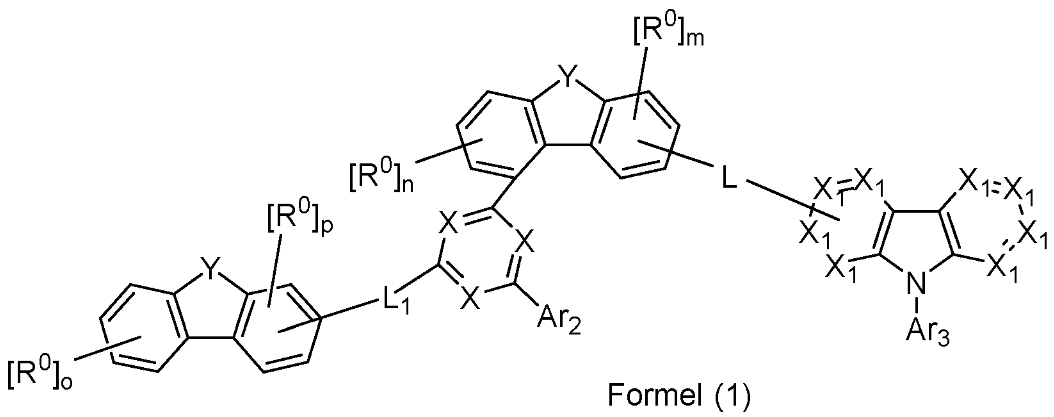

Formel (1)

,

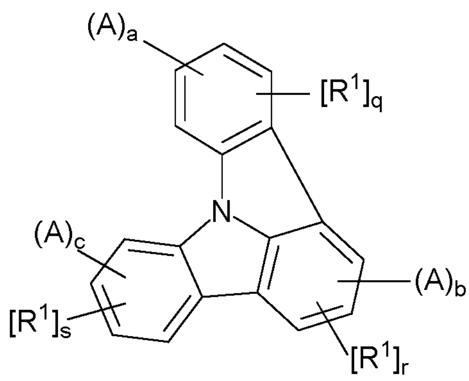

Formel (2) ,

wobei für die verwendeten Symbole und Indizes gilt:

X ist bei jedem Auftreten gleich oder verschieden $CR^0$ oder N, wobei mindestens ein Symbol X für N steht;

$X_1$ ist bei jedem Auftreten gleich oder verschieden CH, CR oder N, wobei maximal 3 Symbole $X_1$ N bedeuten können;

$X_2$ ist bei jedem Auftreten gleich oder verschieden CH, $CR^1$ oder N, wobei maximal 2 Symbole $X_2$ N bedeuten können;

Y ist bei jedem Auftreten gleich oder verschieden ausgewählt aus O oder S;

L und $L_1$ sind bei jedem Auftreten gleich oder verschieden eine Einfachbindung oder ein aromatisches oder heteroaromatisches Ringsystem mit 5 bis 30 aromatischen Ringatomen;

$R^0$ ist bei jedem Auftreten unabhängig voneinander H, D oder ein unsubstituiertes oder teilweise oder vollständig deuteriertes aromatisches Ringsystem mit 6 bis 18 C-Atomen;

R und R# sind bei jedem Auftreten gleich oder verschieden ausgewählt aus der Gruppe bestehend aus D, F, Cl, Br, I, CN, $NO_2$, $C(=O)R^2$, $P(=O)(Ar_1)_2$, $P(Ar_1)_2$, $B(Ar_1)_2$, $Si(Ar_1)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C=CR^2$, $Si(R^2)_2$, C=O, C=S, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 aromatischen Ringatomen, das jeweils mit einem oder mehreren Resten $R^2$ substituiert sein kann, einer Aryloxy- oder Heteroaryloxygruppe mit 5 bis 40 aromatischen Ringatomen, die mit einem oder mehreren Resten $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann; dabei können zwei Substituenten R, die an dasselbe Kohlenstoffatom oder an benachbarte Kohlenstoffatome gebunden sind oder ein Substituent R zusammen mit $Ar_3$ ein monocyclisches oder polycyclisches, aliphatisches, aromatisches oder heteroaromatisches Ringsystem bilden, das mit einem oder mehreren Resten $R^2$ substituiert sein kann;

$R^1$ ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen; dabei können zwei Substituenten $R^1$, die an dasselbe Kohlenstoffatom oder an benachbarte Kohlenstoffatome gebunden sind ein monocyclisches oder polycyclisches, aliphatisches, aromatisches oder heteroaromatisches Ringsystem bilden, das mit einem oder mehreren Resten $R^2$ substituiert sein kann;

$R^2$ ist bei jedem Auftreten gleich oder verschieden ausgewählt aus der Gruppe bestehend aus H, D, F, Cl, Br, I, CN, $NO_2$, $N(Ar_1)_2$, $NH_2$, $N(R^3)_2$, $C(=O)Ar_1$, C(=O)H, $C(=O)R^3$, $P(=O)(Ar_1)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, wobei eine oder mehrere nichtbenachbarte $CH_2$-Gruppen durch HC=CH, $R^3C=CR^3$, C=C, $Si(R^3)_2$, $Ge(R^3)_2$, $Sn(R^3)_2$, C=O, C=S, C=Se,

C=NR$^3$, P(=O)(R$^3$), SO, SO$_2$, NH, NR$^3$, O, S, CONH oder CONR$^3$ ersetzt sein können und wobei ein oder mehrere H-Atome durch D, F, Cl, Br, I, CN oder NO$_2$ ersetzt sein können, einem aromatischen oder heteroaromatischen Ringsystem mit 5 bis 60 aromatischen Ringatomen, das jeweils mit einem oder mehreren Resten R$^3$ substituiert sein kann, einer Aryloxy- oder Heteroaryloxygruppe mit 5 bis 60 aromatischen Ringatomen, die mit einem oder mehreren Resten R$^3$ substituiert sein kann, oder einer Kombination dieser Systeme, wobei optional zwei oder mehr benachbarte Substituenten R$^2$ ein monocyclisches oder polycyclisches, aliphatisches, aromatisches oder heteroaromatisches Ringsystem bilden können, das mit einem oder mehreren Resten R$^3$ substituiert sein kann;

R$^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten R$^3$ miteinander ein mono- oder polycyclisches, aliphatisches Ringsystem bilden;

Ar$_1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R$^3$ substituiert sein kann; dabei können zwei Reste Ar$_1$, welche an dasselbe N-Atom, P-Atom oder B-Atom binden, auch durch eine Einfachbindung oder eine Brücke, ausgewählt aus N(R$^3$), C(R$^3$)$_2$, O oder S, miteinander verbrückt sein;

Ar$_2$ ist jeweils unabhängig voneinander bei jedem Auftreten eine Aryl- oder Heteroarylgruppe mit 5 bis 40 aromatischen Ringatomen, die mit einem oder mehreren Resten R$^2$ substituiert sein kann;

Ar$_3$ ist jeweils unabhängig voneinander bei jedem Auftreten ein aromatisches Ringsystem mit 6 bis 40 aromatischen Ringatomen oder ein heteroaromatisches Ringsystem mit 5 bis 40 aromatischen Ringatomen, das mit einem oder mehreren Resten R$^2$ substituiert sein kann;

A ist bei jedem Auftreten unabhängig voneinander eine Gruppe der Formel (3) oder (4),

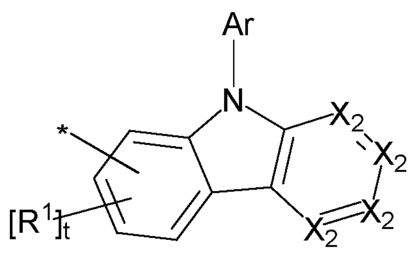

Formel (3) ,

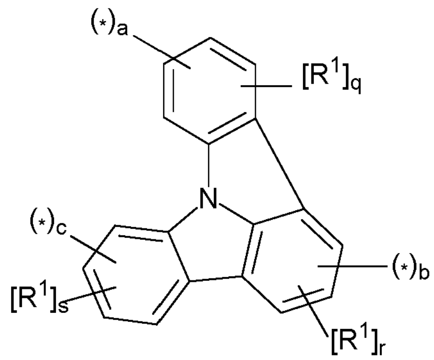

Formel (4) ;

Ar ist jeweils unabhängig voneinander bei jedem Auftreten eine Arylgruppe mit 6 bis 40 aromatischen Ringatomen, die mit einem oder mehreren Resten R# substituiert sein kann, oder eine Heteroarylgruppe mit 5 bis 40 aromatischen Ringatomen, die mit einem oder mehreren Resten R# substituiert sein kann;

* kennzeichnet die Bindungsstelle an die Formel (2);

a, b, c bedeuten jeweils unabhängig voneinander bei jedem Auftreten 0 oder 1, wobei die Summe der Indizes bei jedem Auftreten a+b+c 1 bedeutet;

o bedeutet 1, 2, 3 oder 4;

n, m und p bedeuten unabhängig voneinander bei jedem Auftreten 1, 2 oder 3; und

q, r, s, t bedeuten jeweils unabhängig bei jedem Auftreten 0 oder 1.

Die Ausführungen hinsichtlich der Hostmaterialien der Formeln (1) und (2) sowie deren bevorzugten Ausführungsformen gilt entsprechend auch für die erfindungsgemäße Mischung.

Besonders bevorzugte Mischungen der Hostmaterialien der Formel (1) mit den Hostmaterialien der Formel (2) für die erfindungsgemäße Vorrichtung erhält man durch Kombination der Verbindungen **E1** bis **E48** mit den Verbindungen der Tabelle 3.

Ganz besonders bevorzugte Mischungen der Hostmaterialien der Formel (1) mit den Hostmaterialien der Formel (2) für die erfindungsgemäße Vorrichtung erhält man durch Kombination der Verbindungen **E1** bis **E48** mit den Verbindungen **H1** bis **H21,** wie im Folgenden in Tabelle 5 gezeigt.

Tabelle 5:

| | | | | | | | | |
|---|---|---|---|---|---|---|---|---|
| M1 | **E1** | **H1** | M2 | **E2** | **H1** | M3 | **E3** | **H1** |
| M4 | **E4** | **H1** | M5 | **E5** | **H1** | M6 | **E6** | **H1** |
| M7 | **E7** | **H1** | M8 | **E8** | **H1** | M9 | **E9** | **H1** |
| M10 | **E10** | **H1** | M11 | **E11** | **H1** | M12 | **E12** | **H1** |
| M13 | **E13** | **H1** | M14 | **E14** | **H1** | M15 | **E15** | **H1** |
| M16 | **E16** | **H1** | M17 | **E17** | **H1** | M18 | **E18** | **H1** |
| M19 | **E19** | **H1** | M20 | **E20** | **H1** | M21 | **E21** | **H1** |
| M22 | **E22** | **H1** | M23 | **E23** | **H1** | M24 | **E24** | **H1** |
| M25 | **E25** | **H1** | M26 | **E26** | **H1** | M27 | **E27** | **H1** |
| M28 | **E28** | **H1** | M29 | **E29** | **H1** | M30 | **E30** | **H1** |
| M31 | **E31** | **H1** | M32 | **E32** | **H1** | M33 | **E33** | **H1** |
| M34 | **E34** | **H1** | M35 | **E35** | **H1** | M36 | **E36** | **H1** |
| M37 | **E37** | **H1** | M38 | **E38** | **H1** | M39 | **E39** | **H1** |
| M40 | **E1** | **H2** | M41 | **E2** | **H2** | M42 | **E3** | **H2** |
| M43 | **E4** | **H2** | M44 | **E5** | **H2** | M45 | **E6** | **H2** |
| M46 | **E7** | **H2** | M47 | **E8** | **H2** | M48 | **E9** | **H2** |
| M49 | **E10** | **H2** | M50 | **E11** | **H2** | M51 | **E12** | **H2** |
| M52 | **E13** | **H2** | M53 | **E14** | **H2** | M54 | **E15** | **H2** |
| M55 | **E16** | **H2** | M56 | **E17** | **H2** | M57 | **E18** | **H2** |
| M58 | **E19** | **H2** | M59 | **E20** | **H2** | M60 | **E21** | **H2** |
| M61 | **E22** | **H2** | M62 | **E23** | **H2** | M63 | **E24** | **H2** |
| M64 | **E25** | **H2** | M65 | **E26** | **H2** | M66 | **E27** | **H2** |
| M67 | **E28** | **H2** | M68 | **E29** | **H2** | M69 | **E30** | **H2** |
| M70 | **E31** | **H2** | M71 | **E32** | **H2** | M72 | **E33** | **H2** |
| M73 | **E34** | **H2** | M74 | **E35** | **H2** | M75 | **E36** | **H2** |
| M76 | **E37** | **H2** | M77 | **E38** | **H2** | M78 | **E39** | **H2** |
| M79 | **E1** | **H3** | M80 | **E2** | **H3** | M81 | **E3** | **H3** |
| M82 | **E4** | **H3** | M83 | **E5** | **H3** | M84 | **E6** | **H3** |
| M85 | **E7** | **H3** | M86 | **E8** | **H3** | M87 | **E9** | **H3** |
| M88 | **E10** | **H3** | M89 | **E11** | **H3** | M90 | **E12** | **H3** |
| M91 | **E13** | **H3** | M92 | **E14** | **H3** | M93 | **E15** | **H3** |
| M94 | **E16** | **H3** | M95 | **E17** | **H3** | M96 | **E18** | **H3** |
| M97 | **E19** | **H3** | M98 | **E20** | **H3** | M99 | **E21** | **H3** |
| M100 | **E22** | **H3** | M101 | **E23** | **H3** | M102 | **E24** | **H3** |
| M103 | **E25** | **H3** | M104 | **E26** | **H3** | M105 | **E27** | **H3** |
| M106 | **E28** | **H3** | M107 | **E29** | **H3** | M108 | **E30** | **H3** |
| M109 | **E31** | **H3** | M110 | **E32** | **H3** | M111 | **E33** | **H3** |
| M112 | **E34** | **H3** | M113 | **E35** | **H3** | M114 | **E36** | **H3** |

(fortgesetzt)

| | | | | | | | | |
|---|---|---|---|---|---|---|---|---|
| M115 | **E37** | **H3** | M116 | **E38** | **H3** | M117 | **E39** | **H3** |
| M118 | **E1** | **H4** | M119 | **E2** | **H4** | M120 | **E3** | **H4** |
| M121 | **E4** | **H4** | M122 | **E5** | **H4** | M123 | **E6** | **H4** |
| M124 | **E7** | **H4** | M125 | **E8** | **H4** | M126 | **E9** | **H4** |
| M127 | **E10** | **H4** | M128 | **E11** | **H4** | M129 | **E12** | **H4** |
| M130 | **E13** | **H4** | M131 | **E14** | **H4** | M132 | **E15** | **H4** |
| M133 | **E16** | **H4** | M134 | **E17** | **H4** | M135 | **E18** | **H4** |
| M136 | **E19** | **H4** | M137 | **E20** | **H4** | M138 | **E21** | **H4** |
| M139 | **E22** | **H4** | M140 | **E23** | **H4** | M141 | **E24** | **H4** |
| M142 | **E25** | **H4** | M143 | **E26** | **H4** | M144 | **E27** | **H4** |
| M145 | **E28** | **H4** | M 146 | **E29** | **H4** | M147 | **E30** | **H4** |
| M148 | **E31** | **H4** | M149 | **E32** | **H4** | M150 | **E33** | **H4** |
| M151 | **E34** | **H4** | M152 | **E35** | **H4** | M153 | **E36** | **H4** |
| M154 | **E37** | **H4** | M155 | **E38** | **H4** | M156 | **E39** | **H4** |
| M157 | **E1** | **H5** | M158 | **E2** | **H5** | M159 | **E3** | **H5** |
| M160 | **E4** | **H5** | M161 | **E5** | **H5** | M162 | **E6** | **H5** |
| M163 | **E7** | **H5** | M164 | **E8** | **H5** | M165 | **E9** | **H5** |
| M166 | **E10** | **H5** | M167 | **E11** | **H5** | M168 | **E12** | **H5** |
| M169 | **E13** | **H5** | M170 | **E14** | **H5** | M171 | **E15** | **H5** |
| M172 | **E16** | **H5** | M173 | **E17** | **H5** | M174 | **E18** | **H5** |
| M175 | **E19** | **H5** | M176 | **E20** | **H5** | M177 | **E21** | **H5** |
| M178 | **E22** | **H5** | M179 | **E23** | **H5** | M180 | **E24** | **H5** |
| M181 | **E25** | **H5** | M182 | **E26** | **H5** | M183 | **E27** | **H5** |
| M184 | **E28** | **H5** | M185 | **E29** | **H5** | M186 | **E30** | **H5** |
| M187 | **E31** | **H5** | M188 | **E32** | **H5** | M189 | **E33** | **H5** |
| M190 | **E34** | **H5** | M191 | **E35** | **H5** | M192 | **E36** | **H5** |
| M193 | **E37** | **H5** | M194 | **E38** | **H5** | M195 | **E39** | **H5** |
| M196 | **E1** | **H6** | M197 | **E2** | **H6** | M198 | **E3** | **H6** |
| M199 | **E4** | **H6** | M200 | **E5** | **H6** | M201 | **E6** | **H6** |
| M202 | **E7** | **H6** | M203 | **E8** | **H6** | M204 | **E9** | **H6** |
| M205 | **E10** | **H6** | M206 | **E11** | **H6** | M207 | **E12** | **H6** |
| M208 | **E13** | **H6** | M209 | **E14** | **H6** | M210 | **E15** | **H6** |
| M211 | **E16** | **H6** | M212 | **E17** | **H6** | M213 | **E18** | **H6** |
| M214 | **E19** | **H6** | M215 | **E20** | **H6** | M216 | **E21** | **H6** |
| M217 | **E22** | **H6** | M218 | **E23** | **H6** | M219 | **E24** | **H6** |
| M220 | **E25** | **H6** | M221 | **E26** | **H6** | M222 | **E27** | **H6** |
| M223 | **E28** | **H6** | M224 | **E29** | **H6** | M225 | **E30** | **H6** |
| M226 | **E31** | **H6** | M227 | **E32** | **H6** | M228 | **E33** | **H6** |
| M229 | **E34** | **H6** | M230 | **E35** | **H6** | M231 | **E36** | **H6** |

(fortgesetzt)

| | | | | | | | | |
|---|---|---|---|---|---|---|---|---|
| M232 | **E37** | **H6** | M233 | **E38** | **H6** | M234 | **E39** | **H6** |
| M235 | **E1** | **H7** | M236 | **E2** | **H7** | M237 | **E3** | **H7** |
| M238 | **E4** | **H7** | M239 | **E5** | **H7** | M240 | **E6** | **H7** |
| M241 | **E7** | **H7** | M242 | **E8** | **H7** | M243 | **E9** | **H7** |
| M244 | **E10** | **H7** | M245 | **E11** | **H7** | M246 | **E12** | **H7** |
| M247 | **E13** | **H7** | M248 | **E14** | **H7** | M249 | **E15** | **H7** |
| M250 | **E16** | **H7** | M251 | **E17** | **H7** | M252 | **E18** | **H7** |
| M253 | **E19** | **H7** | M254 | **E20** | **H7** | M255 | **E21** | **H7** |
| M256 | **E22** | **H7** | M257 | **E23** | **H7** | M258 | **E24** | **H7** |
| M259 | **E25** | **H7** | M260 | **E26** | **H7** | M261 | **E27** | **H7** |
| M262 | **E28** | **H7** | M263 | **E29** | **H7** | M264 | **E30** | **H7** |
| M265 | **E31** | **H7** | M266 | **E32** | **H7** | M267 | **E33** | **H7** |
| M268 | **E34** | **H7** | M269 | **E35** | **H7** | M270 | **E36** | **H7** |
| M271 | **E37** | **H7** | M272 | **E38** | **H7** | M273 | **E39** | **H7** |
| M274 | **E1** | **H8** | M275 | **E2** | **H8** | M276 | **E3** | **H8** |
| M277 | **E4** | **H8** | M278 | **E5** | **H8** | M279 | **E6** | **H8** |
| M280 | **E7** | **H8** | M281 | **E8** | **H8** | M282 | **E9** | **H8** |
| M283 | **E10** | **H8** | M284 | **E11** | **H8** | M285 | **E12** | **H8** |
| M286 | **E13** | **H8** | M287 | **E14** | **H8** | M288 | **E15** | **H8** |
| M289 | **E16** | **H8** | M290 | **E17** | **H8** | M291 | **E18** | **H8** |
| M292 | **E19** | **H8** | M293 | **E20** | **H8** | M294 | **E21** | **H8** |
| M295 | **E22** | **H8** | M296 | **E23** | **H8** | M297 | **E24** | **H8** |
| M298 | **E25** | **H8** | M299 | **E26** | **H8** | M300 | **E27** | **H8** |
| M301 | **E28** | **H8** | M302 | **E29** | **H8** | M303 | **E30** | **H8** |
| M304 | **E31** | **H8** | M305 | **E32** | **H8** | M306 | **E33** | **H8** |
| M307 | **E34** | **H8** | M308 | **E35** | **H8** | M309 | **E36** | **H8** |
| M310 | **E37** | **H8** | M311 | **E38** | **H8** | M312 | **E39** | **H8** |
| M313 | **E1** | **H9** | M314 | **E2** | **H9** | M315 | **E3** | **H9** |
| M316 | **E4** | **H9** | M317 | **E5** | **H9** | M318 | **E6** | **H9** |
| M319 | **E7** | **H9** | M320 | **E8** | **H9** | M321 | **E9** | **H9** |
| M322 | **E10** | **H9** | M323 | **E11** | **H9** | M324 | **E12** | **H9** |
| M325 | **E13** | **H9** | M326 | **E14** | **H9** | M327 | **E15** | **H9** |
| M328 | **E16** | **H9** | M329 | **E17** | **H9** | M330 | **E18** | **H9** |
| M331 | **E19** | **H9** | M332 | **E20** | **H9** | M333 | **E21** | **H9** |
| M334 | **E22** | **H9** | M335 | **E23** | **H9** | M336 | **E24** | **H9** |
| M337 | **E25** | **H9** | M338 | **E26** | **H9** | M339 | **E27** | **H9** |
| M340 | **E28** | **H9** | M341 | **E29** | **H9** | M342 | **E30** | **H9** |
| M343 | **E31** | **H9** | M344 | **E32** | **H9** | M345 | **E33** | **H9** |
| M346 | **E34** | **H9** | M347 | **E35** | **H9** | M348 | **E36** | **H9** |

(fortgesetzt)

| | | | | | | | | |
|---|---|---|---|---|---|---|---|---|
| M349 | **E37** | **H9** | M350 | **E38** | **H9** | M351 | **E39** | **H9** |
| M352 | **E1** | **H10** | M353 | **E2** | **H10** | M354 | **E3** | **H10** |
| M355 | **E4** | **H10** | M356 | **E5** | **H10** | M357 | **E6** | **H10** |
| M358 | **E7** | **H10** | M359 | **E8** | **H10** | M360 | **E9** | **H10** |
| M361 | **E10** | **H10** | M362 | **E11** | **H10** | M363 | **E12** | **H10** |
| M364 | **E13** | **H10** | M365 | **E14** | **H10** | M366 | **E15** | **H10** |
| M367 | **E16** | **H10** | M368 | **E17** | **H10** | M369 | **E18** | **H10** |
| M370 | **E19** | **H10** | M371 | **E20** | **H10** | M372 | **E21** | **H10** |
| M373 | **E22** | **H10** | M374 | **E23** | **H10** | M375 | **E24** | **H10** |
| M376 | **E25** | **H10** | M377 | **E26** | **H10** | M378 | **E27** | **H10** |
| M379 | **E28** | **H10** | M380 | **E29** | **H10** | M381 | **E30** | **H10** |
| M382 | **E31** | **H10** | M383 | **E32** | **H10** | M384 | **E33** | **H10** |
| M385 | **E34** | **H10** | M386 | **E35** | **H10** | M387 | **E36** | **H10** |
| M388 | **E37** | **H10** | M389 | **E38** | **H10** | M390 | **E39** | **H10** |
| M391 | **E1** | **H11** | M392 | **E2** | **H11** | M393 | **E3** | **H11** |
| M394 | **E4** | **H11** | M395 | **E5** | **H11** | M396 | **E6** | **H11** |
| M397 | **E7** | **H11** | M398 | **E8** | **H11** | M399 | **E9** | **H11** |
| M400 | **E10** | **H11** | M401 | **E11** | **H11** | M402 | **E12** | **H11** |
| M403 | **E13** | **H11** | M404 | **E14** | **H11** | M405 | **E15** | **H11** |
| M406 | **E16** | **H11** | M407 | **E17** | **H11** | M408 | **E18** | **H11** |
| M409 | **E19** | **H11** | M410 | **E20** | **H11** | M411 | **E21** | **H11** |
| M412 | **E22** | **H11** | M413 | **E23** | **H11** | M414 | **E24** | **H11** |
| M415 | **E25** | **H11** | M416 | **E26** | **H11** | M417 | **E27** | **H11** |
| M418 | **E28** | **H11** | M419 | **E29** | **H11** | M420 | **E30** | **H11** |
| M421 | **E31** | **H11** | M422 | **E32** | **H11** | M423 | **E33** | **H11** |
| M424 | **E34** | **H11** | M425 | **E35** | **H11** | M426 | **E36** | **H11** |
| M427 | **E37** | **H11** | M428 | **E38** | **H11** | M429 | **E39** | **H11** |
| M430 | **E1** | **H12** | M431 | **E2** | **H12** | M432 | **E3** | **H12** |
| M433 | **E4** | **H12** | M434 | **E5** | **H12** | M435 | **E6** | **H12** |
| M436 | **E7** | **H12** | M437 | **E8** | **H12** | M438 | **E9** | **H12** |
| M439 | **E10** | **H12** | M440 | **E11** | **H12** | M441 | **E12** | **H12** |
| M442 | **E13** | **H12** | M443 | **E14** | **H12** | M444 | **E15** | **H12** |
| M445 | **E16** | **H12** | M446 | **E17** | **H12** | M447 | **E18** | **H12** |
| M448 | **E19** | **H12** | M449 | **E20** | **H12** | M450 | **E21** | **H12** |
| M451 | **E22** | **H12** | M452 | **E23** | **H12** | M453 | **E24** | **H12** |
| M454 | **E25** | **H12** | M455 | **E26** | **H12** | M456 | **E27** | **H12** |
| M457 | **E28** | **H12** | M458 | **E29** | **H12** | M459 | **E30** | **H12** |
| M460 | **E31** | **H12** | M461 | **E32** | **H12** | M462 | **E33** | **H12** |
| M463 | **E34** | **H12** | M464 | **E35** | **H12** | M465 | **E36** | **H12** |

(fortgesetzt)

| | | | | | | | | |
|---|---|---|---|---|---|---|---|---|
| M466 | **E37** | **H12** | M467 | **E38** | **H12** | M468 | **E39** | **H12** |
| M469 | **E1** | **H13** | M470 | **E2** | **H13** | M471 | **E3** | **H13** |
| M472 | **E4** | **H13** | M473 | **E5** | **H13** | M474 | **E6** | **H13** |
| M475 | **E7** | **H13** | M476 | **E8** | **H13** | M477 | **E9** | **H13** |
| M478 | **E10** | **H13** | M479 | **E11** | **H13** | M480 | **E12** | **H13** |
| M481 | **E13** | **H13** | M482 | **E14** | **H13** | M483 | **E15** | **H13** |
| M484 | **E16** | **H13** | M485 | **E17** | **H13** | M486 | **E18** | **H13** |
| M487 | **E19** | **H13** | M488 | **E20** | **H13** | M489 | **E21** | **H13** |
| M490 | **E22** | **H13** | M491 | **E23** | **H13** | M492 | **E24** | **H13** |
| M493 | **E25** | **H13** | M494 | **E26** | **H13** | M495 | **E27** | **H13** |
| M496 | **E28** | **H13** | M497 | **E29** | **H13** | M498 | **E30** | **H13** |
| M499 | **E31** | **H13** | M500 | **E32** | **H13** | M501 | **E33** | **H13** |
| M502 | **E34** | **H13** | M503 | **E35** | **H13** | M504 | **E36** | **H13** |
| M505 | **E37** | **H13** | M506 | **E38** | **H13** | M507 | **E39** | **H13** |
| M508 | **E1** | **H14** | M509 | **E2** | **H14** | M510 | **E3** | **H14** |
| M511 | **E4** | **H14** | M512 | **E5** | **H14** | M513 | **E6** | **H14** |
| M514 | **E7** | **H14** | M515 | **E8** | **H14** | M516 | **E9** | **H14** |
| M517 | **E10** | **H14** | M518 | **E11** | **H14** | M519 | **E12** | **H14** |
| M520 | **E13** | **H14** | M521 | **E14** | **H14** | M522 | **E15** | **H14** |
| M523 | **E16** | **H14** | M524 | **E17** | **H14** | M525 | **E18** | **H14** |
| M526 | **E19** | **H14** | M527 | **E20** | **H14** | M528 | **E21** | **H14** |
| M529 | **E22** | **H14** | M530 | **E23** | **H14** | M531 | **E24** | **H14** |
| M532 | **E25** | **H14** | M533 | **E26** | **H14** | M534 | **E27** | **H14** |
| M535 | **E28** | **H14** | M536 | **E29** | **H14** | M537 | **E30** | **H14** |
| M538 | **E31** | **H14** | M539 | **E32** | **H14** | M540 | **E33** | **H14** |
| M541 | **E34** | **H14** | M542 | **E35** | **H14** | M543 | **E36** | **H14** |
| M544 | **E37** | **H14** | M545 | **E38** | **H14** | M546 | **E39** | **H14** |
| M547 | **E1** | **H15** | M548 | **E2** | **H15** | M549 | **E3** | **H15** |
| M550 | **E4** | **H15** | M551 | **E5** | **H15** | M552 | **E6** | **H15** |
| M553 | **E7** | **H15** | M554 | **E8** | **H15** | M555 | **E9** | **H15** |
| M556 | **E10** | **H15** | M557 | **E11** | **H15** | M558 | **E12** | **H15** |
| M559 | **E13** | **H15** | M560 | **E14** | **H15** | M561 | **E15** | **H15** |
| M562 | **E16** | **H15** | M563 | **E17** | **H15** | M564 | **E18** | **H15** |
| M565 | **E19** | **H15** | M 566 | **E20** | **H15** | M567 | **E21** | **H15** |
| M568 | **E22** | **H15** | M569 | **E23** | **H15** | M570 | **E24** | **H15** |
| M571 | **E25** | **H15** | M572 | **E26** | **H15** | M573 | **E27** | **H15** |
| M574 | **E28** | **H15** | M575 | **E29** | **H15** | M576 | **E30** | **H15** |
| M577 | **E31** | **H15** | M578 | **E32** | **H15** | M579 | **E33** | **H15** |
| M580 | **E34** | **H15** | M581 | **E35** | **H15** | M582 | **E36** | **H15** |

(fortgesetzt)

| | | | | | | | | |
|---|---|---|---|---|---|---|---|---|
| M583 | **E37** | **H15** | M584 | **E38** | **H15** | M585 | **E39** | **H15** |
| M586 | **E1** | **H16** | M587 | **E2** | **H16** | M588 | **E3** | **H16** |
| M589 | **E4** | **H16** | M590 | **E5** | **H16** | M591 | **E6** | **H16** |
| M592 | **E7** | **H16** | M593 | **E8** | **H16** | M594 | **E9** | **H16** |
| M595 | **E10** | **H16** | M596 | **E11** | **H16** | M597 | **E12** | **H16** |
| M598 | **E13** | **H16** | M599 | **E14** | **H16** | M600 | **E15** | **H16** |
| M601 | **E16** | **H16** | M602 | **E17** | **H16** | M603 | **E18** | **H16** |
| M604 | **E19** | **H16** | M605 | **E20** | **H16** | M606 | **E21** | **H16** |
| M607 | **E22** | **H16** | M608 | **E23** | **H16** | M609 | **E24** | **H16** |
| M610 | **E25** | **H16** | M611 | **E26** | **H16** | M612 | **E27** | **H16** |
| M613 | **E28** | **H16** | M614 | **E29** | **H16** | M615 | **E30** | **H16** |
| M616 | **E31** | **H16** | M617 | **E32** | **H16** | M618 | **E33** | **H16** |
| M619 | **E34** | **H16** | M620 | **E35** | **H16** | M621 | **E36** | **H16** |
| M622 | **E37** | **H16** | M623 | **E38** | **H16** | M624 | **E39** | **H16** |
| M625 | **E1** | **H17** | M626 | **E2** | **H17** | M627 | **E3** | **H17** |
| M628 | **E4** | **H17** | M629 | **E5** | **H17** | M630 | **E6** | **H17** |
| M631 | **E7** | **H17** | M632 | **E8** | **H17** | M633 | **E9** | **H17** |
| M634 | **E10** | **H17** | M635 | **E11** | **H17** | M636 | **E12** | **H17** |
| M637 | **E13** | **H17** | M638 | **E14** | **H17** | M639 | **E15** | **H17** |
| M640 | **E16** | **H17** | M641 | **E17** | **H17** | M642 | **E18** | **H17** |
| M643 | **E19** | **H17** | M644 | **E20** | **H17** | M645 | **E21** | **H17** |
| M646 | **E22** | **H17** | M647 | **E23** | **H17** | M648 | **E24** | **H17** |
| M649 | **E25** | **H17** | M650 | **E26** | **H17** | M651 | **E27** | **H17** |
| M652 | **E28** | **H17** | M653 | **E29** | **H17** | M654 | **E30** | **H17** |
| M655 | **E31** | **H17** | M656 | **E32** | **H17** | M657 | **E33** | **H17** |
| M658 | **E34** | **H17** | M659 | **E35** | **H17** | M660 | **E36** | **H17** |
| M661 | **E37** | **H17** | M662 | **E38** | **H17** | M663 | **E39** | **H17** |
| M664 | **E1** | **H18** | M665 | **E2** | **H18** | M666 | **E3** | **H18** |
| M667 | **E4** | **H18** | M668 | **E5** | **H18** | M669 | **E6** | **H18** |
| M670 | **E7** | **H18** | M671 | **E8** | **H18** | M672 | **E9** | **H18** |
| M673 | **E10** | **H18** | M674 | **E11** | **H18** | M675 | **E12** | **H18** |
| M676 | **E13** | **H18** | M677 | **E14** | **H18** | M678 | **E15** | **H18** |
| M679 | **E16** | **H18** | M680 | **E17** | **H18** | M681 | **E18** | **H18** |
| M682 | **E19** | **H18** | M683 | **E20** | **H18** | M684 | **E21** | **H18** |
| M685 | **E22** | **H18** | M686 | **E23** | **H18** | M687 | **E24** | **H18** |
| M688 | **E25** | **H18** | M689 | **E26** | **H18** | M690 | **E27** | **H18** |
| M691 | **E28** | **H18** | M692 | **E29** | **H18** | M693 | **E30** | **H18** |
| M694 | **E31** | **H18** | M695 | **E32** | **H18** | M696 | **E33** | **H18** |
| M697 | **E34** | **H18** | M698 | **E35** | **H18** | M699 | **E36** | **H18** |

(fortgesetzt)

| | | | | | | | | |
|---|---|---|---|---|---|---|---|---|
| M700 | **E37** | **H18** | M701 | **E38** | **H18** | M702 | **E39** | **H18** |
| M703 | **E1** | **H19** | M704 | **E2** | **H19** | M705 | **E3** | **H19** |
| M706 | **E4** | **H19** | M707 | **E5** | **H19** | M708 | **E6** | **H19** |
| M709 | **E7** | **H19** | M710 | **E8** | **H19** | M711 | **E9** | **H19** |
| M712 | **E10** | **H19** | M713 | **E11** | **H19** | M714 | **E12** | **H19** |
| M715 | **E13** | **H19** | M716 | **E14** | **H19** | M717 | **E15** | **H19** |
| M718 | **E16** | **H19** | M719 | **E17** | **H19** | M720 | **E18** | **H19** |
| M721 | **E19** | **H19** | M722 | **E20** | **H19** | M723 | **E21** | **H19** |
| M724 | **E22** | **H19** | M725 | **E23** | **H19** | M726 | **E24** | **H19** |
| M727 | **E25** | **H19** | M728 | **E26** | **H19** | M729 | **E27** | **H19** |
| M730 | **E28** | **H19** | M731 | **E29** | **H19** | M732 | **E30** | **H19** |
| M733 | **E31** | **H19** | M734 | **E32** | **H19** | M735 | **E33** | **H19** |
| M736 | **E34** | **H19** | M737 | **E35** | **H19** | M738 | **E36** | **H19** |
| M739 | **E37** | **H19** | M740 | **E38** | **H19** | M741 | **E39** | **H19** |
| M742 | **E1** | **H20** | M743 | **E2** | **H20** | M744 | **E3** | **H20** |
| M745 | **E4** | **H20** | M746 | **E5** | **H20** | M747 | **E6** | **H20** |
| M748 | **E7** | **H20** | M749 | **E8** | **H20** | M750 | **E9** | **H20** |
| M751 | **E10** | **H20** | M752 | **E11** | **H20** | M753 | **E12** | **H20** |
| M754 | **E13** | **H20** | M755 | **E14** | **H20** | M756 | **E15** | **H20** |
| M757 | **E16** | **H20** | M758 | **E17** | **H20** | M759 | **E18** | **H20** |
| M760 | **E19** | **H20** | M761 | **E20** | **H20** | M762 | **E21** | **H20** |
| M763 | **E22** | **H20** | M764 | **E23** | **H20** | M765 | **E24** | **H20** |
| M766 | **E25** | **H20** | M767 | **E26** | **H20** | M768 | **E27** | **H20** |
| M769 | **E28** | **H20** | M770 | **E29** | **H20** | M771 | **E30** | **H20** |
| M772 | **E31** | **H20** | M773 | **E32** | **H20** | M774 | **E33** | **H20** |
| M775 | **E34** | **H20** | M776 | **E35** | **H20** | M777 | **E36** | **H20** |
| M778 | **E37** | **H20** | M779 | **E38** | **H20** | M780 | **E39** | **H20** |
| M781 | **E1** | **H21** | M782 | **E2** | **H21** | M783 | **E3** | **H21** |
| M784 | **E4** | **H21** | M785 | **E5** | **H21** | M786 | **E6** | **H21** |
| M787 | **E7** | **H21** | M788 | **E8** | **H21** | M789 | **E9** | **H21** |
| M790 | **E10** | **H21** | M791 | **E11** | **H21** | M792 | **E12** | **H21** |
| M793 | **E13** | **H21** | M794 | **E14** | **H21** | M795 | **E15** | **H21** |
| M796 | **E16** | **H21** | M797 | **E17** | **H21** | M798 | **E18** | **H21** |
| M799 | **E19** | **H21** | M800 | **E20** | **H21** | M801 | **E21** | **H21** |
| M802 | **E22** | **H21** | M803 | **E23** | **H21** | M804 | **E24** | **H21** |
| M805 | **E25** | **H21** | M806 | **E26** | **H21** | M807 | **E27** | **H21** |
| M808 | **E28** | **H21** | M809 | **E29** | **H21** | M810 | **E30** | **H21** |
| M811 | **E31** | **H21** | M812 | **E32** | **H21** | M813 | **E33** | **H21** |
| M814 | **E34** | **H21** | M815 | **E35** | **H21** | M816 | **E36** | **H21** |

(fortgesetzt)

| | | | | | | | | |
|---|---|---|---|---|---|---|---|---|
| M817 | **E37** | **H21** | M818 | **E38** | **H21** | M819 | **E39** | **H21** |
| M820 | **E40** | **H1** | M821 | **E40** | **H2** | M822 | **E40** | **H3** |
| M823 | **E40** | **H4** | M824 | **E40** | **H5** | M825 | **E40** | **H6** |
| M826 | **E40** | **H7** | M827 | **E40** | **H8** | M828 | **E40** | **H9** |
| M829 | **E40** | **H10** | M830 | **E40** | **H11** | M831 | **E40** | **H12** |
| M832 | **E40** | **H13** | M833 | **E40** | **H14** | M834 | **E40** | **H15** |
| M835 | **E40** | **H16** | M836 | **E40** | **H17** | M837 | **E40** | **H18** |
| M838 | **E40** | **H19** | M839 | **E40** | **H20** | M840 | **E40** | **H21** |
| M841 | **E41** | **H1** | M842 | **E41** | **H2** | M843 | **E41** | **H3** |
| M844 | **E41** | **H4** | M845 | **E41** | **H5** | M846 | **E41** | **H6** |
| M847 | **E41** | **H7** | M848 | **E41** | **H8** | M849 | **E41** | **H9** |
| M850 | **E41** | **H10** | M851 | **E41** | **H11** | M852 | **E41** | **H12** |
| M853 | **E41** | **H13** | M854 | **E41** | **H14** | M855 | **E41** | **H15** |
| M856 | **E41** | **H16** | M857 | **E41** | **H17** | M858 | **E41** | **H18** |
| M859 | **E41** | **H19** | M860 | **E41** | **H20** | M861 | **E41** | **H21** |
| M862 | **E42** | **H1** | M863 | **E42** | **H2** | M864 | **E42** | **H3** |
| M865 | **E42** | **H4** | M866 | **E42** | **H5** | M867 | **E42** | **H6** |
| M868 | **E42** | **H7** | M869 | **E42** | **H8** | M870 | **E42** | **H9** |
| M871 | **E42** | **H10** | M872 | **E42** | **H11** | M873 | **E42** | **H12** |
| M874 | **E42** | **H13** | M875 | **E42** | **H14** | M876 | **E42** | **H15** |
| M877 | **E42** | **H16** | M878 | **E42** | **H17** | M879 | **E42** | **H18** |
| M880 | **E42** | **H19** | M881 | **E42** | **H20** | M882 | **E42** | **H21** |
| M883 | **E43** | **H1** | M884 | **E43** | **H2** | M885 | **E43** | **H3** |
| M886 | **E43** | **H4** | M887 | **E43** | **H5** | M888 | **E43** | **H6** |
| M889 | **E43** | **H7** | M890 | **E43** | **H8** | M891 | **E43** | **H9** |
| M892 | **E43** | **H10** | M893 | **E43** | **H11** | M894 | **E43** | **H12** |
| M895 | **E43** | **H13** | M896 | **E43** | **H14** | M897 | **E43** | **H15** |
| M898 | **E43** | **H16** | M899 | **E43** | **H17** | M900 | **E43** | **H18** |
| M901 | **E43** | **H19** | M902 | **E43** | **H20** | M903 | **E43** | **H21** |
| M904 | **E44** | **H1** | M905 | **E44** | **H2** | M906 | **E44** | **H3** |
| M907 | **E44** | **H4** | M908 | **E44** | **H5** | M909 | **E44** | **H6** |
| M910 | **E44** | **H7** | M911 | **E44** | **H8** | M912 | **E44** | **H9** |
| M913 | **E44** | **H10** | M914 | **E44** | **H11** | M915 | **E44** | **H12** |
| M916 | **E44** | **H13** | M917 | **E44** | **H14** | M918 | **E44** | **H15** |
| M919 | **E44** | **H16** | M920 | **E44** | **H17** | M921 | **E44** | **H18** |
| M922 | **E44** | **H19** | M923 | **E44** | **H20** | M924 | **E44** | **H21** |
| M925 | **E45** | **H1** | M926 | **E45** | **H2** | M927 | **E45** | **H3** |
| M928 | **E45** | **H4** | M929 | **E45** | **H5** | M930 | **E45** | **H6** |
| M931 | **E45** | **H7** | M932 | **E45** | **H8** | M933 | **E45** | **H9** |

(fortgesetzt)

| | | | | | | | | |
|---|---|---|---|---|---|---|---|---|
| M934 | **E45** | **H10** | M935 | **E45** | **H11** | M936 | **E45** | **H12** |
| M937 | **E45** | **H13** | M938 | **E45** | **H14** | M939 | **E45** | **H15** |
| M940 | **E45** | **H16** | M941 | **E45** | **H17** | M942 | **E45** | **H18** |
| M943 | **E45** | **H19** | M944 | **E45** | **H20** | M945 | **E45** | **H21** |
| M946 | **E46** | **H1** | M947 | **E46** | **H2** | M948 | **E46** | **H3** |
| M949 | **E46** | **H4** | M950 | **E46** | **H5** | M951 | **E46** | **H6** |
| M952 | **E46** | **H7** | M953 | **E46** | **H8** | M954 | **E46** | **H9** |
| M955 | **E46** | **H10** | M956 | **E46** | **H11** | M957 | **E46** | **H12** |
| M958 | **E46** | **H13** | M959 | **E46** | **H14** | M960 | **E46** | **H15** |
| M961 | **E46** | **H16** | M962 | **E46** | **H17** | M963 | **E46** | **H18** |
| M964 | **E46** | **H19** | M965 | **E46** | **H20** | M966 | **E46** | **H21** |
| M967 | **E47** | **H1** | M968 | **E47** | **H2** | M969 | **E47** | **H3** |
| M970 | **E47** | **H4** | M971 | **E47** | **H5** | M972 | **E47** | **H6** |
| M973 | **E47** | **H7** | M974 | **E47** | **H8** | M975 | **E47** | **H9** |
| M976 | **E47** | **H10** | M977 | **E47** | **H11** | M978 | **E47** | **H12** |
| M979 | **E47** | **H13** | M980 | **E47** | **H14** | M981 | **E47** | **H15** |
| M982 | **E47** | **H16** | M983 | **E47** | **H17** | M984 | **E47** | **H18** |
| M985 | **E47** | **H19** | M986 | **E47** | **H20** | M987 | **E47** | **H21** |
| M988 | **E48** | **H1** | M989 | **E48** | **H2** | M990 | **E48** | **H3** |
| M991 | **E48** | **H4** | M992 | **E48** | **H5** | M993 | **E48** | **H6** |
| M994 | **E48** | **H7** | M995 | **E48** | **H8** | M996 | **E48** | **H9** |
| M997 | **E48** | **H10** | M998 | **E48** | **H11** | M999 | **E48** | **H12** |
| M1000 | **E48** | **H13** | M1001 | **E48** | **H14** | M1002 | **E48** | **H15** |
| M1003 | **E48** | **H16** | M1004 | **E48** | **H17** | M1005 | **E48** | **H18** |
| M1006 | **E48** | **H19** | M1007 | **E48** | **H20** | M1008 | **E48** | **H21.** |

Die Konzentration des elektronentransportierenden Hostmaterials der Formel (1), wie zuvor beschrieben oder bevorzugt beschrieben, in der erfindungsgemäßen Mischung oder in der lichtemittierenden Schicht der erfindungsgemäßen Vorrichtung liegt im Bereich von 5 Gew.-% bis 90 Gew.-%, bevorzugt im Bereich von 10 Gew.-% bis 85 Gew.-%, mehr bevorzugt im Bereich von 20 Gew.-% bis 85 Gew.-%, noch mehr bevorzugt im Bereich von 30 Gew.-% bis 80 Gew.-%, ganz besonders bevorzugt im Bereich von 20 Gew.-% bis 60 Gew.-% und am meisten bevorzugt im Bereich von 30 Gew.-% bis 50 Gew.-%, bezogen auf die gesamte Mischung oder bezogen auf die gesamte Zusammensetzung der lichtemittierenden Schicht.

Die Konzentration des lochtransportierenden Hostmaterials der Formel (2), wie zuvor beschrieben oder als bevorzugt beschrieben, in der erfindungsgemäßen Mischung oder in der lichtemittierenden Schicht der erfindungsgemäßen Vorrichtung liegt im Bereich von 10 Gew.-% bis 95 Gew.-%, bevorzugt im Bereich von 15 Gew.-% bis 90 Gew.-%, mehr bevorzugt im Bereich von 15 Gew.-% bis 80 Gew.-%, noch mehr bevorzugt im Bereich von 20 Gew.-% bis 70 Gew.-%, ganz besonders bevorzugt im Bereich von 40 Gew.-% bis 80 Gew.-% und am meisten bevorzugt im Bereich von 50 Gew.-% bis 70 Gew.-%, bezogen auf die gesamte Mischung oder bezogen auf die gesamte Zusammensetzung der lichtemittierenden Schicht.

Die vorliegende Erfindung betrifft auch eine Mischung, die neben den vorstehend genannten Hostmaterialien 1 und 2, wie zuvor beschrieben oder bevorzugt beschrieben, insbesondere Mischungen M1 bis M1008, mindestens noch einen phosphoreszierenden Emitter enthält.

Die vorliegende Erfindung betrifft auch eine organische elektrolumineszierende Vorrichtung, wie zuvor beschrieben oder bevorzugt beschrieben, wobei die lichtemittierende Schicht neben den vorstehend genannten Hostmaterialien 1 und 2, wie zuvor beschrieben oder bevorzugt beschrieben, insbesondere den Materialkombinationen M1 bis M1008, mindestens noch einen phosphoreszierenden Emitter enthält.

Vom Begriff phosphoreszierende Emitter sind typischerweise Verbindungen umfasst, bei denen die Lichtemission durch einen spin-verbotenen Übergang aus einem angeregten Zustand mit höherer Spinmultiplizität, also einem Spinzustand > 1, erfolgt, beispielsweise durch einen Übergang aus einem Triplett-Zustand oder einem Zustand mit einer noch höheren Spinquantenzahl, beispielsweise einem Quintett-Zustand. Bevorzugt wird hierbei ein Übergang aus einem Triplett-Zustand verstanden.

Als phosphoreszierende Emitter (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Emitter angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind.

Beispiele der oben beschriebenen Emitter können den Anmeldungen WO 2016/015815, WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2015/036074, WO 2015/117718 und WO 2016/015815 entnommen werden.

Bevorzugte phosphoreszierende Emitter gemäß der vorliegenden Erfindung entsprechen der Formel (III),

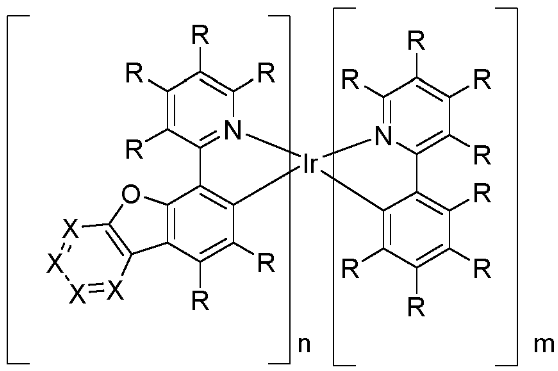

Formel (III)

,

wobei die Symbole und Indizes für diese Formel (III) die Bedeutung haben:

n+m ist 3, n ist 1 oder 2, m ist 2 oder 1,
X ist N oder CR,
R ist H, D oder eine verzweigte oder lineare Alkylgruppe oder eine teilweise oder vollständig deuterierte verzweigte oder lineare Alkylgruppe.

Ein weiterer Gegenstand der Erfindung ist demzufolge eine organische elektrolumineszierende Vorrichtung, wie zuvor beschrieben oder bevorzugt beschrieben, dadurch gekennzeichnet, dass die lichtemittierende Schicht neben den Hostmaterialien 1 und 2 mindestens einen phosphoreszierenden Emitter enthält, der der Formel (III) entspricht, wie zuvor beschrieben.

In Emittern der Formel (III) ist n bevorzugt 1 und m ist bevorzugt 2.

In Emittern der Formel (III) ist bevorzugt ein X ausgewählt aus N und die anderen X bedeuten CR.

In Emittern der Formel (III) ist mindestens ein R bevorzugt unterschiedlich von H. In Emittern der Formel (III) sind bevorzugt zwei R unterschiedlich von H und haben eine der sonst zuvor für die Emitter der Formel (III) angegebenen Bedeutungen.

Bevorzugte phosphoreszierende Emitter gemäß der vorliegenden Erfindung entsprechen den Formeln (Ia), (IIa) oder (IIIa),

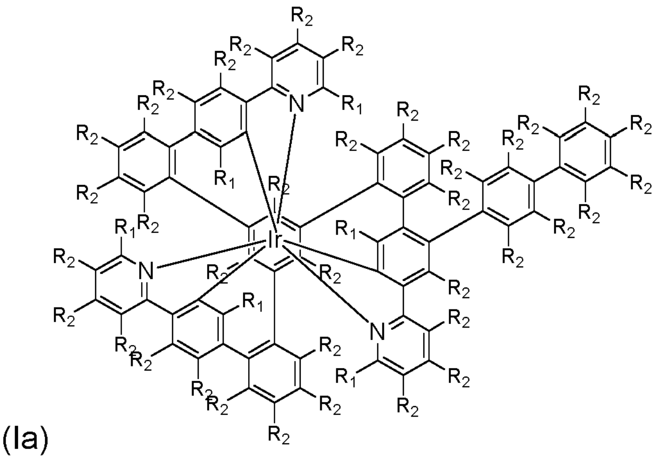

Formel (Ia) ,

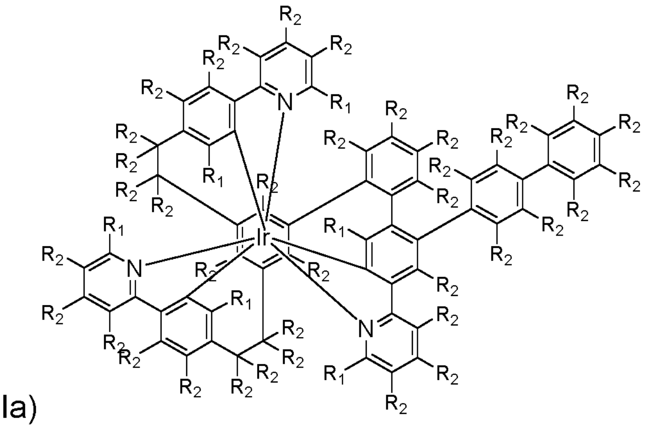

Formel (IIa) ,

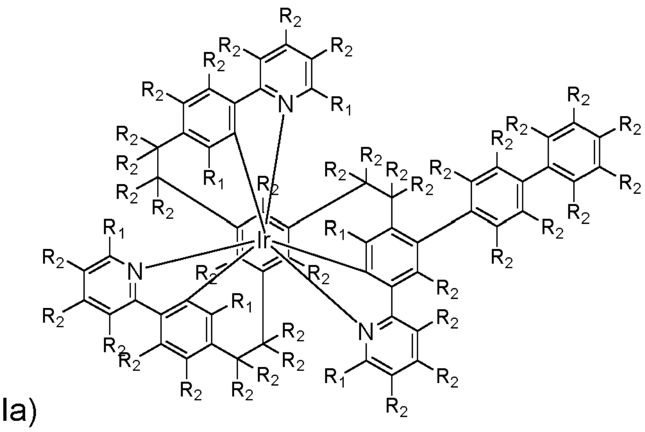

Formel (IIIa) ,

wobei die Symbole und Indizes für diese Formeln (Ia), (IIa) und (IIIa) die Bedeutung haben:

$R_1$ ist H oder D, $R_2$ ist H, D oder eine verzweigte oder lineare Alkylgruppe mit 1 bis 10 C-Atomen oder eine teilweise oder vollständig deuterierte verzweigte oder lineare Alkylgruppe mit 1 bis 10 C-Atomen oder eine Cycloalkylgruppe mit 4 bis 10 C-Atomen, die teilweise oder vollständig mit Deuterium substituiert sein kann.

Bevorzugte phosphoreszierende Emitter gemäß der vorliegenden Erfindung entsprechen den Formeln (IVa), (Va) oder (VIa),

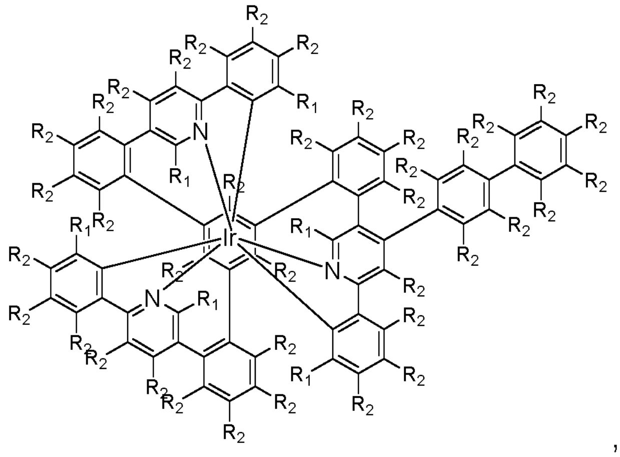

Formel (IVa) ,

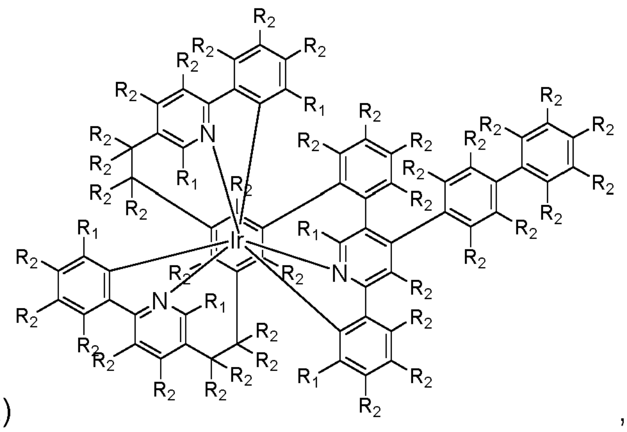

Formel (Va) ,

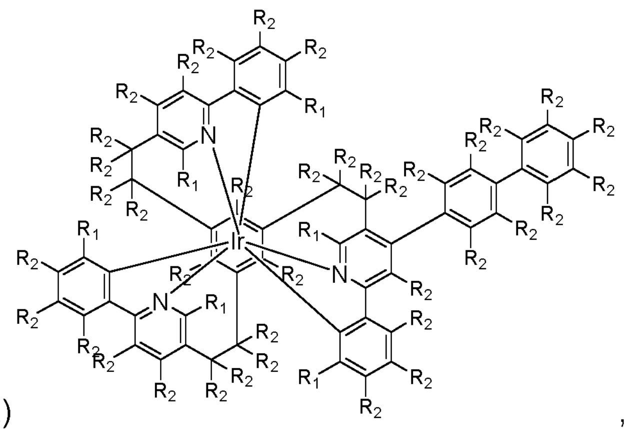

Formel (VIa) ,

wobei die Symbole und Indizes für diese Formeln (IVa), (Va) und (VIa) die Bedeutung haben:
$R_1$ ist H oder D, $R_2$ ist H, D, F oder eine verzweigte oder lineare Alkylgruppe mit 1 bis 10 C-Atomen oder eine teilweise oder vollständig deuterierte verzweigte oder lineare Alkylgruppe mit 1 bis 10 C-Atomen oder eine Cycloalkylgruppe mit 4 bis 10 C-Atomen, die teilweise oder vollständig mit Deuterium substituiert sein kann.

Bevorzugte Beispiele von phosphoreszierenden Emittern sind in der folgenden Tabelle 6 aufgeführt.

Tabelle 6:

| | | |
|---|---|---|
| 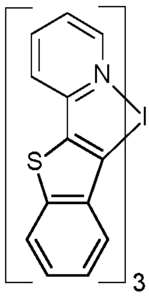 | 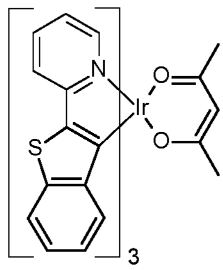 | 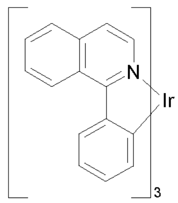 |
| 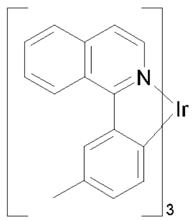 | 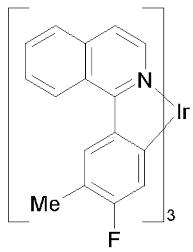 | 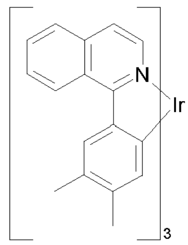 |
| 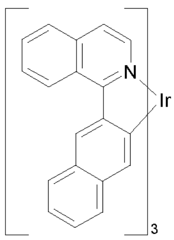 | 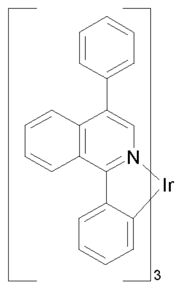 | 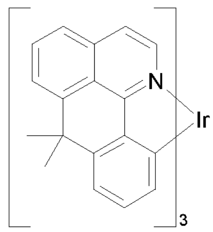 |
| 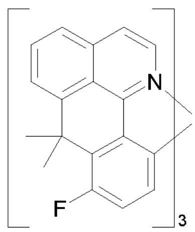 | 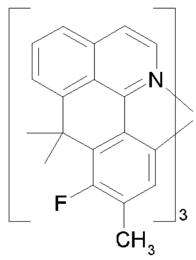 | 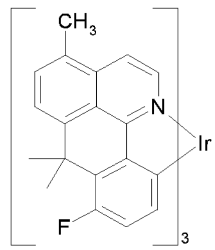 |
| 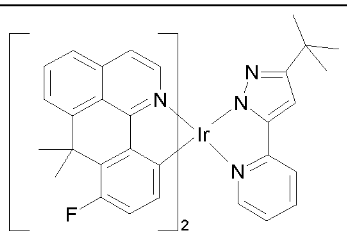 | 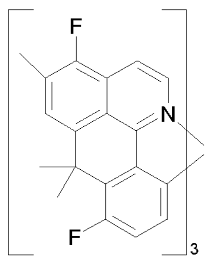 | 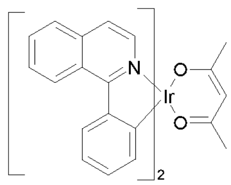 |

| | | |
|---|---|---|
| 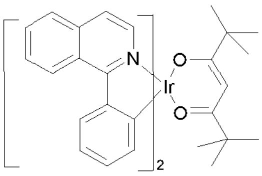 | 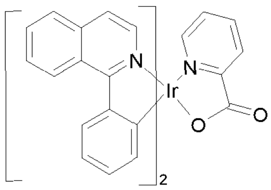 | 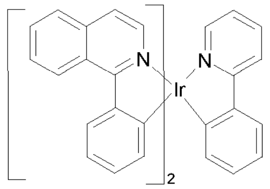 |
| 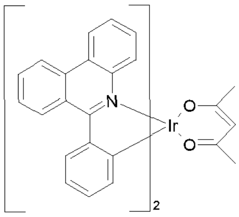 | 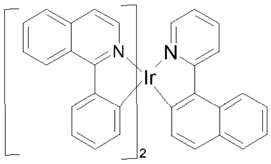 | 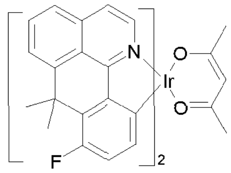 |
| 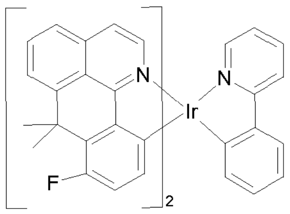 | 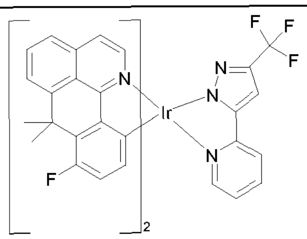 | 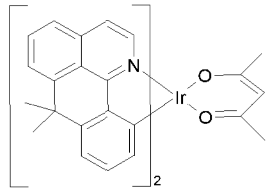 |
| 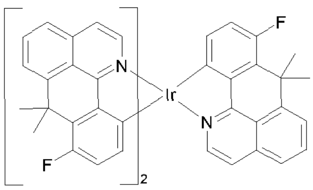 | 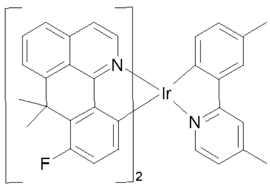 | 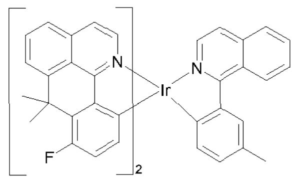 |
| 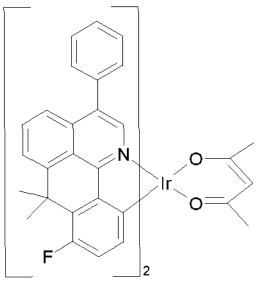 | 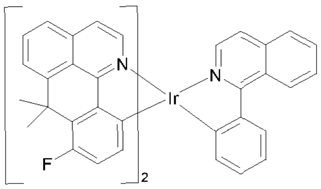 | 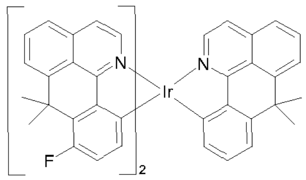 |
| 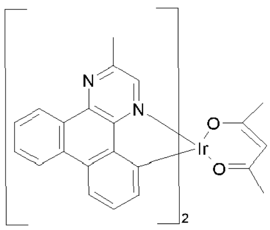 | 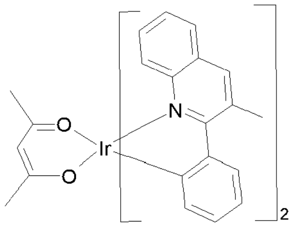 | 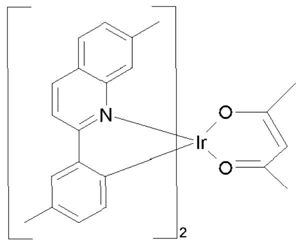 |

| | | |
|---|---|---|
| 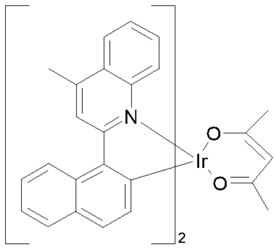 | 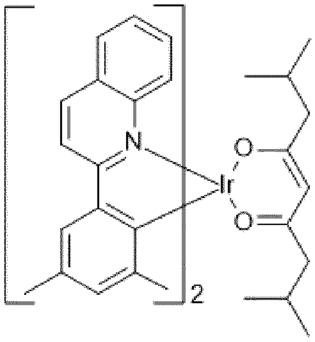 | 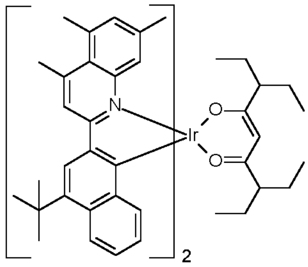 |
| 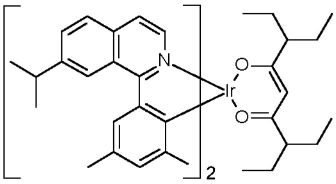 | 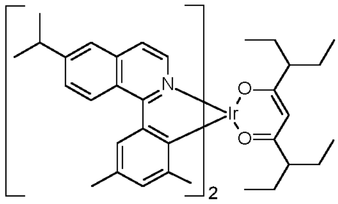 | 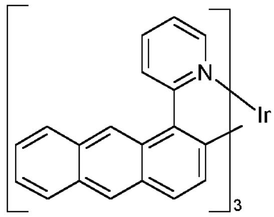 |
| 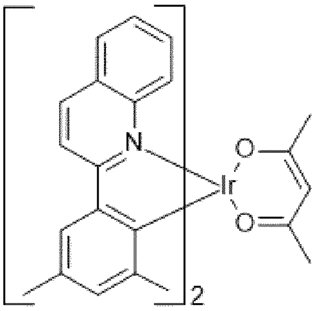 | 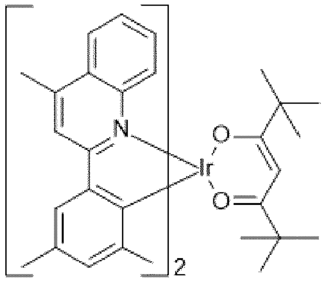 | 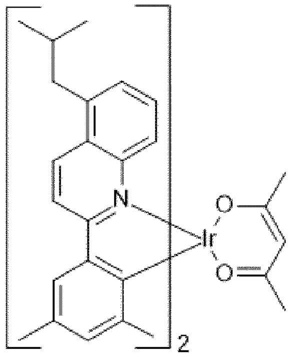 |
| 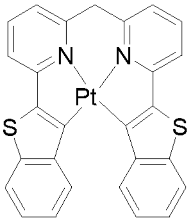 | 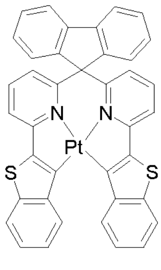 | 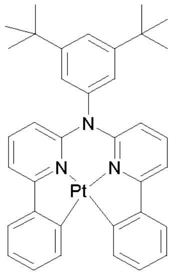 |
| 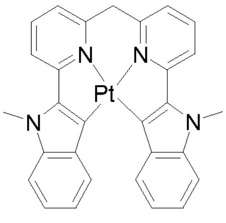 | 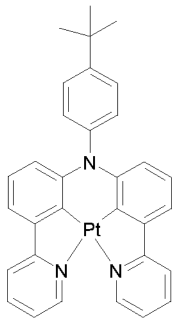 | 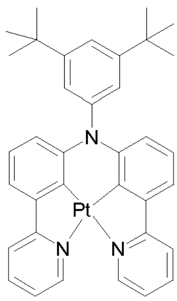 |

| | | |
|---|---|---|
| 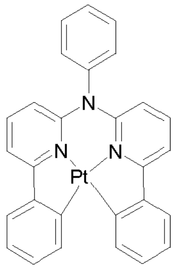 | 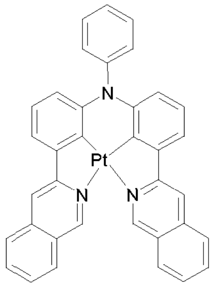 | 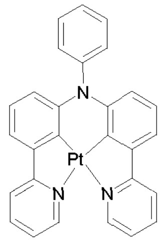 |
| 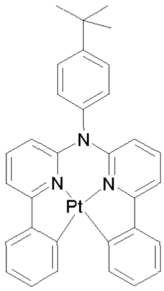 | 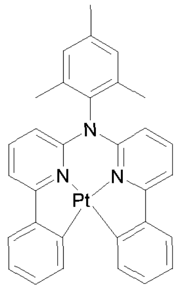 | 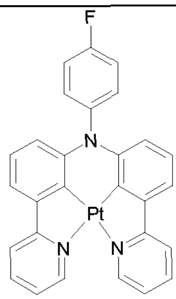 |
| 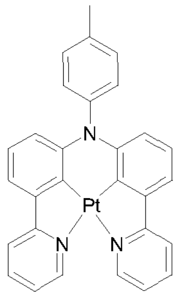 | 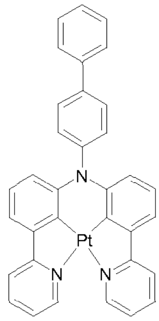 | 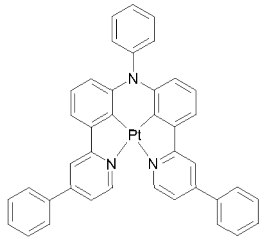 |
| 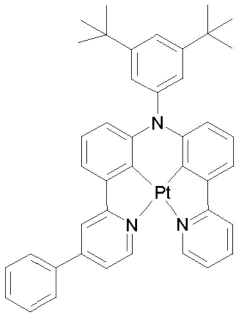 | 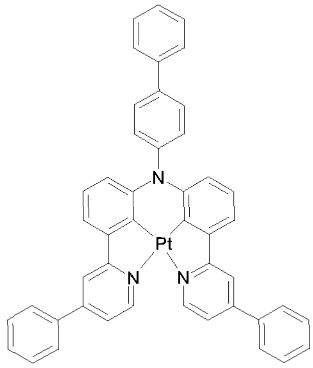 | 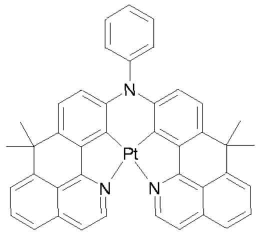 |
| 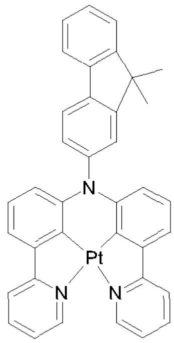 | 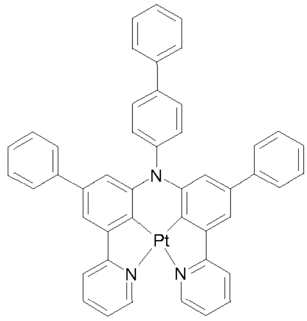 | 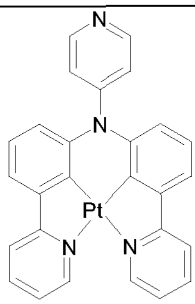 |

| | | |
|---|---|---|
| 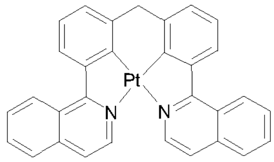 | 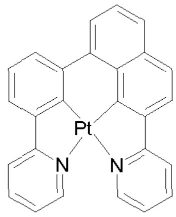 | 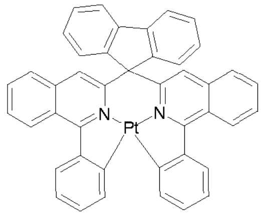 |
| 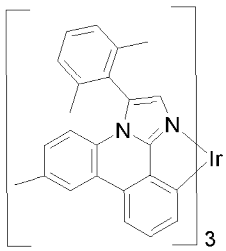 | 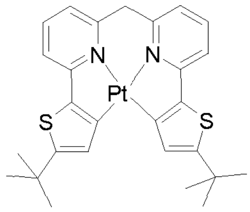 | 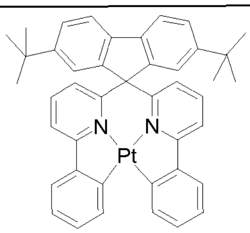 |
| 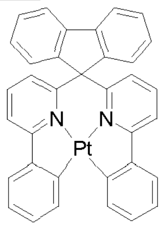 | 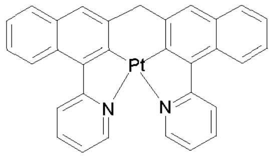 | 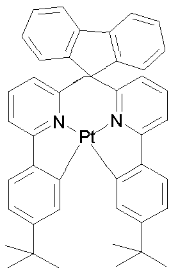 |
| 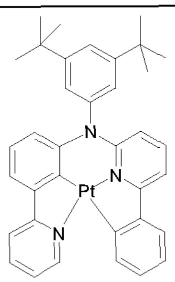 | 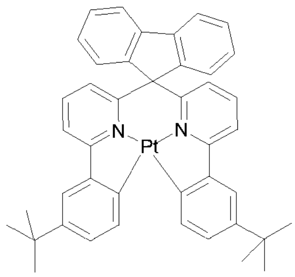 | 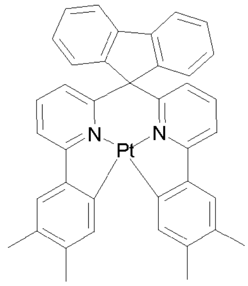 |
| 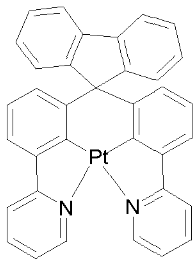 | 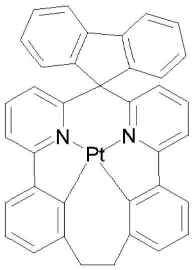 | 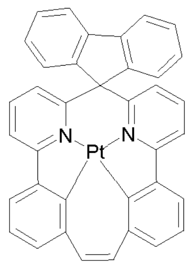 |
| 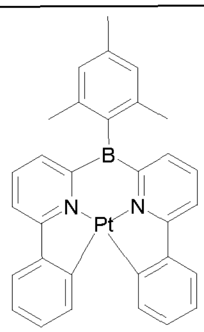 | 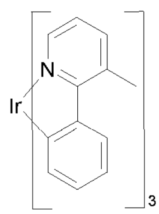 | 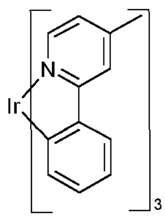 |

| | | |
|---|---|---|
| 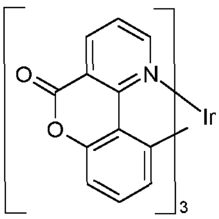 | 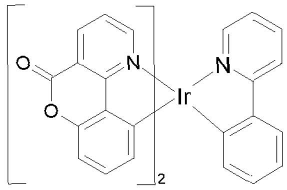 | 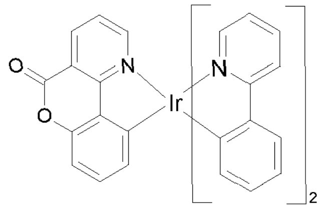 |
| 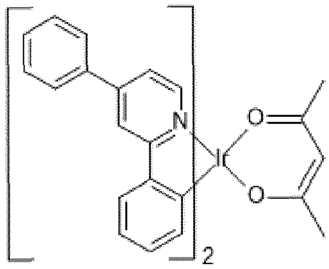 | 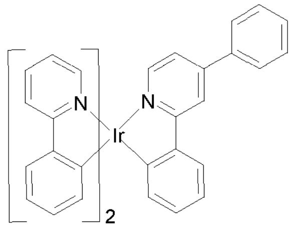 | 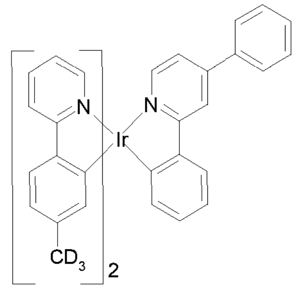 |
| 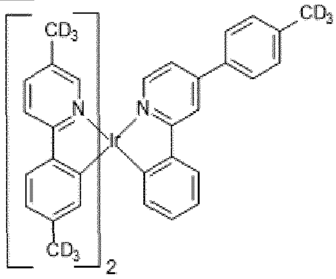 | 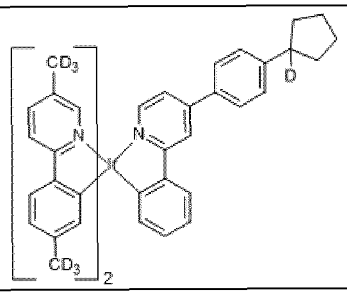 | 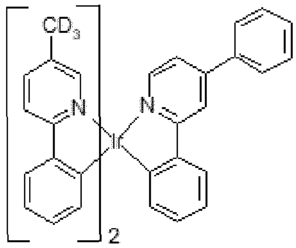 |
| 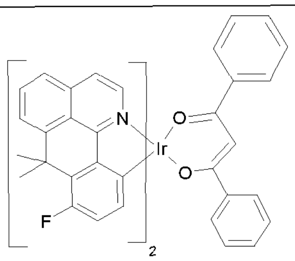 | 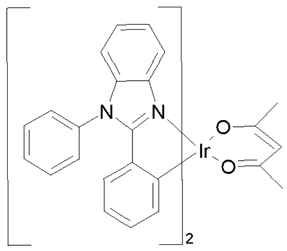 | 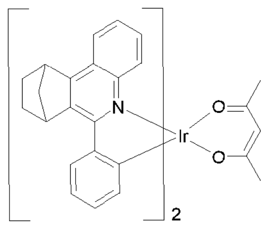 |
| 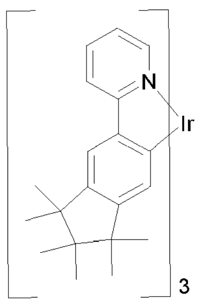 | 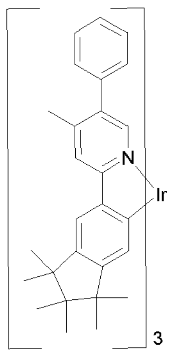 | 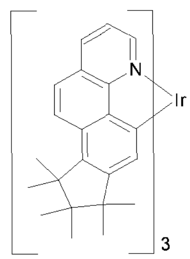 |

| | | |
|---|---|---|
| 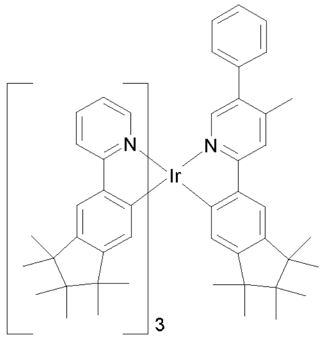 | 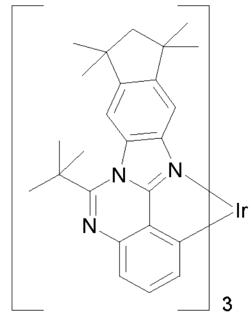 | 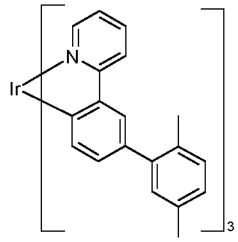 |
| 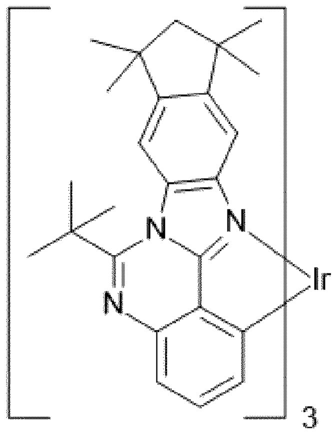 | 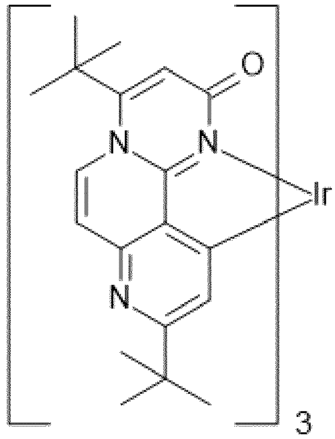 | 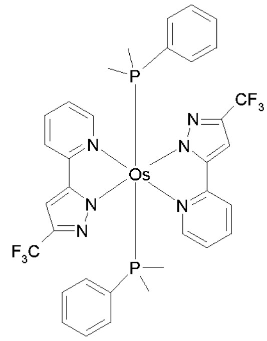 |
| 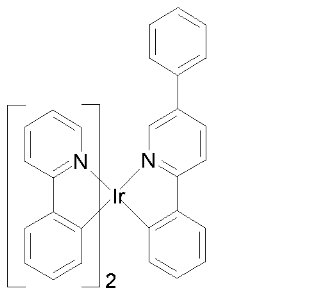 | 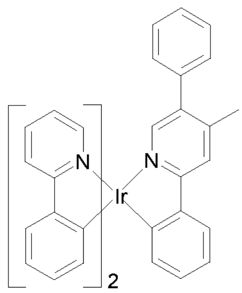 | 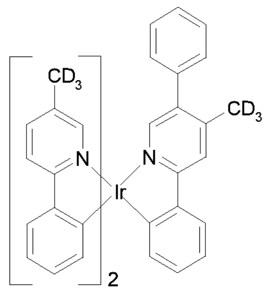 |
| 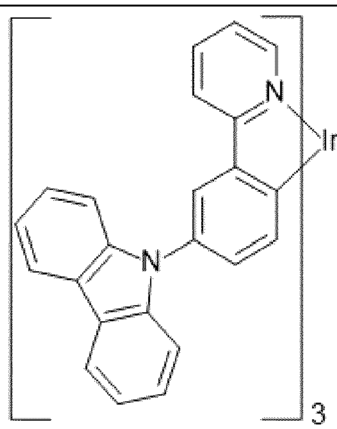 | 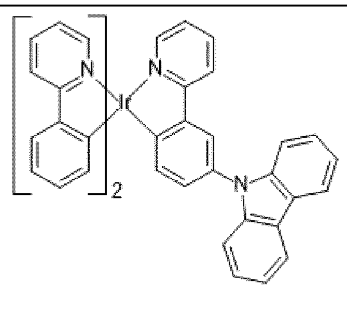 | 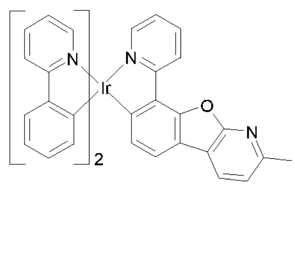 |

| | | |
|---|---|---|
| 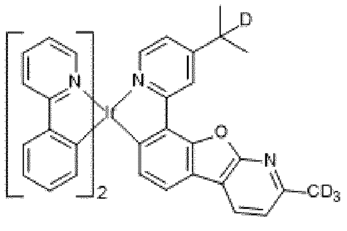 | 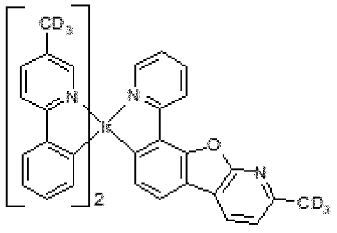 | 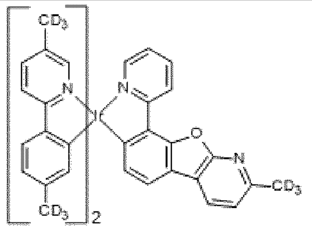 |
| 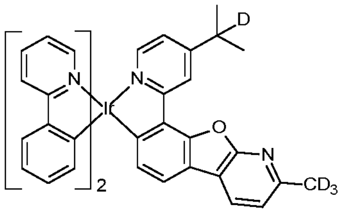 | 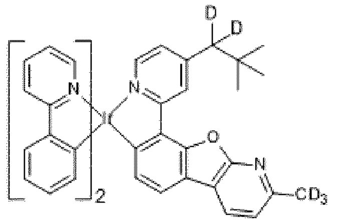 | 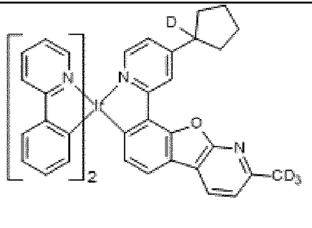 |
| 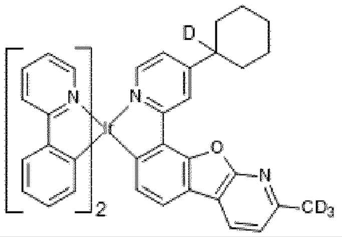 | 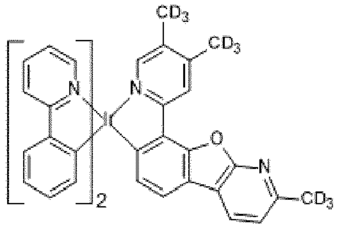 | 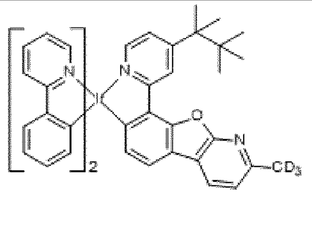 |
| 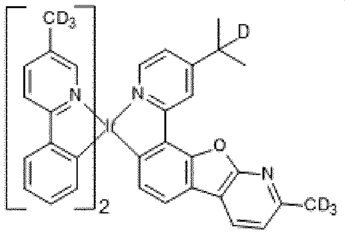 | 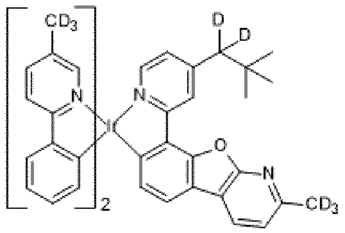 | 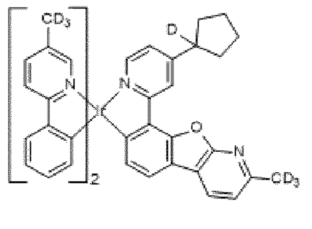 |
| 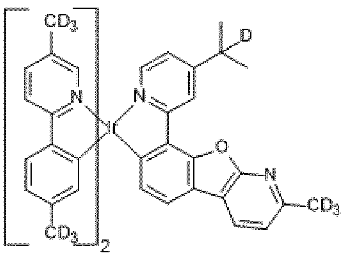 | 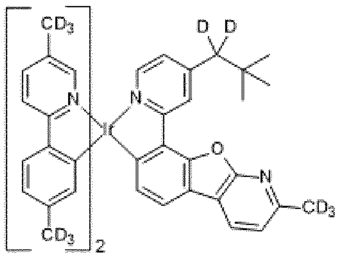 | 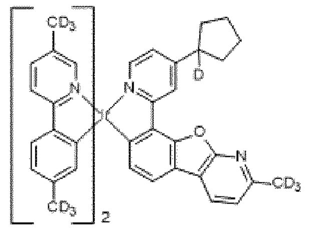 |
| 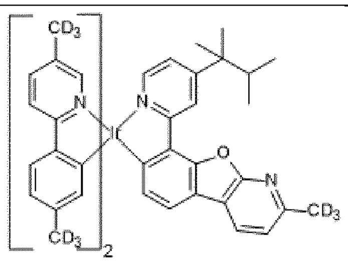 | 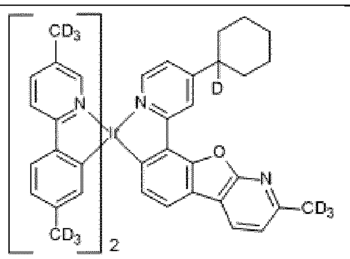 | 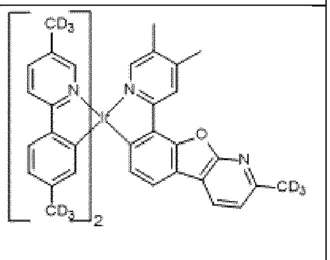 |
| 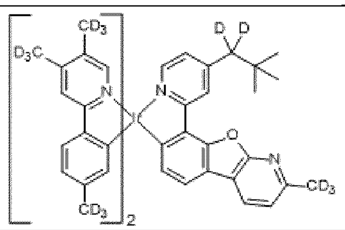 | 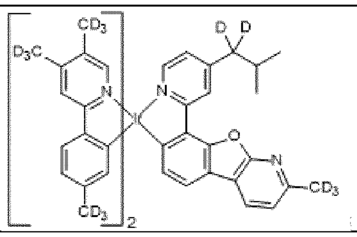 | 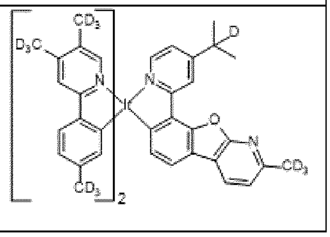 |

| | | |
|---|---|---|
| 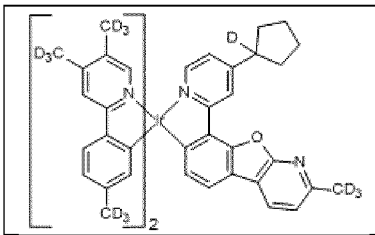 | 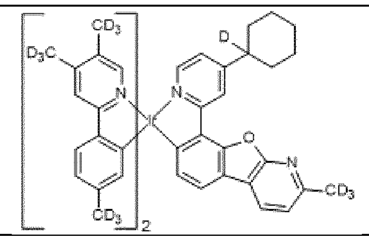 | 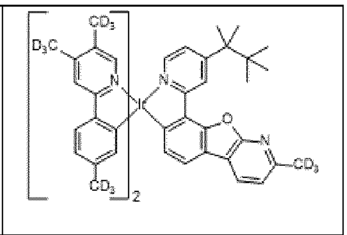 |
| 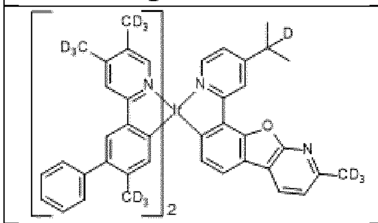 | 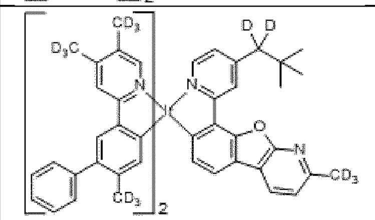 | 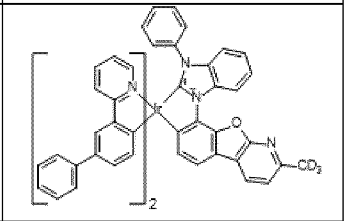 |
| 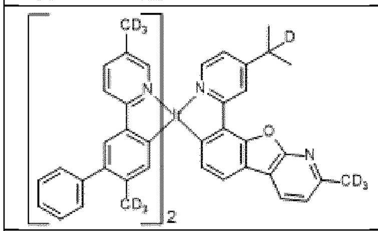 | 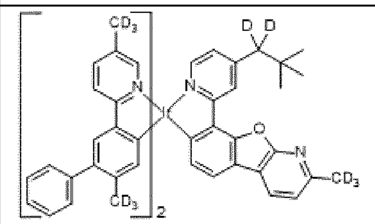 | 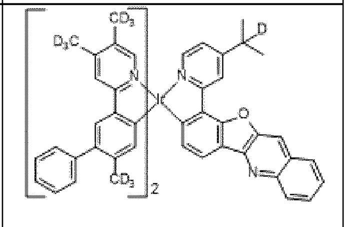 |
| 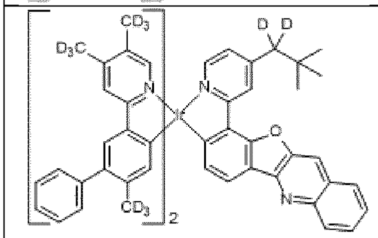 | 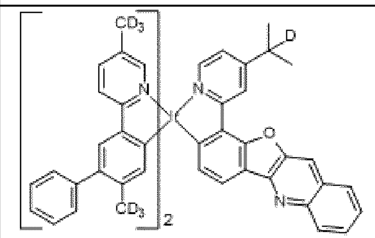 | 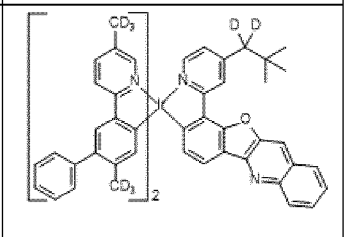 |
| 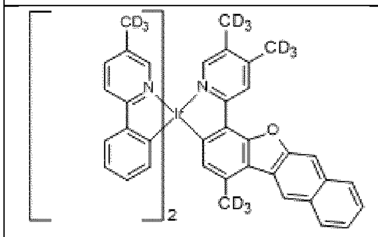 | 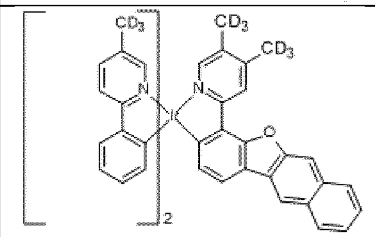 | 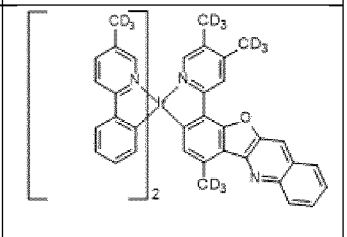 |
| 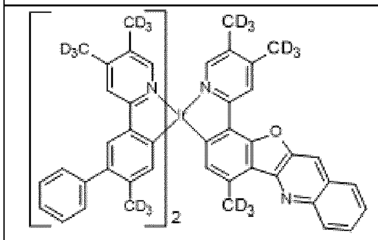 | 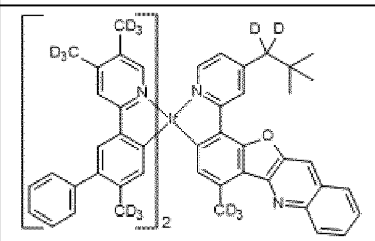 | 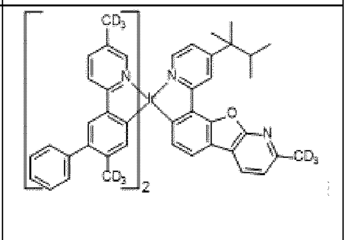 |
| 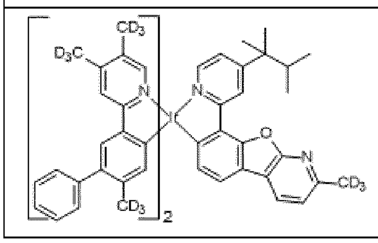 | 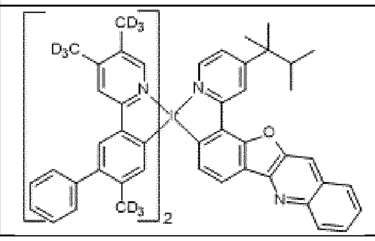 | 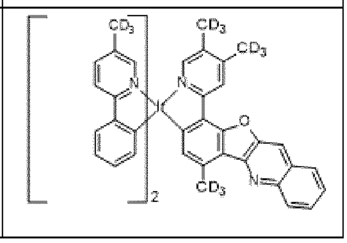 |

| | | |
|---|---|---|
| 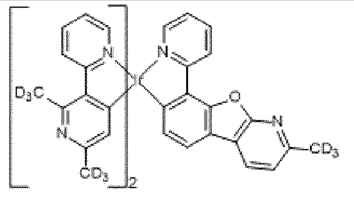 | 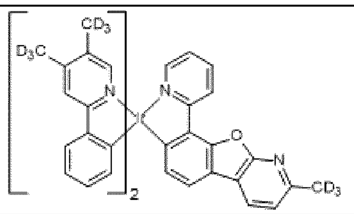 | 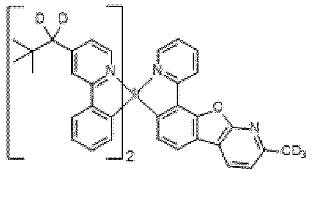 |
| 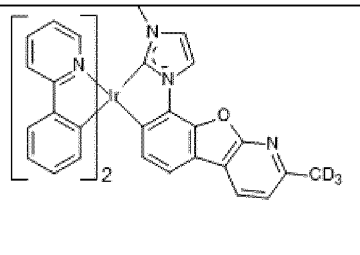 | 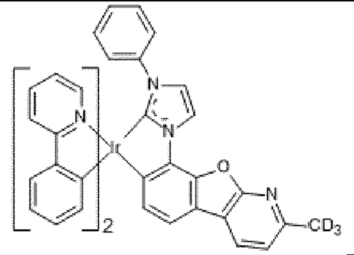 | 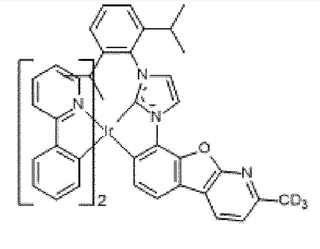 |
| 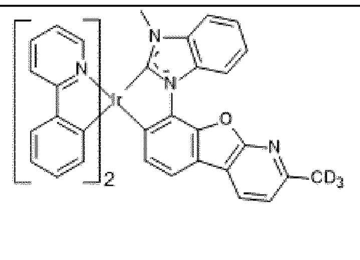 | 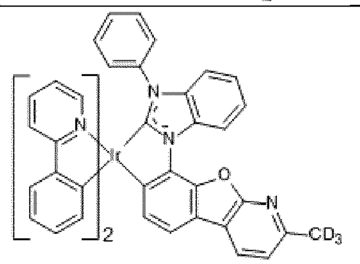 | 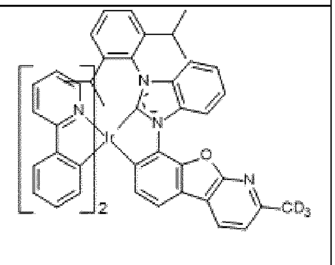 |
| 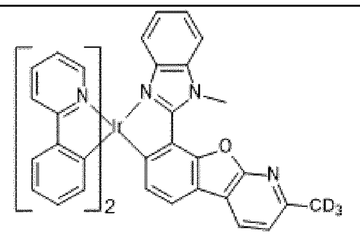 | 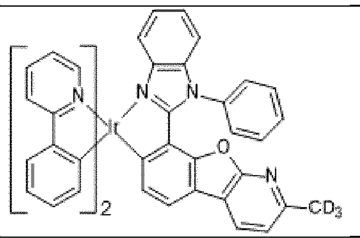 | 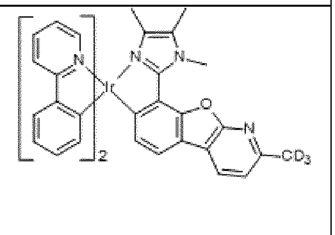 |
| 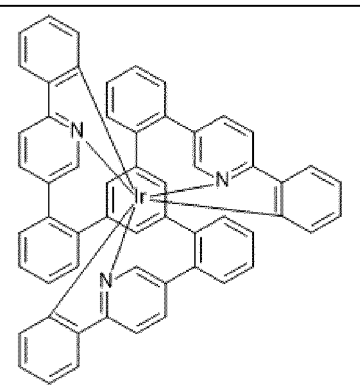 | 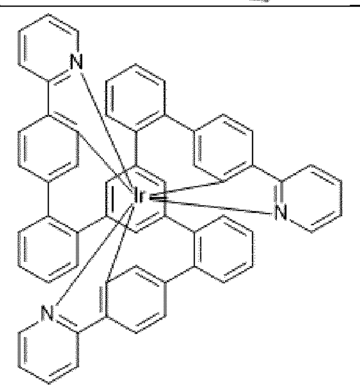 | 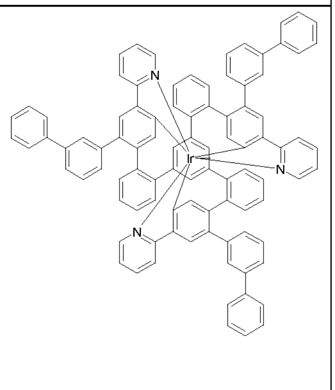 |
| 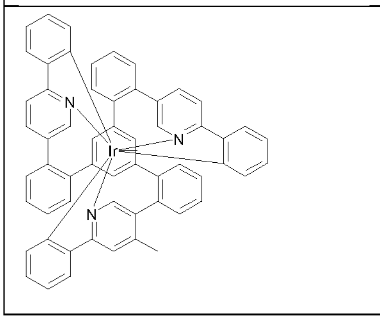 | 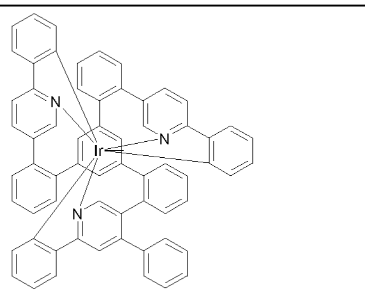 | 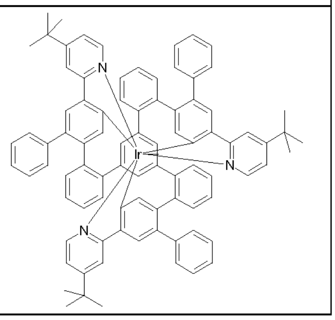 |

| | | |
|---|---|---|
| 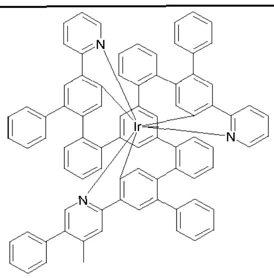 | 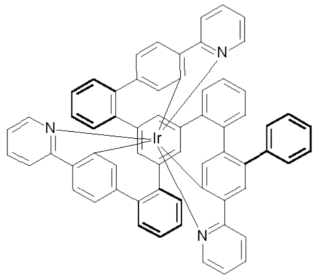 | 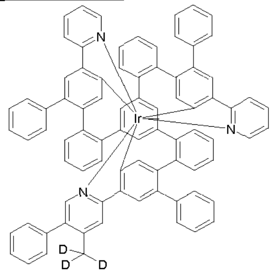 |
| 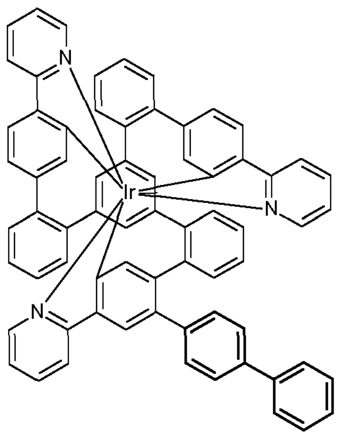 | 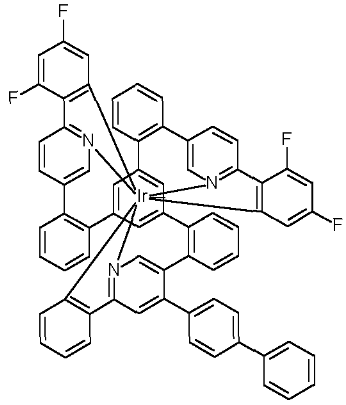 | 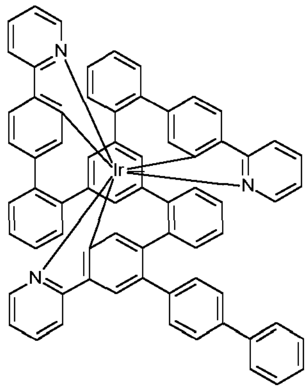 |
| 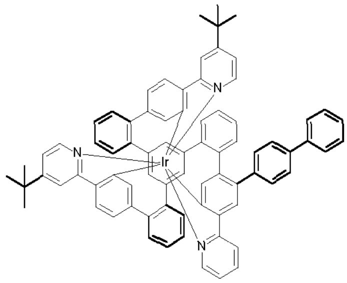 | 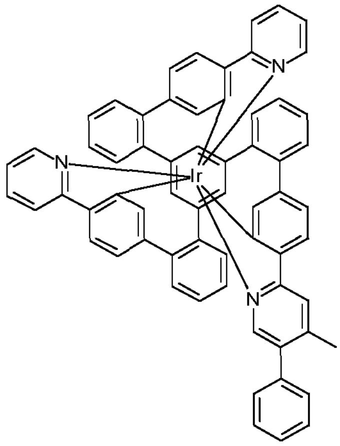 | 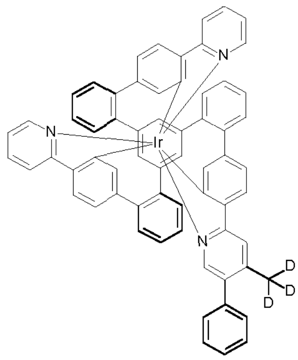 |
| 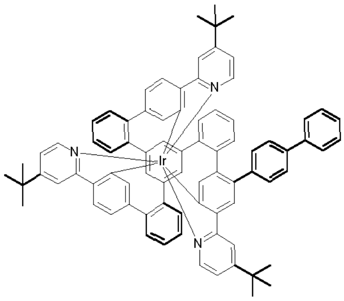 | 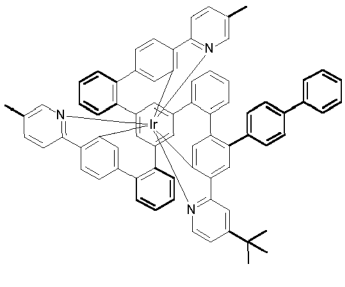 | 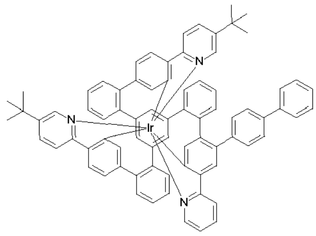 |

| | | |
|---|---|---|
| 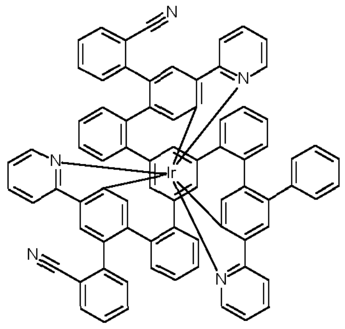 | 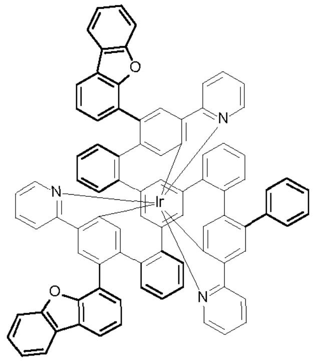 | 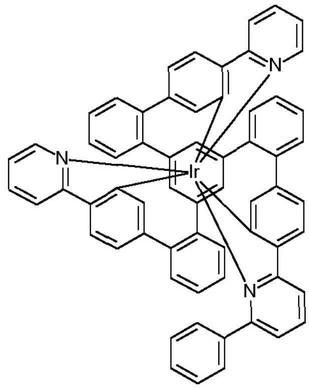 |
| 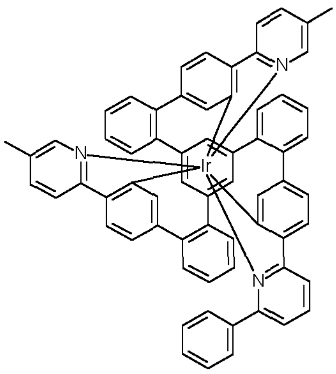 | 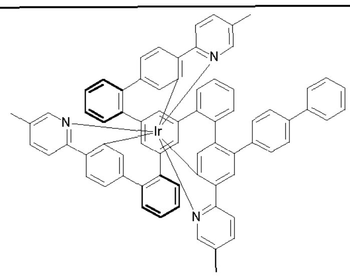 | 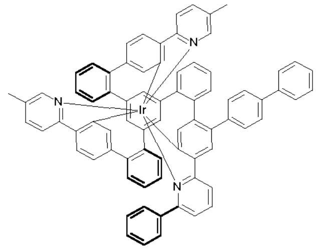 |
| 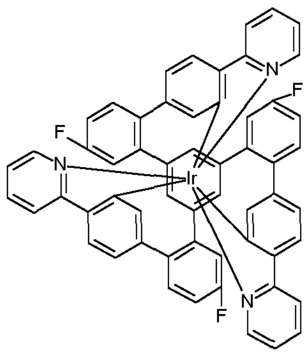 | 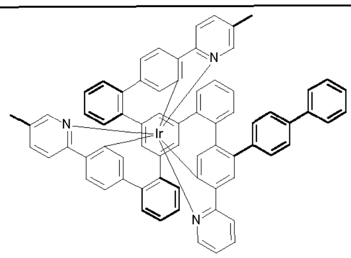 | 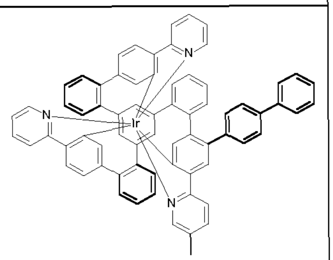 |
| 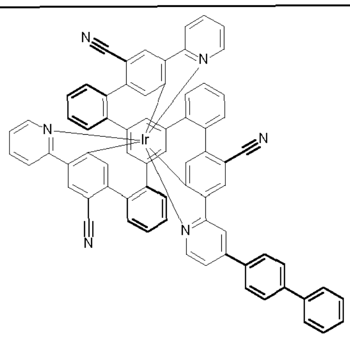 | 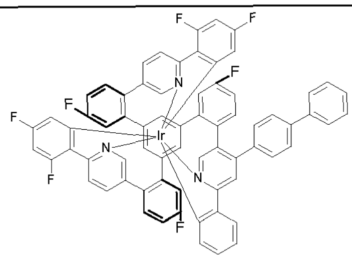 | 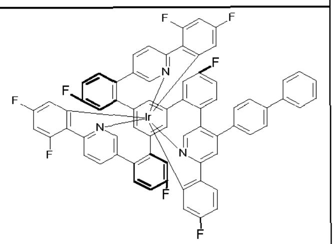 |
| 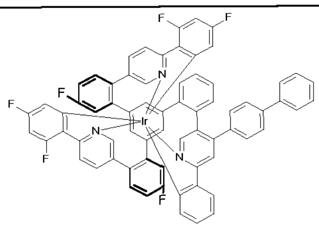 | 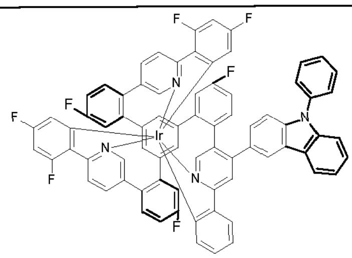 | 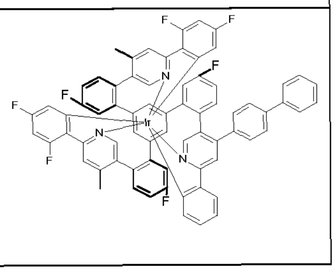 |

| | | |
|---|---|---|
| 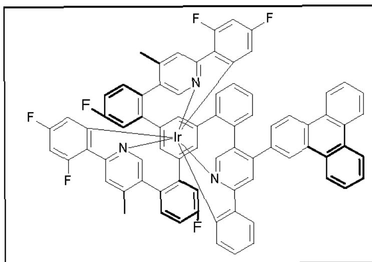 | 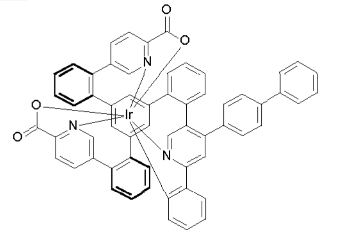 | 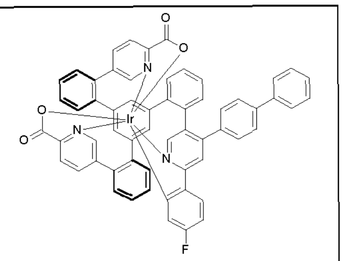 |
| 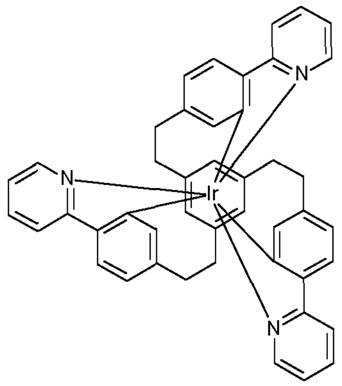 | 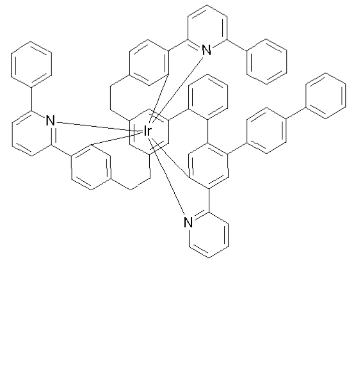 | 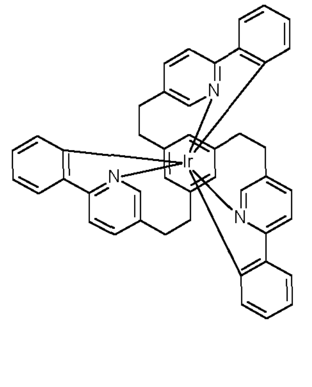 |
| 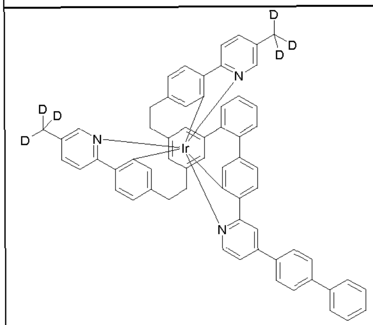 | 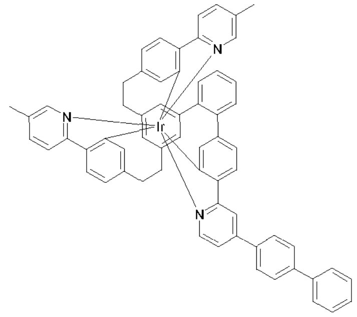 | 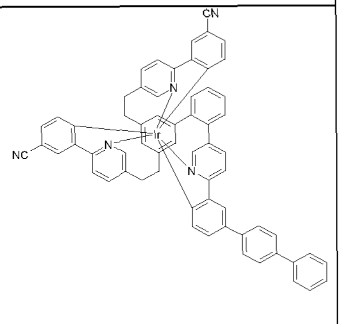 |
| 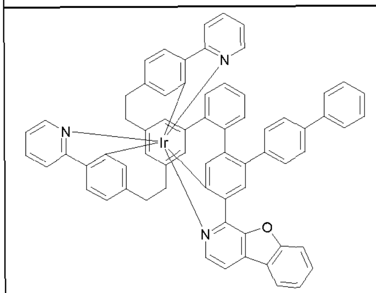 | 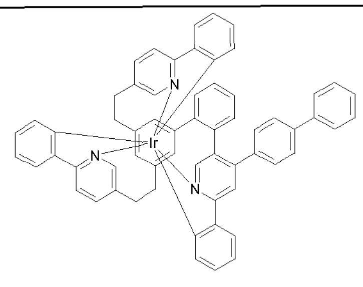 | 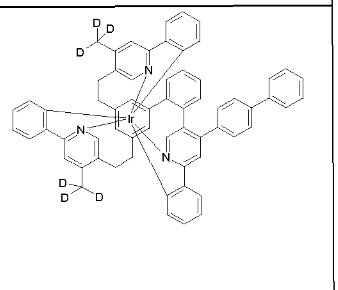 |
| 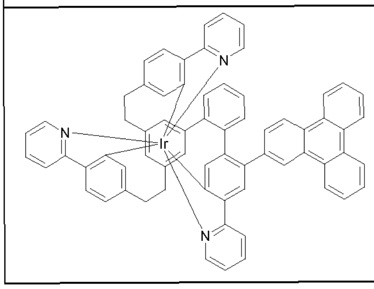 | 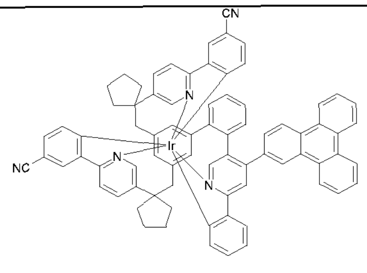 | 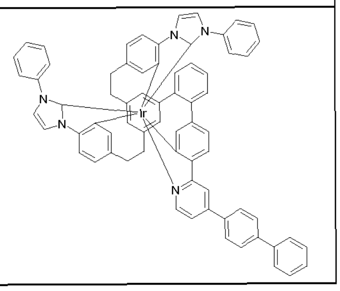 |

| | | |
|---|---|---|
| 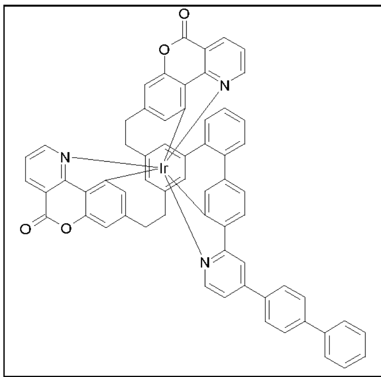 | 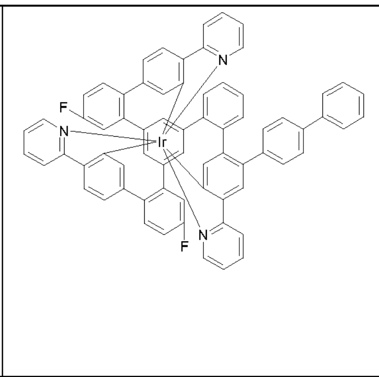 | 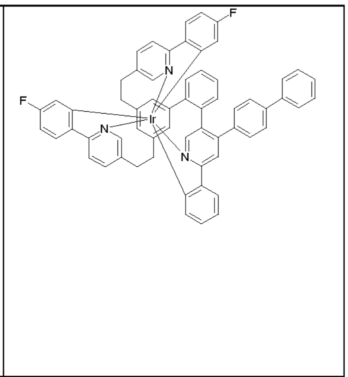 |
| 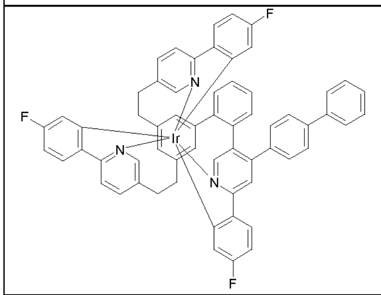 | 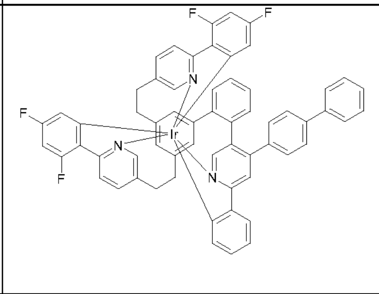 | 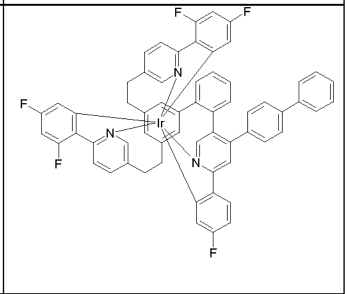 |
| 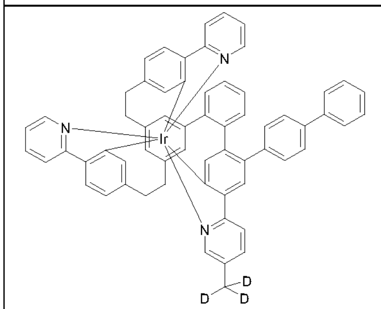 | 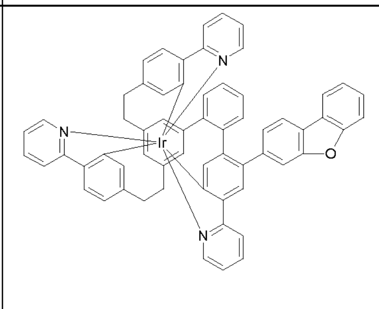 | 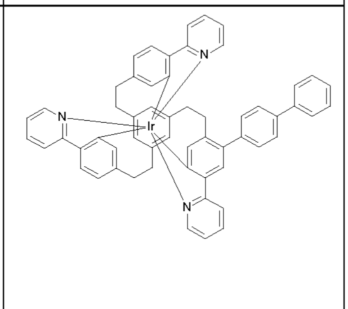 |
| 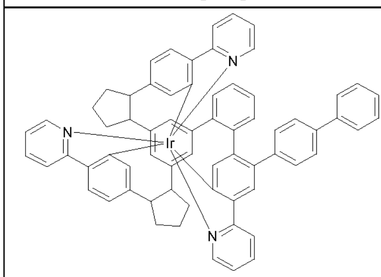 | 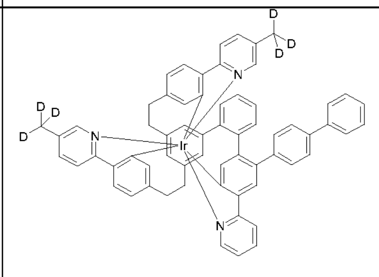 | 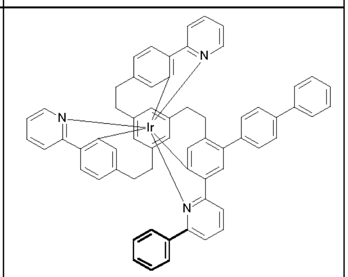 |
| 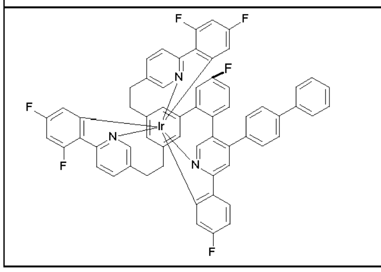 | 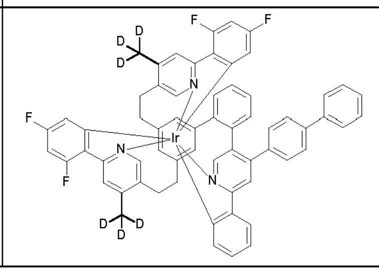 | 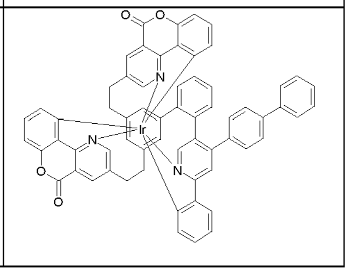 |

| | | |
|---|---|---|
| 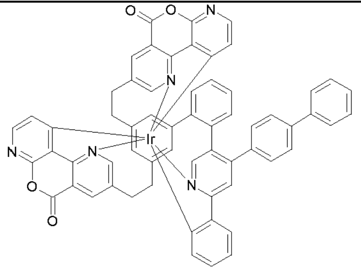 | 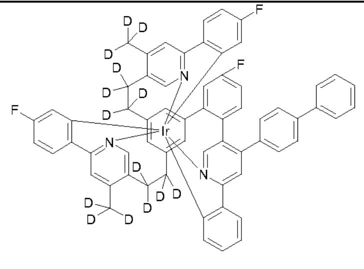 | 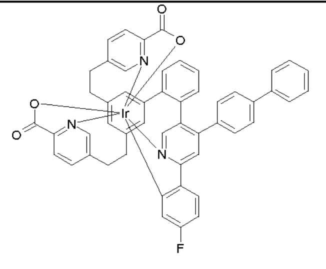 |
| 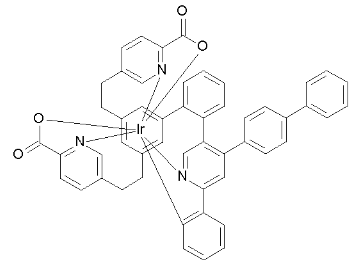 | 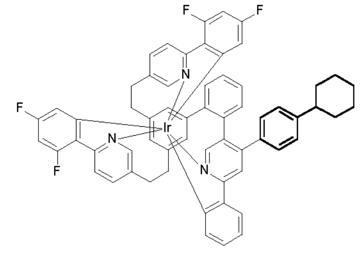 | 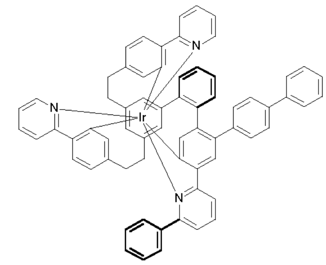 |
| 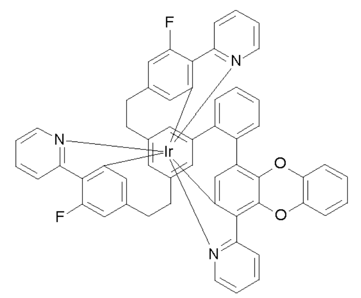 | 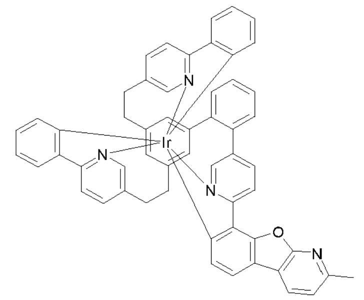 | 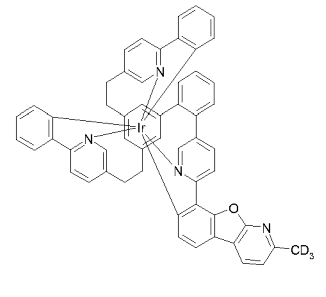 |
| 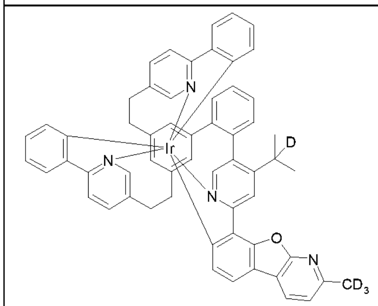 | 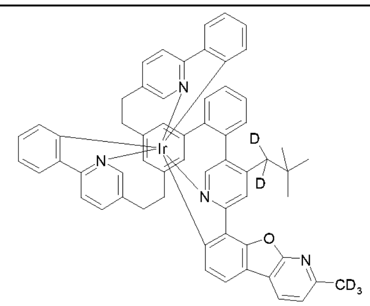 | 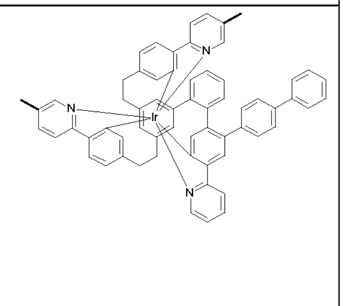 |
| 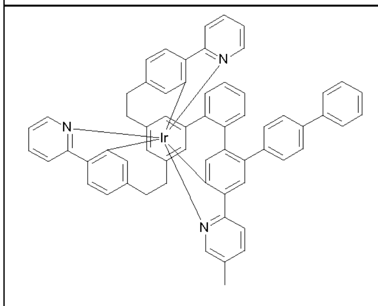 | 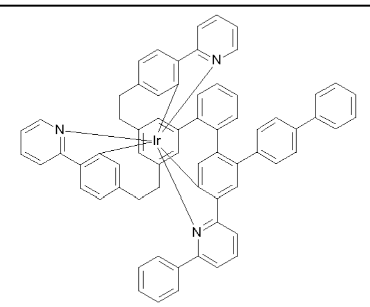 | 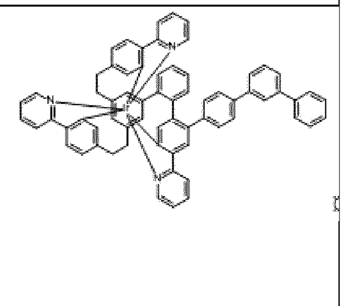 |
| 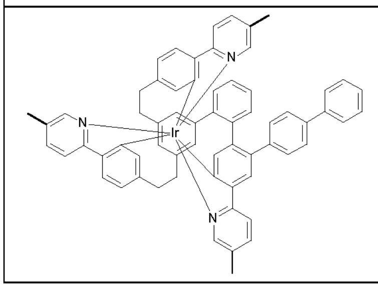 | 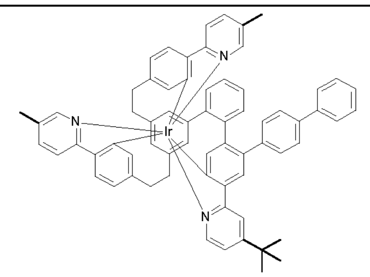 | 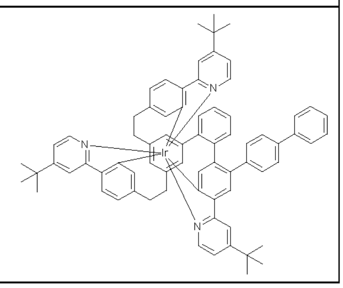 |

| | | |
|---|---|---|
| 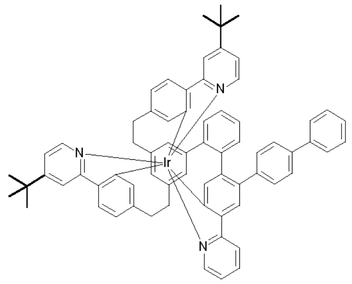 | 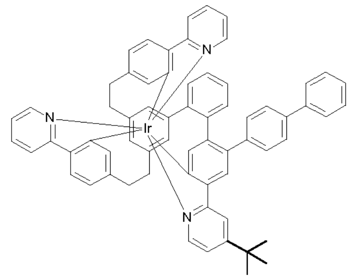 | 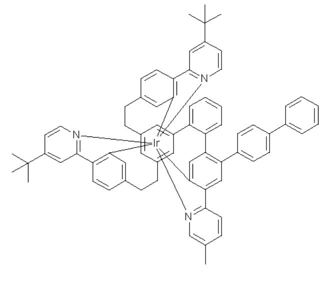 |
| 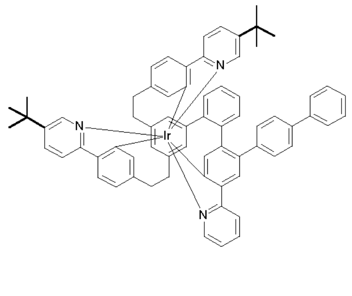 | 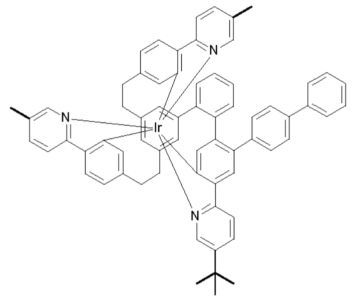 | 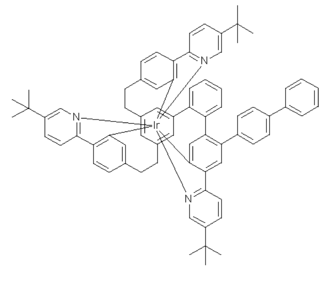 |
| 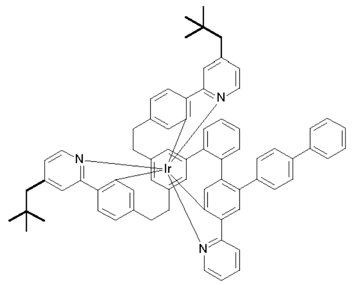 | 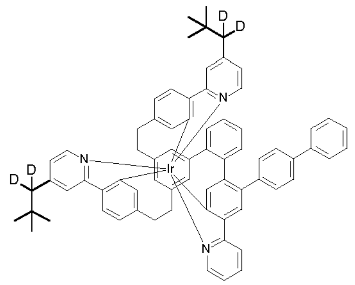 | 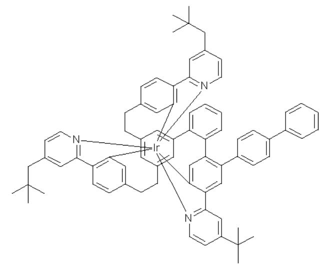 |
| 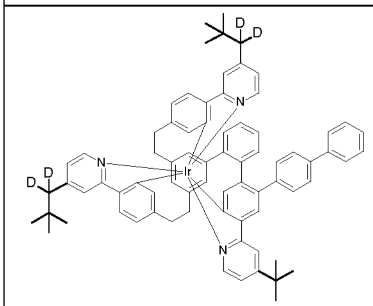 | 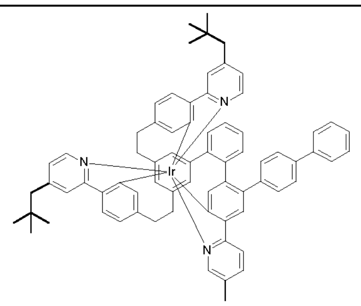 | 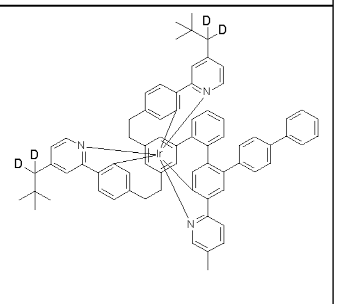 |
| 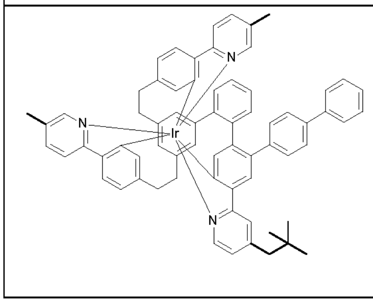 | 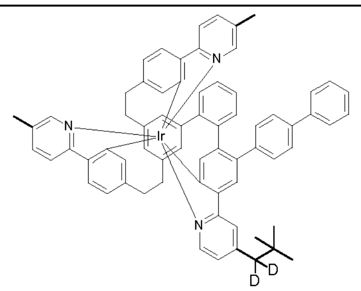 | 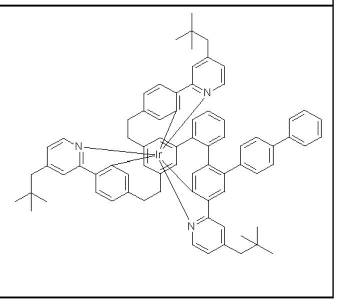 |

| | | |
|---|---|---|
| 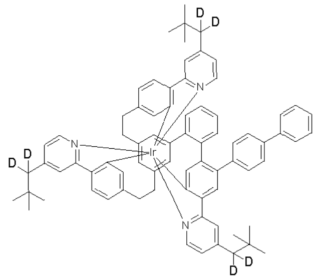 | 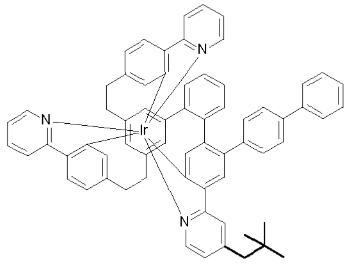 | 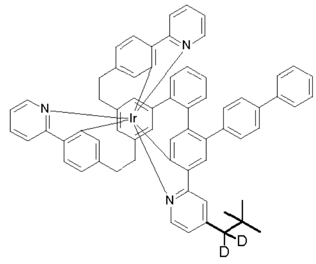 |
| 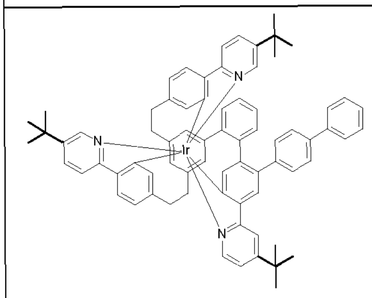 | 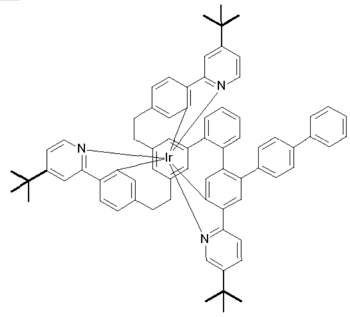 | 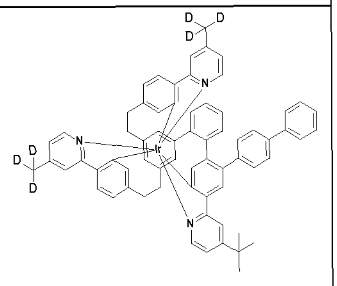 |
| 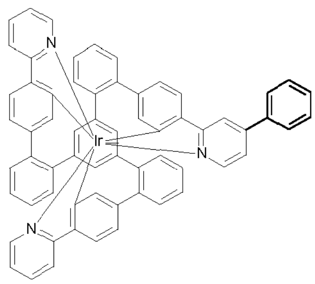 | 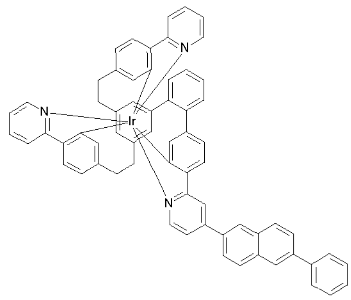 | 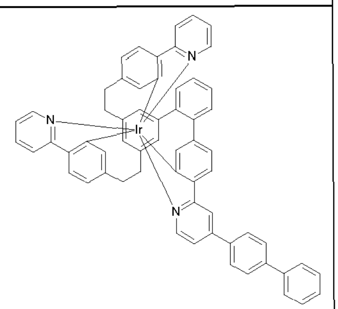 |
| 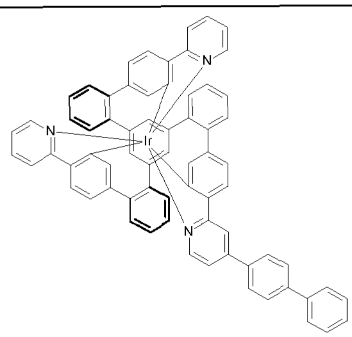 | 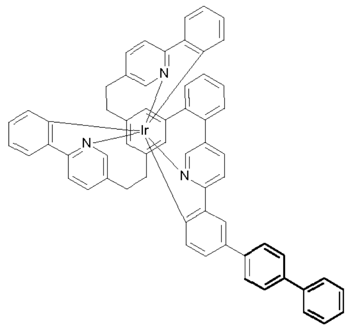 | 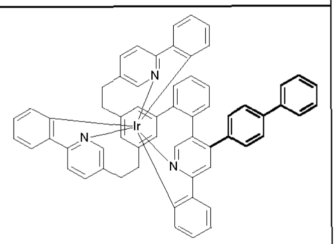 |
| 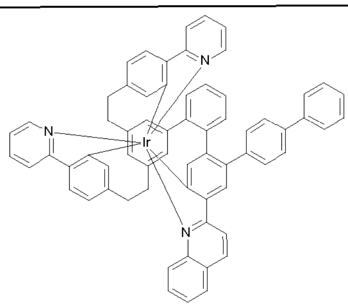 | 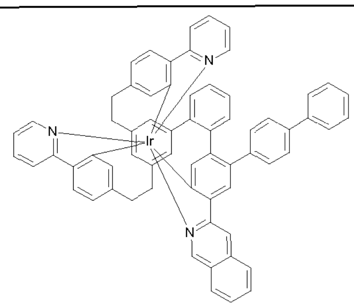 | 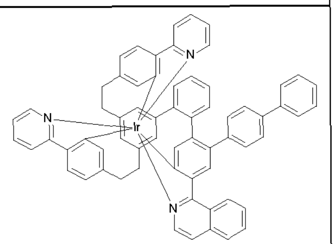 |

| | | |
|---|---|---|
| 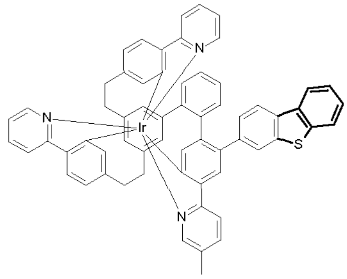 | 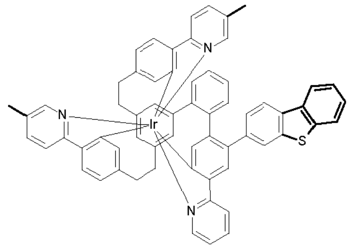 | 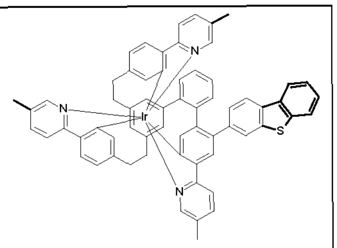 |
| 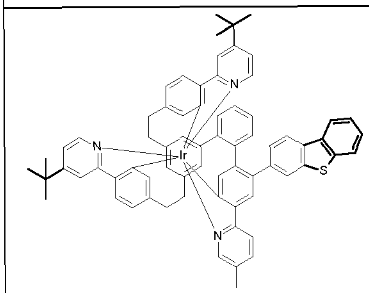 | 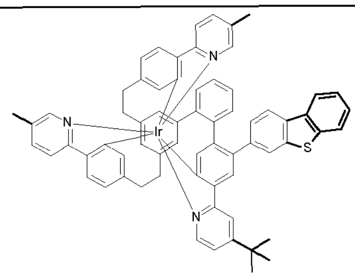 | 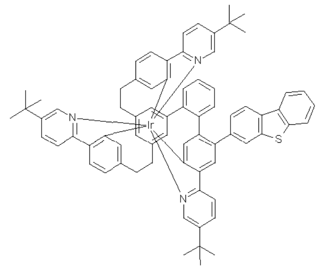 |
| 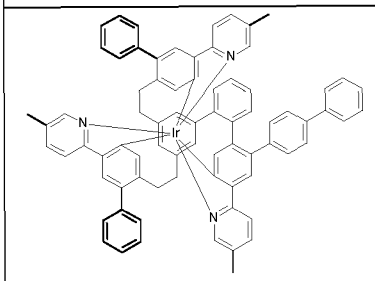 | 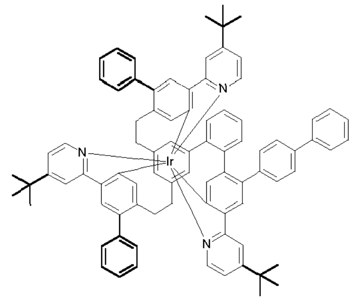 | 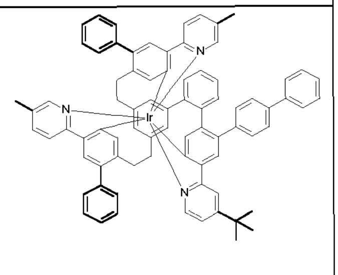 |
| 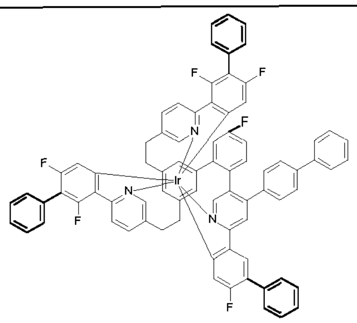 | 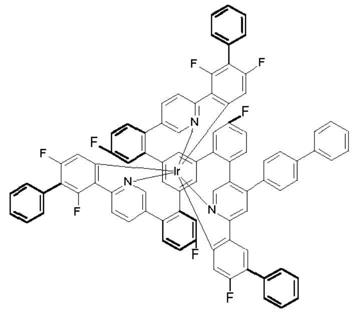 | 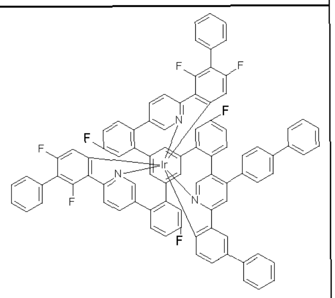 |
| 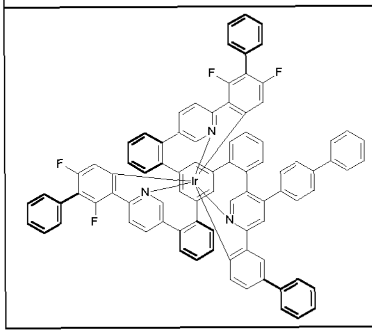 | 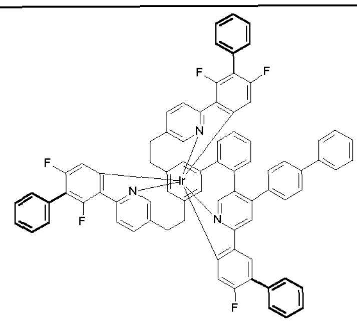 | 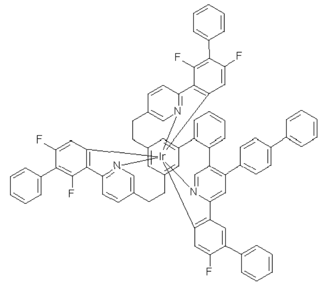 |

| | | |
|---|---|---|
| 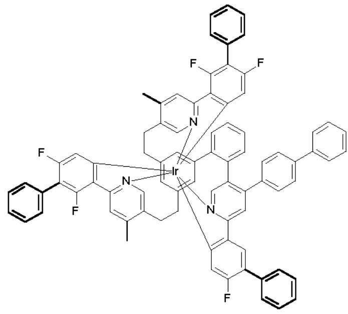 | 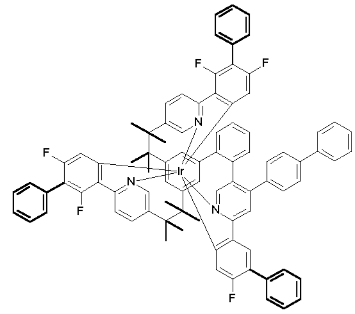 | 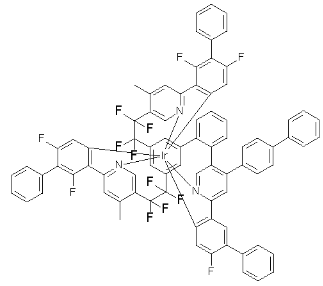 |
| 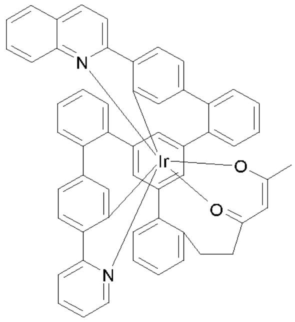 | 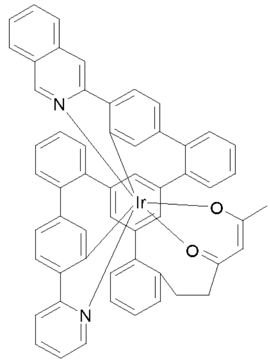 | 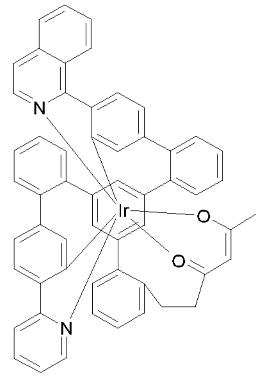 |
| 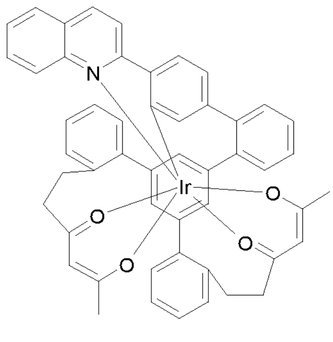 | 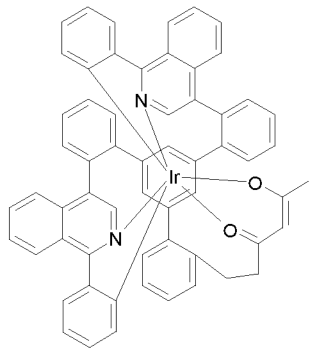 | 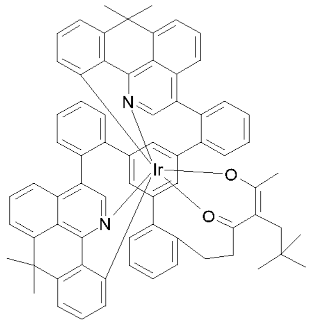 |
| 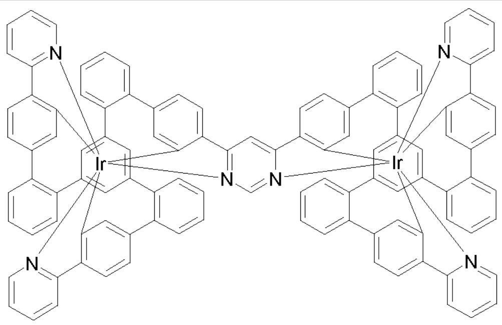 | 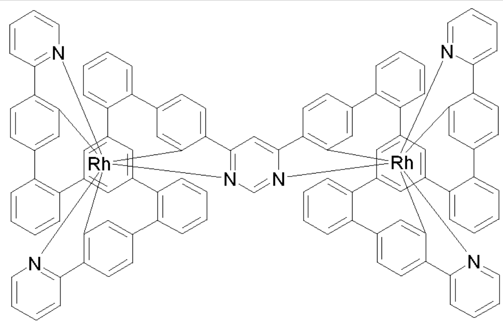 | |

| | |
|---|---|
| 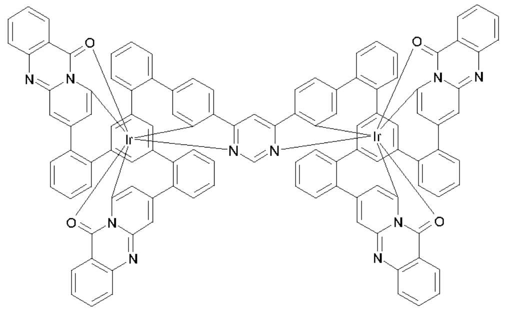 | 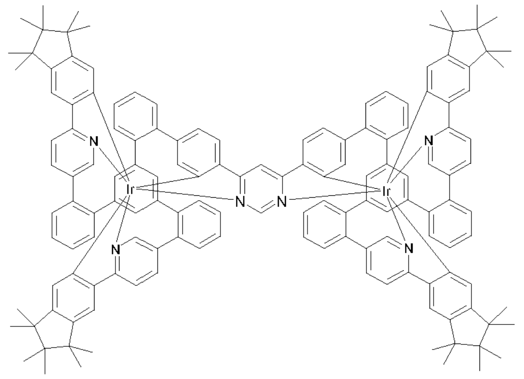 |
| 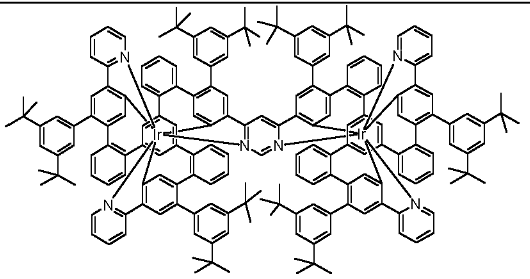 | 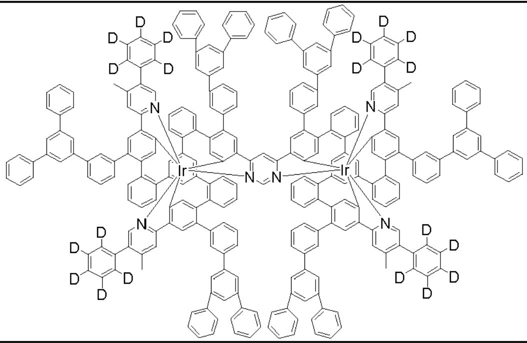 |

Bevorzugte Beispiele von phosphoreszierenden polypodalen Emittern sind in der folgenden Tabelle 7 aufgeführt.

Tabelle 7:

| | | | |
|---|---|---|---|
| CAS-1269508-30-6 | CAS-1989601-68-4 | CAS-1989602-19-8 | CAS-1989602-70-1 |
| CAS-1215692-34-4 | CAS-1989601-69-5 | CAS-1989602-20-1 | CAS-1989602-71-2 |
| CAS-1370364-40-1 | CAS-1989601-70-8 | CAS-1989602-21-2 | CAS-1989602-72-3 |
| CAS-1370364-42-3 | CAS-1989601-71-9 | CAS-1989602-22-3 | CAS-1989602-73-4 |
| CAS-1989600-74-9 | CAS-1989601-72-0 | CAS-1989602-23-4 | CAS-1989602-74-5 |
| CAS-1989600-75-0 | CAS-1989601-73-1 | CAS-1989602-24-5 | CAS-1989602-75-6 |
| CAS-1989600-77-2 | CAS-1989601-74-2 | CAS-1989602-25-6 | CAS-1989602-76-7 |
| CAS-1989600-78-3 | CAS-1989601-75-3 | CAS-1989602-26-7 | CAS-1989602-77-8 |
| CAS-1989600-79-4 | CAS-1989601-76-4 | CAS-1989602-27-8 | CAS-1989602-78-9 |
| CAS-1989600-82-9 | CAS-1989601-77-5 | CAS-1989602-28-9 | CAS-1989602-79-0 |
| CAS-1989600-83-0 | CAS-1989601-78-6 | CAS-1989602-29-0 | CAS-1989602-80-3 |
| CAS-1989600-84-1 | CAS-1989601-79-7 | CAS-1989602-30-3 | CAS-1989602-82-5 |
| CAS-1989600-85-2 | CAS-1989601-80-0 | CAS-1989602-31-4 | CAS-1989602-84-7 |
| CAS-1989600-86-3 | CAS-1989601-81-1 | CAS-1989602-32-5 | CAS-1989602-85-8 |
| CAS-1989600-87-4 | CAS-1989601-82-2 | CAS-1989602-33-6 | CAS-1989602-86-9 |
| CAS-1989600-88-5 | CAS-1989601-83-3 | CAS-1989602-34-7 | CAS-1989602-87-0 |
| CAS-1989600-89-6 | CAS-1989601-84-4 | CAS-1989602-35-8 | CAS-1989602-88-1 |
| CAS-1989601-11-7 | CAS-1989601-85-5 | CAS-1989602-36-9 | CAS-1989604-00-3 |
| CAS-1989601-23-1 | CAS-1989601-86-6 | CAS-1989602-37-0 | CAS-1989604-01-4 |
| CAS-1989601-26-4 | CAS-1989601-87-7 | CAS-1989602-38-1 | CAS-1989604-02-5 |

(fortgesetzt)

| | | | |
|---|---|---|---|
| CAS-1989601-28-6 | CAS-1989601-88-8 | CAS-1989602-39-2 | CAS-1989604-03-6 |
| CAS-1989601-29-7 | CAS-1989601-89-9 | CAS-1989602-40-5 | CAS-1989604-04-7 |
| CAS-1989601-33-3 | CAS-1989601-90-2 | CAS-1989602-41-6 | CAS-1989604-05-8 |
| CAS-1989601-40-2 | CAS-1989601-91-3 | CAS-1989602-42-7 | CAS-1989604-06-9 |
| CAS-1989601-41-3 | CAS-1989601-92-4 | CAS-1989602-43-8 | CAS-1989604-07-0 |
| CAS-1989601-42-4 | CAS-1989601-93-5 | CAS-1989602-44-9 | CAS-1989604-08-1 |
| CAS-1989601-43-5 | CAS-1989601-94-6 | CAS-1989602-45-0 | CAS-1989604-09-2 |
| CAS-1989601-44-6 | CAS-1989601-95-7 | CAS-1989602-46-1 | CAS-1989604-10-5 |
| CAS-1989601-45-7 | CAS-1989601-96-8 | CAS-1989602-47-2 | CAS-1989604-11-6 |
| CAS-1989601-46-8 | CAS-1989601-97-9 | CAS-1989602-48-3 | CAS-1989604-13-8 |
| CAS-1989601-47-9 | CAS-1989601-98-0 | CAS-1989602-49-4 | CAS-1989604-14-9 |
| CAS-1989601-48-0 | CAS-1989601-99-1 | CAS-1989602-50-7 | CAS-1989604-15-0 |
| CAS-1989601-49-1 | CAS-1989602-00-7 | CAS-1989602-51-8 | CAS-1989604-16-1 |
| CAS-1989601-50-4 | CAS-1989602-01-8 | CAS-1989602-52-9 | CAS-1989604-17-2 |
| CAS-1989601-51-5 | CAS-1989602-02-9 | CAS-1989602-53-0 | CAS-1989604-18-3 |
| CAS-1989601-52-6 | CAS-1989602-03-0 | CAS-1989602-54-1 | CAS-1989604-19-4 |
| CAS-1989601-53-7 | CAS-1989602-04-1 | CAS-1989602-55-2 | CAS-1989604-20-7 |
| CAS-1989601-54-8 | CAS-1989602-05-2 | CAS-1989602-56-3 | CAS-1989604-21-8 |
| CAS-1989601-55-9 | CAS-1989602-06-3 | CAS-1989602-57-4 | CAS-1989604-22-9 |
| CAS-1989601-56-0 | CAS-1989602-07-4 | CAS-1989602-58-5 | CAS-1989604-23-0 |
| CAS-1989601-57-1 | CAS-1989602-08-5 | CAS-1989602-59-6 | CAS-1989604-24-1 |
| CAS-1989601-58-2 | CAS-1989602-09-6 | CAS-1989602-60-9 | CAS-1989604-25-2 |
| CAS-1989601-59-3 | CAS-1989602-10-9 | CAS-1989602-61-0 | CAS-1989604-26-3 |
| CAS-1989601-60-6 | CAS-1989602-11-0 | CAS-1989602-62-1 | CAS-1989604-27-4 |
| CAS-1989601-61-7 | CAS-1989602-12-1 | CAS-1989602-63-2 | CAS-1989604-28-5 |
| CAS-1989601-62-8 | CAS-1989602-13-2 | CAS-1989602-64-3 | CAS-1989604-29-6 |
| CAS-1989601-63-9 | CAS-1989602-14-3 | CAS-1989602-65-4 | CAS-1989604-30-9 |
| CAS-1989601-64-0 | CAS-1989602-15-4 | CAS-1989602-66-5 | CAS-1989604-31-0 |
| CAS-1989601-65-1 | CAS-1989602-16-5 | CAS-1989602-67-6 | CAS-1989604-32-1 |
| CAS-1989601-66-2 | CAS-1989602-17-6 | CAS-1989602-68-7 | CAS-1989604-33-2 |
| CAS-1989601-67-3 | CAS-1989602-18-7 | CAS-1989602-69-8 | CAS-1989604-34-3 |
| CAS-1989604-35-4 | CAS-1989604-88-7 | CAS-1989605-52-8 | CAS-1989606-07-6 |
| CAS-1989604-36-5 | CAS-1989604-89-8 | CAS-1989605-53-9 | CAS-1989606-08-7 |
| CAS-1989604-37-6 | CAS-1989604-90-1 | CAS-1989605-54-0 | CAS-1989606-09-8 |
| CAS-1989604-38-7 | CAS-1989604-92-3 | CAS-1989605-55-1 | CAS-1989606-10-1 |
| CAS-1989604-39-8 | CAS-1989604-93-4 | CAS-1989605-56-2 | CAS-1989606-11-2 |
| CAS-1989604-40-1 | CAS-1989604-94-5 | CAS-1989605-57-3 | CAS-1989606-12-3 |
| CAS-1989604-41-2 | CAS-1989604-95-6 | CAS-1989605-58-4 | CAS-1989606-13-4 |
| CAS-1989604-42-3 | CAS-1989604-96-7 | CAS-1989605-59-5 | CAS-1989606-14-5 |

(fortgesetzt)

| | | | |
|---|---|---|---|
| CAS-1989604-43-4 | CAS-1989604-97-8 | CAS-1989605-61-9 | CAS-1989606-15-6 |
| CAS-1989604-45-6 | CAS-1989605-09-5 | CAS-1989605-62-0 | CAS-1989606-16-7 |
| CAS-1989604-46-7 | CAS-1989605-10-8 | CAS-1989605-63-1 | CAS-1989606-17-8 |
| CAS-1989604-47-8 | CAS-1989605-11-9 | CAS-1989605-64-2 | CAS-1989606-18-9 |
| CAS-1989604-48-9 | CAS-1989605-13-1 | CAS-1989605-65-3 | CAS-1989606-19-0 |
| CAS-1989604-49-0 | CAS-1989605-14-2 | CAS-1989605-66-4 | CAS-1989606-20-3 |
| CAS-1989604-50-3 | CAS-1989605-15-3 | CAS-1989605-67-5 | CAS-1989606-21-4 |
| CAS-1989604-52-5 | CAS-1989605-16-4 | CAS-1989605-68-6 | CAS-1989606-22-5 |
| CAS-1989604-53-6 | CAS-1989605-17-5 | CAS-1989605-69-7 | CAS-1989606-23-6 |
| CAS-1989604-54-7 | CAS-1989605-18-6 | CAS-1989605-70-0 | CAS-1989606-24-7 |
| CAS-1989604-55-8 | CAS-1989605-19-7 | CAS-1989605-71-1 | CAS-1989606-26-9 |
| CAS-1989604-56-9 | CAS-1989605-20-0 | CAS-1989605-72-2 | CAS-1989606-27-0 |
| CAS-1989604-57-0 | CAS-1989605-21-1 | CAS-1989605-73-3 | CAS-1989606-28-1 |
| CAS-1989604-58-1 | CAS-1989605-22-2 | CAS-1989605-74-4 | CAS-1989606-29-2 |
| CAS-1989604-59-2 | CAS-1989605-23-3 | CAS-1989605-75-5 | CAS-1989606-30-5 |
| CAS-1989604-60-5 | CAS-1989605-24-4 | CAS-1989605-76-6 | CAS-1989606-31-6 |
| CAS-1989604-61-6 | CAS-1989605-25-5 | CAS-1989605-77-7 | CAS-1989606-32-7 |
| CAS-1989604-62-7 | CAS-1989605-26-6 | CAS-1989605-78-8 | CAS-1989606-33-8 |
| CAS-1989604-63-8 | CAS-1989605-27-7 | CAS-1989605-79-9 | CAS-1989606-34-9 |
| CAS-1989604-64-9 | CAS-1989605-28-8 | CAS-1989605-81-3 | CAS-1989606-35-0 |
| CAS-1989604-65-0 | CAS-1989605-29-9 | CAS-1989605-82-4 | CAS-1989606-36-1 |
| CAS-1989604-66-1 | CAS-1989605-30-2 | CAS-1989605-83-5 | CAS-1989606-37-2 |
| CAS-1989604-67-2 | CAS-1989605-31-3 | CAS-1989605-84-6 | CAS-1989606-38-3 |
| CAS-1989604-68-3 | CAS-1989605-32-4 | CAS-1989605-85-7 | CAS-1989606-39-4 |
| CAS-1989604-69-4 | CAS-1989605-33-5 | CAS-1989605-86-8 | CAS-1989606-40-7 |
| CAS-1989604-70-7 | CAS-1989605-34-6 | CAS-1989605-87-9 | CAS-1989606-41-8 |
| CAS-1989604-71-8 | CAS-1989605-35-7 | CAS-1989605-88-0 | CAS-1989606-42-9 |
| CAS-1989604-72-9 | CAS-1989605-36-8 | CAS-1989605-89-1 | CAS-1989606-43-0 |
| CAS-1989604-73-0 | CAS-1989605-37-9 | CAS-1989605-90-4 | CAS-1989606-44-1 |
| CAS-1989604-74-1 | CAS-1989605-38-0 | CAS-1989605-91-5 | CAS-1989606-45-2 |
| CAS-1989604-75-2 | CAS-1989605-39-1 | CAS-1989605-92-6 | CAS-1989606-46-3 |
| CAS-1989604-76-3 | CAS-1989605-40-4 | CAS-1989605-93-7 | CAS-1989606-48-5 |
| CAS-1989604-77-4 | CAS-1989605-41-5 | CAS-1989605-94-8 | CAS-1989606-49-6 |
| CAS-1989604-78-5 | CAS-1989605-42-6 | CAS-1989605-95-9 | CAS-1989606-53-2 |
| CAS-1989604-79-6 | CAS-1989605-43-7 | CAS-1989605-96-0 | CAS-1989606-55-4 |
| CAS-1989604-80-9 | CAS-1989605-44-8 | CAS-1989605-97-1 | CAS-1989606-56-5 |
| CAS-1989604-81-0 | CAS-1989605-45-9 | CAS-1989605-98-2 | CAS-1989606-61-2 |
| CAS-1989604-82-1 | CAS-1989605-46-0 | CAS-1989605-99-3 | CAS-1989606-62-3 |
| CAS-1989604-83-2 | CAS-1989605-47-1 | CAS-1989606-00-9 | CAS-1989606-63-4 |

(fortgesetzt)

| | | | |
|---|---|---|---|
| CAS-1989604-84-3 | CAS-1989605-48-2 | CAS-1989606-01-0 | CAS-1989606-67-8 |
| CAS-1989604-85-4 | CAS-1989605-49-3 | CAS-1989606-04-3 | CAS-1989606-69-0 |
| CAS-1989604-86-5 | CAS-1989605-50-6 | CAS-1989606-05-4 | CAS-1989606-70-3 |
| CAS-1989604-87-6 | CAS-1989605-51-7 | CAS-1989606-06-5 | CAS-1989606-74-7 |
| CAS-1989658-39-0 | CAS-2088184-56-7 | CAS-2088185-07-1 | CAS-2088185-66-2 |
| CAS-1989658-41-4 | CAS-2088184-57-8 | CAS-2088185-08-2 | CAS-2088185-67-3 |
| CAS-1989658-43-6 | CAS-2088184-58-9 | CAS-2088185-09-3 | CAS-2088185-68-4 |
| CAS-1989658-47-0 | CAS-2088184-59-0 | CAS-2088185-10-6 | CAS-2088185-69-5 |
| CAS-1989658-49-2 | CAS-2088184-60-3 | CAS-2088185-11-7 | CAS-2088185-70-8 |
| CAS-2088184-07-8 | CAS-2088184-61-4 | CAS-2088185-12-8 | CAS-2088185-71-9 |
| CAS-2088184-08-9 | CAS-2088184-62-5 | CAS-2088185-13-9 | CAS-2088185-72-0 |
| CAS-2088184-09-0 | CAS-2088184-63-6 | CAS-2088185-14-0 | CAS-2088185-73-1 |
| CAS-2088184-10-3 | CAS-2088184-64-7 | CAS-2088185-15-1 | CAS-2088185-74-2 |
| CAS-2088184-11-4 | CAS-2088184-65-8 | CAS-2088185-16-2 | CAS-2088185-75-3 |
| CAS-2088184-13-6 | CAS-2088184-66-9 | CAS-2088185-17-3 | CAS-2088185-76-4 |
| CAS-2088184-14-7 | CAS-2088184-67-0 | CAS-2088185-18-4 | CAS-2088185-77-5 |
| CAS-2088184-15-8 | CAS-2088184-68-1 | CAS-2088185-19-5 | CAS-2088185-78-6 |
| CAS-2088184-16-9 | CAS-2088184-69-2 | CAS-2088185-20-8 | CAS-2088185-79-7 |
| CAS-2088184-17-0 | CAS-2088184-70-5 | CAS-2088185-21-9 | CAS-2088185-80-0 |
| CAS-2088184-18-1 | CAS-2088184-71-6 | CAS-2088185-22-0 | CAS-2088185-81-1 |
| CAS-2088184-19-2 | CAS-2088184-72-7 | CAS-2088185-23-1 | CAS-2088185-82-2 |
| CAS-2088184-20-5 | CAS-2088184-73-8 | CAS-2088185-32-2 | CAS-2088185-83-3 |
| CAS-2088184-21-6 | CAS-2088184-74-9 | CAS-2088185-33-3 | CAS-2088185-84-4 |
| CAS-2088184-22-7 | CAS-2088184-75-0 | CAS-2088185-34-4 | CAS-2088185-85-5 |
| CAS-2088184-23-8 | CAS-2088184-76-1 | CAS-2088185-35-5 | CAS-2088185-86-6 |
| CAS-2088184-24-9 | CAS-2088184-77-2 | CAS-2088185-36-6 | CAS-2088185-87-7 |
| CAS-2088184-25-0 | CAS-2088184-78-3 | CAS-2088185-37-7 | CAS-2088185-88-8 |
| CAS-2088184-26-1 | CAS-2088184-79-4 | CAS-2088185-38-8 | CAS-2088185-89-9 |
| CAS-2088184-27-2 | CAS-2088184-80-7 | CAS-2088185-39-9 | CAS-2088185-90-2 |
| CAS-2088184-28-3 | CAS-2088184-81-8 | CAS-2088185-40-2 | CAS-2088185-91-3 |
| CAS-2088184-29-4 | CAS-2088184-82-9 | CAS-2088185-41-3 | CAS-2088185-92-4 |
| CAS-2088184-30-7 | CAS-2088184-83-0 | CAS-2088185-42-4 | CAS-2088185-93-5 |
| CAS-2088184-32-9 | CAS-2088184-84-1 | CAS-2088185-43-5 | CAS-2088185-94-6 |
| CAS-2088184-34-1 | CAS-2088184-85-2 | CAS-2088185-44-6 | CAS-2088185-95-7 |
| CAS-2088184-35-2 | CAS-2088184-86-3 | CAS-2088185-45-7 | CAS-2088185-96-8 |
| CAS-2088184-36-3 | CAS-2088184-87-4 | CAS-2088185-46-8 | CAS-2088185-97-9 |
| CAS-2088184-37-4 | CAS-2088184-88-5 | CAS-2088185-47-9 | CAS-2088185-98-0 |
| CAS-2088184-38-5 | CAS-2088184-89-6 | CAS-2088185-48-0 | CAS-2088185-99-1 |
| CAS-2088184-39-6 | CAS-2088184-90-9 | CAS-2088185-49-1 | CAS-2088186-00-7 |

(fortgesetzt)

| | | | |
|---|---|---|---|
| CAS-2088184-40-9 | CAS-2088184-91-0 | CAS-2088185-50-4 | CAS-2088186-01-8 |
| CAS-2088184-41-0 | CAS-2088184-92-1 | CAS-2088185-51-5 | CAS-2088186-02-9 |
| CAS-2088184-42-1 | CAS-2088184-93-2 | CAS-2088185-52-6 | CAS-2088195-88-2 |
| CAS-2088184-43-2 | CAS-2088184-94-3 | CAS-2088185-53-7 | CAS-2088195-89-3 |
| CAS-2088184-44-3 | CAS-2088184-95-4 | CAS-2088185-54-8 | CAS-2088195-90-6 |
| CAS-2088184-45-4 | CAS-2088184-96-5 | CAS-2088185-55-9 | CAS-2088195-91-7 |
| CAS-2088184-46-5 | CAS-2088184-97-6 | CAS-2088185-56-0 | CAS-861806-70-4 |
| CAS-2088184-47-6 | CAS-2088184-98-7 | CAS-2088185-57-1 | CAS-1269508-30-6 |
| CAS-2088184-48-7 | CAS-2088184-99-8 | CAS-2088185-58-2 | |
| CAS-2088184-49-8 | CAS-2088185-00-4 | CAS-2088185-59-3 | |
| CAS-2088184-50-1 | CAS-2088185-01-5 | CAS-2088185-60-6 | |
| CAS-2088184-51-2 | CAS-2088185-02-6 | CAS-2088185-61-7 | |
| CAS-2088184-52-3 | CAS-2088185-03-7 | CAS-2088185-62-8 | |
| CAS-2088184-53-4 | CAS-2088185-04-8 | CAS-2088185-63-9 | |
| CAS-2088184-54-5 | CAS-2088185-05-9 | CAS-2088185-64-0 | |
| CAS-2088184-55-6 | CAS-2088185-06-0 | CAS-2088185-65-1 | |

In den erfindungsgemäßen Mischungen oder in der lichtemittierenden Schicht der erfindungsgemäßen Vorrichtung wird bevorzugt jede Mischung M1, M2, M3, M4, M5, M6, M7, M8, M9, M10, M11, M12, M13, M14, M15, M16, M17, M18, M19, M20, M21, M22, M23, M24, M25, M26, M27, M28, M29, M30, M31, M32, M33, M34, M35, M36, M37, M38, M39, M40, M41, M42, M43, M44, M45, M46, M47, M48, M49, M50, M51, M52, M53, M54, M55, M56, M57, M58, M59, M60, M61, M62, M63, M64, M65, M66, M67, M68, M69, M70, M71, M72, M73, M74, M75, M76, M77, M78, M79, M80, M81, M82, M83, M84, M85, M86, M87, M88, M89, M90, M91, M92, M93, M94, M95, M96, M97, M98, M99, M100, M101, M102, M103, M104, M105, M106, M107, M108, M109, M110, M111, M112, M113, M114, M115, M116, M117, M118, M119, M120, M121, M122, M123, M124, M125, M126, M127, M128, M129, M130, M131, M132, M133, M134, M135, M136, M137, M138, M139, M140, M141, M142, M143, M144, M145, M146, M147, M148, M149, M150, M151, M152, M153, M154, M155, M156, M157, M158, M159, M160, M161, M162, M163, M164, M165, M166, M167, M168, M169, M170, M171, M172, M173, M174, M175, M176, M177, M178, M179, M180, M181, M182, M183, M184, M185, M186, M187, M188, M189, M190, M191, M192, M193, M194, M195, M196, M197, M198, M199, M200, M201, M202, M203, M204, M205, M206, M207, M208, M209, M210, M211, M212, M213, M214, M215, M216, M217, M218, M219, M220, M221, M222, M223, M224, M225, M226, M227, M228, M229, M230, M231, M232, M233, M234, M235, M236, M237, M238, M239, M240, M241, M242, M243, M244, M245, M246, M247, M248, M249, M250, M251, M252, M253, M254, M255, M256, M257, M258, M259, M260, M261, M262, M263, M264, M265, M266, M267, M268, M269, M270, M271, M272, M273, M274, M275, M276, M277, M278, M279, M280, M281, M282, M283, M284, M285, M286, M287, M288, M289, M290, M291, M292, M293, M294, M295, M296, M297, M298, M299, M300, M301, M302, M303, M304, M305, M306, M307, M308, M309, M310, M311, M312, M313, M314, M315, M316, M317, M318, M319, M320, M321, M322, M323, M324, M325, M326, M327, M328, M329, M330, M331, M332, M333, M334, M335, M336, M337, M338, M339, M340, M341, M342, M343, M344, M345, M346, M347, M348, M349, M350, M351, M352, M353, M354, M355, M356, M357, M358, M359, M360, M361, M362, M363, M364, M365, M366, M367, M368, M369, M370, M371, M372, M373, M374, M375, M376, M377, M378, M379, M380, M381, M382, M383, M384, M385, M386, M387, M388, M389, M390, M391, M392, M393, M394, M395, M396, M397, M398, M399, M400, M401, M402, M403, M404, M405, M406, M407, M408, M409, M410, M411, M412, M413, M414, M415, M416, M417, M418, M419, M420, M421, M422, M423, M424, M425, M426, M427, M428, M429, M430, M431, M432, M433, M434, M435, M436, M437, M438, M439, M440, M441, M442, M443, M444, M445, M446, M447, M448, M449, M450, M451, M452, M453, M454, M455, M456, M457, M458, M459, M460, M461, M462, M463, M464, M465, M466, M467, M468, M469, M470, M471, M472, M473, M474, M475, M476, M477, M478, M479, M480, M481, M482, M483, M484, M485, M486, M487, M488, M489, M490, M491, M492, M493, M494, M495, M496, M497, M498, M499, M500, M501, M502, M503, M504, M505, M506, M507, M508, M509, M510, M511, M512, M513, M514, M515, M516, M517, M518, M519, M520, M521, M522, M523, M524, M525, M526, M527, M528, M529, M530, M531, M532, M533, M534, M535, M536, M537, M538, M539, M540, M541, M542,

M543, M544, M545, M546, M547, M548, M549, M550, M551, M552, M553, M554, M555, M556, M557, M558, M559, M560, M561, M562, M563, M564, M565, M566, M567, M568, M569, M570, M571, M572, M573, M574, M575, M576, M577, M578, M579, M580, M581, M582, M583, M584, M585, M586, M587, M588, M589, M590, M591, M592, M593, M594, M595, M596, M597, M598, M599, M600, M601, M602, M603, M604, M605, M606, M607, M608, M609, M610, M611, M612, M613, M614, M615, M616, M617, M618, M619, M620, M621, M622, M623, M624, M625, M626, M627, M628, M629, M630, M631, M632, M633, M634, M635, M636, M637, M638, M639, M640, M641, M642, M643, M644, M645, M646, M647, M648, M649, M650, M651, M652, M653, M654, M655, M656, M657, M658, M659, M660, M661, M662, M663, M664, M665, M666, M667, M668, M669, M670, M671, M672, M673, M674, M675, M676, M677, M678, M679, M680, M681, M682, M683, M684, M685, M686, M687, M688, M689, M690, M691, M692, M693, M694, M695, M696, M697, M698, M699, M700, M701, M702, M703, M704, M705, M706, M707, M708, M709, M710, M711, M712, M713, M714, M715, M716, M717, M718, M719, M720, M721, M722, M723, M724, M725, M726, M727, M728, M729, M730, M731, M732, M733, M734, M735, M736, M737, M738, M739, M740, M741, M742, M743, M744, M745, M746, M747, M748, M749, M750, M751, M752, M753, M754, M755, M756, M757, M758, M759, M760, M761, M762, M763, M764, M765, M766, M767, M768, M769, M770, M771, M772, M773, M774, M775, M776, M777, M778, M779, M780, M781, M782, M783, M784, M785, M786, M787, M788, M789, M790, M791, M792, M793, M794, M795, M796, M797, M798, M799, M800, M801, M802, M803, M804, M805, M806, M807, M808, M809, M810, M811, M812, M813, M814, M815, M816, M817, M818, M819, M820, M821, M822, M823, M824, M825, M826, M827, M828, M829, M830, M831, M832, M833, M834, M835, M836, M837, M838, M839, M840, M841, M842, M843, M844, M845, M846, M847, M848, M849, M850, M851, M852, M853, M854, M855, M856, M857, M858, M859, M860, M861, M862, M863, M864, M865, M866, M867, M868, M869, M870, M871, M872, M873, M874, M875, M876, M877, M878, M879, M880, M881, M882, M883, M884, M885, M886, M887, M888, M889, M890, M891, M892, M893, M894, M895, M896, M897, M898, M899, M900, M901, M902, M903, M904, M905, M906, M907, M908, M909, M910, M911, M912, M913, M914, M915, M916, M917, M918, M919, M920, M921, M922, M923, M924, M925, M926, M927, M928, M929, M930, M931, M932, M933, M934, M935, M936, M937, M938, M939, M940, M941, M942, M943, M944, M945, M946, M947, M948, M949, M950, M951, M952, M953, M954, M955, M956, M957, M958, M959, M960, M961, M962, M963, M964, M965, M966, M967, M968, M969, M970, M971, M972, M973, M974, M975, M976, M977, M978, M979, M980, M981, M982, M983, M984, M985, M986, M987, M988, M989, M990, M991, M992, M993, M994, M995, M996, M997, M998, M999, M1000, M1001, M1002, M1003, M1004, M1005, M1006, M1007, M1008 mit einer Verbindung der Formel (III) oder einer Verbindung der Formeln (Ia), (IIa), (IIIa), (IVa), (Va), (VIa) oder einer Verbindung aus Tabelle 6 oder 7 kombiniert.

Die lichtemittierende Schicht in der erfindungsgemäßen organischen elektrolumineszierenden Vorrichtung enthaltend mindestens einen phosphoreszierenden Emitter ist bevorzugt eine infra-rot emittierende, gelb, orange, rot, grün, blau oder ultra-violett emittierende Schicht, besonders bevorzugt eine gelb oder grün emittierende Schicht und ganz besonders bevorzugt eine grün emittierende Schicht.

Dabei wird unter einer gelb emittierenden Schicht eine Schicht verstanden, deren Photolumineszenzmaximum im Bereich von 540 bis 570 nm liegt. Unter einer orange emittierenden Schicht wird eine Schicht verstanden, deren Photolumineszenzmaximum im Bereich von 570 bis 600 nm liegt. Unter einer rot emittierenden Schicht wird eine Schicht verstanden, deren Photolumineszenzmaximum im Bereich von 600 bis 750 nm liegt. Unter einer grün emittierenden Schicht wird eine Schicht verstanden, deren Photolumineszenzmaximum im Bereich von 490 bis 540 nm liegt. Unter einer blau emittierenden Schicht wird eine Schicht verstanden, deren Photolumineszenzmaximum im Bereich von 440 bis 490 nm liegt. Dabei wird das Photolumineszenzmaximum der Schicht durch Messung des Photolumineszenzspektrums der Schicht mit einer Schichtdicke von 50 nm bei Raumtemperatur bestimmt, wobei die Schicht die erfindungsgemäße Kombination der Hostmaterialien der Formeln (1) und (2) und den entsprechenden Emitter enthält.

Die Aufnahme des Photolumineszenzspektrums der Schicht erfolgt beispielsweise mit einem handelsüblichen Photolumineszenzspektrometer.

Das Photolumineszenzspektrum des gewählten Emitters wird in der Regel in Sauerstoff-freier Lösung, 10-5 molar, gemessen, wobei die Messung bei Raumtemperatur erfolgt und jedes Lösemittel geeignet ist, in dem sich der gewählte Emitter in der genannten Konzentration löst. Besonders geeignete Lösemittel sind üblicherweise Toluol oder 2-Methyl-THF, aber auch Dichlormethan. Gemessen wird mit einem handelsüblichen Photolumineszenzspektrometer. Die Triplettenergie T1 in eV wird aus den Photolumineszenzspektren der Emitter bestimmt. Es wird zunächst das Peakmaximum Plmax. (in nm) des Photolumineszenzspektrums bestimmt. Das Peakmaximum Plmax. (in nm) wird dann in eV umgerechnet gemäß: E(TI in eV) = 1240 / E(T1 in nm) = 1240 / PLmax. (in nm).

Bevorzugte phosphoreszierende Emitter sind demzufolge infra-rote Emitter, vorzugsweise der Formel (III), (Ia), (IIa), (IIIa), (IVa), (Va), (VIa) oder aus Tabelle 6 oder 7, deren Triplettenergie $T_1$ bevorzugt bei ~1.9 eV bis ~1.0 eV liegt.

Bevorzugte phosphoreszierende Emitter sind demzufolge rote Emitter, vorzugsweise der Formel (III), (Ia), (IIa), (IIIa), (IVa), (Va), (VIa) oder aus Tabelle 6 oder 7, deren Triplettenergie $T_1$ bevorzugt bei ~2.1 eV bis ~1.9 eV liegt.

Bevorzugte phosphoreszierende Emitter sind demzufolge gelbe Emitter, vorzugsweise der Formel (III), (Ia), (IIa), (IIIa), (IVa), (Va), (VIa) oder aus Tabelle 6 oder 7, deren Triplettenergie $T_1$ bevorzugt bei ~2.3 eV bis ~2.1 eV liegt.

Bevorzugte phosphoreszierende Emitter sind demzufolge grüne Emitter, vorzugsweise der Formel (III), (Ia),

(IIa), (IIIa), (IVa), (Va), (VIa) oder aus Tabelle 6 oder 7, deren Triplettenergie $T_1$ bevorzugt bei ~2.5 eV bis ~2.3 eV liegt.

Bevorzugte phosphoreszierende Emitter sind demzufolge blaue Emitter, vorzugsweise der Formel (III), (Ia), (IIa), (IIIa), (IVa), (Va), (VIa) oder aus Tabelle 6 oder 7, deren Triplettenergie $T_1$ bevorzugt bei ~3.1 eV bis ~2.5 eV liegt.

Bevorzugte phosphoreszierende Emitter sind demzufolge ultra-violette Emitter, der Formel (III), (Ia), (IIa), (IIIa), (IVa), (Va), (VIa) oder aus Tabelle 6 oder 7, deren Triplettenergie $T_1$ bevorzugt bei ~4.0 eV bis ~3.1 eV liegt.

Besonders bevorzugte phosphoreszierende Emitter sind demzufolge grüne oder gelbe Emitter, vorzugsweise der Formel (III), (Ia), (IIa), (IIIa), (IVa), (Va), (VIa) oder aus Tabelle 6 oder 7, wie zuvor beschrieben.

Ganz besonders bevorzugte phosphoreszierende Emitter sind demzufolge grüne Emitter, vorzugsweise der Formel (III), (Ia), (IIa), (IIIa), (IVa), (Va), (VIa) oder aus Tabelle 6 oder 7, deren Triplettenergie $T_1$ bevorzugt bei ~2.5 eV bis ~2.3 eV liegt.

Ganz besonders bevorzugt werden grüne Emitter, vorzugsweise der Formel (III) oder aus Tabelle 6 oder 7, wie zuvor beschrieben, für die erfindungsgemäße Zusammensetzung bzw. erfindungsgemäße emittierende Schicht ausgewählt.

In der lichtemittierenden Schicht der erfindungsgemäßen Vorrichtung können auch fluoreszierende Emitter enthalten sein.

Bevorzugte fluoreszierende Emitter sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

In einer weiteren bevorzugten Ausführungsform der Erfindung kann die mindestens eine lichtemittierende Schicht der organischen elektrolumineszierenden Vorrichtung neben den Hostmaterialien 1 und 2, wie zuvor beschrieben oder als bevorzugt beschrieben, weitere Hostmaterialien oder Matrixmaterialien umfassen, sogenannte Mixed-Matrix-Systeme. Die Mixed-Matrix-Systeme umfassen bevorzugt drei oder vier verschiedene Matrixmaterialien, besonders bevorzugt drei verschiedene Matrixmaterialien (das heißt, eine weitere Matrixkomponente zusätzlich zu den Hostmaterialien 1 und 2, wie zuvor beschrieben). Besonders geeignete Matrixmaterialien, welche in Kombination als Matrixkomponente eines Mixed-Matrix-Systems verwendet werden können, sind ausgewählt aus wide-band-gap-Materialien, bipolaren Hostmaterialien, Elektronentransportmaterialien (ETM) und Lochtransportmaterialien (HTM).

Unter wide-band-gap-Material wird hierin ein Material im Sinne der Offenbarung von US 7,294,849 verstanden, das durch eine Bandlücke von mindestens 3.5 eV charakterisiert ist, wobei unter Bandlücke der Abstand zwischen HOMO und LUMO-Energie eines Materials verstanden wird.

Gemäß einer Ausführungsform der vorliegenden Erfindung enthält die Mischung neben den Bestandteilen elektronentransportierendes Hostmaterial der Formel (1) und lochtransportierendes Hostmaterial der Formel (2) keine weiteren Bestandteile, das heißt, funktionellen Materialien. Es handelt sich um Materialmischungen, die als solches zur Herstellung der lichtemittierenden Schicht, verwendet werden. Man bezeichnet diese Mischungen auch als Premix-Systeme, die als einzige Materialquelle bei der Aufdampfung der Hostmaterialien für die lichtemittierende Schicht verwendet wird und die ein konstantes Mischungsverhältnis bei der Aufdampfung haben. Dadurch lässt sich auf einfache und schnelle Art und Weise das Aufdampfen einer Schicht mit gleichmäßiger Verteilung der Komponenten erreichen, ohne dass eine präzise Ansteuerung einer Vielzahl an Materialquellen notwendig ist.

Gemäß einer alternativen Ausführungsform der vorliegenden Erfindung enthält die Mischung neben den Bestandteilen elektronentransportierendes Hostmaterial der Formel (1) und lochtransportierendes Hostmaterial der Formel (2) noch den phosphoreszierenden Emitter, wie zuvor beschrieben. Bei geeignetem Mischungsverhältnis bei der Aufdampfung kann auch diese Mischung als einzige Materialquelle verwendet werden, wie zuvor beschrieben.

Die Komponenten bzw. Bestandteile der lichtemittierenden Schicht der erfindungsgemäßen Vorrichtung können somit durch Aufdampfen oder aus Lösung prozessiert werden. Die Materialkombination der Hostmaterialien 1 und 2, wie zuvor beschrieben oder bevorzugt beschrieben, gegebenenfalls mit dem phosphoreszierenden Emitter, wie zuvor beschrieben oder bevorzugt beschrieben, werden dazu in einer Formulierung bereitgestellt, die mindestens ein Lösemittel enthält. Diese Formulierungen können beispielsweise Lösungen, Dispersionen oder Emulsionen sein. Es kann bevorzugt

sein, hierfür Mischungen aus zwei oder mehr Lösemitteln zu verwenden.

Ein weiterer Gegenstand der vorliegenden Erfindung ist daher eine Formulierung enthaltend eine erfindungsgemäße Mischung an Hostmaterialien 1 und 2, wie zuvor beschrieben, gegebenenfalls in Kombination mit einem phosphoreszierenden Emitter, wie zuvor beschrieben oder bevorzugt beschrieben, und mindestens ein Lösemittel.

Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Die Formulierung kann dabei auch mindestens eine weitere organische oder anorganische Verbindung enthalten, die ebenfalls in der lichtemittierenden Schicht der erfindungsgemäßen Vorrichtung eingesetzt wird, insbesondere eine weitere emittierende Verbindung und/oder ein weiteres Matrixmaterial.

Die lichtemittierende Schicht in der erfindungsgemäßen Vorrichtung gemäß den bevorzugten Ausführungsformen und der emittierenden Verbindung enthält vorzugsweise zwischen 99,9 und 1 Vol.-%, weiter vorzugsweise zwischen 99 und 10 Vol.-%, besonders bevorzugt zwischen 98 und 60 Vol.-%, ganz besonders bevorzugt zwischen 97 und 80 Vol.-% an Matrixmaterial aus mindestens einer Verbindung der Formel (1) und mindestens einer Verbindung der Formel (2) gemäß den bevorzugten Ausführungsformen, bezogen auf die gesamte Zusammensetzung aus Emitter und Matrixmaterial. Entsprechend enthält die lichtemittierende Schicht in der erfindungsgemäßen Vorrichtung vorzugsweise zwischen 0,1 und 99 Vol.-%, weiter vorzugsweise zwischen 1 und 90 Vol.-%, besonders bevorzugt zwischen 2 und 40 Vol.-%, ganz besonders bevorzugt zwischen 3 und 20 Vol.-% des Emitters bezogen auf die gesamte Zusammensetzung der aus Emitter und Matrixmaterial bestehenden lichtemittierenden Schicht. Werden die Verbindungen aus Lösung verarbeitet, so werden statt der oben angegebenen Mengen in Vol.-% bevorzugt die entsprechenden Mengen in Gew.-% verwendet.

Die lichtemittierende Schicht in der erfindungsgemäßen Vorrichtung gemäß den bevorzugten Ausführungsformen und der emittierenden Verbindung enthält vorzugsweise das Matrixmaterial der Formel (1) und das Matrixmaterial der Formel (2) in einem Volumenprozentverhältnis zwischen 3:1 und 1:3, bevorzugt zwischen 1:2.5 und 1:1, besonders bevorzugt zwischen 1:2 und 1:1. Werden die Verbindungen aus Lösung verarbeitet, so werden statt des oben angegebenen Verhältnisses in Vol.-% bevorzugt das entsprechende Verhältnis in Gew.-% verwendet.

Die Abfolge der Schichten in der erfindungsgemäßen organischen Elektrolumineszenzvorrichtung ist bevorzugt die folgende:
Anode / Lochinjektionsschicht / Lochtransportschicht / emittierende Schicht / Elektronentransportschicht / Elektroneninjektionsschicht / Kathode.

Diese Abfolge der Schichten ist eine bevorzugte Abfolge.

Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Mindestens eine der emittierenden Schichten ist die erfindungsgemäße lichtemittierende Schicht enthaltend mindestens eine Verbindung der Formel (1) als Hostmaterial 1 und mindestens eine Verbindung der Formel (2) als Hostmaterial 2, wie zuvor beschrieben. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B.

WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß

dem Stand der Technik als Elektronentransportmaterialien in der Elektronentransportschicht verwendet werden. Insbesondere eignen sich Aluminiumkomplexe, beispielsweise $Alq_3$, Zirkoniumkomplexe, beispielsweise $Zrq_4$, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Lochtransportmaterialien sind insbesondere Materialien bevorzugt, die in einer Lochtransport-, Lochinjektions- oder Elektronenblockierschicht verwendet werden können, wie Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß WO 2014/015937, WO 2014/015938 und WO 2014/015935), Spiro-Dibenzopyran-Amine (z. B. gemäß WO 2013/083216) und Dihydroacridin-Derivate (z. B. WO 2012/150001).

Als Kathode der erfindungsgemäßen Vorrichtung eignen sich Metalle mit geringer Austrittsarbeit, Metalllegierungen oder mehrlagige Strukturen aus verschiedenen Metallen, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die erfindungsgemäße organische elektrolumineszierende Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

Die Herstellung der erfindungsgemäßen Vorrichtung ist hierbei nicht eingeschränkt. Es ist möglich, dass eine oder mehrere organische Schichten, auch die lichtemittierende Schicht, mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist die erfindungsgemäße organische Elektrolumineszenzvorrichtung dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist die erfindungsgemäße organische Elektrolumineszenzvorrichtung dadurch gekennzeichnet, dass eine oder mehrere organische Schichten enthaltend die erfindungsgemäße Zusammensetzung aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Hostmaterialien 1 und 2 und phosphoreszierende Emitter nötig. Das Verarbeiten aus Lösung hat den Vorteil, dass beispielsweise die lichtemittierende Schicht sehr einfach und kostengünstig aufgebracht werden kann. Diese Technik eignet sich insbesondere für die Massenproduktion organischer elektrolumineszierender Vorrichtungen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können auf organische Elektrolumineszenzvorrichtungen angewandt werden.

Ein weiterer Gegenstand der Erfindung ist daher ein Verfahren zur Herstellung der erfindungsgemäßen organischen elektrolumineszierenden Vorrichtung, wie zuvor beschrieben oder bevorzugt beschrieben, dadurch gekennzeichnet, dass die lichtemittierende Schicht durch Gasphasenabscheidung, insbesondere mit einem Sublimationsverfahren und/oder mit einem OVPD (Organic Vapour Phase Deposition) Verfahren und/oder mit Hilfe einer Trägergassublimation, oder aus Lösung, insbesondere durch Spincoating oder mit einem Druckverfahren, aufgebracht wird.

Bei der Herstellung mittels Gasphasenabscheidung bestehen grundsätzlich zwei Möglichkeiten, wie die erfindungsgemäße lichtemittierende Schicht, auf ein beliebiges Substrat bzw. die vorherige Schicht aufgebracht bzw. aufgedampft werden kann. Zum einen können die verwendeten Materialien jeweils in einer Materialquelle vorgelegt und schließlich aus den verschiedenen Materialquellen verdampft werden ("co-evaporation"). Zum anderen können die verschiedenen Materialien vorgemischt ("premixed", Premix-Systeme) und das Gemisch in einer einzigen Materialquelle vorgelegt werden, aus der es schließlich verdampft wird ("premixevaporation"). Dadurch lässt sich auf einfache und schnelle Art und Weise das Aufdampfen der lichtemittierenden Schicht mit gleichmäßiger Verteilung der Komponenten erreichen, ohne dass eine präzise Ansteuerung einer Vielzahl an Materialquellen notwendig ist.

Ein weiterer Gegenstand der Erfindung ist demzufolge ein Verfahren zur Herstellung der erfindungsgemäßen Vorrichtung, dadurch gekennzeichnet, dass die mindestens eine Verbindung der Formel (1), wie zuvor beschrieben oder als bevorzugt beschrieben, und die mindestens eine Verbindung der Formel (2), wie zuvor beschrieben oder als bevorzugt beschrieben, nacheinander oder gleichzeitig aus mindestens zwei Materialquellen, gegebenenfalls mit dem mindestens einen phosphoreszierenden Emitter, wie zuvor beschrieben oder bevorzugt beschrieben, aus der Gasphase abgeschieden werden und die lichtemittierende Schicht bilden.

In einer bevorzugten Ausführungsform der vorliegenden Erfindung wird die lichtemittierende Schicht mittels Gasphasenabscheidung aufgebracht, wobei die Bestandeile der Zusammensetzung vorgemischt und aus einer einzigen Materialquelle verdampft werden.

Ein weiterer Gegenstand der Erfindung ist demzufolge ein Verfahren zur Herstellung der erfindungsgemäßen Vorrichtung, dadurch gekennzeichnet, dass die mindestens eine Verbindung der Formel (1) und die mindestens eine Verbindung der Formel (2) als Mischung, nacheinander oder gleichzeitig mit dem mindestens einen phosphoreszierenden Emitter, aus der Gasphase abgeschieden werden und die lichtemittierende Schicht bilden.

Ein weiterer Gegenstand der Erfindung ist ein Verfahren zur Herstellung der erfindungsgemäßen Vorrichtung, wie zuvor beschrieben oder bevorzugt beschrieben, dadurch gekennzeichnet, dass die mindestens eine Verbindung der Formel (1) und die mindestens eine Verbindung der Formel (2), wie zuvor beschrieben oder bevorzugt beschrieben, zusammen mit dem mindestens einen phosphoreszierenden Emitter, aus Lösung aufgebracht werden, um die lichtemittierende Schicht zu bilden.

Die erfindungsgemäßen Vorrichtungen zeichnen sich durch folgende überraschende Vorteile gegenüber dem Stand der Technik aus:
Die Verwendung der beschriebenen Materialkombination der Hostmaterialien 1 und 2, wie zuvor beschrieben, führt insbesondere zu einer Steigerung der Lebensdauer der Vorrichtungen.

Wie im nachfolgend angegebenen Beispiel ersichtlich, lässt sich durch Vergleich der Daten für OLEDs mit Kombinationen aus dem Stand der Technik feststellen, dass die erfindungsgemäßen Kombinationen an Matrixmaterialien in der EML zu Vorrichtungen führen, deren Lebensdauer um ca. 20 bis 70% erhöht sind, unabhängig von der Emitterkonzentration.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbarte Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn, dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Die mit der vorliegenden Erfindung offengelegte Lehre zum technischen Handeln kann abstrahiert und mit anderen Beispielen kombiniert werden. Die Erfindung wird durch die nachfolgenden Beispiele näher erläutert, ohne sie dadurch einschränken zu wollen.

Allgemeine Methoden:

In allen quantenchemischen Berechnungen wird das Programmpaket Gaussian16 (Rev. B.01) verwendet. Der neutrale Singulettgrundzustand wird auf dem B3LYP/6-31G(d)-Niveau optimiert. HOMO- und LUMO-Werte werden auf dem B3LYP/6-31G(d)-Niveau für die mit B3LYP/6-31G(d) optimierte Grundzustandsenergie bestimmt. Daraufhin werden TD-DFT-Singulett- und Triplettanregungen (vertikale Anregungen) mit der gleichen Methode (B3LYP/6-31G(d)) und der optimierten Grundzustandsgeometrie berechnet. Die Standardeinstellungen für SCF- und Gradientenkonvergenz werden verwendet.

Aus der Energierechnung erhält man das HOMO als das letzte mit zwei Elektronen besetze Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht. Daraus wird der anhand von Cyclovoltammetriemessungen kalibrierte HOMO- und LUMO-Wert in Elektronenvolt wie folgt bestimmt:

$$\text{HOMOcorr} = 0.90603 * \text{HOMO} - 0.84836$$

$$\text{LUMOcorr} = 0.99687 * \text{LUMO} - 0.72445$$

Das Triplett-Niveau T1 eines Materials ist definiert als die relative Anregungsenergie (in eV) des Triplettzustands mit der niedrigsten Energie, der sich aus der quantenchemischen Energierechnung ergibt.

Das Singulett-Niveau S1 eines Materials ist definiert als die relative Anregungsenergie (in eV) des Singulettzustands mit der zweitniedrigsten Energie, der sich aus der quantenchemischen Energierechnung ergibt. Der energetisch niedrigste Singulettzustand wird als S0 bezeichnet.

Die hierin beschriebene Methode ist unabhängig von dem verwendeten Softwarepaket und liefert immer dieselben Ergebnisse. Beispiele oft benutzter Programme für diesen Zweck sind "Gaussian09" (Gaussian Inc.) und Q-Chem 4.1 (Q-Chem, Inc.). Vorliegend wird zur Berechnung der Energien das Programmpaket "Gaussian16 (Rev. B.01)" verwendet.

Beispiel 1: **Herstellung der OLEDs**

In den folgenden Beispielen (siehe Tabellen 8 bis 10) wird der Einsatz der erfindungsgemäßen Materialkombinationen in OLEDs in Vergleich zu Materialkombinationen aus dem Stand der Technik vorgestellt.

**Vorbehandlung für die Beispiele V1 bis V6 und E1a bis E6f:**

Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 8 zu entnehmen. Die zur Herstellung der OLEDs benötigten Materialien, sofern sie nicht schon zuvor beschrieben wurden, sind in Tabelle 10 gezeigt. Die Device Daten der OLEDs sind in Tabelle 9 aufgelistet.

Die Beispiele V1 und V6 sind Vergleichsbeispiele der Hostmaterialien E40 bis E45, die aus WO19007866 bekannt sind, mit einem Biscarbazol als lochtransportierenden Host gemäß Stand der Technik, beispielsweise WO 19007866.

Die Beispiele E1a bis E6f zeigen Daten von erfindungsgemäßen OLEDs.

Alle Materialien werden in einer Vakuumkammer thermisch aufgedampft. Dabei besteht die Emissionsschicht immer aus mindestens zwei Matrixmaterialien und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie E40:BCbz1:TE2 (32%:60%:8%) bedeutet hierbei, dass das Material E40 in einem Volumenanteil von 32%, BCbz1 in einem Anteil von 60% und TE2 in einem Anteil von 8% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren und Strom-Spannungs-Leuchtdichte-Kennlinien (IUL-Kennlinien) gemessen. EQE und die Stromeffizienz SE (in cd/A) werden daraus berechnet. Die Berechnung der SE erfolgt unter Annahme einer lambertschen Abstrahlcharakteristik.

Die Elektrolumineszenzspektren werden bei einer Leuchtdichte von 1000 cd/m$^2$ bestimmt und daraus die CIE 1931 x und y Farbkoordinaten berechnet. Die Angabe U10 in Tabelle 2 bezeichnet die Spannung, die für eine Stromdichte von 10 mA/cm$^2$ benötigt wird. SE10 und EQE10 bezeichnen die Stromeffizienz bzw. die externe Quanteneffizienz, die bei 10 mA/cm$^2$ erreicht werden.

Als Lebensdauer LD wird die Zeit definiert, nach der die Leuchtdichte gemessen in cd/m$^2$ in Vorwärtsrichtung, bei Betrieb mit konstanter Stromdichte $j_0$ von der Startleuchtdichte auf einen gewissen Anteil L1 absinkt. Eine Angabe L1=80% in Tabelle 9 bedeutet, dass die in Spalte LD angegebene Lebensdauer der Zeit entspricht, nach der die Leuchtdichte in cd/m$^2$ auf 80% ihres Anfangswertes absinkt.

**Verwendung** von **erfindungsgemäßen Mischungen in OLEDs**

Die erfindungsgemäßen Materialkombinationen werden in den Beispielen E1a-i, E2a-f, E3a-f, E4a-f, E5a-g, E6a-f als Matrixmaterialen in der Emissionsschicht von grün phosphoreszierenden OLEDs eingesetzt. Als Vergleich zum Stand der Technik kommen die Materialien E40 bis E45 mit BCbz1 bis BCbz3 in den Beispielen V1 bis V6 zum Einsatz.

Beim Vergleich der erfindungsgemäßen Beispiele mit den entsprechenden Vergleichsbeispielen, ist deutlich ersichtlich, dass die erfindungsgemäßen Beispiele jeweils einen deutlichen Vorteil in der Device Lebensdauer aufzeigen, bei ansonsten vergleichbaren Leistungsdaten der OLED.

Tabelle 8: Aufbau der OLEDs

| Bsp | HIL Dicke | HTL Dicke | EBL Dicke | EML Dicke | HBL Dicke | ETL Dicke | EIL Dicke |
|---|---|---|---|---|---|---|---|
| V1 | SpMA1:PD1 (95%:5%) 20nm | SpMA1 200nm | SpMA2 20nm | E40:BCbz1:TE2 (32%: 60%:8%) 40nm | ST2 5nm | ST2:LiQ (50%:50%) 30nm | LiQ 1nm |
| E1a | SpMA1:PD1 (95%:5%) 20nm | SpMA1 200nm | SpMA2 20nm | E40:H3:TE2 (32%:60%: 8%) 40nm | ST2 5nm | ST2:LiQ (50%:50%) 30nm | LiQ 1nm |
| E1b | SpMA1:PD1 (95%:5%) 20nm | SpMA1 200nm | SpMA2 20nm | E40:H7:TE2 (32%:60%: 8%) 40nm | ST2 5nm | ST2:LiQ (50%:50%) 30nm | LiQ 1nm |
| E1c | SpMA1:PD1 (95%:5%) 20nm | SpMA1 200nm | SpMA2 20nm | E40:H12:TE2 (32%:60%: 8%) 40nm | ST2 5nm | ST2:LiQ (50%:50%) 30nm | LiQ 1nm |
| E1d | SpMA1:PD1 (95%:5%) 20nm | SpMA1 200nm | SpMA2 20nm | E1:H13:TE2 (32%:60%: 8%) 40nm | ST2 5nm | ST2:LiQ (50%:50%) 30nm | LiQ 1nm |
| E1e | SpMA1:PD1 (95%:5%) 20nm | SpMA1 200nm | SpMA2 20nm | E3:H18:TE2 (32%:60%: 8%) 40nm | ST2 5nm | ST2:LiQ (50%:50%) 30nm | LiQ 1nm |
| E1f | SpMA1:PD1 (95%:5%) 20nm | SpMA1 200nm | SpMA2 20nm | E10:H16:TE2 (32%:60%: 8%) 40nm | ST2 5nm | ST2:LiQ (50%:50%) 30nm | LiQ 1nm |
| E1g | SpMA1:PD1 (95%:5%) 20nm | SpMA1 200nm | SpMA2 20nm | E14:H3:TE2 (32%:60%: 8%) 40nm | ST2 5nm | ST2:LiQ (50%:50%) 30nm | LiQ 1nm |
| E1h | SpMA1:PD1 (95%:5%) 20nm | SpMA1 200nm | SpMA2 20nm | E29:H16:TE2 (32%:60%: 8%) 40nm | ST2 5nm | ST2:LiQ (50%:50%) 30nm | LiQ 1nm |
| E1i | SpMA1:PD1 (95%:5%) 20nm | SpMA1 200nm | SpMA2 20nm | E34:H13:TE2 (32%:60%: 8%) 40nm | ST2 5nm | ST2:LiQ (50%:50%) 30nm | LiQ 1nm |

(fortgesetzt)

| Bsp | HIL Dicke | HTL Dicke | EBL Dicke | EML Dicke | HBL Dicke | ETL Dicke | EIL Dicke |
|---|---|---|---|---|---|---|---|
| | | | | | | | |
| V2 | SpMA1:PD1 (95%:5%) 20nm | SpMA1 200nm | SpMA2 20nm | E41:BCbz1:TE2 (32%:60%:8%) 40nm | ST2 5nm | ST2:LiQ (50%:50%) 30nm | LiQ 1nm |
| E2a | SpMA1:PD1 (95%:5%) 20nm | SpMA1 200nm | SpMA2 20nm | E41:H5:TE2 (32%:60%:8%) 40nm | ST2 5nm | ST2:LiQ (50%:50%) 30nm | LiQ 1nm |
| E2b | SpMA1:PD1 (95%:5%) 20nm | SpMA1 200nm | SpMA2 20nm | E41:H8:TE2 (32%:60%:8%) 40nm | ST2 5nm | ST2:LiQ (50%:50%) 30nm | LiQ 1nm |
| E2c | SpMA1:PD1 (95%:5%) 20nm | SpMA1 200nm | SpMA2 20nm | E41:H10:TE2 (32%:60%:8%) 40nm | ST2 5nm | ST2:LiQ (50%:50%) 30nm | LiQ 1nm |
| E2d | SpMA1:PD1 (95%:5%) 20nm | SpMA1 200nm | SpMA2 20nm | E6:H5:TE2 (32%:60%:8%) 40nm | ST2 5nm | ST2:LiQ (50%:50%) 30nm | LiQ 1nm |
| E2e | SpMA1:PD1 (95%:5%) 20nm | SpMA1 200nm | SpMA2 20nm | E8:H17:TE2 (32%:60%:8%) 40nm | ST2 5nm | ST2:LiQ (50%:50%) 30nm | LiQ 1nm |
| E2f | SpMA1:PD1 (95%:5%) 20nm | SpMA1 200nm | SpMA2 20nm | E48:H17:TE2 (32%:60%:8%) 40nm | ST2 5nm | ST2:LiQ (50%:50%) 30nm | LiQ 1nm |
| | | | | | | | |
| V3 | SpMA1:PD1 (95%:5%) 20nm | SpMA1 200nm | SpMA2 20nm | E42:BCbz2:TE2 (32%:60%:8%) 40nm | ST2 5nm | ST2:LiQ (50%:50%) 30nm | LiQ 1nm |
| E3a | SpMA1:PD1 (95%:5%) 20nm | SpMA1 200nm | SpMA2 20nm | E42:H3:TE2 (32%:60%:8%) 40nm | ST2 5nm | ST2:LiQ (50%:50%) 30nm | LiQ 1nm |
| E3b | SpMA1:PD1 (95%:5%) 20nm | SpMA1 200nm | SpMA2 20nm | E28:H3:TE2 (32%:60%:8%) 40nm | ST2 5nm | ST2:LiQ (50%:50%) 30nm | LiQ 1nm |
| E3c | SpMA1:PD1 (95%:5%) 20nm | SpMA1 200nm | SpMA2 20nm | E26:H5:TE2 (32%:60%:8%) 40nm | ST2 5nm | ST2:LiQ (50%:50%) 30nm | LiQ 1nm |
| E3d | SpMA1:PD1 (95%:5%) 20nm | SpMA1 200nm | SpMA2 20nm | E24:H7:TE2 (32%:60%:8%) 40nm | ST2 5nm | ST2:LiQ (50%:50%) 30nm | LiQ 1nm |
| E3e | SpMA1:PD1 (95%:5%) 20nm | SpMA1 200nm | SpMA2 20nm | E33:H12:TE2 (32%:60%:8%) 40nm | ST2 5nm | ST2:LiQ (50%:50%) 30nm | LiQ 1nm |
| E3f | SpMA1:PD1 (95%:5%) 20nm | SpMA1 200nm | SpMA2 20nm | E46:H7:TE2 (32%:60%:8%) 40nm | ST2 5nm | ST2:LiQ (50%:50%) 30nm | LiQ 1nm |
| | | | | | | | |

(fortgesetzt)

| Bsp | HIL Dicke | HTL Dicke | EBL Dicke | EML Dicke | HBL Dicke | ETL Dicke | EIL Dicke |
|---|---|---|---|---|---|---|---|
| V4 | SpMA1:PD1 (95%:5%) 20nm | SpMA1 200nm | SpMA2 20nm | E43:BCbz2:TE1 (32%: 60%:8%) 40nm | ST2 5nm | ST2:LiQ (50%:50%) 30nm | LiQ 1nm |
| E4a | SpMA1:PD1 (95%:5%) 20nm | SpMA1 200nm | SpMA2 20nm | E43:H5:TE1 (32%:60%: 8%) 40nm | ST2 5nm | ST2:LiQ (50%:50%) 30nm | LiQ 1nm |
| E4b | SpMA1:PD1 (95%:5%) 20nm | SpMA1 200nm | SpMA2 20nm | E4:H7:TE1 (32%:60%:8%) 40nm | ST2 5nm | ST2:LiQ (50%:50%) 30nm | LiQ 1nm |
| E4c | SpMA1:PD1 (95%:5%) 20nm | SpMA1 200nm | SpMA2 20nm | E20:H8:TE1 (32%:60%: 8%) 40nm | ST2 5nm | ST2:LiQ (50%:50%) 30nm | LiQ 1nm |
| E4d | SpMA1:PD1 (95%:5%) 20nm | SpMA1 200nm | SpMA2 20nm | E19:H5:TE1 (32%:60%: 8%) 40nm | ST2 5nm | ST2:LiQ (50%:50%) 30nm | LiQ 1nm |
| E4e | SpMA1:PD1 (95%:5%) 20nm | SpMA1 200nm | SpMA2 20nm | E22:H3:TE1 (32%:60%: 8%) 40nm | ST2 5nm | ST2:LiQ (50%:50%) 30nm | LiQ 1nm |
| E4f | SpMA1:PD1 (95%:5%) 20nm | SpMA1 200nm | SpMA2 20nm | E32:H18:TE1 (32%:60%: 8%) 40nm | ST2 5nm | ST2:LiQ (50%:50%) 30nm | LiQ 1nm |
| | | | | | | | |
| V5 | SpMA1:PD1 (95%:5%) 20nm | SpMA1 200nm | SpMA2 20nm | E44:BCbz3:TE1 (38%: 50%:12%) 40nm | ST2 5nm | ST2:LiQ (50%:50%) 30nm | LiQ 1nm |
| E5a | SpMA1:PD1 (95%:5%) 20nm | SpMA1 200nm | SpMA2 20nm | E44:H3:TE1 (38%:50%: 12%) 40nm | ST2 5nm | ST2:LiQ (50%:50%) 30nm | LiQ 1nm |
| E5b | SpMA1:PD1 (95%:5%) 20nm | SpMA1 200nm | SpMA2 20nm | E5:H14:TE1 (38%:50%: 12%) 40nm | ST2 5nm | ST2:LiQ (50%:50%) 30nm | LiQ 1nm |
| E5c | SpMA1:PD1 (95%:5%) 20nm | SpMA1 200nm | SpMA2 20nm | E14:H3:TE1 (38%:50%: 12%) 40nm | ST2 5nm | ST2:LiQ (50%:50%) 30nm | LiQ 1nm |
| E5d | SpMA1:PD1 (95%:5%) 20nm | SpMA1 200nm | SpMA2 20nm | E21:H8:TE1 (38%:50%: 12%) 40nm | ST2 5nm | ST2:LiQ (50%:50%) 30nm | LiQ 1nm |
| E5e | SpMA1:PD1 (95%:5%) 20nm | SpMA1 200nm | SpMA2 20nm | E16:H12:TE1 (38%:50%: 12%) 40nm | ST2 5nm | ST2:LiQ (50%:50%) 30nm | LiQ 1nm |
| E5f | SpMA1:PD1 (95%:5%) 20nm | SpMA1 200nm | SpMA2 20nm | E30:H3:TE1 (38%:50%: 12%) 40nm | ST2 5nm | ST2:LiQ (50%:50%) 30nm | LiQ 1nm |
| E5g | SpMA1:PD1 (95%:5%) 20nm | SpMA1 200nm | SpMA2 20nm | E47:H5:TE1 (38%:50%: 12%) 40nm | ST2 5nm | ST2:LiQ (50%:50%) 30nm | LiQ 1nm |

(fortgesetzt)

| Bsp | HIL Dicke | HTL Dicke | EBL Dicke | EML Dicke | HBL Dicke | ETL Dicke | EIL Dicke |
|---|---|---|---|---|---|---|---|
| | | | | | | | |
| V6 | SpMA1:PD1 (95%:5%) 20nm | SpMA1 200nm | SpMA2 20nm | E45:BCbz3:TE2 (32%: 60%:8%) 40nm | ST2 5nm | ST2:LiQ (50%:50%) 30nm | LiQ 1nm |
| E6a | SpMA1:PD1 (95%:5%) 20nm | SpMA1 200nm | SpMA2 20nm | E45:H3:TE2 (32%:60%: 8%) 40nm | ST2 5nm | ST2:LiQ (50%:50%) 30nm | LiQ 1nm |
| E6b | SpMA1:PD1 (95%:5%) 20nm | SpMA1 200nm | SpMA2 20nm | E7:H3:TE2 (32%:60%:8%) 40nm | ST2 5nm | ST2:LiQ (50%:50%) 30nm | LiQ 1nm |
| E6c | SpMA1:PD1 (95%:5%) 20nm | SpMA1 200nm | SpMA2 20nm | E12:H7:TE2 (32%:60%: 8%) 40nm | ST2 5nm | ST2:LiQ (50%:50%) 30nm | LiQ 1nm |
| E6d | SpMA1:PD1 (95%:5%) 20nm | SpMA1 200nm | SpMA2 20nm | E18:H13:TE2 (32%:60%: 8%) 40nm | ST2 5nm | ST2:LiQ (50%:50%) 30nm | LiQ 1nm |
| E6e | SpMA1:PD1 (95%:5%) 20nm | SpMA1 200nm | SpMA2 20nm | E31:H5:TE2 (32%:60%: 8%) 40nm | ST2 5nm | ST2:LiQ (50%:50%) 30nm | LiQ 1nm |
| E6f | SpMA1:PD1 (95%:5%) 20nm | SpMA1 200nm | SpMA2 20nm | E36:H3:TE2 (32%:60%: 8%) 40nm | ST2 5nm | ST2:LiQ (50%:50%) 30nm | LiQ 1nm |
| | | | | | | | |

Tabelle 9: Daten der OLEDs

| Bsp. | U10 (V) | EQE10 (%) | CIE x/y bei 1000 cd/$m^2$ | $j_0$ (mA/$cm^2$) | L1 (%) | LD (h) |
|---|---|---|---|---|---|---|
| V1 | 4.4 | 21.5 | 0.35/0.63 | 40 | 80 | 620 |
| E1a | 4.5 | 21.6 | 0.35/0.63 | 40 | 80 | 790 |
| E1b | 4.4 | 21.5 | 0.35/0.63 | 40 | 80 | 820 |
| E1c | 4.6 | 21.4 | 0.35/0.63 | 40 | 80 | 840 |
| E1d | 4.4 | 22.7 | 0.35/0.63 | 40 | 80 | 785 |
| E1e | 4.4 | 22.5 | 0.35/0.63 | 40 | 80 | 740 |
| E1f | 4.6 | 22.0 | 0.35/0.63 | 40 | 80 | 710 |
| E1d | 4.3 | 21.9 | 0.35/0.63 | 40 | 80 | 800 |
| E1h | 4.7 | 21.9 | 0.35/0.63 | 40 | 80 | 710 |
| E1i | 4.5 | 22.0 | 0.35/0.63 | 40 | 80 | 825 |
| | | | | | | |
| V2 | 4.4 | 23.7 | 0.35/0.63 | 40 | 80 | 510 |
| E2a | 4.5 | 22.2 | 0.35/0.63 | 40 | 80 | 665 |
| E2b | 4.6 | 22.9 | 0.35/0.63 | 40 | 80 | 740 |
| E2c | 4.4 | 22.6 | 0.35/0.63 | 40 | 80 | 575 |

(fortgesetzt)

| Bsp. | U10 (V) | EQE10 (%) | CIE x/y bei 1000 cd/$m^2$ | $j_0$ (mA/$cm^2$) | L1 (%) | LD (h) |
|---|---|---|---|---|---|---|
| E2d | 4.6 | 21.7 | 0.35/0.63 | 40 | 80 | 800 |
| E2e | 4.6 | 22.1 | 0.35/0.63 | 40 | 80 | 640 |
| E2f | 4.6 | 21.3 | 0.34/0.63 | 40 | 80 | 665 |
| | | | | | | |
| V3 | 4.4 | 23.0 | 0.35/0.63 | 40 | 80 | 480 |
| E3a | 4.5 | 22.7 | 0.35/0.63 | 40 | 80 | 630 |
| E3b | 4.6 | 22.2 | 0.35/0.63 | 40 | 80 | 710 |
| E3c | 4.45 | 21.8 | 0.35/0.63 | 40 | 80 | 770 |
| E3d | 4.8 | 22.2 | 0.35/0.63 | 40 | 80 | 660 |
| E3e | 4.4 | 23.1 | 0.35/0.63 | 40 | 80 | 665 |
| E3f | 4.6 | 22.4 | 0.34/0.62 | 40 | 80 | 710 |
| | | | | | | |
| V4 | 5.0 | 19.2 | 0.34/0.62 | 40 | 80 | 675 |
| E4a | 5.2 | 19.3 | 0.34/0.62 | 40 | 80 | 1040 |
| E4b | 4.9 | 18.6 | 0.34/0.62 | 40 | 80 | 1160 |
| E4c | 4.9 | 19.5 | 0.34/0.62 | 40 | 80 | 1265 |
| E4d | 5.0 | 18.4 | 0.34/0.62 | 40 | 80 | 1140 |
| E4e | 4.9 | 19.3 | 0.34/0.62 | 40 | 80 | 830 |
| E4f | 5.1 | 18.2 | 0.34/0.62 | 40 | 80 | 880 |
| | | | | | | |
| V5 | 4.5 | 18.1 | 0.34/0.62 | 40 | 80 | 1070 |
| E5a | 4.6 | 18.4 | 0.34/0.62 | 40 | 80 | 1815 |
| E5b | 4.4 | 17.3 | 0.34/0.62 | 40 | 80 | 1320 |
| E5c | 4.4 | 18.7 | 0.34/0.62 | 40 | 80 | 1650 |
| E5d | 4.3 | 18.6 | 0.34/0.62 | 40 | 80 | 1295 |
| E5e | 4.7 | 18.4 | 0.34/0.62 | 40 | 80 | 1400 |
| E5f | 4.5 | 16.5 | 0.34/0.62 | 40 | 80 | 1980 |
| E5g | 4.4 | 18.8 | 0.34/0.63 | 40 | 80 | 1690 |
| | | | | | | |
| V6 | 4.5 | 21.9 | 0.35/0.63 | 40 | 80 | 630 |
| E6a | 4.4 | 22.4 | 0.35/0.63 | 40 | 80 | 765 |
| E6b | 4.4 | 22.9 | 0.35/0.63 | 40 | 80 | 720 |
| E6c | 4.3 | 23.1 | 0.35/0.63 | 40 | 80 | 990 |
| E6d | 4.5 | 22.1 | 0.35/0.63 | 40 | 80 | 790 |
| E6e | 4.6 | 22.5 | 0.35/0.63 | 40 | 80 | 835 |
| E6f | 4.3 | 21.5 | 0.35/0.63 | 40 | 80 | 745 |

Tabelle 10: Strukturformeln der verwendeten Materialien der OLEDs, sofern nicht schon zuvor beschrieben

| | |
|---|---|
| 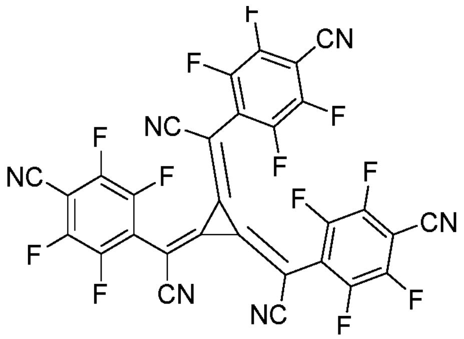 | 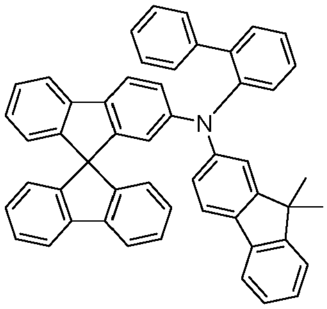 |
| PD1 (CAS Reg. No. 1224447-88-4) | SpMA1 |
| 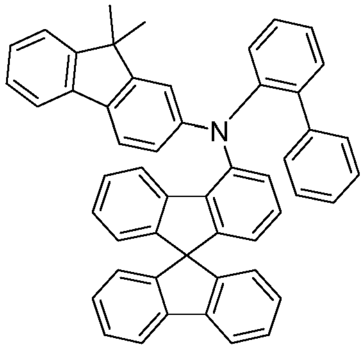 | 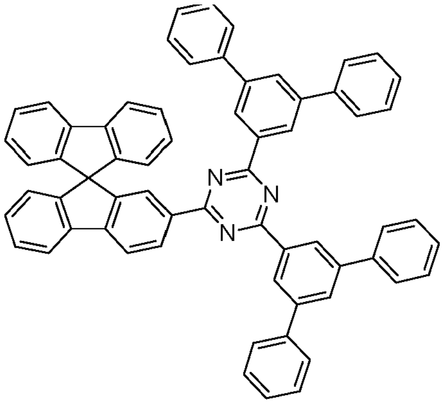 |
| SpMA2 | ST2 |
| 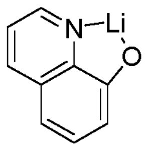 | 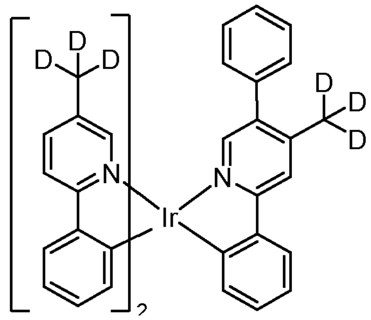 |
| LiQ | TE1 |
| 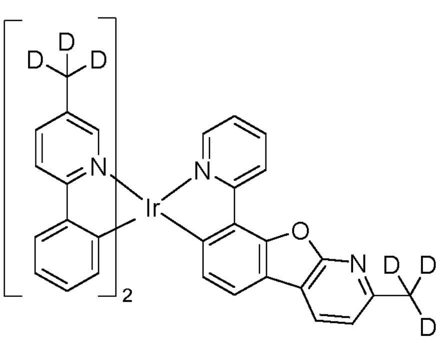 | 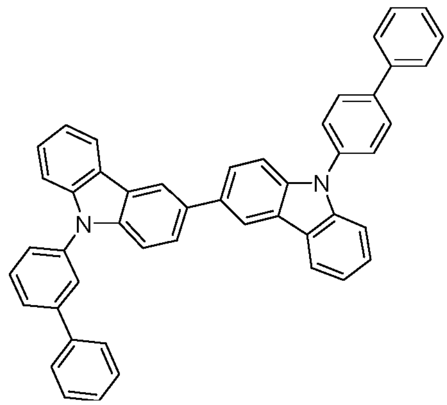 |
| TE2 | BCbz1 |

(fortgesetzt)

| | |
|---|---|
| 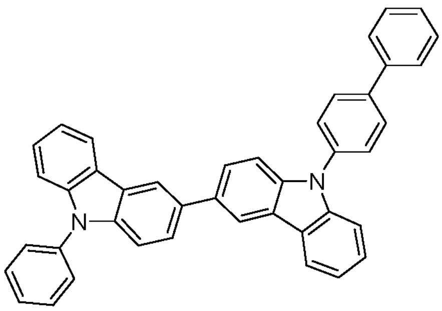 | 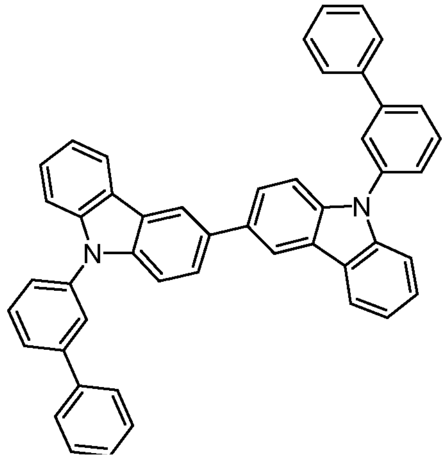 |
| BCbz2 | BCbz3 |

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

**Synthon S1a**

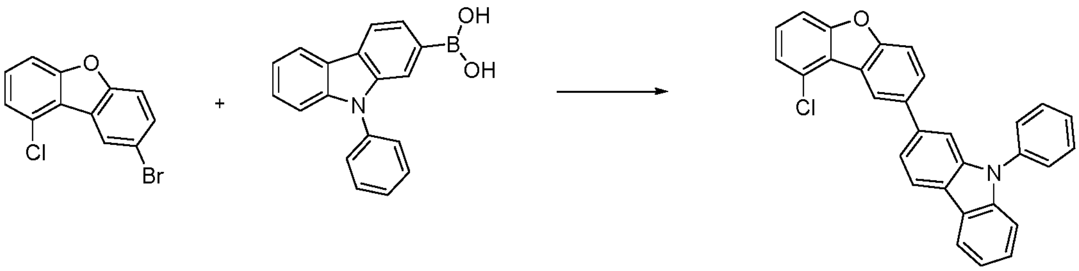

1-Chlor-8-Bromdibenzofuran (100 g, 353.6 mmol) [CAS-2225909-61-3] und B-(9-phenyl-9H-carbazol-2-yl)boronsäure (106.6 g, 371.3 mmol) [1001911-63-2] werden in Toluol (800 mL), 1,4-Dioxan (800 mL) und Wasser (400 mL) unter inerter Atmosphäre vorgelegt, mit $Na_2CO_3$ und (74.95 g, 0.71 mol) Tetrakis(triphenylphosphin)palladium(0) (4.09 g, 3.54 mmol) versetzt und 16 h unter Rückfluss gerührt.

Nach dem Abkühlen saugt man den Ansatz über eine mit Celite gefüllte Fritte ab und arbeitet den Ansatz extraktiv mit Toluol und Wasser auf. Die die wässrige Phase wird 2-mal mit Toluol (je 500 mL) extrahiert und die vereinigten organischen Phasen über $Na_2SO_4$ getrocknet. Das Lösemittel wird am Rotationsverdampfer entfernt und das Rohprodukt wird mit Ethanol (1200 mL) aufgeschlämmt und 2 h unter Rückfluss gerührt. Der Feststoff wird abgesaugt, mit Ethanol gewaschen und im Vakuumtrockenschrank getrocknet.
Ausbeute: 138.5 g (312.2 mmol, 88%), 97%ig nach $^1$H-NMR
Analog können folgende Verbindungen dargestellt werden. Zur Aufreinigung kann auch Säuien-chro¬ma¬tographie verwendet werden, oder zur Umkristallisation oder Heißex¬trak¬tion können andere gängige Lösungsmittel wie Ethanol, Butanol, Aceton, Ethylacetat, Acetonitril, Toluol, Xylol, Dichlormethan, Methanol, Tetrahydrofuran, n Butylacetat, 1,4-Dioxan oder zur Umkristallisation Hoch¬sieder wie Dimethylsulfoxid, N,N Dimethyl-formamid, N,N-Di¬methyl-acet¬amid, N-Methylpyrrolidon, etc. verwendet werden.

| Edukt 1 | Edukt 2 | Produkt | Ausbeute |
|---|---|---|---|
| 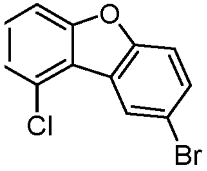 CAS-2225909-61-3 | 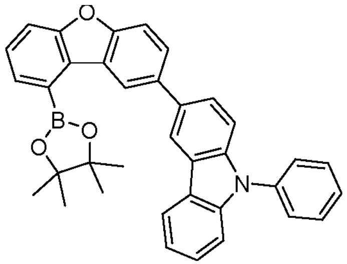 CAS-2239299-50-2 | 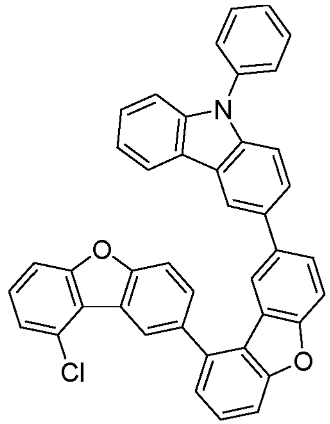 S2a | 63% |
| 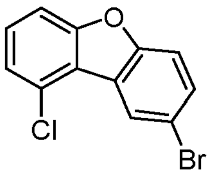 CAS-2225909-61-3 | 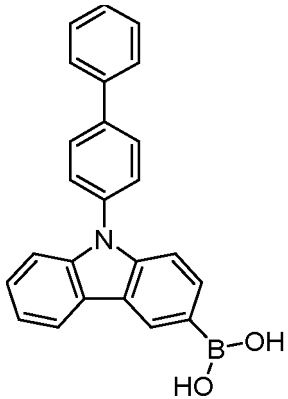 CAS-1028648-22-7 | 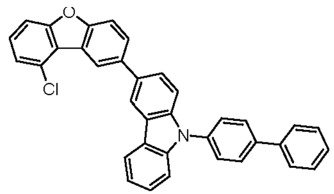 S3a | 62% |

(fortgesetzt)

| Edukt 1 | Edukt 2 | Produkt | Ausbeute |
|---|---|---|---|
| 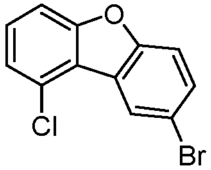 CAS-2225909-61-3 | 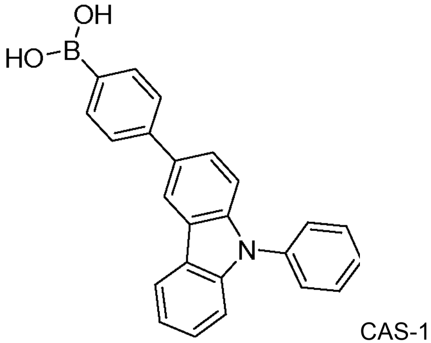 CAS-1240963-55-6 | 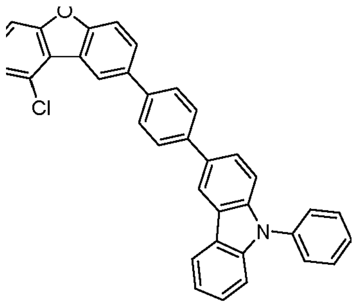 S4a | 71% |
| 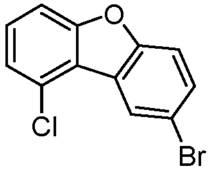 CAS-2225909-61-3 | 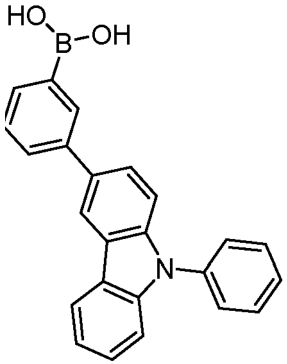 CAS-854952-60-6 | 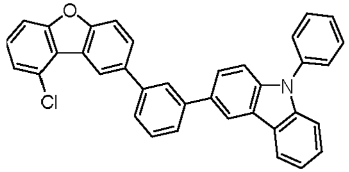 S5a | 77% |
| 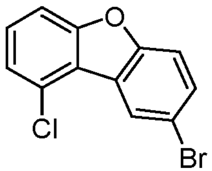 CAS-2225909-61-3 | 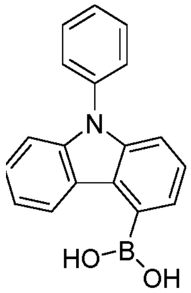 CAS-1370555-65-9 | 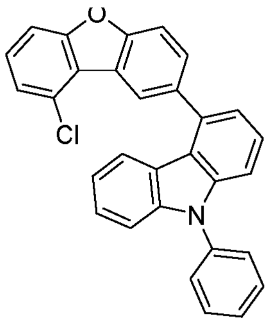 S6a | 56% |

(fortgesetzt)

| Edukt 1 | Edukt 2 | Produkt | Ausbeute |
|---|---|---|---|
| 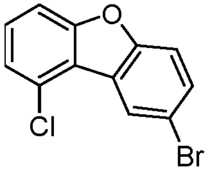 CAS-2225909-61-3 | 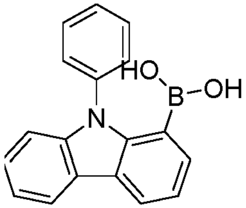 CAS-1333002-41-7 | 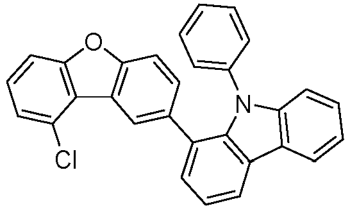 S7a | 52% |
| 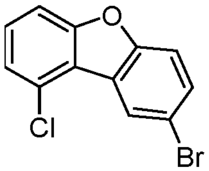 CAS-2225909-61-3 | 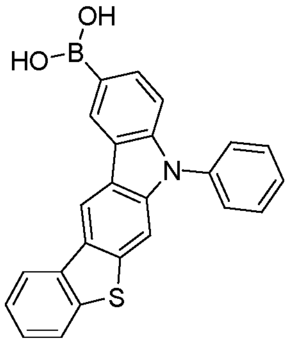 CAS-2128690-41-3 | 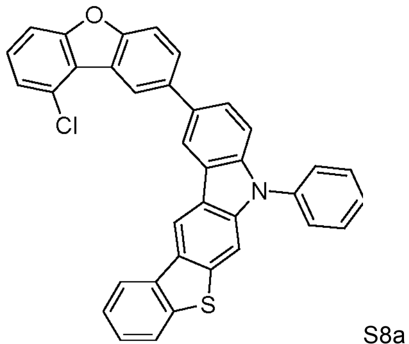 S8a | 68% |
| 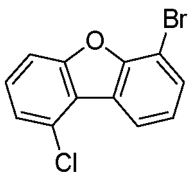 CAS- 2144800-21-3 | 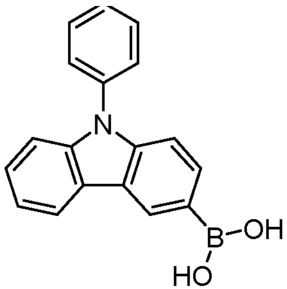 CAS-854952-58-2 | 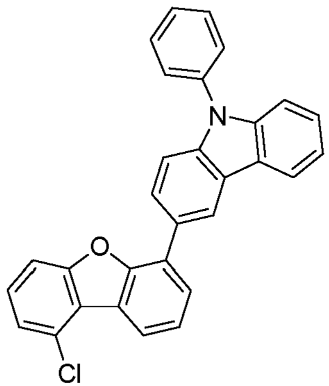 S9a | 69% |

(fortgesetzt)

| Edukt 1 | Edukt 2 | Produkt | Ausbeute |
|---|---|---|---|
| 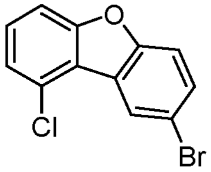 CAS-2225909-61-3 | 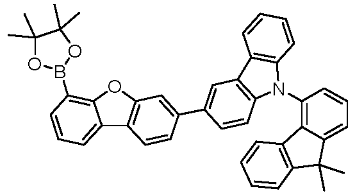 | 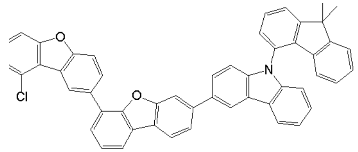 S10a | 67% |
| 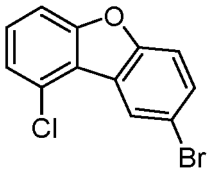 CAS-2225909-61-3 | 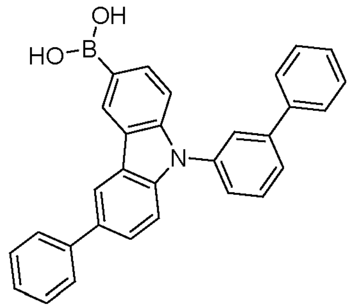 | 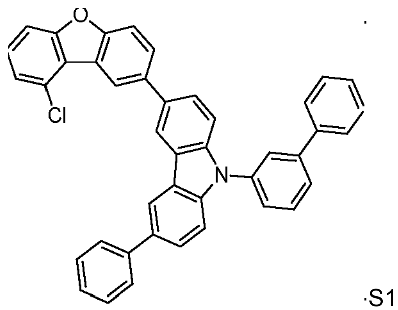 ·S11a | 52% |
| 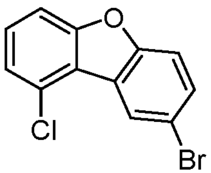 CAS-2225909-61-3 | 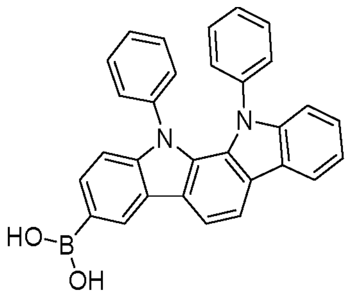 CAS-1373359-67-1 | 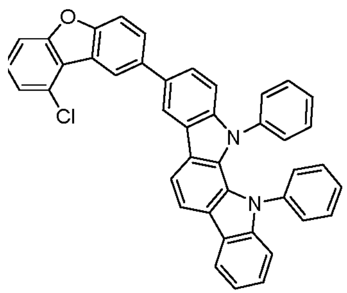 S12a | 68% |

(fortgesetzt)

| Edukt 1 | Edukt 2 | Produkt | Ausbeute |
|---|---|---|---|
| 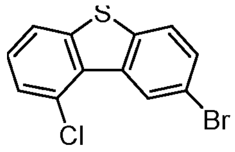 CAS-2055864-36-1 | 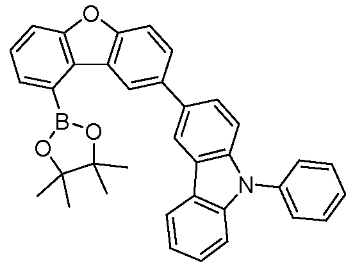 CAS-2239299-50-2 | 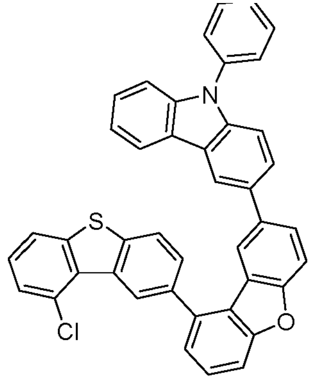 S13a | 72% |
| 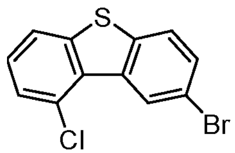 CAS-2055864-36-1 | 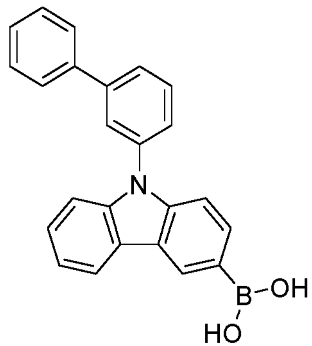 CAS-1416814-68-0 | 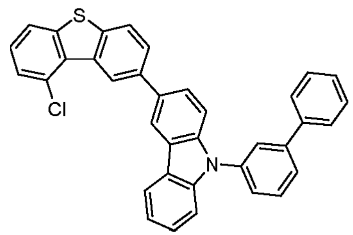 S14a | 80% |

(fortgesetzt)

| Edukt 1 | Edukt 2 | Produkt | Ausbeute |
|---|---|---|---|
| 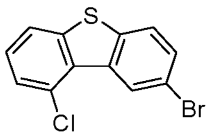 CAS-2055864-36-1 | 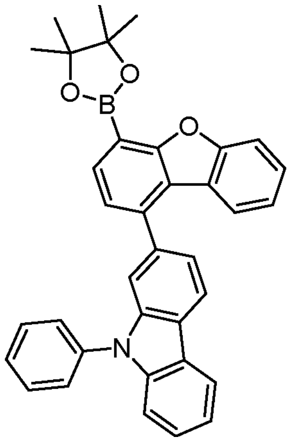 | 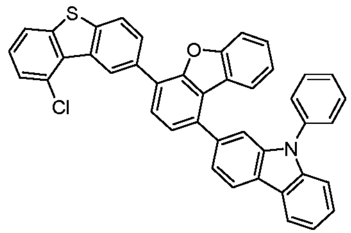 S15a | 73% |
| 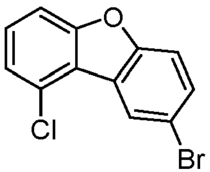 CAS-2225909-61-3 | 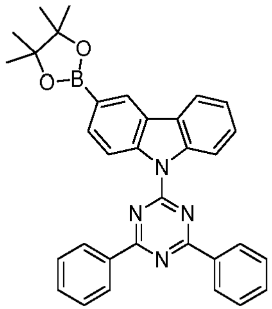 CAS-1361094-91-8 | 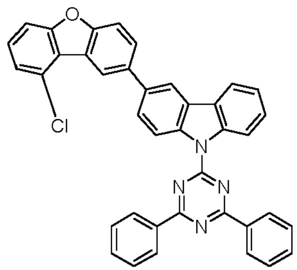 S16a | 66% |
| 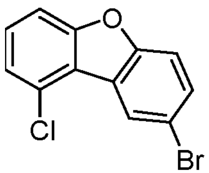 CAS-2225909-61-3 | 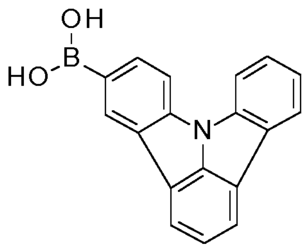 CAS-1174032-92-8 | 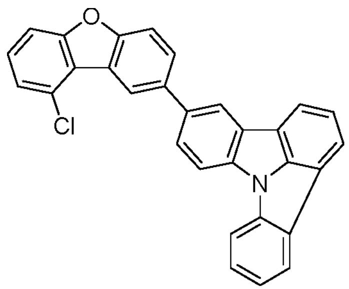 S17a | 60% |

**S1b:**

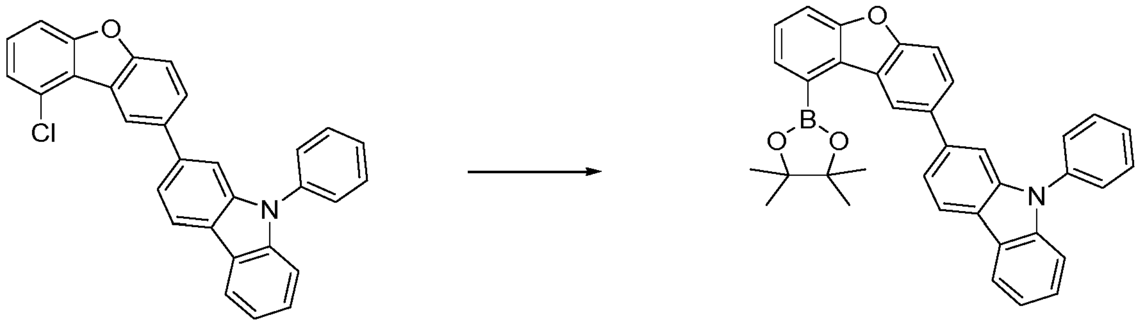

S1a (124.30 g, 280 mmol) und 4,4,5,5-tetramethyl-2-(4,4,5,5-tetramethyl-1,3,2-dioxaborolan-2-yl)-1,3,2-dioxaborolane ( 76.20 g, 300 mmol) werden in 1,4-Dioxan (2000 mL) unter inerter Atmosphäre vorgelegt und mit Kaliumacetat (82.33 g, 1.40 mol) und trans-Dichlorbis(tricyclohexylphos-phin)palladium(II) (6.21 g, 83.9 mmol) versetzt und für 32 h unter Rückfluss gerührt. Nach dem Abkühlen wird das Lösungsmittel am Rotationsverdampfer abrotiert, der Rückstand wird extraktiv mit Toluol/Wasser aufgearbeitet, die organische Phase über $Na_2SO_4$ getrocknet. Das Rohprodukt wird mit Ethanol (1100 mL) unter Rückfluss ausgerührt, der Feststoff nach dem Abkühlen abgesaugt und mit Ethanol gewaschen.

Ausbeute: 113.5 g (212.5 mmol, 76%), 95%ig nach $^{1}H$-NMR.

Analog können folgende Verbindungen dargestellt werden. Zur Aufreinigung kann auch Säuien-chro¬ma¬tographie verwendet werden, oder zur Umkristallisation oder Heißex¬trak¬tion können andere gängige Lösungsmittel wie Ethanol, Butanol, Aceton, Ethylacetat, Acetonitril, Toluol, Xylol, Dichlormethan, Methanol, Tetrahydrofuran, n Butylacetat, 1,4-Dioxan oder zur Umkristallisation Hoch¬sieder wie Dimethylsulfoxid, N,N Dimethyl-formamid, N,N-Di¬methyl-acet¬amid, N-Methylpyrrolidon, etc. verwendet werden.

| Edukt 1 | Produkt | Ausbeute |
|---|---|---|
| 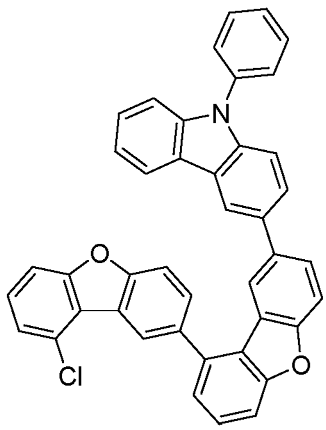 S2a | 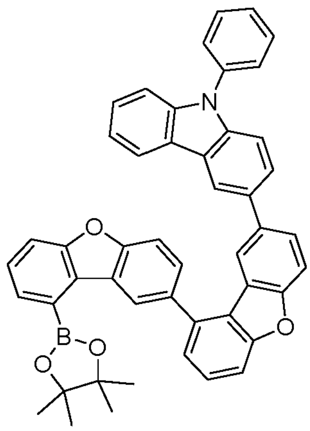 S2b | 79% |
| 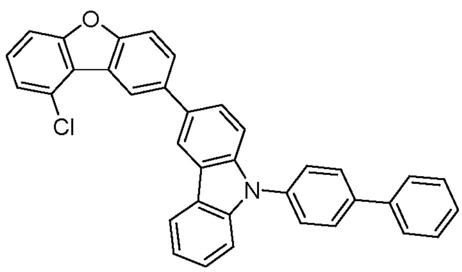 S3a | 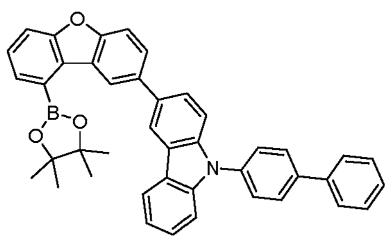 S3b | 70% |

(fortgesetzt)

| Edukt 1 | Produkt | Ausbeute |
|---|---|---|
| 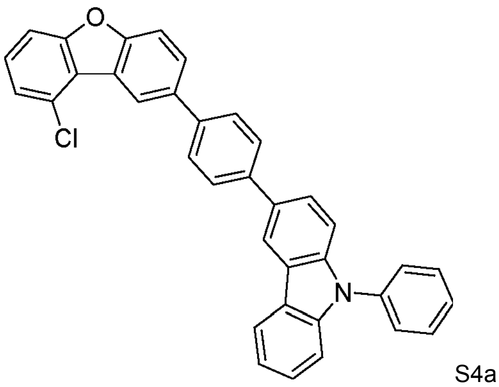 S4a | 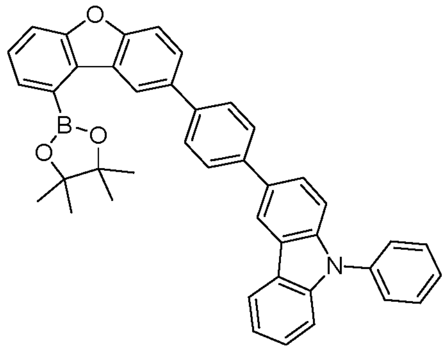 S4b | 66% |
| 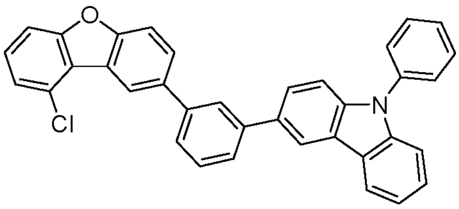 S5a | 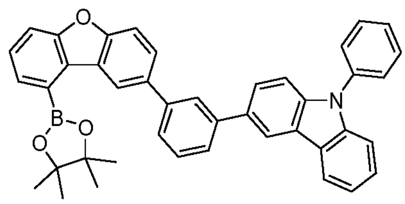 S5b | 67% |
| 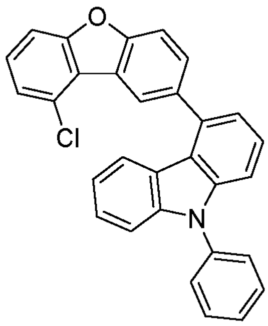 S6a | 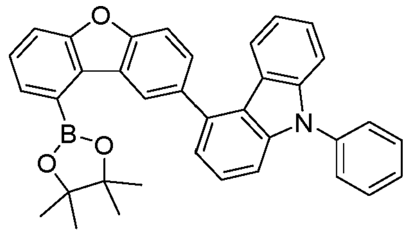 S6b | 72% |
| 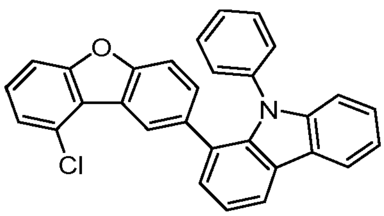 S7a | 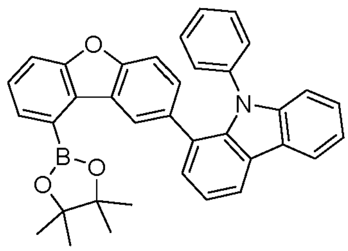 S7b | 64% |
| 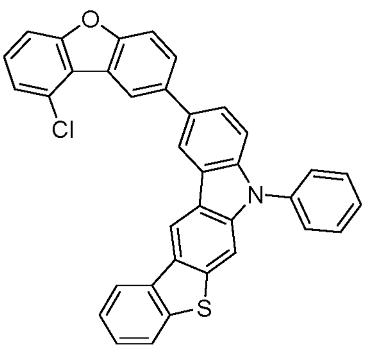 S8a | 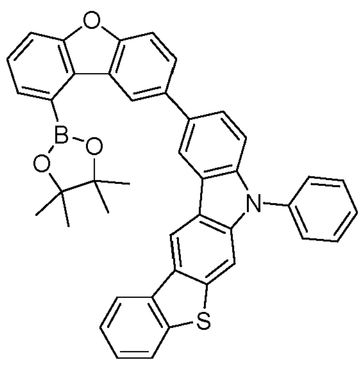 S8b | 66% |

(fortgesetzt)

| Edukt 1 | Produkt | Ausbeute |
|---|---|---|
| 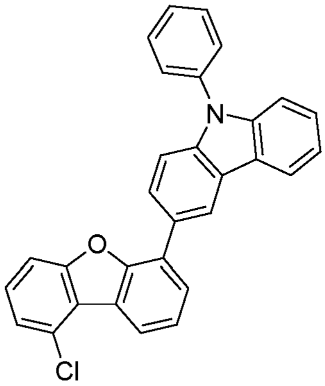 S9a | 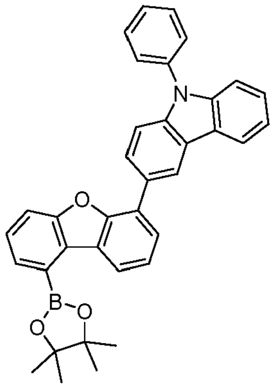 S9b | 78% |
| 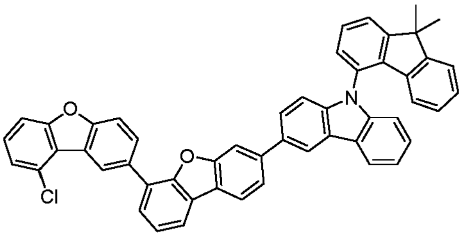 S10a | 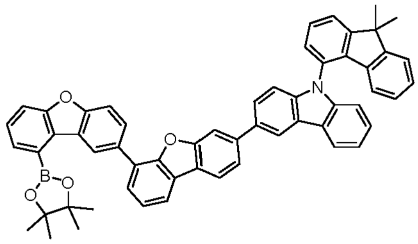 S10b | 59% |
| 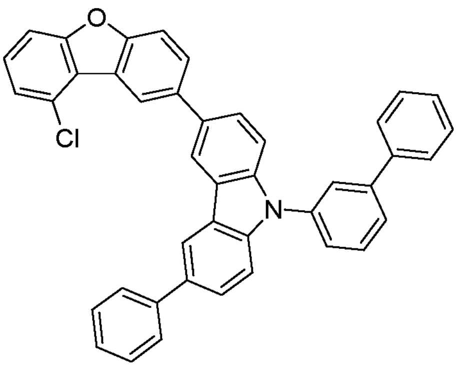 S11a | 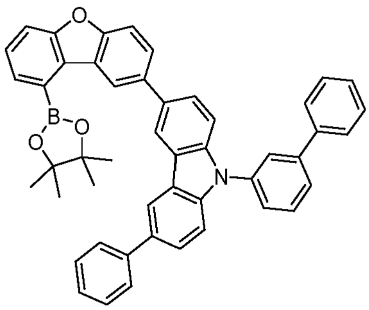 S11b | 71% |
| 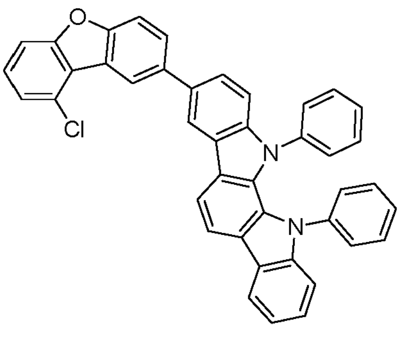 S12a | 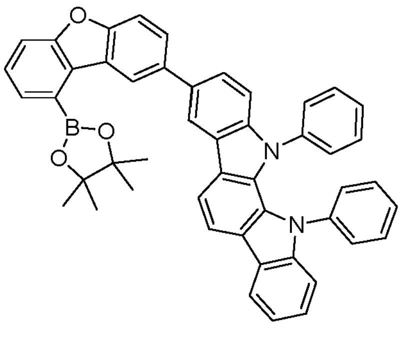 S12b | 62% |

(fortgesetzt)

| Edukt 1 | Produkt | Ausbeute |
|---|---|---|
| 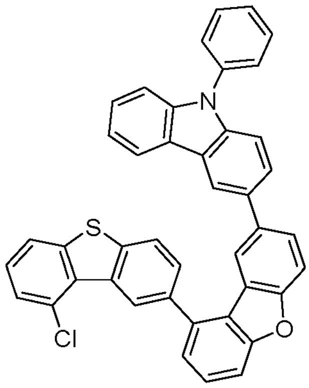 S13a | 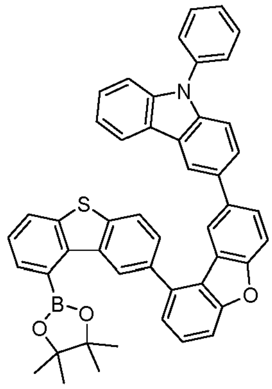 S13b | 67% |
| 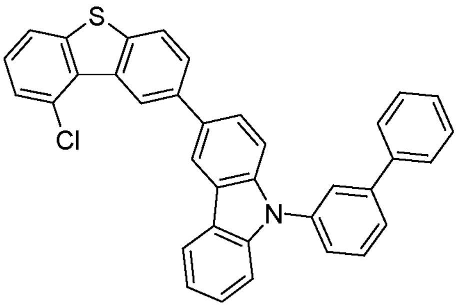 S14a | 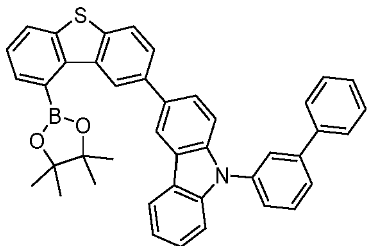 S14b | 80% |
| 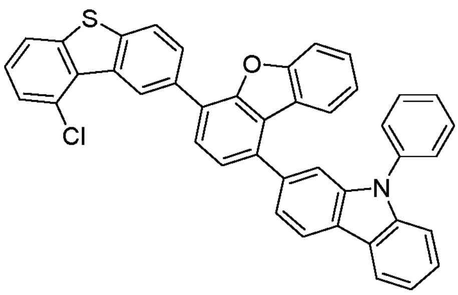 S15a | 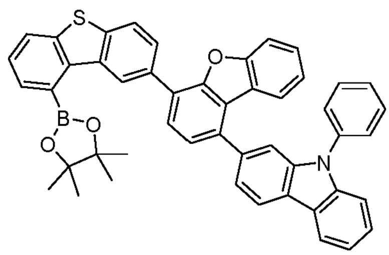 S15b | 82% |
| 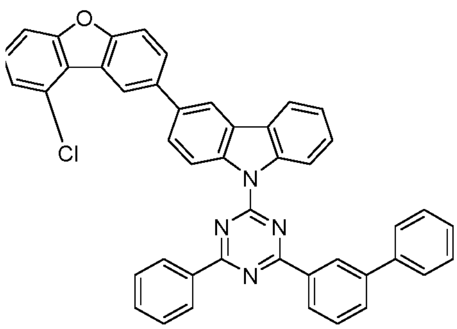 S16a | 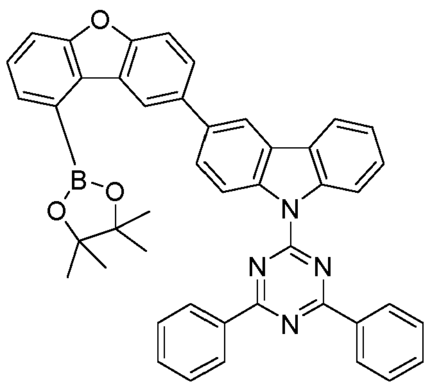 S16b | 72% |

(fortgesetzt)

| Edukt 1 | Produkt | Ausbeute |
|---|---|---|
| 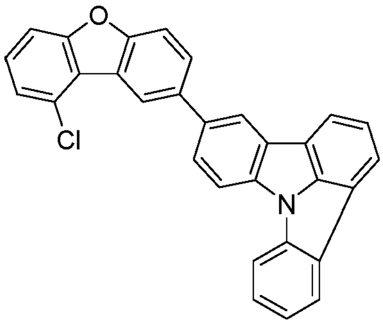 S17a | 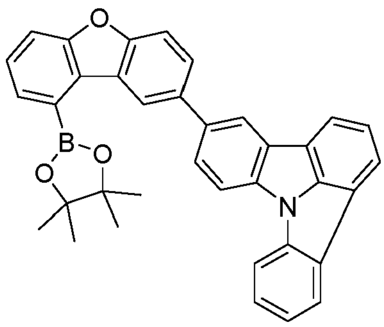 S17b | 76% |

**E1**

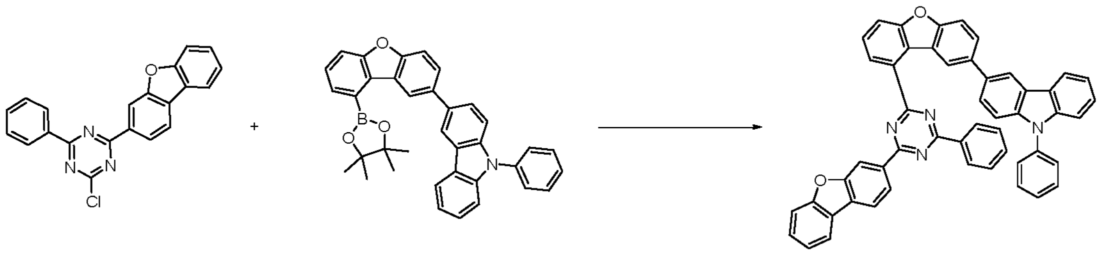

9-Phenyl-3-[9-(4,4,5,5-tetramethyl-[1,3,2]dioxaborolan-2-yl)-dibenzofuran-2-yl]-9H-carbazole (22.00 g, 41.1 mmol) [2239299-50-2], 2-chloro-4-{8-oxatricyclo[7.4.0.0$^{2}$,7]tri-deca-1(13),2(7),3,5,9,11-hexaen-5-yl}-6-phenyl-1,3,5-triazine (14.70 g, 41.1 mmol) [CAS-2142681-84-1] werden in THF (650 mL) und Wasser (250 mL) unter inerter Atmosphäre vorgelegt, mit $Na_2CO_3$ (9.58 g, 90.4 mmol) und Tetrakis(triphenylphosphin)palladium(0) (710 mg, 0.62 mmol) versetzt und für 16 h unter Rückfluss gerührt. Nach dem Abkühlen wird der Ansatz extraktiv mit Toluol/Wasser aufgearbeitet, die wässrige Phase 3-mal mit Toluol (je 250 mL) extrahiert und die vereinigten organischen Phasen über $Na_2SO_4$ getrocknet. Das Rohprodukt wir 2-mal mit Heptan/Toluol heißextrahiert, 2 mal aus n-Butylacetat umkristallisiert und abschließend im Hochvakuum sublimiert.

Ausbeute: 15.5 g (21.2 mmol, 52%); Reinheit > 99.9% nach HPLC.

Analog können folgende Verbindungen dargestellt werden. Hierbei kann als Katalysatorsystem (Palladiumquelle und Ligand) auch $Pd_2(dba)_3$ mit SPhos [657408-07-6] oder Bis(triphenylphosphin)-palladium(II)chlorid [13965-03-2] verwendet werden. Zur Aufreinigung kann auch Säulen-chro¬ma¬tographie verwendet werden, oder zur Umkristallisation oder Heißex¬trak¬tion können andere gängige Lösungsmittel wie Ethanol, Butanol, Aceton, Ethylacetat, Acetonitril, Toluol, Xylol, Dichlormethan, Methanol, Tetrahydrofuran, n Butylacetat, 1,4-Dioxan oder zur Umkristallisation Hoch¬sieder wie Dimethylsulfoxid, N,N Dimethyl-formamid, N,N-Di¬methylacet¬amid, N-Methylpyrrolidon, etc. verwendet werden.

| Edukt 1 | Edukt 2 | Produkt | Ausbeute |
|---|---|---|---|
| 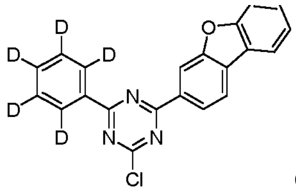 CAS-2226747-65-3 | 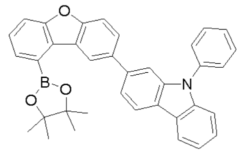 S1b | 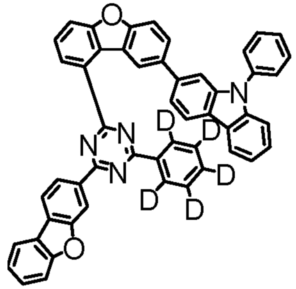 E2 | 54% |
| 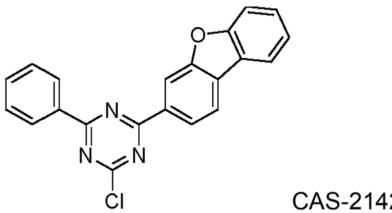 CAS-2142681-84-1 | 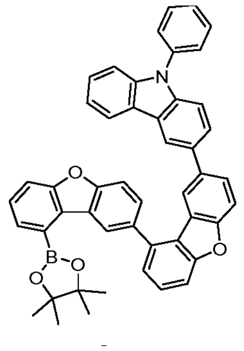 S2b | 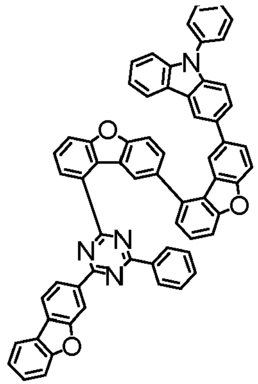 E3 | 46% |
| 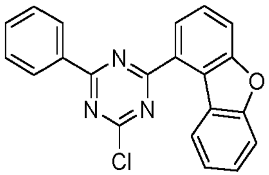 CAS-1883265-32-4 | 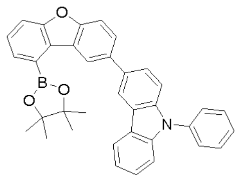 CAS-2239299-50-2 | 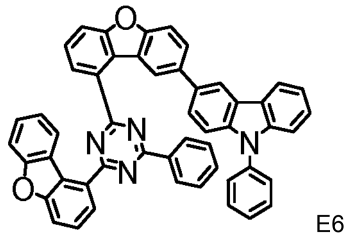 E6 | 51% |

(fortgesetzt)

| Edukt 1 | Edukt 2 | Produkt | Ausbeute |
|---|---|---|---|
| 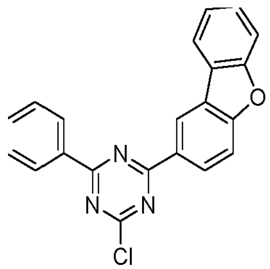 CAS-1618107-00-8 | 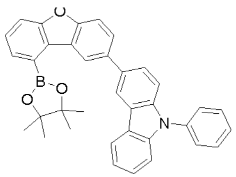 CAS-2239299-50-2 | 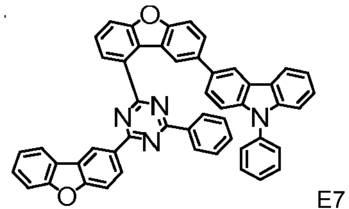 E7 | 57% |
| 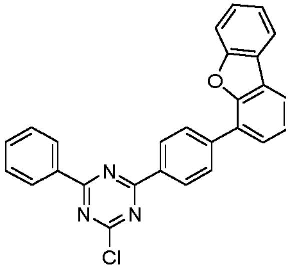 CAS-2074632-09-8 | 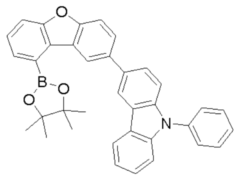 CAS-2239299-50-2 | 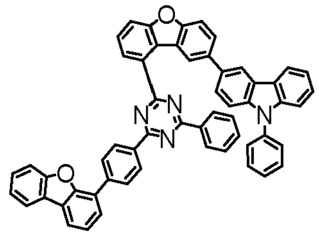 E8 | 62% |
| 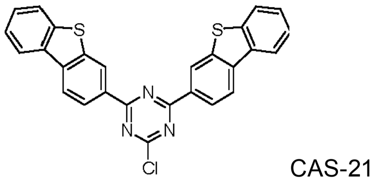 CAS-2172944-02-2 | 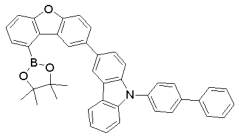 S3b | 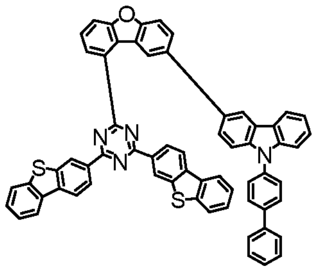 E9 | 60% |
| 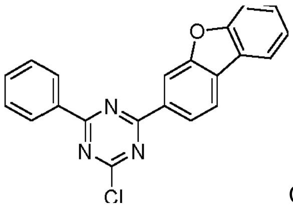 CAS-2142681-84-1 | 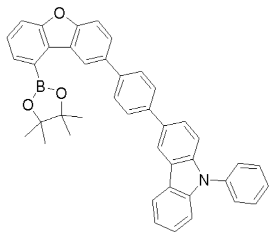 S4b | 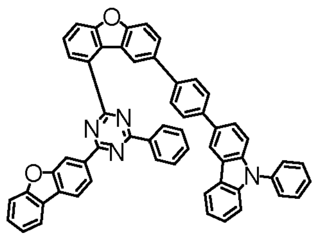 E11 | 54% |

(fortgesetzt)

| Edukt 1 | Edukt 2 | Produkt | Ausbeute |
|---|---|---|---|
| 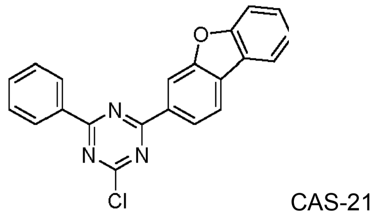 CAS-2142681-84- | 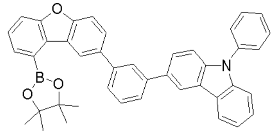 S5b | 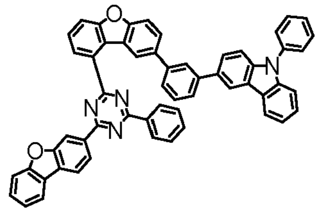 E12 | 44% |
| 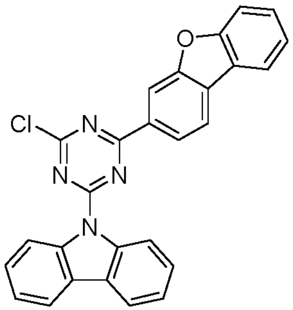 CAS-2260688-95-5 | 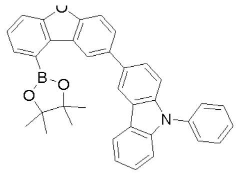 CAS-2239299-50-2 | 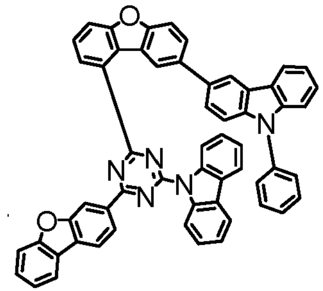 E13 | 46% |
| 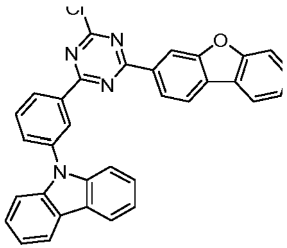 | 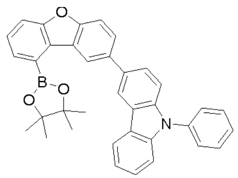 CAS-2239299-50-2 | 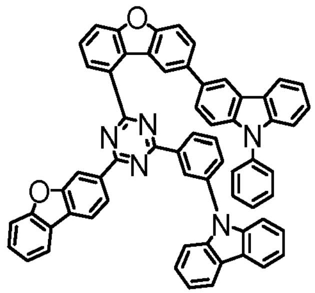 E15 | 44% |

(fortgesetzt)

| Edukt 1 | Edukt 2 | Produkt | Ausbeute |
|---|---|---|---|
| 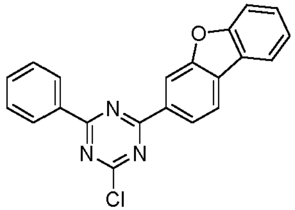 CAS-2142681-84- | 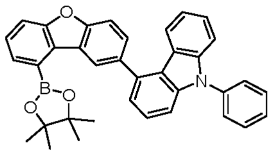 S6b | 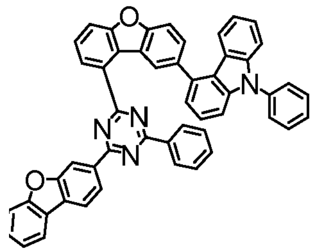 E16 | 49% |
| 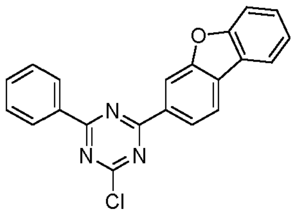 CAS-2142681-84- | 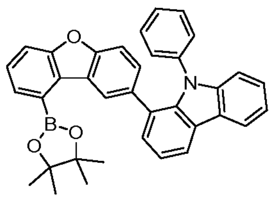 S7b | 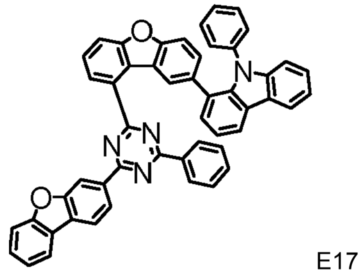 E17 | 38% |
| 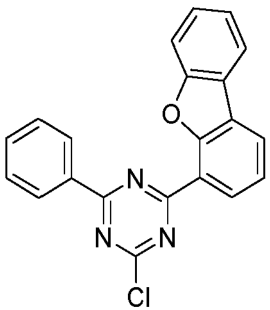 CAS-1472729-25-1 | 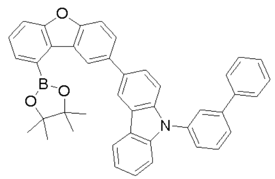 | 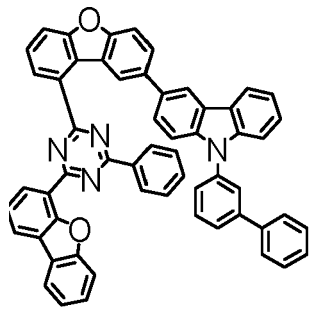 E18 | 39% |

(fortgesetzt)

| Edukt 1 | Edukt 2 | Produkt | Ausbeute |
|---|---|---|---|
| 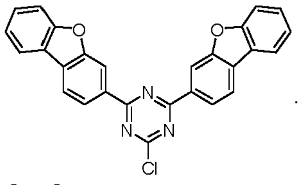 CAS-2251105-15-2 | 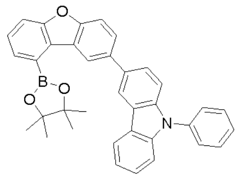 CAS-2239299-50-2 | 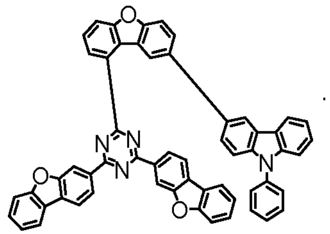 E20 | 55% |
| 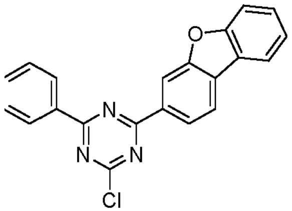 CAS-2142681-84 | 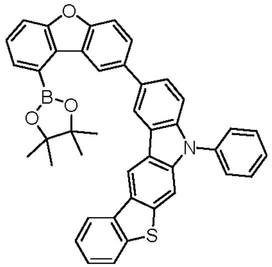 S8b | 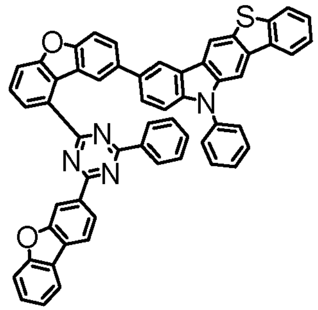 E21 | 41% |
| 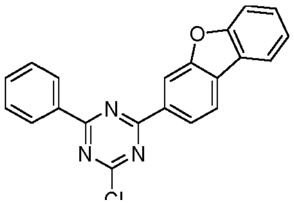 CAS-2142681-84 | 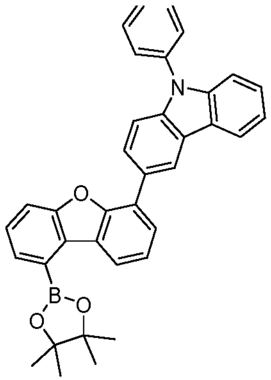 S9b | 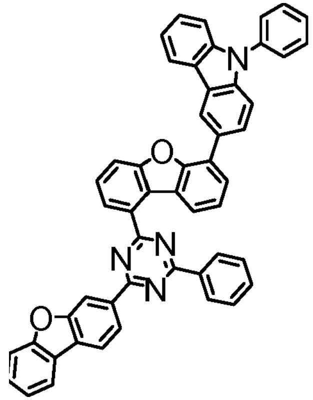 E25 | 53% |

(fortgesetzt)

| Edukt 1 | Edukt 2 | Produkt | Ausbeute |
|---|---|---|---|
| 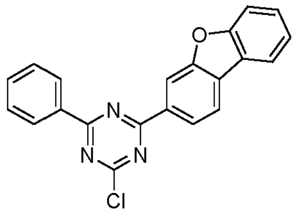 CAS-2142681-84 | 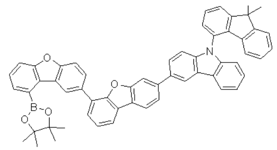 S10b | 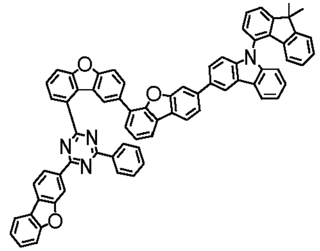 E27 | 59% |
| 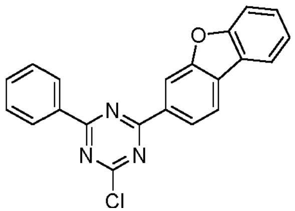 CAS-2142681-84 | 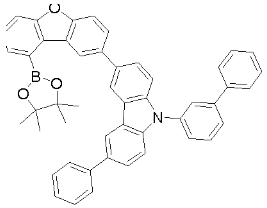 S11b | 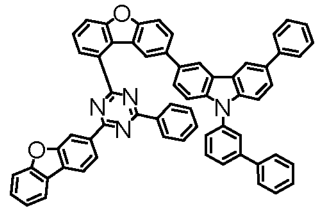 E29 | 56% |
| 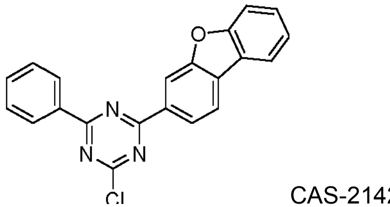 CAS-2142681-84 | 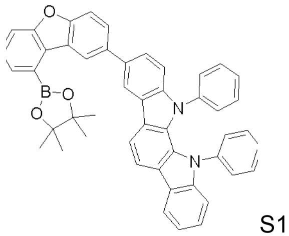 S12b | 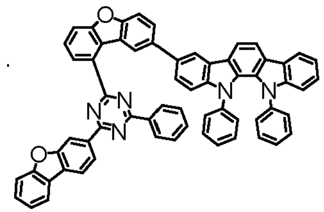 E31 | 54% |

(fortgesetzt)

| Edukt 1 | Edukt 2 | Produkt | Ausbeute |
|---|---|---|---|
| 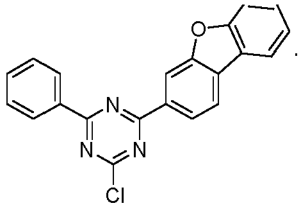 CAS-2142681-84 | 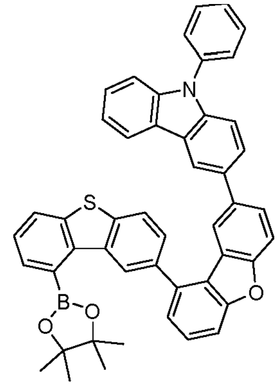 S13b | 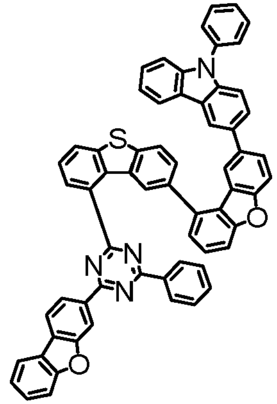 E32 | 45% |
| 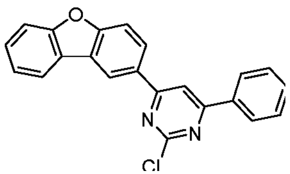 CAS-1421599-32-7 | 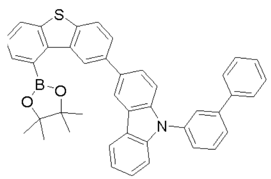 S14b | 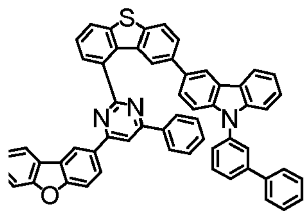 E33 | 46% |
| 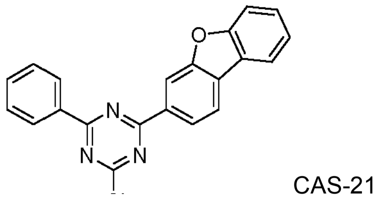 CAS-2142681-84 | 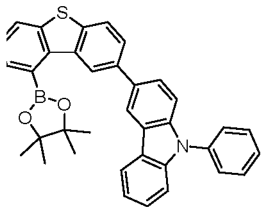 CAS-2299272-36-7 | 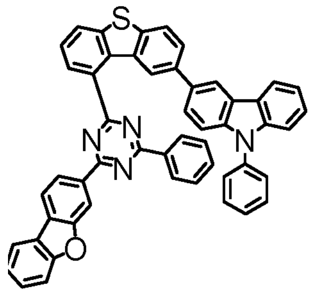 E34 | 58% |

(fortgesetzt)

| Edukt 1 | Edukt 2 | Produkt | Ausbeute |
|---|---|---|---|
| 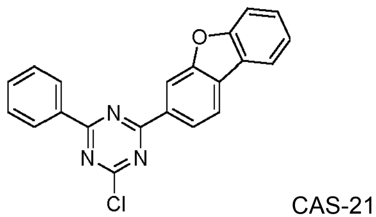 CAS-2142681-84 | 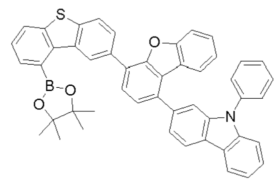 S15b | 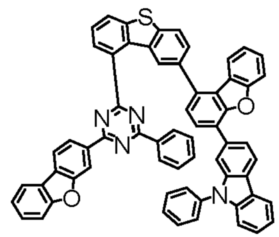 E36 | 32% |
| 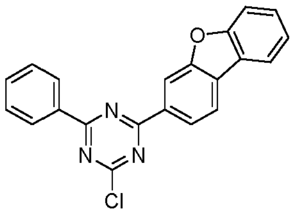 CAS-2142681-84 | 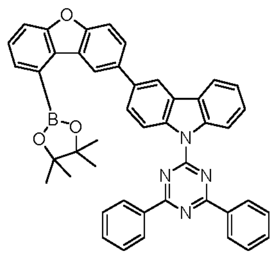 S16b | 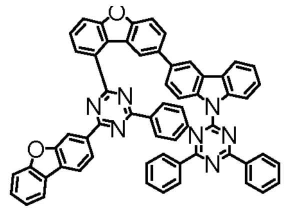 E38 | 38% |
| 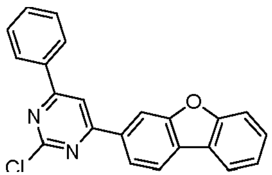 CAS-2286234-09-9 | 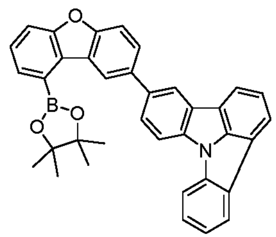 S17b | 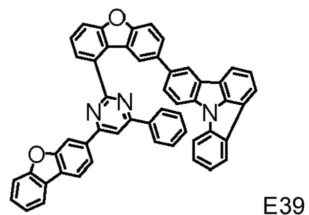 E39 | 40% |

**H3**

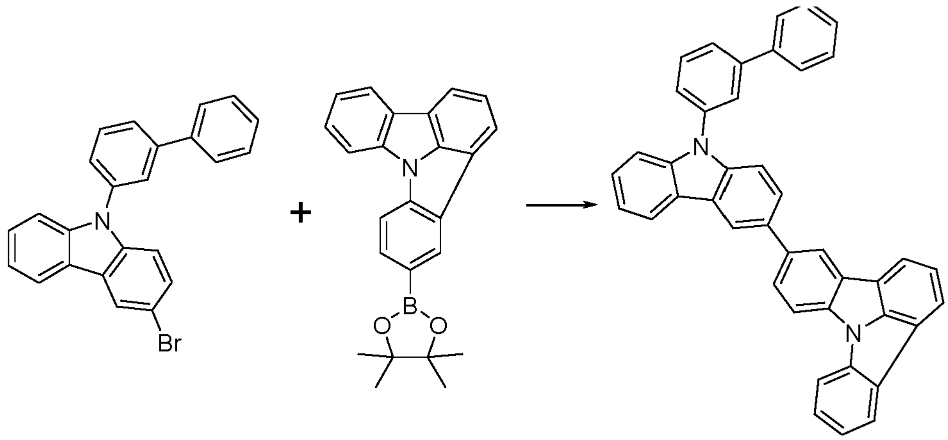

9-[1,1'-biphenyl]-3-yl-3-bromo-9H-carbazole (59.88 g, 150.3 mmol) [CAS-1428551-28-3], 5-(4,4,5,5-tetramethyl-1,3,2-dioxaborolan-2-yl)-indolo[3,2,1-jk]carbazole (51.1 g, 147.3 mmol) [CAS- 1454807-26-1] werden in Toluol (1200 mL), 1,4-Dioxan (1200 mL) und Wasser (600 mL) unter inerter Atmosphäre vorgelegt, mit $K_3PO_4$ (95.7 g, 451 mmol) Tri-(ortho-tolyl)phosphin (2.33 g, 7.52 mmol) und $Pd(OAc)_2$ (840 mg, 3.76 mmol) versetzt und für 32 h unter Rückfluss gerührt. Nach dem Abkühlen wird der Ansatz extraktiv mit Toluol/Wasser aufgearbeitet, die wässrige Phase 3-mal mit Toluol (je 500 mL) extrahiert und die vereinigten organischen Phasen über $Na_2SO_4$ getrocknet. Das Rohprodukt wird zunächst in EtOH (1500 mL) ausgerührt. Der abgesaugte Feststoff wir 2-mal mit Heptan/Toluol heißextrahiert, 2-mal aus DMAc umkristallisiert und abschließend im Hochvakuum sublimiert.

Ausbeute: 40.5 g (72.5 mmol, 48%); Reinheit > 99.9% nach HPLC.

Analog können folgende Verbindungen dargestellt werden. Hierbei kann als Katalysatorsystem (Palladiumquelle und Ligand) auch $Pd_2(dba)_3$ mit SPhos [657408-07-6] oder, Tetrakis(triphenylphosphin)palladium(0) oder Bis(triphenylphosphin)-palladium(II)chlorid [13965-03-2] verwendet werden. Zur Aufreinigung kann auch Säuien-chro¬ma¬tographie verwendet werden, oder zur Umkristallisation oder Heiß¬ex¬trak¬tion können andere gängige Lösungsmittel wie Ethanol, Butanol, Aceton, Ethylacetat, Acetonitril, Toluol, Xylol, Dichlormethan, Methanol, Tetrahydrofuran, n Butylacetat, 1,4-Dioxan oder zur Umkristallisation Hoch¬sieder wie Dimethylsulfoxid, N,N Dimethyl-formamid, N,N-Di¬methylacet¬amid, N-Methylpyrrolidon, etc. verwendet werden.

| Edukt 1 | Edukt 2 | Produkt | Ausbeute |
|---|---|---|---|
| 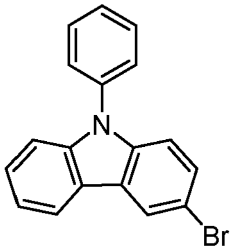 CAS-1153-85-1 | 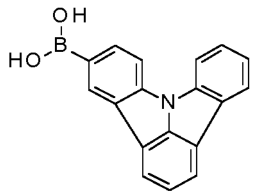 CAS-1174032-92-8 | 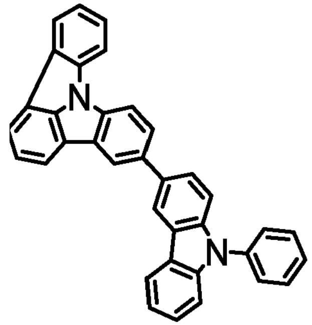 1 H1 | 47% |
| 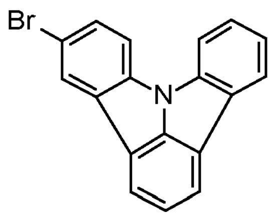 CAS-109589-98-2 | 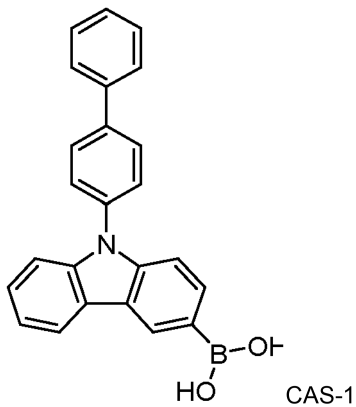 CAS-1028648-22- | 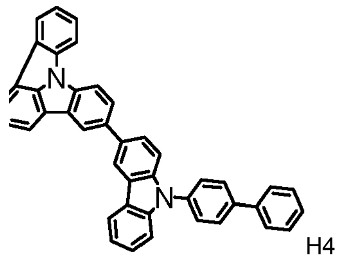 H4 | 59% |
| 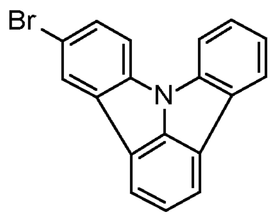 CAS-109589-98-2 | 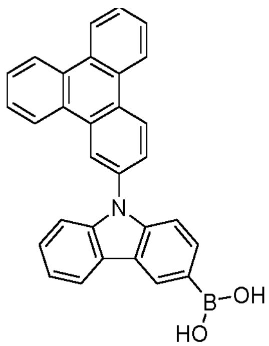 CAS-854952-58-2 | 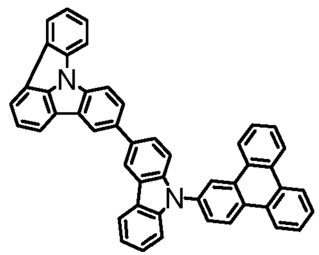 H5 | 64% |

(fortgesetzt)

| Edukt 1 | Edukt 2 | Produkt | Ausbeute |
|---|---|---|---|
| 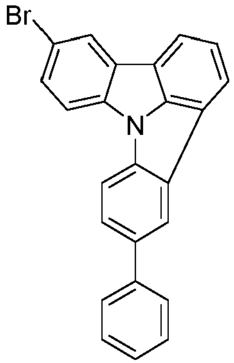 CAS-2108078-02-8 | 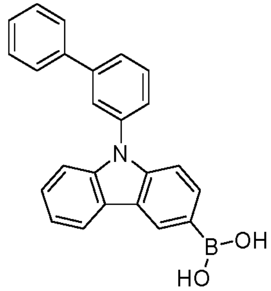 CAS-1416814-68-0 | 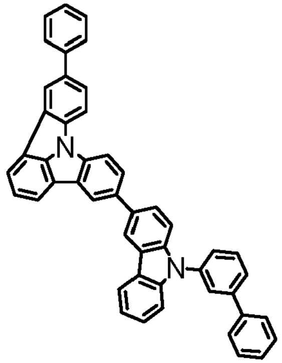 H7 | 55% |
| 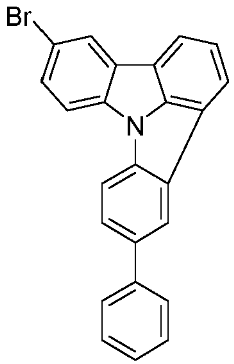 CAS-2108078-02- | 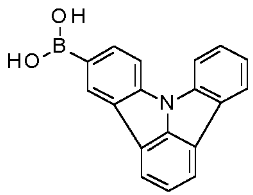 CAS-1174032-92-8 | 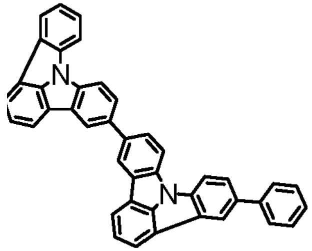 H8 | 50% |

(fortgesetzt)

| Edukt 1 | Edukt 2 | Produkt | Ausbeute |
|---|---|---|---|
| 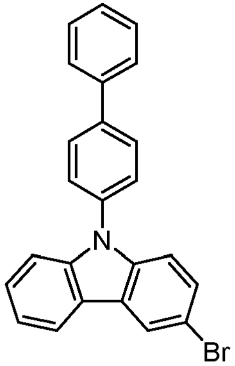 CAS-894791-46-9 | 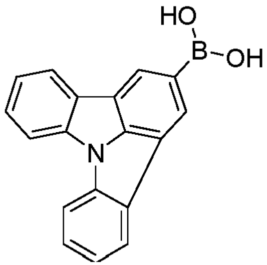 CAS-1174032-85-9 | 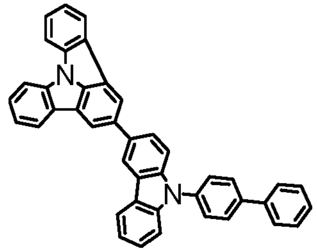 H9 | 45% |
| 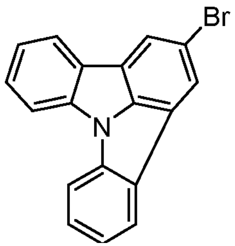 CAS-1174032-81-5 | 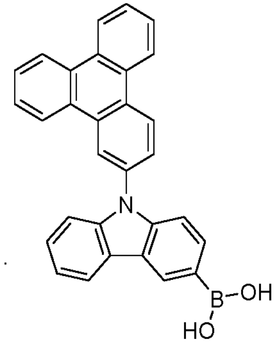 CAS-854952-58-2 | 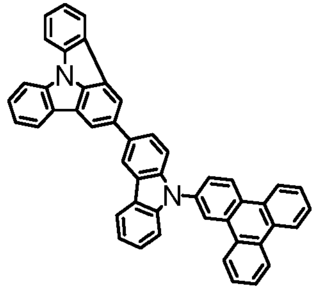 H10 | 58% |
| 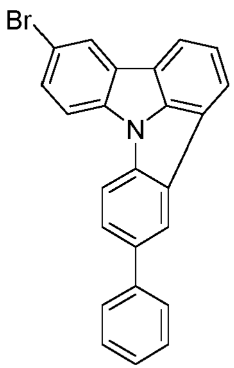 CAS-2108078-02- | 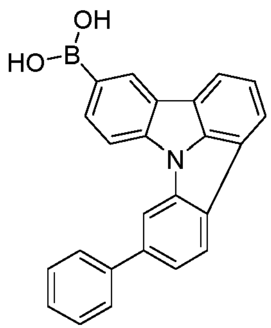 | 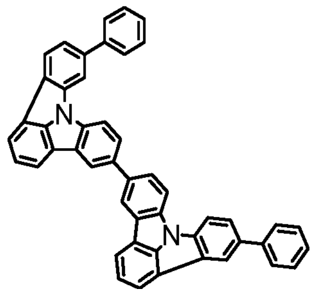 H12 | 62% |

(fortgesetzt)

| Edukt 1 | Edukt 2 | Produkt | Ausbeute |
|---|---|---|---|
| 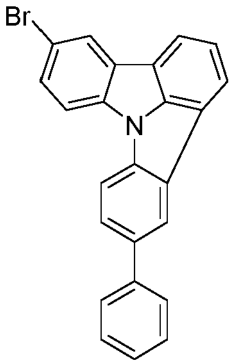 CAS-2108078-02 | 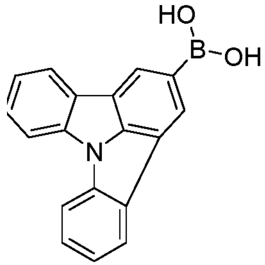 CAS-1174032-85-9 | 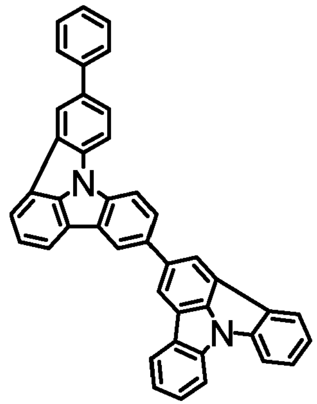 H13 | 38% |
| 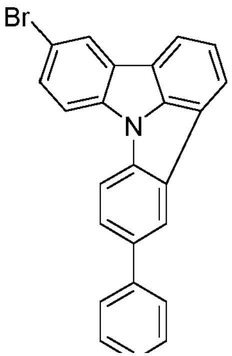 CAS-2108078-02- | 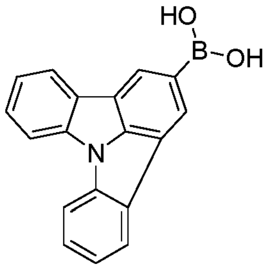 CAS-1174032-85-9 | 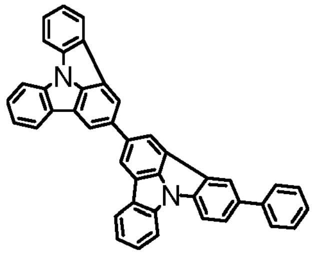 H14 | 46% |

(fortgesetzt)

| Edukt 1 | Edukt 2 | Produkt | Ausbeute |
|---|---|---|---|
| 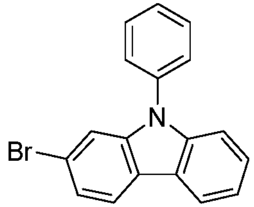 CAS-94994-62-4 | 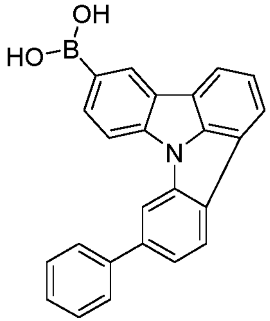 | 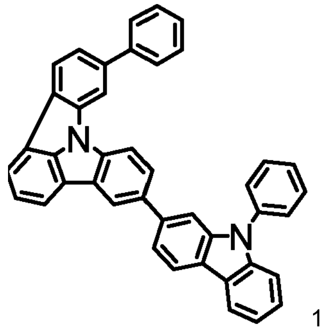 1 H16 | 40% |
| 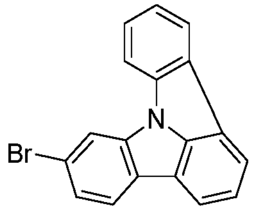 CAS-858507-84-3 | 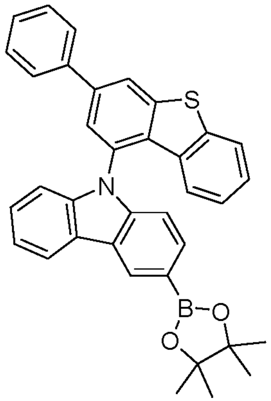 | 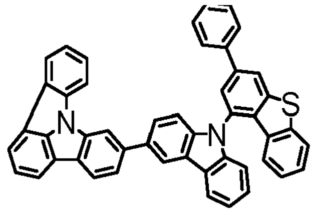 H18 | 52% |

(fortgesetzt)

| Edukt 1 | Edukt 2 | Produkt | Ausbeute |
|---|---|---|---|
| 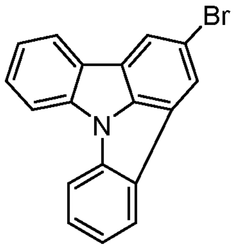 CAS-1174032-81-5 | 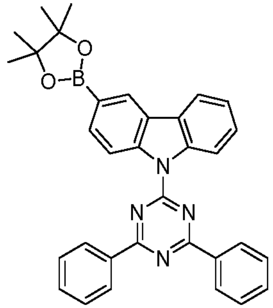 CAS-1361094-91-8 | 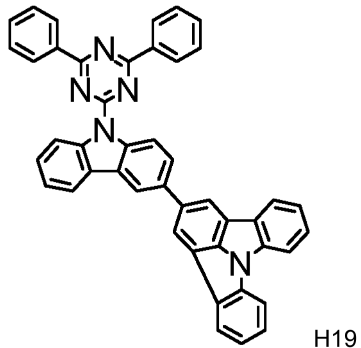 H19 | 41% |

## Patentansprüche

1. Organische elektrolumineszierende Vorrichtung umfassend eine Anode, eine Kathode und mindestens eine organische Schicht, enthaltend mindestens eine lichtemittierende Schicht, wobei die mindestens eine lichtemittierende Schicht mindestens eine Verbindung der Formel (1) als Hostmaterial 1 und mindestens eine Verbindung der Formel (2) als Hostmaterial 2 enthält,

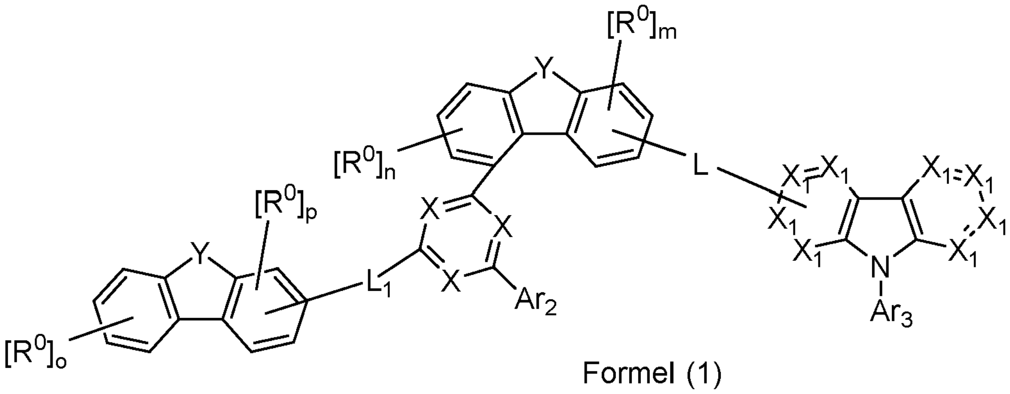

Formel (1) ,

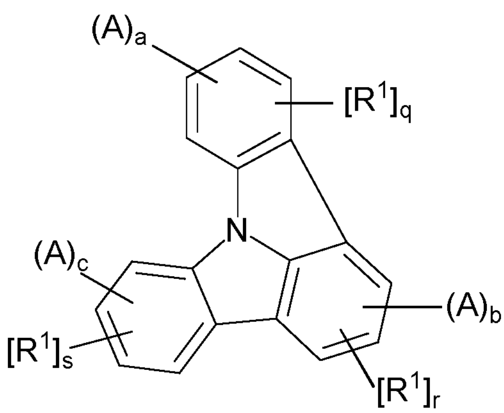

Formel (2) ,

wobei für die verwendeten Symbole und Indizes gilt:

X ist bei jedem Auftreten gleich oder verschieden $CR^0$ oder N, wobei mindestens ein Symbol X für N steht;
$X_1$ ist bei jedem Auftreten gleich oder verschieden CH, CR oder N, wobei maximal 3 Symbole $X_1$ N bedeuten können;
$X_2$ ist bei jedem Auftreten gleich oder verschieden CH, $CR^1$ oder N, wobei maximal 2 Symbole $X_2$ N bedeuten können;
Y ist bei jedem Auftreten gleich oder verschieden ausgewählt aus O oder S;
L und $L_1$ sind bei jedem Auftreten gleich oder verschieden eine Einfachbindung oder ein aromatisches oder heteroaromatisches Ringsystem mit 5 bis 30 aromatischen Ringatomen;
$R^0$ ist bei jedem Auftreten unabhängig voneinander H, D oder ein unsubstituiertes oder teilweise oder vollständig deuteriertes aromatisches Ringsystem mit 6 bis 18 C-Atomen;
R und R# sind bei jedem Auftreten gleich oder verschieden ausgewählt aus der Gruppe bestehend aus D, F, Cl, Br, I, CN, $NO_2$, $C(=O)R^2$, $P(=O)(Ar_1)_2$, $P(Ar_1)_2$, $B(Ar_1)_2$, $Si(Ar_1)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C=CR^2$, $Si(R^2)_2$, C=O, C=S, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 aromatischen Ringatomen, das jeweils mit einem oder meh-

reren Resten $R^2$ substituiert sein kann, einer Aryloxy- oder Heteroaryloxygruppe mit 5 bis 40 aromatischen Ringatomen, die mit einem oder mehreren Resten $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann; dabei können zwei Substituenten R, die an dasselbe Kohlenstoffatom oder an benachbarte Kohlenstoffatome gebunden sind oder ein Substituent R zusammen mit $Ar_3$ ein monocyclisches oder polycyclisches, aliphatisches, aromatisches oder heteroaromatisches Ringsystem bilden, das mit einem oder mehreren Resten $R^2$ substituiert sein kann;

$R^1$ ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen; dabei können zwei Substituenten $R^1$, die an dasselbe Kohlenstoffatom oder an benachbarte Kohlenstoffatome gebunden sind ein monocyclisches oder polycyclisches, aliphatisches, aromatisches oder heteroaromatisches Ringsystem bilden, das mit einem oder mehreren Resten $R^2$ substituiert sein kann;

$R^2$ ist bei jedem Auftreten gleich oder verschieden ausgewählt aus der Gruppe bestehend aus H, D, F, Cl, Br, I, CN, $NO_2$, $N(Ar_1)_2$, $NH_2$, $N(R^3)_2$, $C(=O)Ar_1$, C(=O)H, $C(=O)R^3$, $P(=O)(Ar_1)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, wobei eine oder mehrere nicht-benachbarte $CH_2$-Gruppen durch HC=CH, $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH oder $CONR^3$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 aromatischen Ringatomen, das jeweils mit einem oder mehreren Resten $R^3$ substituiert sein kann, einer Aryloxy- oder Heteroaryloxygruppe mit 5 bis 60 aromatischen Ringatomen, die mit einem oder mehreren Resten $R^3$ substituiert sein kann, oder einer Kombination dieser Systeme, wobei optional zwei oder mehr benachbarte Substituenten $R^2$ ein monocyclisches oder polycyclisches, aliphatisches, aromatisches oder heteroaromatisches Ringsystem bilden können, das mit einem oder mehreren Resten $R^3$ substituiert sein kann;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^3$ miteinander ein mono- oder polycyclisches, aliphatisches Ringsystem bilden;

$Ar_1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^3$ substituiert sein kann; dabei können zwei Reste $Ar_1$, welche an dasselbe N-Atom, P-Atom oder B-Atom binden, auch durch eine Einfachbindung oder eine Brücke, ausgewählt aus $N(R^3)$, $C(R^3)_2$, O oder S, miteinander verbrückt sein;

$Ar_2$ ist jeweils unabhängig voneinander bei jedem Auftreten eine Aryl- oder Heteroarylgruppe mit 5 bis 40 aromatischen Ringatomen, die mit einem oder mehreren Resten $R^2$ substituiert sein kann;

$Ar_3$ ist jeweils unabhängig voneinander bei jedem Auftreten ein aromatisches Ringsystem mit 6 bis 40 aromatischen Ringatomen oder ein heteroaromatisches Ringsystem mit 5 bis 40 aromatischen Ringatomen, das mit einem oder mehreren Resten $R^2$ substituiert sein kann;

A ist bei jedem Auftreten unabhängig voneinander eine Gruppe der Formel (3) oder (4),

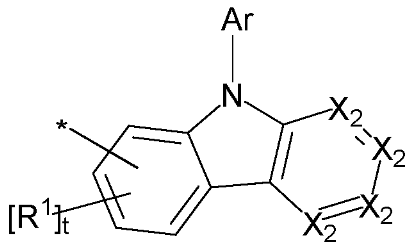

Formel (3) ,

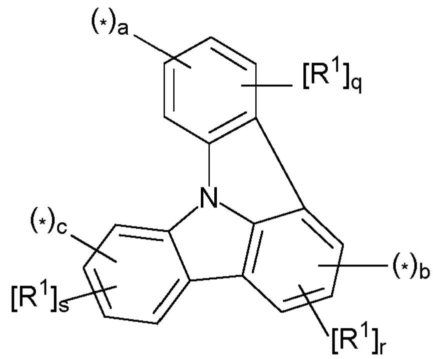

Formel (4) ;

Ar ist jeweils unabhängig voneinander bei jedem Auftreten eine Arylgruppe mit 6 bis 40 aromatischen Ringatomen, die mit einem oder mehreren Resten R# substituiert sein kann, oder eine Heteroarylgruppe mit 5 bis 40 aromatischen Ringatomen, die mit einem oder mehreren Resten R# substituiert sein kann;
* kennzeichnet die Bindungsstelle an die Formel (2);
a, b, c bedeuten jeweils unabhängig voneinander bei jedem Auftreten 0 oder 1, wobei die Summe der Indizes bei jedem Auftreten a+b+c 1 bedeutet;
o bedeutet 1, 2, 3 oder 4;
n, m und p bedeuten unabhängig voneinander bei jedem Auftreten 1, 2 oder 3; und
q, r, s, t bedeuten jeweils unabhängig bei jedem Auftreten 0 oder 1.

**2.** Organische elektrolumineszierende Vorrichtung nach Anspruch 1, **dadurch gekennzeichnet, dass** im Hostmaterial 1 L ausgewählt ist aus einer Bindung oder den Linkern der Gruppe L-1 bis L-33,

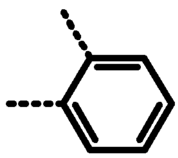

**L-1**

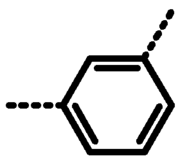

**L-2**

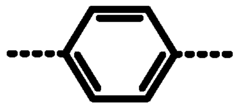

**L-3**

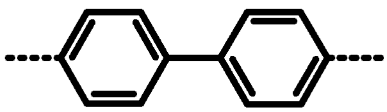

**L-4**

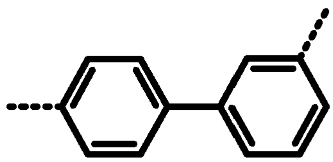

**L-5**

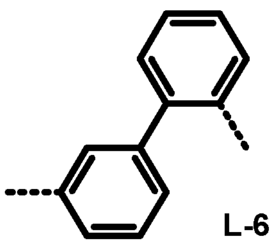

**L-6**

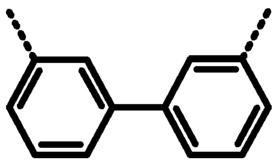

**L-7**

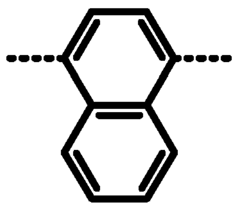

**L-8**

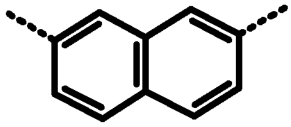

L-9

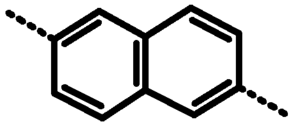

L-10

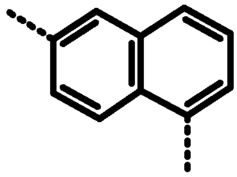

L-11

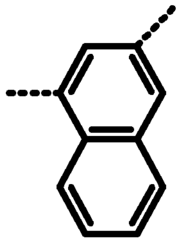

L-12

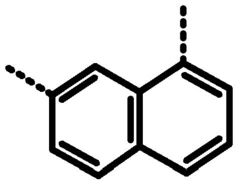

L-13

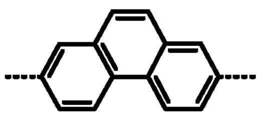

L-14

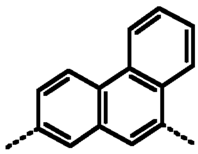

L-15

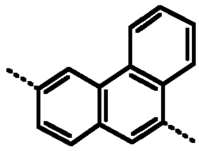

L-16

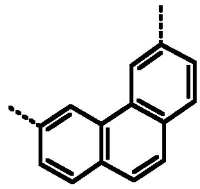

L-17

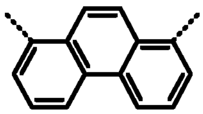

L-18

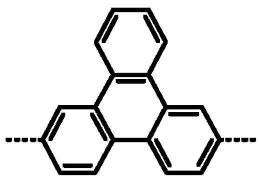

L-19

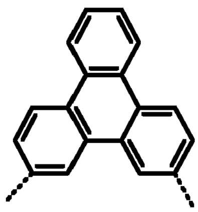

L-20

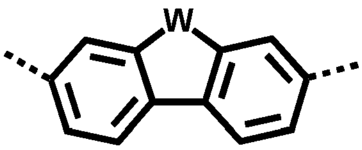

L-21

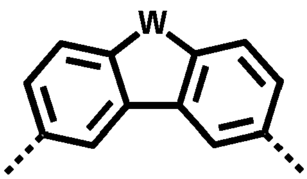

L-22

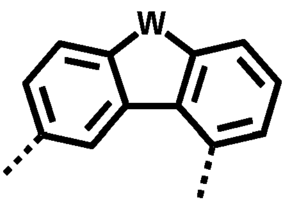

L-23

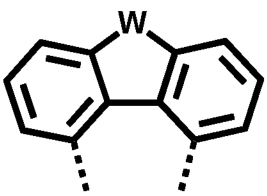

L-24

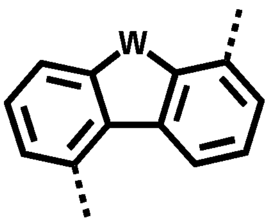

L-25

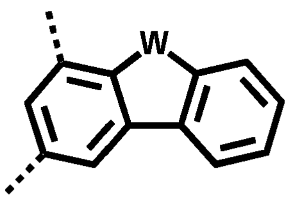

L-26

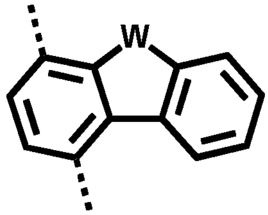

**L-27**

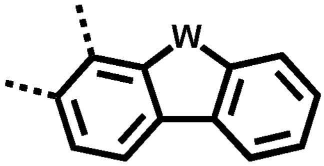

**L-28**

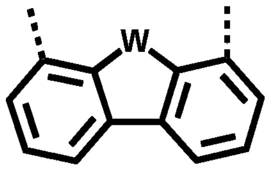

**L-29**

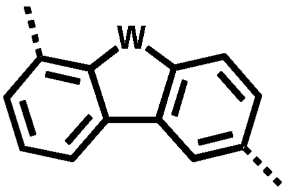

**L-30**

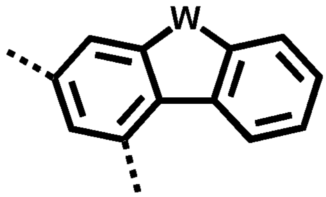

**L-31**

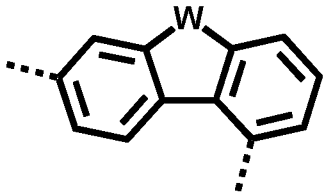

**L-32**

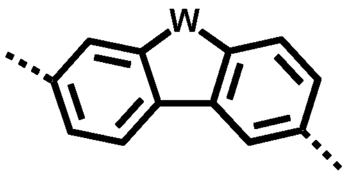

**L-33**

,

wobei W jeweils unabhängig voneinander O oder S bedeutet.

**3.** Organische elektrolumineszierende Vorrichtung nach Anspruch 1 oder 2, **dadurch gekennzeichnet, dass** das Hostmaterial 2 einer der Formeln (2a), (2b) oder (2c) entspricht,

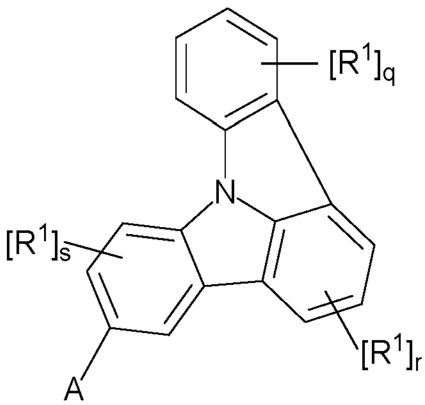

Formel (2a) ,

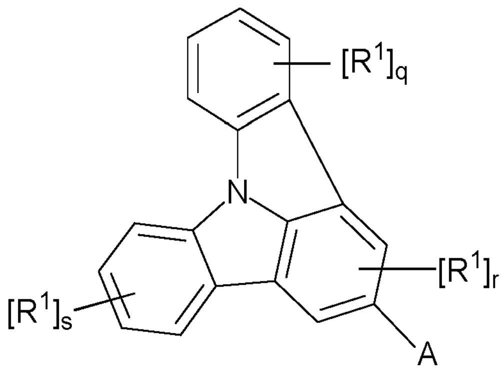

Formel (2b) ,

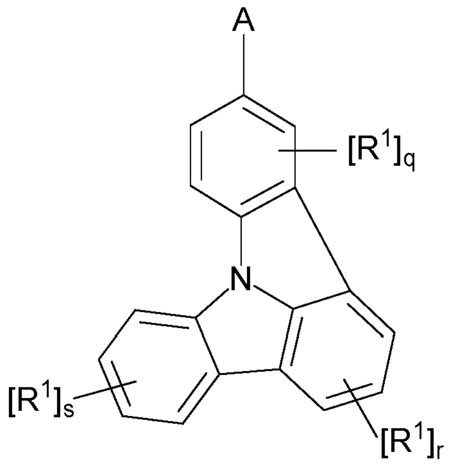

Formel (2c)

,

wobei die verwendeten Symbole und Indizes A, $R^1$, q, r und s eine Bedeutung wie in Anspruch 1 haben.

4. Organische elektrolumineszierende Vorrichtung nach einem oder mehreren der Ansprüche 1 bis 3, **dadurch gekennzeichnet, dass** im Hostmaterial 1, $L_1$ eine einfache Bindung ist.

5. Organische elektrolumineszierende Vorrichtung nach einem oder mehreren der Ansprüche 1 bis 4, **dadurch gekennzeichnet, dass** es sich um eine Elektrolumineszenzvorrichtung handelt, die ausgewählt ist aus den organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und den organischen lichtemittierenden Dioden (OLEDs).

6. Organische elektrolumineszierende Vorrichtung nach einem oder mehreren der Ansprüche 1 bis 5, **dadurch gekennzeichnet, dass** diese neben der Licht-emittierenden Schicht (EML), eine Lochinjektionsschicht (HIL), eine Lochtransportschicht (HTL), eine Elektronentransportschicht (ETL), eine Elektroneninjektionsschicht (EIL) und/oder eine Lochblockierschicht (HBL) enthält.

7. Organische elektrolumineszierende Vorrichtung nach einem oder mehreren der Ansprüche 1 bis 6, **dadurch gekennzeichnet, dass** die lichtemittierende Schicht neben dem mindestens einen Hostmaterial 1 und dem mindestens einen Hostmaterial 2 mindestens einen phosphoreszierenden Emitter enthält.

8. Organische elektrolumineszierende Vorrichtung nach Anspruch 7, **dadurch gekennzeichnet, dass** der phosphoreszierende Emitter der Formel (III) entspricht,

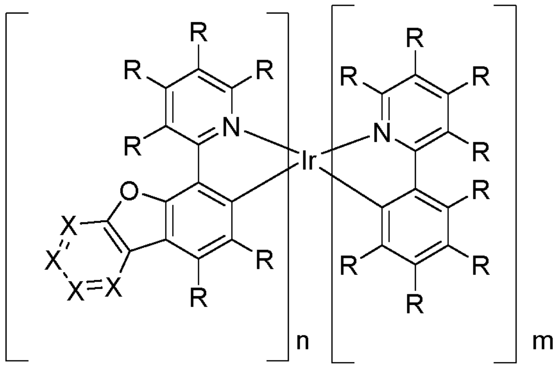

Formel (III)

,

wobei die Symbole und Indizes für diese Formel (III) die Bedeutung haben:

n+m ist 3, n ist 1 oder 2, m ist 2 oder 1,
X ist N oder CR,
R ist H, D oder eine verzweigte oder lineare Alkylgruppe oder eine teilweise oder vollständig deuterierte verzweigte oder lineare Alkylgruppe.

**9.** Verfahren zur Herstellung einer Vorrichtung nach einem oder mehreren der Ansprüche 1 bis 8, **dadurch gekennzeichnet, dass** die lichtemittierende Schicht durch Gasphasenabscheidung oder aus Lösung aufgebracht wird.

**10.** Verfahren nach Anspruch 9, **dadurch gekennzeichnet, dass** die mindestens eine Verbindung der Formel (1) und die mindestens eine Verbindung der Formel (2), nacheinander oder gleichzeitig aus mindestens zwei Materialquellen, gegebenenfalls mit dem mindestens einen phosphoreszierenden Emitter, aus der Gasphase abgeschieden werden und die lichtemittierende Schicht bilden.

**11.** Verfahren nach Anspruch 9, **dadurch gekennzeichnet, dass** die mindestens eine Verbindung der Formel (1) und die mindestens eine Verbindung der Formel (2) als Mischung, nacheinander oder gleichzeitig mit dem mindestens einen phosphoreszierenden Emitter, aus der Gasphase abgeschieden werden und die lichtemittierende Schicht bilden.

**12.** Verfahren nach Anspruch 9, **dadurch gekennzeichnet, dass** die mindestens eine Verbindung der Formel (1) und die mindestens eine Verbindung der Formel (2) zusammen mit dem mindestens einen phosphoreszierenden Emitter, aus einer Lösung aufgebracht werden, um die lichtemittierende Schicht zu bilden.

**13.** Mischung enthaltend mindestens eine Verbindung der Formel (1) als Hostmaterial 1 und mindestens eine Verbindung der Formel (2) als Hostmaterial 2,

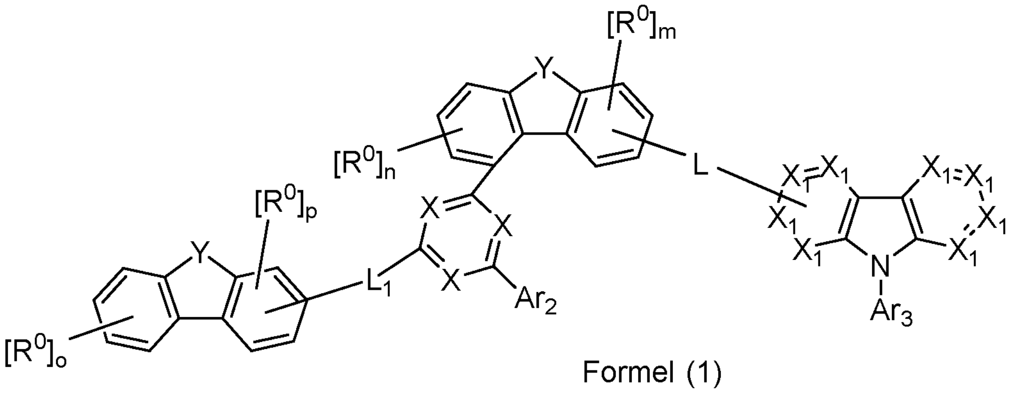

Formel (1)

,

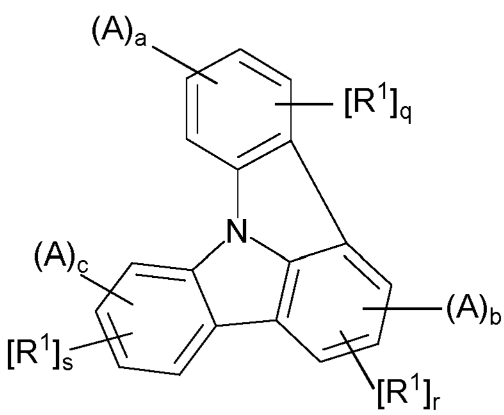

Formel (2)

,

wobei für die verwendeten Symbole und Indizes gilt:

X ist bei jedem Auftreten gleich oder verschieden $CR^0$ oder N, wobei mindestens ein Symbol X für N steht;
$X_1$ ist bei jedem Auftreten gleich oder verschieden CH, CR oder N, wobei maximal 3 Symbole $X_1$ N bedeuten können;
$X_2$ ist bei jedem Auftreten gleich oder verschieden CH, $CR^1$ oder N, wobei maximal 2 Symbole $X_2$ N bedeuten können;
Y ist bei jedem Auftreten gleich oder verschieden ausgewählt aus O oder S;
L und $L_1$ sind bei jedem Auftreten gleich oder verschieden eine Einfachbindung oder ein aromatisches oder heteroaromatisches Ringsystem mit 5 bis 30 aromatischen Ringatomen;
$R^0$ ist bei jedem Auftreten unabhängig voneinander H, D oder ein unsubstituiertes oder teilweise oder vollständig deuteriertes aromatisches Ringsystem mit 6 bis 18 C-Atomen;
R und R# sind bei jedem Auftreten gleich oder verschieden ausgewählt aus der Gruppe bestehend aus D, F, Cl, Br, I, CN, $NO_2$, $C(=O)R^2$, $P(=O)(Ar_1)_2$, $P(Ar_1)_2$, $B(Ar_1)_2$, $Si(Ar_1)_3$, $Si(R^2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nichtbenachbarte $CH_2$-Gruppen durch $R^2C=CR^2$, $Si(R^2)_2$, C=O, C=S, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 aromatischen Ringatomen, das jeweils mit einem oder mehreren Resten $R^2$ substituiert sein kann, einer Aryloxy- oder Heteroaryloxygruppe mit 5 bis 40 aromatischen Ringatomen, die mit einem oder mehreren Resten $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann; dabei können zwei Substituenten R, die an dasselbe Kohlenstoffatom oder an benachbarte Kohlenstoffatome gebunden sind oder ein Substituent R zusammen mit $Ar_3$ ein monocyclisches oder polycyclisches, aliphatisches, aromatisches oder heteroaromatisches Ringsystem bilden, das mit einem oder mehreren Resten $R^2$ substituiert sein kann;
$R^1$ ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen; dabei können zwei Substituenten $R^1$, die an dasselbe Kohlenstoffatom oder an benachbarte Kohlenstoffatome gebunden sind ein monocyclisches oder polycyclisches, aliphatisches, aromatisches oder heteroaromatisches Ringsystem bilden, das mit einem oder mehreren Resten $R^2$ substituiert sein kann;
$R^2$ ist bei jedem Auftreten gleich oder verschieden ausgewählt aus der Gruppe bestehend aus H, D, F, Cl, Br, I, CN, $NO_2$, $N(Ar_1)_2$, $NH_2$, $N(R^3)_2$, $C(=O)Ar_1$, C(=O)H, $C(=O)R^3$, $P(=O)(Ar_1)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, wobei eine oder mehrere nicht-benachbarte $CH_2$-Gruppen durch HC=CH, $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH oder $CONR^3$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 aromatischen Ringatomen, das jeweils mit einem oder mehreren Resten $R^3$ substituiert sein kann, einer Aryloxy- oder Heteroaryloxygruppe mit 5 bis 60 aromatischen Ringatomen, die mit einem oder mehreren Resten $R^3$ substituiert sein kann, oder einer Kombination dieser Systeme, wobei optional zwei oder mehr benachbarte Substituenten $R^2$ ein monocyclisches oder polycyclisches, aliphatisches, aromatisches oder heteroaromatisches Ringsystem bilden können, das mit einem oder mehreren Resten $R^3$ substituiert sein kann;
$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^3$ miteinander ein mono- oder polycyclisches, aliphatisches Ringsystem bilden;
$Ar_1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^3$ substituiert sein kann; dabei können zwei Reste Ar1, welche an dasselbe N-Atom, P-Atom oder B-Atom binden, auch durch eine Einfachbindung oder eine Brücke, ausgewählt aus $N(R^3)$, $C(R^3)_2$, O oder S, miteinander verbrückt sein;
$Ar_2$ ist jeweils unabhängig voneinander bei jedem Auftreten eine Aryl- oder Heteroarylgruppe mit 5 bis 40 aromatischen Ringatomen, die mit einem oder mehreren Resten $R^2$ substituiert sein kann;

$Ar_3$ ist jeweils unabhängig voneinander bei jedem Auftreten ein aromatisches Ringsystem mit 6 bis 40 aromatischen Ringatomen oder ein heteroaromatisches Ringsystem mit 5 bis 40 aromatischen Ringatomen, das mit einem oder mehreren Resten $R^2$ substituiert sein kann;
A ist bei jedem Auftreten unabhängig voneinander eine Gruppe der Formel (3) oder (4),

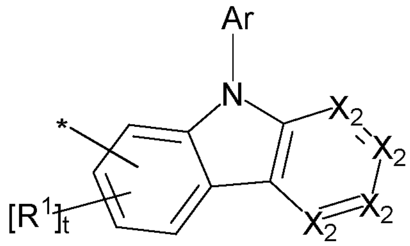

Formel (3) ,

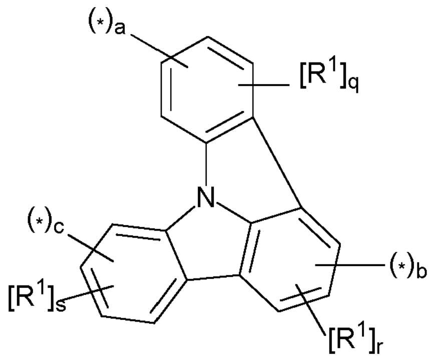

Formel (4) ;

Ar ist jeweils unabhängig voneinander bei jedem Auftreten eine Arylgruppe mit 6 bis 40 aromatischen Ringatomen, die mit einem oder mehreren Resten R# substituiert sein kann, oder eine Heteroarylgruppe mit 5 bis 40 aromatischen Ringatomen, die mit einem oder mehreren Resten R# substituiert sein kann;
* kennzeichnet die Bindungsstelle an die Formel (2);
a, b, c bedeuten jeweils unabhängig voneinander bei jedem Auftreten 0 oder 1, wobei die Summe der Indizes bei jedem Auftreten a+b+c 1 bedeutet;
o bedeutet 1, 2, 3 oder 4;
n, m und p bedeuten unabhängig voneinander bei jedem Auftreten 1, 2 oder 3; und
q, r, s, t bedeuten jeweils unabhängig bei jedem Auftreten 0 oder 1.

**14.** Mischung nach Anspruch 13, **dadurch gekennzeichnet, dass** die Mischung aus mindestens einer Verbindung der Formel (1), mindestens einer Verbindung der Formel (2) und einem phosphoreszierenden Emitter besteht.

**15.** Formulierung enthaltend eine Mischung nach Anspruch 13 oder 14 sowie mindestens ein Lösemittel.

## Claims

**1.** Organic electroluminescent device comprising an anode, a cathode and at least one organic layer, comprising at least one light-emitting layer, where the at least one light-emitting layer comprises at least one compound of the formula (1) as host material 1 and at least one compound of the formula (2) as host material 2

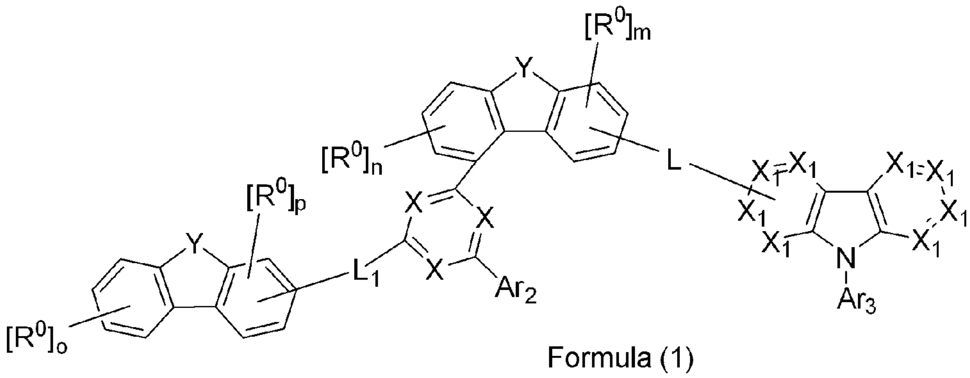

Formula (1)

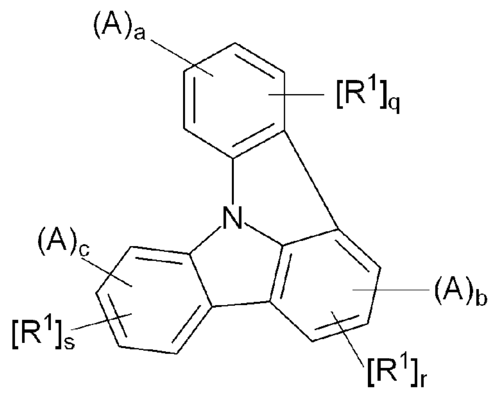

Formula (2)

where the following applies to the symbols and indices used:

X is on each occurrence, identically or differently, $CR^0$ or N, where at least one symbol X stands for N;
$X_1$ is on each occurrence, identically or differently, CH, CR or N, where a maximum of 3 symbols $X_1$ can denote N;
$X_2$ is on each occurrence, identically or differently, CH, $CR^1$ or N, where a maximum of 2 symbols $X_2$ can denote N;
Y is selected on each occurrence, identically or differently, from O or S;
L and $L_1$ are on each occurrence, identically or differently, a single bond or an aromatic or heteroaromatic ring system having 5 to 30 aromatic ring atoms;
R° is on each occurrence, independently of one another, H, D or an unsubstituted or partially or fully deuterated aromatic ring system having 6 to 18 C atoms;
R and R# are selected on each occurrence, identically or differently, from the group consisting of D, F, Cl, Br, I, CN, $NO_2$, $C(=O)R^2$, $P(=O)(Ar_1)_2$, $P(Ar_1)_2$, $B(Ar_1)_2$, $Si(Ar_1)_3$, $Si(R^2)_3$, a straightchain alkyl, alkoxy or thioalkyl group having 1 to 20 C atoms or a branched or cyclic alkyl, alkoxy or thioalkyl group having 3 to 20 C atoms or an alkenyl group having 2 to 20 C atoms, which may in each case be substituted by one or more radicals $R^2$, where one or more non-adjacent $CH_2$ groups may be replaced by $R^2C=CR^2$, $Si(R^2)_2$, C=O, C=S, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, an aromatic or heteroaromatic ring system having 5 to 40 aromatic ring atoms, which may in each case be substituted by one or more radicals $R^2$, an aryloxy or heteroaryloxy group having 5 to 40 aromatic ring atoms, which may be substituted by one or more radicals $R^2$, or an aralkyl or heteroaralkyl group having 5 to 40 aromatic ring atoms, which may be substituted by one or more radicals $R^2$; two substituents R that are bonded to the same carbon atom or to adjacent carbon atoms or a substituent R together with $Ar_3$ may form a monocyclic or polycyclic, aliphatic, aromatic or heteroaromatic ring system, which may be substituted by one or more radicals $R^2$;
$R^1$ is selected on each occurrence, identically or differently, from the group consisting of CN, a straight-chain alkyl, alkoxy or thioalkyl group having 1 to 20 C atoms or a branched or cyclic alkyl, alkoxy or thioalkyl group having 3 to 20 C atoms, an aromatic or heteroaromatic ring system having 5 to 40 aromatic ring atoms, an aryloxy or heteroaryloxy group having 5 to 40 aromatic ring atoms, or an aralkyl or heteroaralkyl group having 5 to 40 aromatic ring atoms; two substituents $R^1$ that are bonded to the same carbon atom or to adjacent carbon atoms may form a monocyclic or polycyclic, aliphatic, aromatic or heteroaromatic ring system, which may be substituted by one or more radicals $R^2$;
$R^2$ is selected on each occurrence, identically or differently, from the group consisting of H, D, F, Cl, Br, I, CN, $NO_2$, $N(Ar_1)_2$, $NH_2$, $N(R^3)_2$, $C(=O)Ar_1$, C(=O)H, $C(=O)R^3$, $P(=O)(Ar_1)_2$, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals $R^3$, where one or more non-adjacent $CH_2$ groups may be replaced by HC=CH, $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH or $CONR^3$ and where one or more H atoms may be replaced by D, F, Cl, Br, I, CN or $NO_2$, an aromatic or heteroaromatic ring system having 5 to 60 aromatic ring atoms, which may in each case be substituted by one or more radicals $R^3$, an aryloxy or heteroaryloxy group having 5 to 60 aromatic ring atoms, which may be substituted by one or more radicals $R^3$, or a combination of these systems; where two or more adjacent substituents $R^2$ may optionally form a monocyclic or polycyclic, aliphatic, aromatic or heteroaromatic ring system, which may be substituted by one or more radicals $R^3$;

$R^3$ is selected on each occurrence, identically or differently, from the group consisting of H, D, F, CN, an aliphatic hydrocarbon radical having 1 to 20 C atoms or an aromatic or heteroaromatic ring system having 5 to 30 aromatic ring atoms, in which one or more H atoms may be replaced by D, F, Cl, Br, I or CN and which may be substituted by one or more alkyl groups having 1 to 4 carbon atoms each; two or more adjacent substituents $R^3$ may form a mono- or polycyclic, aliphatic ring system with one another;

$Ar_1$ is on each occurrence, identically or differently, an aromatic or heteroaromatic ring system having 5 to 30 aromatic ring atoms, which may be substituted by one or more non-aromatic radicals $R^3$; two radicals $Ar_1$ that are bonded to the same N atom, P atom or B atom may also be bridged to one another by a single bond or a bridge selected from $N(R^3)$, $C(R^3)_2$, O or S;

$Ar_2$ is on each occurrence, in each case independently of one another, an aryl or heteroaryl group having 5 to 40 aromatic ring atoms, which may be substituted by one or more radicals $R^2$;

$Ar_3$ is on each occurrence, in each case independently of one another, an aromatic ring system having 6 to 40 aromatic ring atoms or a heteroaromatic ring system having 5 to 40 aromatic ring atoms, which may be substituted by one or more radicals $R^2$;

A is on each occurrence, independently of one another, a group of the formula (3) or (4),

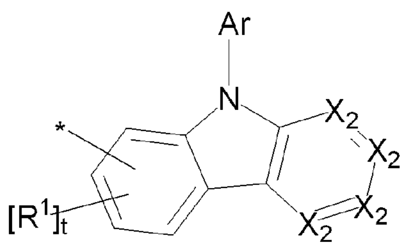

Formula (3) ,

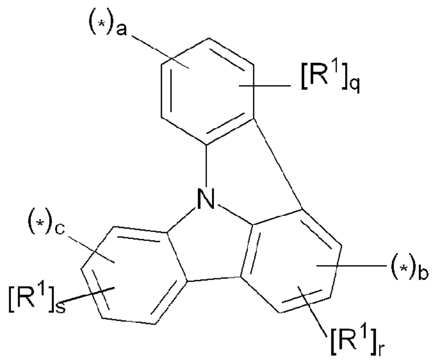

Formula (4) ;

Ar is on each occurrence, in each case independently of one another, an aryl group having 6 to 40 aromatic ring atoms, which may be substituted by one or more radicals R#, or a heteroaryl group having 5 to 40 aromatic ring atoms, which may be substituted by one or more radicals R#;

* denotes the bonding site to the formula (2);

a, b, c on each occurrence, in each case independently of one another, denote 0 or 1, where the sum of the indices a+b+c on each occurrence is 1;

o denotes 1, 2, 3 or 4;

n, m and p on each occurrence, independently of one another, denote 1, 2 or 3; and

q, r, s, t on each occurrence, in each case independently, denote 0 or 1.

2. Organic electroluminescent device according to Claim 1, **characterised in that**, in host material 1, L is selected from a bond or the linkers from the group L-1 to L-33,

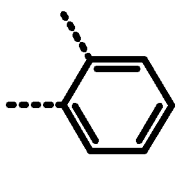

**L-1**

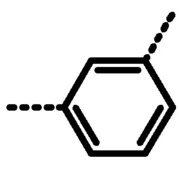

**L-2**

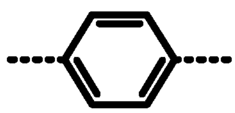

**L-3**

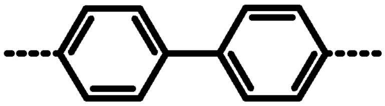

L-4

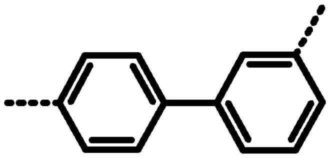

L-5

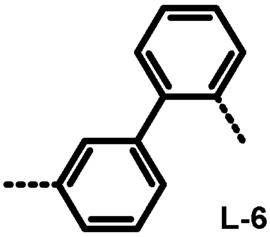

L-6

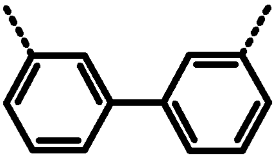

L-7

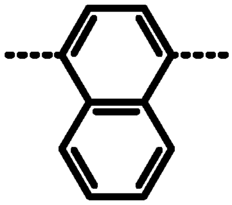

L-8

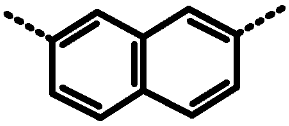

L-9

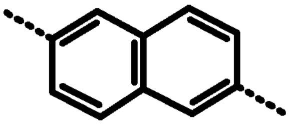

L-10

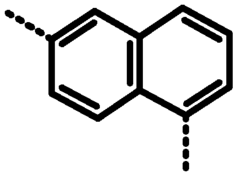

L-11

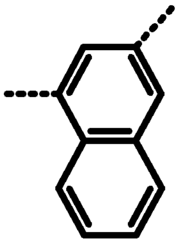

L-12

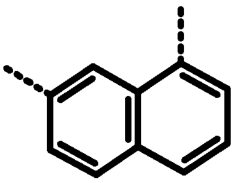

L-13

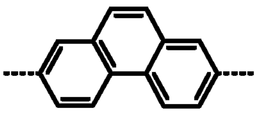

L-14

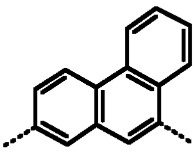

L-15

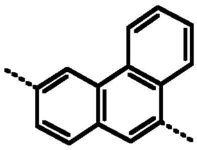

L-16

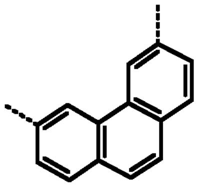

L-17

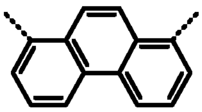

L-18

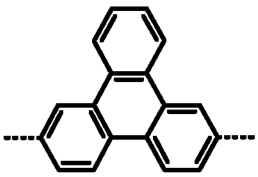

L-19

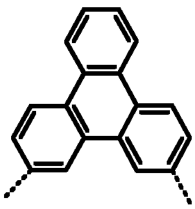

L-20

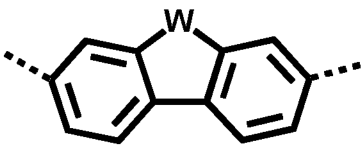 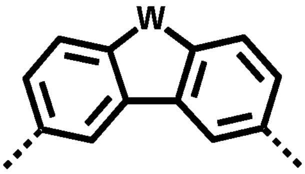 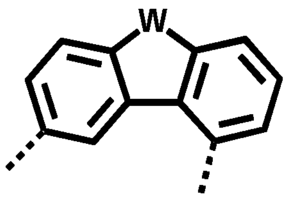

**L-21** **L-22** **L-23**

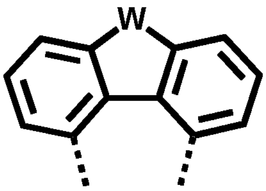 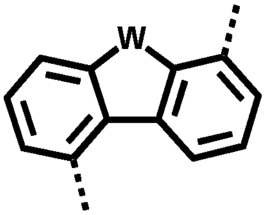 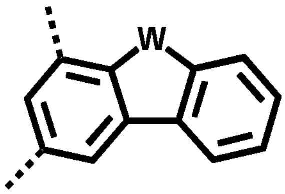

**L-24** **L-25** **L-26**

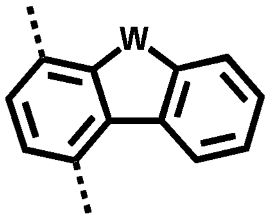 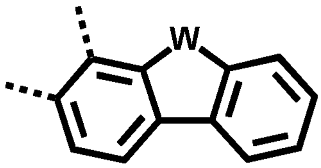 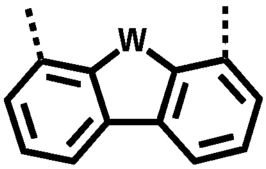

**L-27** **L-28** **L-29**

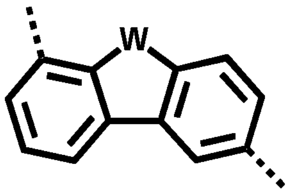 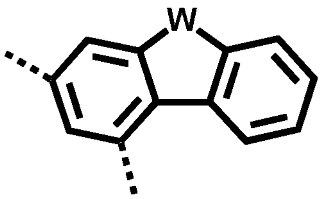 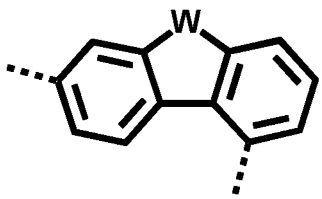

**L-30** **L-31** **L-32**

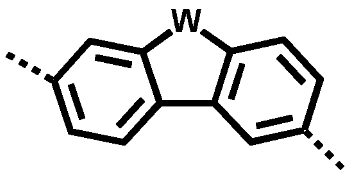

**L-33**

,

where W in each case, independently of one another, denotes O or S.

3. Organic electroluminescent device according to Claim 1 or 2, **characterised in that** the host material 2 conforms to one of the formulae (2a), (2b) or (2c)

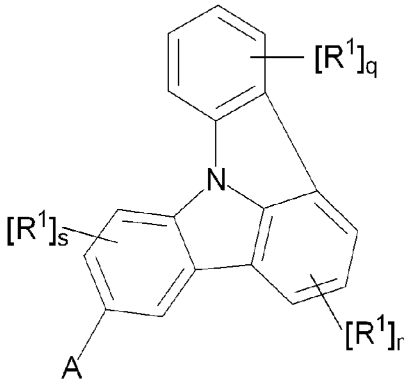

Formula (2a) ,

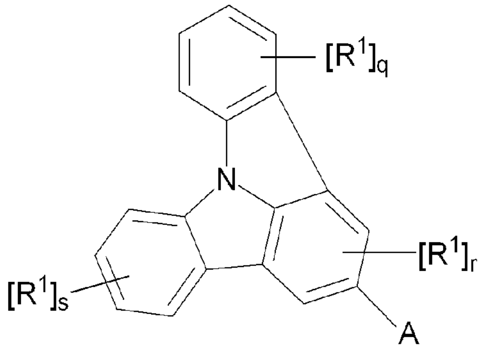

Formula (2b) ,

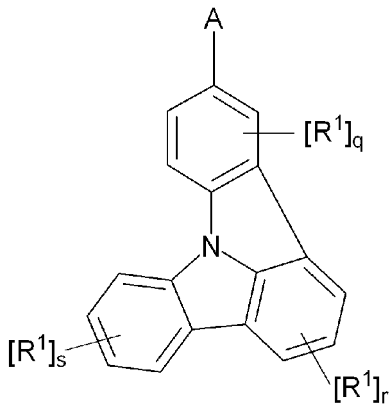

Formula (2c)

where the symbols and indices A, $R^1$, q, r and s used have a meaning as in Claim 1.

**4.** Organic electroluminescent device according to one or more of Claims 1 to 3, **characterised in that**, in host material 1, $L_1$ is a single bond.

**5.** Organic electroluminescent device according to one or more of Claims 1 to 4, **characterised in that** it is an electroluminescent device selected from organic light-emitting transistors (OLETs), organic field-quench devices (OFQDs), organic light-emitting electrochemical cells (OLECs, LECs, LEECs), organic laser diodes (O-lasers) and organic light-emitting diodes (OLEDs).

**6.** Organic electroluminescent device according to one or more of Claims 1 to 5, **characterised in that**, besides the light-emitting layer (EML), it comprises a hole-injection layer (HIL), a hole-transport layer (HTL), an electron-transport layer (ETL), an electron-injection layer (EIL) and/or a hole-blocking layer (HBL).

**7.** Organic electroluminescent device according to one or more of Claims 1 to 6, **characterised in that**, besides the at least one host material 1 and the at least one host material 2, the light-emitting layer comprises at least one phosphorescent emitter.

**8.** Organic electroluminescent device according to Claim 7, **characterised in that** the phosphorescent emitter conforms to the formula (III)

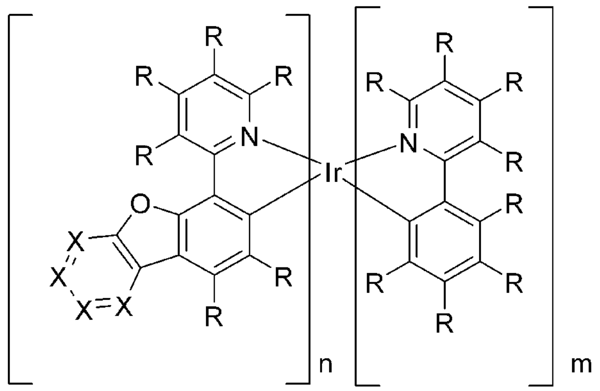

Formula (III)

where the symbols and indices for this formula (III) have the meaning:

n+m is 3, n is 1 or 2, m is 2 or 1,
X is N or CR,
R is H, D or a branched or linear alkyl group or a partially or fully deuterated branched or linear alkyl group.

**9.** Process for the production of a device according to one or more of Claims 1 to 8, **characterised in that** the light-emitting layer is applied by gas-phase deposition or from solution.

**10.** Process according to Claim 9, **characterised in that** the at least one compound of the formula (1) and the at least one compound of the formula (2) are deposited from the gas phase, successively or simultaneously from at least two material sources, optionally with the at least one phosphorescent emitter, and form the light-emitting layer.

**11.** Process according to Claim 9, **characterised in that** the at least one compound of the formula (1) and the at least one compound of the formula (2) are deposited from the gas phase as a mixture, successively or simultaneously with the at least one phosphorescent emitter, and form the light-emitting layer.

**12.** Process according to Claim 9, **characterised in that** the at least one compound of the formula (1) and the at least one compound of the formula (2) are applied from a solution together with the at least one phosphorescent emitter in order to form the light-emitting layer.

**13.** Mixture comprising at least one compound of the formula (1) as host material 1 and at least one compound of the formula (2) as host material 2

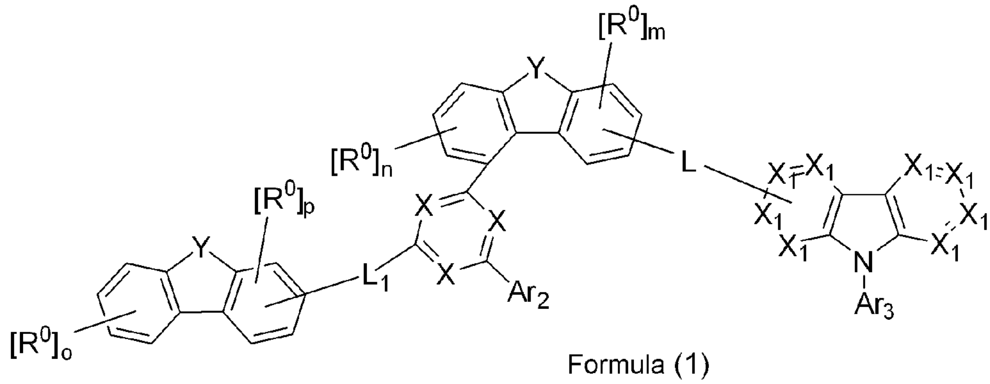

Formula (1)

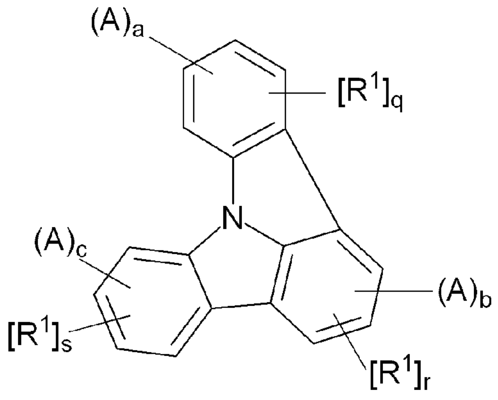

Formula (2)

where the following applies to the symbols and indices used:

X is on each occurrence, identically or differently, $CR^0$ or N, where at least one symbol X stands for N;
$X_1$ is on each occurrence, identically or differently, CH, CR or N, where a maximum of 3 symbols $X_1$ can denote N;
$X_2$ is on each occurrence, identically or differently, CH, $CR^1$ or N, where a maximum of 2 symbols $X_2$ can denote N;
Y is selected on each occurrence, identically or differently, from O or S;
L and $L_1$ are on each occurrence, identically or differently, a single bond or an aromatic or heteroaromatic ring system having 5 to 30 aromatic ring atoms;
R° is on each occurrence, independently of one another, H, D or an unsubstituted or partially or fully deuterated aromatic ring system having 6 to 18 C atoms;
R and R# are selected on each occurrence, identically or differently, from the group consisting of D, F, Cl, Br, I, CN, $NO_2$, $C(=O)R^2$, $P(=O)(Ar_1)_2$, $P(Ar_1)_2$, $B(Ar_1)2$, $Si(Ar_1)3$, $Si(R^2)_3$, a straight-chain alkyl, alkoxy or thioalkyl group having 1 to 20 C atoms or a branched or cyclic alkyl, alkoxy or thioalkyl group having 3 to 20 C atoms or an alkenyl group having 2 to 20 C atoms, which may in each case may be substituted by one or more radicals $R^2$, where one or more non-adjacent $CH_2$ groups may be replaced by $R^2C=CR^2$, $Si(R^2)_2$, C=O, C=S, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, an aromatic or heteroaromatic ring system having 5 to 40 aromatic ring atoms, which may in each case be substituted by one or more radicals $R^2$, an aryloxy or heteroaryloxy group having 5 to 40 aromatic ring atoms, which may be substituted by one or more radicals $R^2$, or an aralkyl or heteroaralkyl group having 5 to 40 aromatic ring atoms, which may be substituted by one or more radicals $R^2$; two substituents R that are bonded to the same carbon atom or to adjacent carbon atoms or a substituent R together with $Ar_3$ may form a monocyclic or polycyclic, aliphatic, aromatic or heteroaromatic ring system, which may be substituted by one or more radicals $R^2$;
$R^1$ is selected on each occurrence, identically or differently, from the group consisting of CN, a straight-chain alkyl, alkoxy or thioalkyl group having 1 to 20 C atoms or a branched or cyclic alkyl, alkoxy or thioalkyl group having 3 to 20 C atoms, an aromatic or heteroaromatic ring system having 5 to 40 aromatic ring atoms, an aryloxy or heteroaryloxy group having 5 to 40 aromatic ring atoms, or an aralkyl or heteroaralkyl group having 5 to 40 aromatic ring atoms; two substituents $R^1$ that are bonded to the same carbon atom or to adjacent carbon atoms may form a monocyclic or polycyclic, aliphatic, aromatic or heteroaromatic ring system, which may be substituted by one or more radicals $R^2$;
$R^2$ is selected on each occurrence, identically or differently, from the group consisting of H, D, F, Cl, Br, I, CN, $NO_2$, $N(Ar_1)_2$, $NH_2$, $N(R^3)_2$, $C(=O)Ar_1$, C(=O)H, $C(=O)R^3$, $P(=O)(Ar_1)_2$, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals $R^3$, where one or more non-adjacent $CH_2$ groups may be replaced by HC=CH, $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH or $CONR^3$ and where one or more H atoms may be replaced by D, F, Cl, Br, I, CN or $NO_2$, an aromatic or heteroaromatic ring system having 5 to 60 aromatic ring atoms, which may in each case be substituted by one or more radicals $R^3$, an aryloxy or heteroaryloxy group having 5 to 60 aromatic ring atoms, which may be substituted by one or more radicals $R^3$, or a combination of these systems; where two or more adjacent substituents $R^2$ may optionally form a monocyclic or polycyclic, aliphatic, aromatic or heteroaromatic ring system, which may be substituted by one or more radicals $R^3$;

$R^3$ is selected on each occurrence, identically or differently, from the group consisting of H, D, F, CN, an aliphatic hydrocarbon radical having 1 to 20 C atoms, or an aromatic or heteroaromatic ring system having 5 to 30 aromatic ring atoms, in which one or more H atoms may be replaced by D, F, Cl, Br, I or CN and which may be substituted by one or more alkyl groups having 1 to 4 carbon atoms each; two or more adjacent substituents $R^3$ may form a mono- or polycyclic, aliphatic ring system with one another;

$Ar_1$ is on each occurrence, identically or differently, an aromatic or heteroaromatic ring system having 5 to 30 aromatic ring atoms, which may be substituted by one or more non-aromatic radicals $R^3$; two radicals $Ar_1$ that are bonded to the same N atom, P atom or B atom may also be bridged to one another by a single bond or a bridge selected from $N(R^3)$, $C(R^3)_2$, O or S;

$Ar_2$ is on each occurrence, in each case independently of one another, an aryl or heteroaryl group having 5 to 40 aromatic ring atoms, which may be substituted by one or more radicals $R^2$;

$Ar_3$ is on each occurrence, in each case independently of one another, an aromatic ring system having 6 to 40 aromatic ring atoms or a heteroaromatic ring system having 5 to 40 aromatic ring atoms, which may be substituted by one or more radicals $R^2$;

A is on each occurrence, independently of one another, a group of the formula (3) or (4),

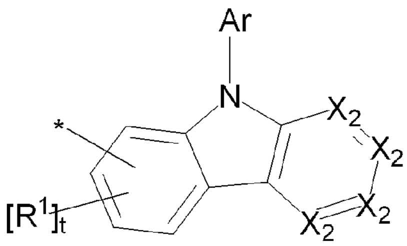

Formula (3) ,

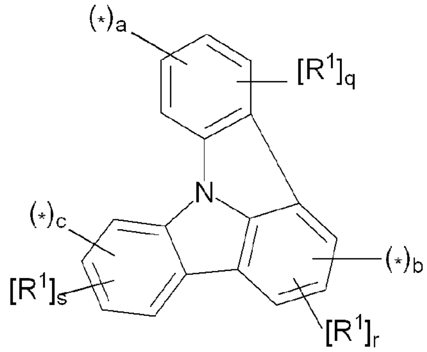

Formula (4) ;

Ar is on each occurrence, in each case independently of one another, an aryl group having 6 to 40 aromatic ring atoms, which may be substituted by one or more radicals R#, or a heteroaryl group having 5 to 40 aromatic ring atoms, which may be substituted by one or more radicals R#;

* denotes the bonding site to the formula (2);

a, b, c on each occurrence, in each case independently of one another, denote 0 or 1, where the sum of the indices a+b+c on each occurrence is 1;

o denotes 1, 2, 3 or 4;

n, m and p on each occurrence, independently of one another, denote 1, 2 or 3; and

q, r, s, t on each occurrence, in each case independently, denote 0 or 1.

14. Mixture according to Claim 13, **characterised in that** the mixture consists of at least one compound of the formula (1), at least one compound of the formula (2) and a phosphorescent emitter.

15. Formulation comprising a mixture according to Claim 13 or 14 and at least one solvent.

## Revendications

1. Dispositif électroluminescent organique comprenant une anode, une cathode et au moins une couche organique, comprenant au moins une couche émettrice de lumière, où la au moins une couche émettrice de lumière comprend au moins un composé de formule (1) comme matériau hôte 1 et au moins un composé de formule (2) comme matériau hôte 2

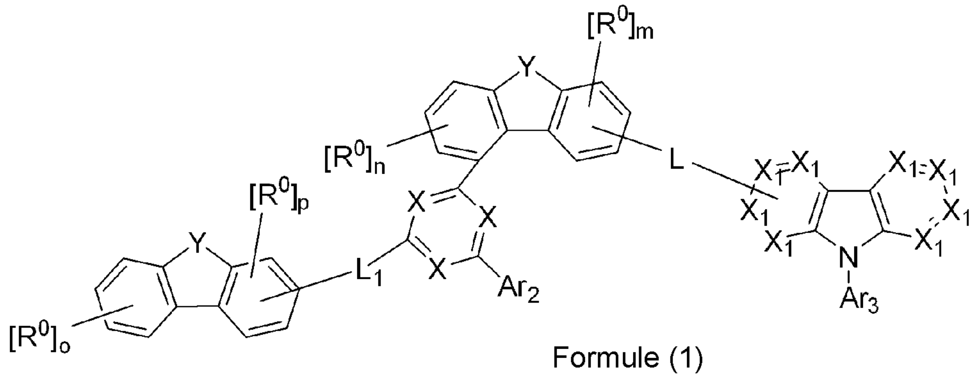

Formule (1)

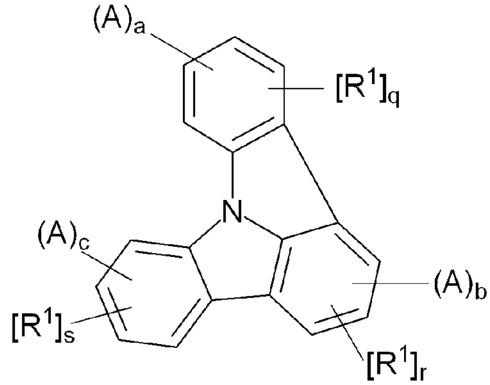

Formule (2)

dans lesquelles ce qui suit s'applique aux symboles et indices utilisés :

X est à chaque occurrence, de manière identique ou différente, $CR^0$ ou N, où au moins un symbole X représente N ;

$X_1$ est à chaque occurrence, de manière identique ou différente, CH, CR ou N, où un maximum de 3 symboles $X_1$ peuvent désigner N ;

$X_2$ est à chaque occurrence, de manière identique ou différente, CH, $CR^1$ ou N, où un maximum de 2 symboles $X_2$ peuvent désigner N ;

Y est choisi à chaque occurrence, de manière identique ou différente, parmi O ou S ;

L et $L_1$ sont à chaque occurrence, de manière identique ou différente, une liaison simple ou un noyau aromatique ou hétéro-aromatique ayant de 5 à 30 atomes de cycle aromatique ;

$R^0$ est à chaque occurrence, indépendamment les uns des autres, H, D ou un noyau aromatique non substitué ou partiellement ou totalement deutérié ayant de 6 à 18 atomes de C ;

R et R# sont choisis à chaque occurrence, de manière identique ou différente, dans le groupe constitué par D, F, Cl, Br, I, CN, $NO_2$, $C(=O)R^2$, $P(=O)(Ar_1)_2$, $P(Ar_1)_2$, $B(Ar_1)_2$, $Si(Ar_1)_3$, $Si(R^2)_3$, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ayant de 2 à 20 atomes de C, pouvant dans chaque cas être substitué par un ou plusieurs radicaux $R^2$, où un ou plusieurs groupements $CH_2$ non adjacents peuvent être remplacés par $R^2C=CR^2$, $Si(R^2)_2$, C=O, C=S, $C=NR^2$, $P(=O)(R^2)$, SO, $SO_2$, $NR^2$, O, S ou $CONR^2$ et où un ou plusieurs atomes de H peuvent être remplacés par D, F, Cl, Br, I, CN ou $NO_2$, un noyau aromatique ou hétéroaromatique ayant de 5 à 40 atomes de cycle aromatique, pouvant dans chaque cas être substitué par un ou plusieurs radicaux $R^2$, un groupement aryloxy ou hétéroaryloxy ayant de 5 à 40 atomes de cycle aromatique, pouvant être substitué par un ou plusieurs radicaux $R^2$, ou un groupement aralkyle ou hétéroaralkyle ayant de 5 à 40 atomes de cycle aromatique, pouvant être substitué par un ou plusieurs radicaux $R^2$; deux substituants R qui sont liés au même atome de carbone ou à des atomes de carbone adjacents ou un substituant R conjointement avec $Ar_3$ peuvent former un noyau monocyclique ou polycyclique, aliphatique, aromatique ou hétéroaromatique, qui peut être substitué par un ou plusieurs radicaux $R^2$;

$R^1$ est choisi à chaque occurrence, de manière identique ou différente, dans le groupe constitué par CN, un

groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C, un noyau aromatique ou hétéroaromatique ayant de 5 à 40 atomes de cycle aromatique, un groupement aryl-oxy ou hétéroaryloxy ayant de 5 à 40 atomes de cycle aromatique, ou un groupement aralkyle ou hétéroaralkyle ayant de 5 à 40 atomes de cycle aromatique ; deux substituants $R^1$ qui sont liés au même atome de carbone ou à des atomes de carbone adjacents peuvent former un noyau monocyclique ou polycyclique, aliphatique, aromatique ou hétéroaromatique, qui peut être substitué par un ou plusieurs radicaux $R^2$;

$R^2$ est choisi à chaque occurrence, de manière identique ou différente, dans le groupe constitué par H, D, F, Cl, Br, I, CN, $NO_2$, $N(Ar_1)_2$, $NH_2$, $N(R^3)_2$, $C(=O)Ar_1$, C(=O)H, $C(=O)R^3$, $P(=O)(Ar_1)_2$, un groupement alkyle, alcoxy ou thioalkyle à chaîne linéaire ayant de 1 à 40 atomes de C ou un groupement alkyle, alcoxy ou thioalkyle ramifié ou cyclique ayant de 3 à 40 atomes de C ou un groupement alcényle ou alcynyle ayant de 2 à 40 atomes de C, pouvant dans chaque cas être substitué par un ou plusieurs radicaux $R^3$, où un ou plusieurs groupements $CH_2$ non adjacents peuvent être rem-placés par HC=CH, $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH ou $CONR^3$ et où un ou plusieurs atomes de H peuvent être remplacés par D, F, Cl, Br, I, CN ou $NO_2$, un noyau aromatique ou hétéroaromatique ayant de 5 à 60 atomes de cycle aromatique, pouvant dans chaque cas être substitué par un ou plusieurs radi-caux $R^3$, un groupement aryloxy ou hétéroaryloxy ayant de 5 à 60 atomes de cycle aromatique, pouvant être substitué par un ou plusieurs radicaux $R^3$, ou une combinaison de ces systèmes ; où deux, ou plus, substituants $R^2$ adjacents peuvent éventuellement former un noyau monocyclique ou polycyclique, aliphatique, aromatique ou hétéroaromatique, qui peut être substitué par un ou plusieurs radicaux $R^3$;

$R^3$ est choisi à chaque occurrence, de manière identique ou différente, dans le groupe constitué par H, D, F, CN, un radical hydrocarboné aliphatique ayant de 1 à 20 atomes de C ou un noyau aromatique ou hétéroaromatique ayant de 5 à 30 atomes de cycle aromatique, dans lequel un ou plusieurs atomes de H peuvent être remplacés par D, F, Cl, Br, I ou CN et pouvant être substitué par un ou plusieurs groupements alkyle ayant de 1 à 4 atomes de carbone chacun ; deux, ou plus, substituants $R^3$ adjacents peuvent former un noyau mono- ou polycyclique, aliphatique, les uns avec les autres ;

$Ar_1$ est à chaque occurrence, de manière identique ou différente, un noyau aromatique ou hétéroaromatique ayant de 5 à 30 atomes de cycle aromatique, pouvant être substitué par un ou plusieurs radicaux non aromatiques $R^3$; deux radicaux $Ar_1$ qui sont liés au même atome de N, atome de P ou atome de B peuvent également être pontés l'un avec l'autre par une liaison simple ou un pont choisi parmi $N(R^3)$, $C(R^3)_2$, O ou S;

$Ar_2$ est à chaque occurrence, dans chaque cas indépendamment les uns des autres, un groupement aryle ou hétéroaryle ayant de 5 à 40 atomes de cycle aromatique, pouvant être substitué par un ou plusieurs radicaux $R^2$;

$Ar_3$ est à chaque occurrence, dans chaque cas indépendamment les uns des autres, un noyau aromatique ayant de 6 à 40 atomes de cycle aromatique ou un noyau hétéroaromatique ayant de 5 à 40 atomes de cycle aromatique, pouvant être substitué par un ou plusieurs radicaux $R^2$;

A est à chaque occurrence, indépendamment les uns des autres, un groupement de formule (3) ou (4),

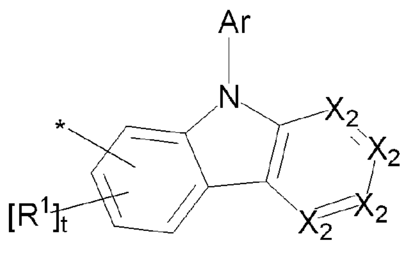

Formule (3)

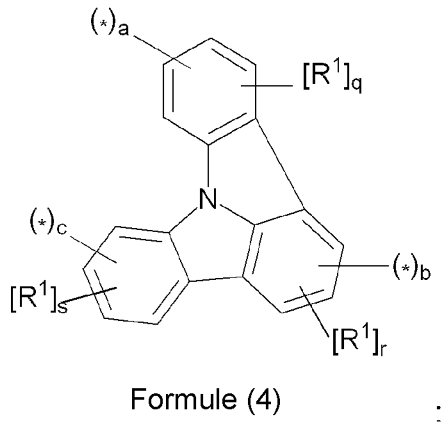

Formule (4)

;

Ar est à chaque occurrence, dans chaque cas indépendamment les uns des autres, un groupement aryle ayant de 6 à 40 atomes de cycle aromatique, pouvant être substitué par un ou plusieurs radicaux R#, ou un groupement hétéroaryle ayant de 5 à 40 atomes de cycle aromatique, pouvant être substitué par un ou plusieurs radicaux R# ;

* désigne le site de fixation à la formule (2) ;

a, b, c à chaque occurrence, dans chaque cas indépendamment les uns des autres, désignent 0 ou 1, où la somme des indices a+b+c à chaque occurrence vaut 1 ;

o désigne 1, 2, 3 ou 4 ;
n, m et p à chaque occurrence, indépendamment les uns des autres, désignent 1, 2 ou 3 ; et
q, r, s, t à chaque occurrence, dans chaque cas indépendamment, désignent 0 ou 1.

**2.** Dispositif électroluminescent organique selon la revendication 1, **caractérisé en ce que**, dans le matériau hôte 1, L est choisi parmi une liaison ou les bras de liaison du groupe constitué par L-1 à L-33,

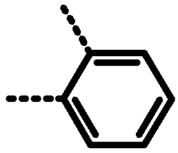

**L-1**

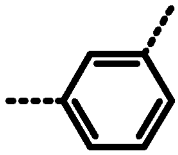

**L-2**

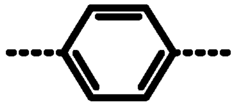

**L-3**

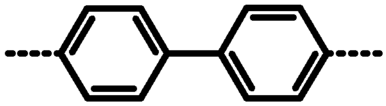

**L-4**

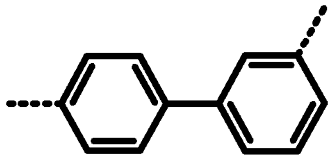

**L-5**

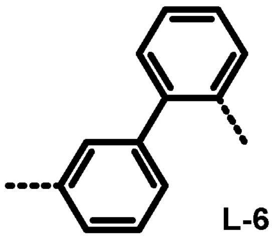

**L-6**

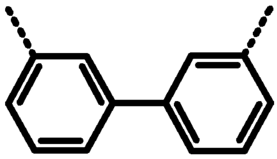

**L-7**

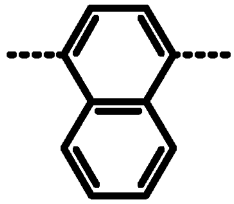

**L-8**

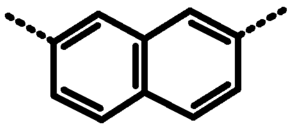

**L-9**

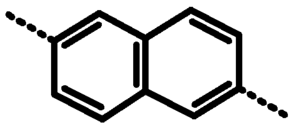

**L-10**

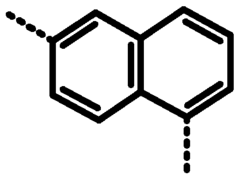

**L-11**

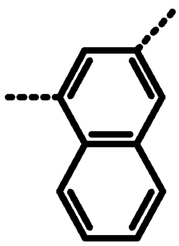

**L-12**

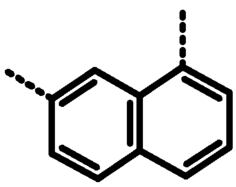

**L-13**

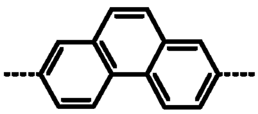
L-14

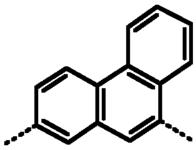
L-15

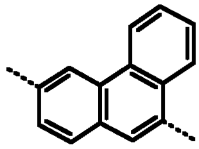
L-16

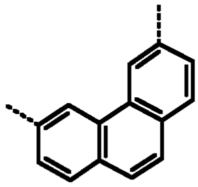
L-17

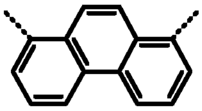
L-18

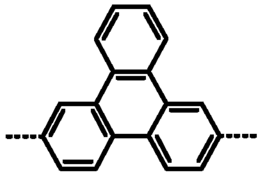
L-19

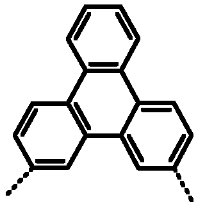
L-20

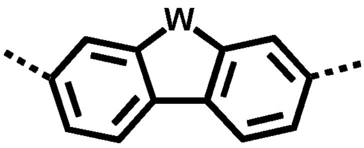
L-21

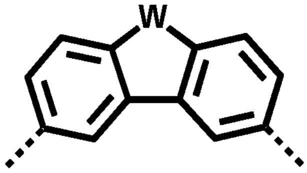
L-22

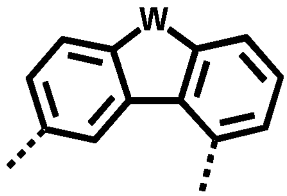
L-23

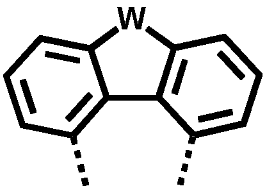
L-24

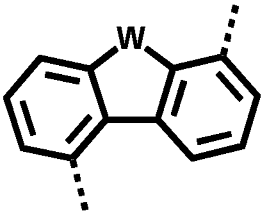
L-25

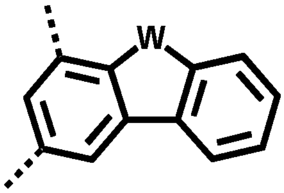
L-26

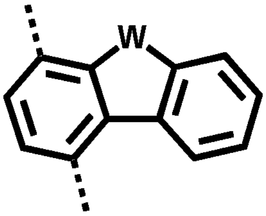
L-27

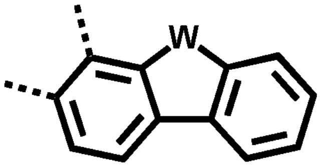
L-28

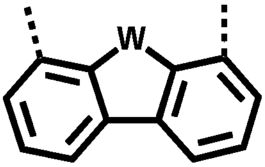
L-29

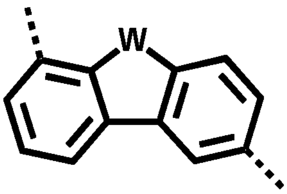
L-30

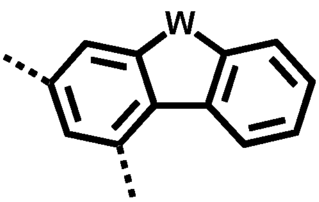
L-31

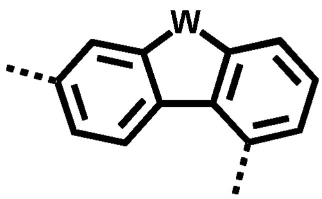
L-32

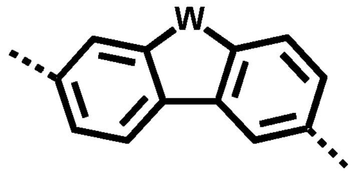

L-33

,

dans lesquels W dans chaque cas, indépendamment les uns des autres, désigne O ou S.

3. Dispositif électroluminescent organique selon la revendication 1 ou 2, **caractérisé en ce que** le matériau hôte 2 répond à l'une des formules (2a), (2b) ou (2c)

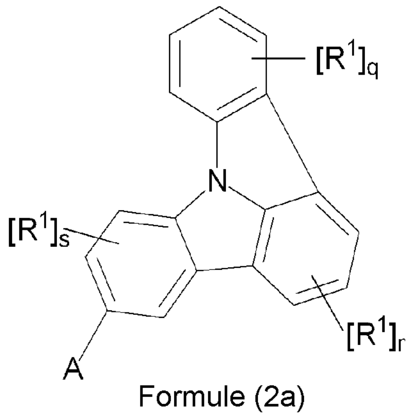

Formule (2a)

,

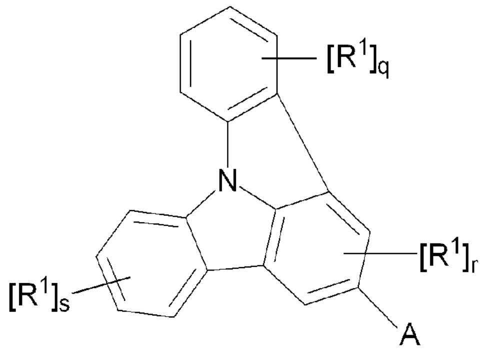

Formule (2b)

,

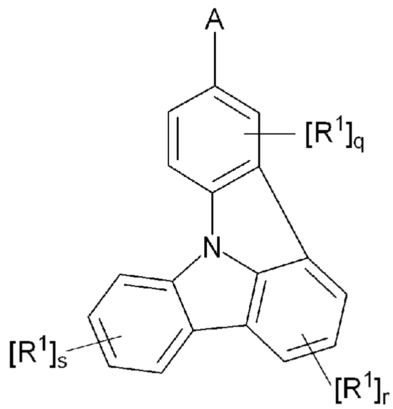

Formule (2c)

dans lesquelles les symboles et indices A, $R^1$, q, r et s utilisés revêtent une signification selon la revendication 1.

4. Dispositif électroluminescent organique selon l'une ou plusieurs parmi les revendications 1 à 3, **caractérisé en ce que**, dans le matériau hôte 1, $L_1$ est une liaison simple.

5. Dispositif électroluminescent organique selon l'une ou plusieurs parmi les revendications 1 à 4, **caractérisé en ce qu'**il s'agit d'un dispositif électroluminescent choisi parmi les transistors organiques émetteurs de lumière (OLET), les dispositifs organiques à extinction de champ (OFQD), les cellules électrochimiques organiques émettrices de lumière (OLEC, LEC, LEEC), les diodes laser organiques (O-lasers) et les diodes électroluminescentes organiques (OLED).

6. Dispositif électroluminescent organique selon l'une ou plusieurs parmi les revendications 1 à 5, **caractérisé en ce**

**que**, outre la couche émettrice de lumière (EML), il comprend une couche d'injection de trous (HIL), une couche de transport de trous (HTL), une couche de transport d'électrons (ETL), une couche d'injection d'électrons (EIL) et/ou une couche de blocage de trous (HBL).

**7.** Dispositif électroluminescent organique selon l'une ou plusieurs parmi les revendications 1 à 6, **caractérisé en ce que**, outre le au moins un matériau hôte 1 et le au moins un matériau hôte 2, la couche émettrice de lumière comprend au moins un émetteur phosphorescent.

**8.** Dispositif électroluminescent organique selon la revendication 7, **caractérisé en ce que** l'émetteur phosphorescent correspond à la formule (III)

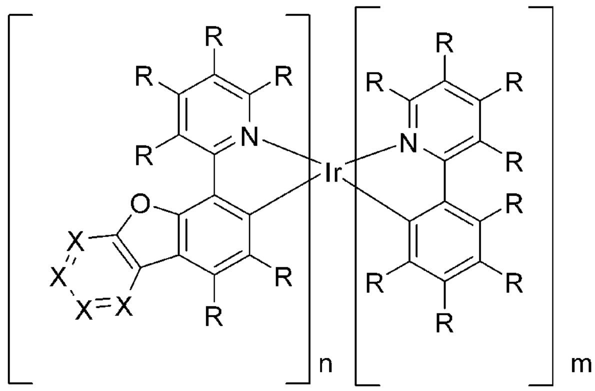

Formule (III)

dans laquelle les symboles et indices pour cette formule (III) revêtent la signification :

n+m vaut 3, n vaut 1 ou 2, m vaut 2 ou 1,
X est N ou CR,
R est H, D ou un groupement alkyle ramifié ou linéaire ou un groupement alkyle ramifié ou linéaire partiellement ou totalement deutérié.

**9.** Procédé de production d'un dispositif selon l'une ou plusieurs parmi les revendications 1 à 8, **caractérisé en ce que** la couche émettrice de lumière est appliquée par dépôt en phase gazeuse ou à partir d'une solution.

**10.** Procédé selon la revendication 9, **caractérisé en ce que** le au moins un composé de formule (1) et le au moins un composé de formule (2) sont déposés à partir de la phase gazeuse successivement ou simultanément à partir d'au moins deux sources de matériau, éventuellement avec le au moins un émetteur phosphorescent, et forment la couche émettrice de lumière.

**11.** Procédé selon la revendication 9, **caractérisé en ce que** le au moins un composé de formule (1) et le au moins un composé de formule (2) sont déposés à partir de la phase gazeuse sous forme de mélange, successivement ou simultanément avec le au moins un émetteur phosphorescent, et forment la couche émettrice de lumière.

**12.** Procédé selon la revendication 9, **caractérisé en ce que** le au moins un composé de formule (1) et le au moins un composé de formule (2) sont appliqués à partir d'une solution conjointement avec le au moins un émetteur phosphorescent afin de former la couche émettrice de lumière.

**13.** Mélange comprenant au moins un composé de formule (1) comme matériau hôte 1 et au moins un composé de formule (2) comme matériau hôte 2

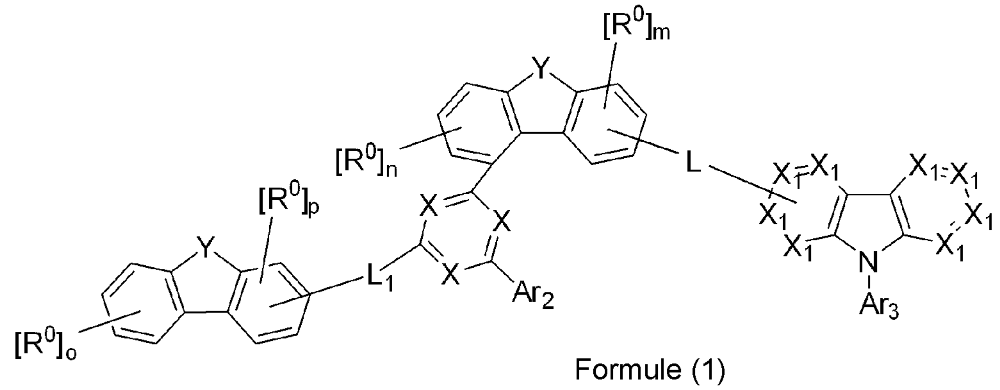

Formule (1)

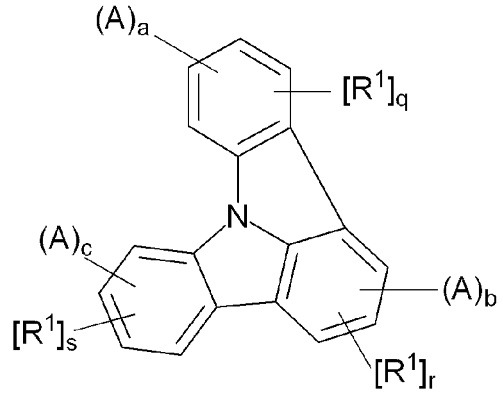

Formule (2)

dans lesquelles ce qui suit s'applique aux symboles et indices utilisés :

X est à chaque occurrence, de manière identique ou différente, $CR^0$ ou N, où au moins un symbole X représente N ;

$X_1$ est à chaque occurrence, de manière identique ou différente, CH, CR ou N, où un maximum de 3 symboles $X_1$ peuvent désigner N ;

$X_2$ est à chaque occurrence, de manière identique ou différente, CH, $CR^1$ ou N, où un maximum de 2 symboles $X_2$ peuvent désigner N ;

Y est choisi à chaque occurrence, de manière identique ou différente, parmi O ou S ;

L et $L_1$ sont à chaque occurrence, de manière identique ou différente, une liaison simple ou un noyau aromatique ou hétéro-aromatique ayant de 5 à 30 atomes de cycle aromatique ;

$R^0$ est à chaque occurrence, indépendamment les uns des autres, H, D ou un noyau aromatique non substitué ou partiellement ou totalement deutérié ayant de 6 à 18 atomes de C ;

R et R# sont choisis à chaque occurrence, de manière identique ou différente, dans le groupe constitué par D, F, Cl, Br, I, CN, $NO_2$, $C(=O)R^2$, $P(=O)(Ar_1)_2$, $P(Ar_1)_2$, $B(Ar_1)_2$, $Si(Ar_1)_3$, $Si(R^2)_3$, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ayant de 2 à 20 atomes de C, pouvant dans chaque cas être substitué par un ou plusieurs radicaux $R^2$, où un ou plusieurs groupements $CH_2$ non adjacents peuvent être remplacés par $R^2C=CR^2$, $Si(R^2)_2$, C=O, C=S, $C=NR^2$, $P(=O)(R^2)$, SO, $SO_2$, $NR^2$, O, S ou $CONR^2$ et où un ou plusieurs atomes de H peuvent être remplacés par D, F, Cl, Br, I, CN ou $NO_2$, un noyau aromatique ou hétéroaromatique ayant de 5 à 40 atomes de cycle aromatique, pouvant dans chaque cas être substitué par un ou plusieurs radicaux $R^2$, un groupement aryloxy ou hétéroaryloxy ayant de 5 à 40 atomes de cycle aromatique, pouvant être substitué par un ou plusieurs radicaux $R^2$, ou un groupement aralkyle ou hétéroaralkyle ayant de 5 à 40 atomes de cycle aromatique, pouvant être substitué par un ou plusieurs radicaux $R^2$; deux substituants R qui sont liés au même atome de carbone ou à des atomes de carbone adjacents ou un substituant R conjointement avec $Ar_3$ peuvent former un noyau monocyclique ou polycyclique, aliphatique, aromatique ou hétéroaromatique, qui peut être substitué par un ou plusieurs radicaux $R^2$;

$R^1$ est choisi à chaque occurrence, de manière identique ou différente, dans le groupe constitué par CN, un

groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C, un noyau aromatique ou hétéroaromatique ayant de 5 à 40 atomes de cycle aromatique, un groupement aryloxy ou hétéroaryloxy ayant de 5 à 40 atomes de cycle aromatique, ou un groupement aralkyle ou hétéroaralkyle ayant de 5 à 40 atomes de cycle aromatique ; deux substituants $R^1$ qui sont liés au même atome de carbone ou à des atomes de carbone adjacents peuvent former un noyau monocyclique ou polycyclique, aliphatique, aromatique ou hétéroaromatique, qui peut être substitué par un ou plusieurs radicaux $R^2$;

$R^2$ est choisi à chaque occurrence, de manière identique ou différente, dans le groupe constitué par H, D, F, Cl, Br, I, CN, $NO_2$, $N(Ar_1)_2$, $NH_2$, $N(R^3)_2$, $C(=O)Ar_1$, C(=O)H, $C(=O)R^3$, $P(=O)(Ar_1)_2$, un groupement alkyle, alcoxy ou thioalkyle à chaîne linéaire ayant de 1 à 40 atomes de C ou un groupement alkyle, alcoxy ou thioalkyle ramifié ou cyclique ayant de 3 à 40 atomes de C ou un groupement alcényle ou alcynyle ayant de 2 à 40 atomes de C, pouvant dans chaque cas être substitué par un ou plusieurs radicaux $R^3$, où un ou plusieurs groupements $CH_2$ non adjacents peuvent être remplacés par HC=CH, $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, NH, $NR^3$, O, S, CONH ou $CONR^3$ et où un ou plusieurs atomes de H peuvent être remplacés par D, F, Cl, Br, I, CN ou $NO_2$, un noyau aromatique ou hétéroaromatique ayant de 5 à 60 atomes de cycle aromatique, pouvant dans chaque cas être substitué par un ou plusieurs radicaux $R^3$, un groupement aryloxy ou hétéroaryloxy ayant de 5 à 60 atomes de cycle aromatique, pouvant être substitué par un ou plusieurs radicaux $R^3$, ou une combinaison de ces systèmes ; où deux, ou plus, substituants $R^2$ adjacents peuvent éventuellement former un noyau monocyclique ou polycyclique, aliphatique, aromatique ou hétéroaromatique, qui peut être substitué par un ou plusieurs radicaux $R^3$;

$R^3$ est choisi à chaque occurrence, de manière identique ou différente, dans le groupe constitué par H, D, F, CN, un radical hydrocarboné aliphatique ayant de 1 à 20 atomes de C, ou un noyau aromatique ou hétéroaromatique ayant de 5 à 30 atomes de cycle aromatique, dans lequel un ou plusieurs atomes de H peuvent être remplacés par D, F, Cl, Br, I ou CN et pouvant être substitué par un ou plusieurs groupements alkyle ayant de 1 à 4 atomes de carbone chacun ; deux, ou plus, substituants $R^3$ adjacents peuvent former un noyau mono- ou polycyclique, aliphatique, les uns avec les autres ;

$Ar_1$ est à chaque occurrence, de manière identique ou différente, un noyau aromatique ou hétéroaromatique ayant de 5 à 30 atomes de cycle aromatique, pouvant être substitué par un ou plusieurs radicaux non aromatiques $R^3$; deux radicaux $Ar_1$ qui sont liés au même atome de N, atome de P ou atome de B peuvent également être pontés l'un avec l'autre par une liaison simple ou un pont choisi parmi $N(R^3)$, $C(R^3)_2$, O ou S;

$Ar_2$ est à chaque occurrence, dans chaque cas indépendamment les uns des autres, un groupement aryle ou hétéroaryle ayant de 5 à 40 atomes de cycle aromatique, pouvant être substitué par un ou plusieurs radicaux $R^2$;

$Ar_3$ est à chaque occurrence, dans chaque cas indépendamment les uns des autres, un noyau aromatique ayant de 6 à 40 atomes de cycle aromatique ou un noyau hétéroaromatique ayant de 5 à 40 atomes de cycle aromatique, pouvant être substitué par un ou plusieurs radicaux $R^2$;

A est à chaque occurrence, indépendamment les uns des autres, un groupement de formule (3) ou (4),

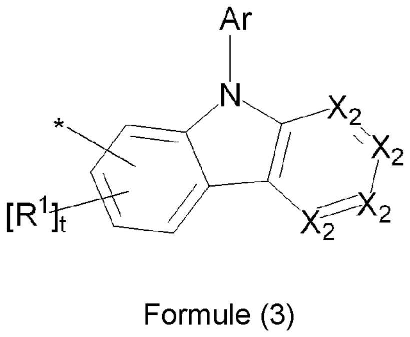

Formule (3)

,

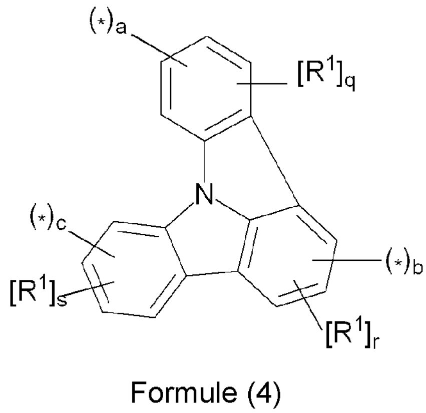

Formule (4)

;

Ar est à chaque occurrence, dans chaque cas indépendamment les uns des autres, un groupement aryle ayant de 6 à 40 atomes de cycle aromatique, pouvant être substitué par un ou plusieurs radicaux R#, ou un groupement hétéroaryle ayant de 5 à 40 atomes de cycle aromatique, pouvant être substitué par un ou plusieurs radicaux R# ;

* désigne le site de fixation à la formule (2) ;

a, b, c à chaque occurrence, dans chaque cas indépendamment les uns des autres, désignent 0 ou 1, où la somme des indices a+b+c à chaque occurrence vaut 1 ;

o désigne 1, 2, 3 ou 4 ;
n, m et p à chaque occurrence, indépendamment les uns des autres, désignent 1, 2 ou 3 ; et
q, r, s, t à chaque occurrence, dans chaque cas indépendamment, désignent 0 ou 1.

**14.** Mélange selon la revendication 13, **caractérisé en ce que** le mélange est constitué d'au moins un composé de formule (1), d'au moins un composé de formule (2) et d'un émetteur phosphorescent.

**15.** Formulation comprenant un mélange selon la revendication 13 ou 14 et au moins un solvant.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 6392250 B1
- US 6803720 B1
- WO 2011088877 A
- WO 2015156587 A
- WO 2015169412 A
- US 9771373 B
- KR 20160046077
- WO 2016015810 A
- KR 2018010149
- WO 2018174678 A
- WO 2018174679 A
- KR 20170113318
- KR 20170113320
- CN 107973786
- US 20190006590 A
- US 2019047991 A
- WO 19031679 A
- WO 19007866 A
- WO 2020022860 A
- US 2015333273 A
- KR 20170113320 A
- WO 2016015815 A
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016124304 A
- WO 2017032439 A
- WO 2006108497 A
- WO 2006122630 A
- WO 2008006449 A
- WO 2007140847 A
- WO 2010012328 A
- US 7294849 B
- WO 2005011013 A
- JP 2000053957 A
- WO 2003060956 A
- WO 2004028217 A
- WO 2004080975 A
- WO 2010072300 A
- WO 06122630 A
- WO 06100896 A
- EP 1661888 A
- WO 01049806 A
- US 5061569 A
- WO 9509147 A
- WO 08006449 A
- WO 07140847 A
- WO 2012034627 A
- EP 12000929
- WO 2014015937 A
- WO 2014015938 A
- WO 2014015935 A
- WO 2013083216 A
- WO 2012150001 A

### In der Beschreibung aufgeführte Nicht-Patentliteratur

- *CHEMICAL ABSTRACTS,* 1269508-30-6
- *CHEMICAL ABSTRACTS,* 1989601-68-4
- *CHEMICAL ABSTRACTS,* 1989602-19-8
- *CHEMICAL ABSTRACTS,* 1989602-70-1
- *CHEMICAL ABSTRACTS,* 1215692-34-4
- *CHEMICAL ABSTRACTS,* 1989601-69-5
- *CHEMICAL ABSTRACTS,* 1989602-20-1
- *CHEMICAL ABSTRACTS,* 1989602-71-2
- *CHEMICAL ABSTRACTS,* 1370364-40-1
- *CHEMICAL ABSTRACTS,* 1989601-70-8

- *CHEMICAL ABSTRACTS,* 1989602-21-2
- *CHEMICAL ABSTRACTS,* 1989602-72-3
- *CHEMICAL ABSTRACTS,* 1370364-42-3
- *CHEMICAL ABSTRACTS,* 1989601-71-9
- *CHEMICAL ABSTRACTS,* 1989602-22-3
- *CHEMICAL ABSTRACTS,* 1989602-73-4
- *CHEMICAL ABSTRACTS,* 1989600-74-9
- *CHEMICAL ABSTRACTS,* 1989601-72-0
- *CHEMICAL ABSTRACTS,* 1989602-23-4
- *CHEMICAL ABSTRACTS,* 1989602-74-5
- *CHEMICAL ABSTRACTS,* 1989600-75-0
- *CHEMICAL ABSTRACTS,* 1989601-73-1
- *CHEMICAL ABSTRACTS,* 1989602-24-5
- *CHEMICAL ABSTRACTS,* 1989602-75-6
- *CHEMICAL ABSTRACTS,* 1989600-77-2
- *CHEMICAL ABSTRACTS,* 1989601-74-2
- *CHEMICAL ABSTRACTS,* 1989602-25-6
- *CHEMICAL ABSTRACTS,* 1989602-76-7
- *CHEMICAL ABSTRACTS,* 1989600-78-3
- *CHEMICAL ABSTRACTS,* 1989601-75-3
- *CHEMICAL ABSTRACTS,* 1989602-26-7
- *CHEMICAL ABSTRACTS,* 1989602-77-8
- *CHEMICAL ABSTRACTS,* 1989600-79-4
- *CHEMICAL ABSTRACTS,* 1989601-76-4
- *CHEMICAL ABSTRACTS,* 1989602-27-8
- *CHEMICAL ABSTRACTS,* 1989602-78-9
- *CHEMICAL ABSTRACTS,* 1989600-82-9
- *CHEMICAL ABSTRACTS,* 1989601-77-5
- *CHEMICAL ABSTRACTS,* 1989602-28-9
- *CHEMICAL ABSTRACTS,* 1989602-79-0
- *CHEMICAL ABSTRACTS,* 1989600-83-0
- *CHEMICAL ABSTRACTS,* 1989601-78-6
- *CHEMICAL ABSTRACTS,* 1989602-29-0
- *CHEMICAL ABSTRACTS,* 1989602-80-3
- *CHEMICAL ABSTRACTS,* 1989600-84-1
- *CHEMICAL ABSTRACTS,* 1989601-79-7
- *CHEMICAL ABSTRACTS,* 1989602-30-3
- *CHEMICAL ABSTRACTS,* 1989602-82-5
- *CHEMICAL ABSTRACTS,* 1989600-85-2
- *CHEMICAL ABSTRACTS,* 1989601-80-0
- *CHEMICAL ABSTRACTS,* 1989602-31-4
- *CHEMICAL ABSTRACTS,* 1989602-84-7
- *CHEMICAL ABSTRACTS,* 1989600-86-3
- *CHEMICAL ABSTRACTS,* 1989601-81-1
- *CHEMICAL ABSTRACTS,* 1989602-32-5
- *CHEMICAL ABSTRACTS,* 1989602-85-8
- *CHEMICAL ABSTRACTS,* 1989600-87-4
- *CHEMICAL ABSTRACTS,* 1989601-82-2
- *CHEMICAL ABSTRACTS,* 1989602-33-6
- *CHEMICAL ABSTRACTS,* 1989602-86-9
- *CHEMICAL ABSTRACTS,* 1989600-88-5
- *CHEMICAL ABSTRACTS,* 1989601-83-3
- *CHEMICAL ABSTRACTS,* 1989602-34-7
- *CHEMICAL ABSTRACTS,* 1989602-87-0
- *CHEMICAL ABSTRACTS,* 1989600-89-6
- *CHEMICAL ABSTRACTS,* 1989601-84-4
- *CHEMICAL ABSTRACTS,* 1989602-35-8
- *CHEMICAL ABSTRACTS,* 1989602-88-1
- *CHEMICAL ABSTRACTS,* 1989601-11-7
- *CHEMICAL ABSTRACTS,* 1989601-85-5
- *CHEMICAL ABSTRACTS,* 1989602-36-9
- *CHEMICAL ABSTRACTS,* 1989604-00-3
- *CHEMICAL ABSTRACTS,* 1989601-23-1
- *CHEMICAL ABSTRACTS,* 1989601-86-6
- *CHEMICAL ABSTRACTS,* 1989602-37-0
- *CHEMICAL ABSTRACTS,* 1989604-01-4
- *CHEMICAL ABSTRACTS,* 1989601-26-4
- *CHEMICAL ABSTRACTS,* 1989601-87-7
- *CHEMICAL ABSTRACTS,* 1989602-38-1
- *CHEMICAL ABSTRACTS,* 1989604-02-5
- *CHEMICAL ABSTRACTS,* 1989601-28-6
- *CHEMICAL ABSTRACTS,* 1989601-88-8
- *CHEMICAL ABSTRACTS,* 1989602-39-2
- *CHEMICAL ABSTRACTS,* 1989604-03-6
- *CHEMICAL ABSTRACTS,* 1989601-29-7
- *CHEMICAL ABSTRACTS,* 1989601-89-9
- *CHEMICAL ABSTRACTS,* 1989602-40-5
- *CHEMICAL ABSTRACTS,* 1989604-04-7
- *CHEMICAL ABSTRACTS,* 1989601-33-3
- *CHEMICAL ABSTRACTS,* 1989601-90-2
- *CHEMICAL ABSTRACTS,* 1989602-41-6
- *CHEMICAL ABSTRACTS,* 1989604-05-8
- *CHEMICAL ABSTRACTS,* 1989601-40-2
- *CHEMICAL ABSTRACTS,* 1989601-91-3
- *CHEMICAL ABSTRACTS,* 1989602-42-7
- *CHEMICAL ABSTRACTS,* 1989604-06-9
- *CHEMICAL ABSTRACTS,* 1989601-41-3
- *CHEMICAL ABSTRACTS,* 1989601-92-4
- *CHEMICAL ABSTRACTS,* 1989602-43-8
- *CHEMICAL ABSTRACTS,* 1989604-07-0
- *CHEMICAL ABSTRACTS,* 1989601-42-4
- *CHEMICAL ABSTRACTS,* 1989601-93-5
- *CHEMICAL ABSTRACTS,* 1989602-44-9
- *CHEMICAL ABSTRACTS,* 1989604-08-1
- *CHEMICAL ABSTRACTS,* 1989601-43-5
- *CHEMICAL ABSTRACTS,* 1989601-94-6
- *CHEMICAL ABSTRACTS,* 1989602-45-0
- *CHEMICAL ABSTRACTS,* 1989604-09-2
- *CHEMICAL ABSTRACTS,* 1989601-44-6
- *CHEMICAL ABSTRACTS,* 1989601-95-7
- *CHEMICAL ABSTRACTS,* 1989602-46-1
- *CHEMICAL ABSTRACTS,* 1989604-10-5
- *CHEMICAL ABSTRACTS,* 1989601-45-7
- *CHEMICAL ABSTRACTS,* 1989601-96-8
- *CHEMICAL ABSTRACTS,* 1989602-47-2
- *CHEMICAL ABSTRACTS,* 1989604-11-6
- *CHEMICAL ABSTRACTS,* 1989601-46-8
- *CHEMICAL ABSTRACTS,* 1989601-97-9
- *CHEMICAL ABSTRACTS,* 1989602-48-3
- *CHEMICAL ABSTRACTS,* 1989604-13-8
- *CHEMICAL ABSTRACTS,* 1989601-47-9
- *CHEMICAL ABSTRACTS,* 1989601-98-0
- *CHEMICAL ABSTRACTS,* 1989602-49-4
- *CHEMICAL ABSTRACTS,* 1989604-14-9
- *CHEMICAL ABSTRACTS,* 1989601-48-0
- *CHEMICAL ABSTRACTS,* 1989601-99-1

- *CHEMICAL ABSTRACTS,* 1989602-50-7
- *CHEMICAL ABSTRACTS,* 1989604-15-0
- *CHEMICAL ABSTRACTS,* 1989601-49-1
- *CHEMICAL ABSTRACTS,* 1989602-00-7
- *CHEMICAL ABSTRACTS,* 1989602-51-8
- *CHEMICAL ABSTRACTS,* 1989604-16-1
- *CHEMICAL ABSTRACTS,* 1989601-50-4
- *CHEMICAL ABSTRACTS,* 1989602-01-8
- *CHEMICAL ABSTRACTS,* 1989602-52-9
- *CHEMICAL ABSTRACTS,* 1989604-17-2
- *CHEMICAL ABSTRACTS,* 1989601-51-5
- *CHEMICAL ABSTRACTS,* 1989602-02-9
- *CHEMICAL ABSTRACTS,* 1989602-53-0
- *CHEMICAL ABSTRACTS,* 1989604-18-3
- *CHEMICAL ABSTRACTS,* 1989601-52-6
- *CHEMICAL ABSTRACTS,* 1989602-03-0
- *CHEMICAL ABSTRACTS,* 1989602-54-1
- *CHEMICAL ABSTRACTS,* 1989604-19-4
- *CHEMICAL ABSTRACTS,* 1989601-53-7
- *CHEMICAL ABSTRACTS,* 1989602-04-1
- *CHEMICAL ABSTRACTS,* 1989602-55-2
- *CHEMICAL ABSTRACTS,* 1989604-20-7
- *CHEMICAL ABSTRACTS,* 1989601-54-8
- *CHEMICAL ABSTRACTS,* 1989602-05-2
- *CHEMICAL ABSTRACTS,* 1989602-56-3
- *CHEMICAL ABSTRACTS,* 1989604-21-8
- *CHEMICAL ABSTRACTS,* 1989601-55-9
- *CHEMICAL ABSTRACTS,* 1989602-06-3
- *CHEMICAL ABSTRACTS,* 1989602-57-4
- *CHEMICAL ABSTRACTS,* 1989604-22-9
- *CHEMICAL ABSTRACTS,* 1989601-56-0
- *CHEMICAL ABSTRACTS,* 1989602-07-4
- *CHEMICAL ABSTRACTS,* 1989602-58-5
- *CHEMICAL ABSTRACTS,* 1989604-23-0
- *CHEMICAL ABSTRACTS,* 1989601-57-1
- *CHEMICAL ABSTRACTS,* 1989602-08-5
- *CHEMICAL ABSTRACTS,* 1989602-59-6
- *CHEMICAL ABSTRACTS,* 1989604-24-1
- *CHEMICAL ABSTRACTS,* 1989601-58-2
- *CHEMICAL ABSTRACTS,* 1989602-09-6
- *CHEMICAL ABSTRACTS,* 1989602-60-9
- *CHEMICAL ABSTRACTS,* 1989604-25-2
- *CHEMICAL ABSTRACTS,* 1989601-59-3
- *CHEMICAL ABSTRACTS,* 1989602-10-9
- *CHEMICAL ABSTRACTS,* 1989602-61-0
- *CHEMICAL ABSTRACTS,* 1989604-26-3
- *CHEMICAL ABSTRACTS,* 1989601-60-6
- *CHEMICAL ABSTRACTS,* 1989602-11-0
- *CHEMICAL ABSTRACTS,* 1989602-62-1
- *CHEMICAL ABSTRACTS,* 1989604-27-4
- *CHEMICAL ABSTRACTS,* 1989601-61-7
- *CHEMICAL ABSTRACTS,* 1989602-12-1
- *CHEMICAL ABSTRACTS,* 1989602-63-2
- *CHEMICAL ABSTRACTS,* 1989604-28-5
- *CHEMICAL ABSTRACTS,* 1989601-62-8
- *CHEMICAL ABSTRACTS,* 1989602-13-2
- *CHEMICAL ABSTRACTS,* 1989602-64-3
- *CHEMICAL ABSTRACTS,* 1989604-29-6
- *CHEMICAL ABSTRACTS,* 1989601-63-9
- *CHEMICAL ABSTRACTS,* 1989602-14-3
- *CHEMICAL ABSTRACTS,* 1989602-65-4
- *CHEMICAL ABSTRACTS,* 1989604-30-9
- *CHEMICAL ABSTRACTS,* 1989601-64-0
- *CHEMICAL ABSTRACTS,* 1989602-15-4
- *CHEMICAL ABSTRACTS,* 1989602-66-5
- *CHEMICAL ABSTRACTS,* 1989604-31-0
- *CHEMICAL ABSTRACTS,* 1989601-65-1
- *CHEMICAL ABSTRACTS,* 1989602-16-5
- *CHEMICAL ABSTRACTS,* 1989602-67-6
- *CHEMICAL ABSTRACTS,* 1989604-32-1
- *CHEMICAL ABSTRACTS,* 1989601-66-2
- *CHEMICAL ABSTRACTS,* 1989602-17-6
- *CHEMICAL ABSTRACTS,* 1989602-68-7
- *CHEMICAL ABSTRACTS,* 1989604-33-2
- *CHEMICAL ABSTRACTS,* 1989601-67-3
- *CHEMICAL ABSTRACTS,* 1989602-18-7
- *CHEMICAL ABSTRACTS,* 1989602-69-8
- *CHEMICAL ABSTRACTS,* 1989604-34-3
- *CHEMICAL ABSTRACTS,* 1989604-35-4
- *CHEMICAL ABSTRACTS,* 1989604-88-7
- *CHEMICAL ABSTRACTS,* 1989605-52-8
- *CHEMICAL ABSTRACTS,* 1989606-07-6
- *CHEMICAL ABSTRACTS,* 1989604-36-5
- *CHEMICAL ABSTRACTS,* 1989604-89-8
- *CHEMICAL ABSTRACTS,* 1989605-53-9
- *CHEMICAL ABSTRACTS,* 1989606-08-7
- *CHEMICAL ABSTRACTS,* 1989604-37-6
- *CHEMICAL ABSTRACTS,* 1989604-90-1
- *CHEMICAL ABSTRACTS,* 1989605-54-0
- *CHEMICAL ABSTRACTS,* 1989606-09-8
- *CHEMICAL ABSTRACTS,* 1989604-38-7
- *CHEMICAL ABSTRACTS,* 1989604-92-3
- *CHEMICAL ABSTRACTS,* 1989605-55-1
- *CHEMICAL ABSTRACTS,* 1989606-10-1
- *CHEMICAL ABSTRACTS,* 1989604-39-8
- *CHEMICAL ABSTRACTS,* 1989604-93-4
- *CHEMICAL ABSTRACTS,* 1989605-56-2
- *CHEMICAL ABSTRACTS,* 1989606-11-2
- *CHEMICAL ABSTRACTS,* 1989604-40-1
- *CHEMICAL ABSTRACTS,* 1989604-94-5
- *CHEMICAL ABSTRACTS,* 1989605-57-3
- *CHEMICAL ABSTRACTS,* 1989606-12-3
- *CHEMICAL ABSTRACTS,* 1989604-41-2
- *CHEMICAL ABSTRACTS,* 1989604-95-6
- *CHEMICAL ABSTRACTS,* 1989605-58-4
- *CHEMICAL ABSTRACTS,* 1989606-13-4
- *CHEMICAL ABSTRACTS,* 1989604-42-3
- *CHEMICAL ABSTRACTS,* 1989604-96-7
- *CHEMICAL ABSTRACTS,* 1989605-59-5
- *CHEMICAL ABSTRACTS,* 1989606-14-5
- *CHEMICAL ABSTRACTS,* 1989604-43-4
- *CHEMICAL ABSTRACTS,* 1989604-97-8
- *CHEMICAL ABSTRACTS,* 1989605-61-9
- *CHEMICAL ABSTRACTS,* 1989606-15-6
- *CHEMICAL ABSTRACTS,* 1989604-45-6
- *CHEMICAL ABSTRACTS,* 1989605-09-5

- *CHEMICAL ABSTRACTS,* 1989605-62-0
- *CHEMICAL ABSTRACTS,* 1989606-16-7
- *CHEMICAL ABSTRACTS,* 1989604-46-7
- *CHEMICAL ABSTRACTS,* 1989605-10-8
- *CHEMICAL ABSTRACTS,* 1989605-63-1
- *CHEMICAL ABSTRACTS,* 1989606-17-8
- *CHEMICAL ABSTRACTS,* 1989604-47-8
- *CHEMICAL ABSTRACTS,* 1989605-11-9
- *CHEMICAL ABSTRACTS,* 1989605-64-2
- *CHEMICAL ABSTRACTS,* 1989606-18-9
- *CHEMICAL ABSTRACTS,* 1989604-48-9
- *CHEMICAL ABSTRACTS,* 1989605-13-1
- *CHEMICAL ABSTRACTS,* 1989605-65-3
- *CHEMICAL ABSTRACTS,* 1989606-19-0
- *CHEMICAL ABSTRACTS,* 1989604-49-0
- *CHEMICAL ABSTRACTS,* 1989605-14-2
- *CHEMICAL ABSTRACTS,* 1989605-66-4
- *CHEMICAL ABSTRACTS,* 1989606-20-3
- *CHEMICAL ABSTRACTS,* 1989604-50-3
- *CHEMICAL ABSTRACTS,* 1989605-15-3
- *CHEMICAL ABSTRACTS,* 1989605-67-5
- *CHEMICAL ABSTRACTS,* 1989606-21-4
- *CHEMICAL ABSTRACTS,* 1989604-52-5
- *CHEMICAL ABSTRACTS,* 1989605-16-4
- *CHEMICAL ABSTRACTS,* 1989605-68-6
- *CHEMICAL ABSTRACTS,* 1989606-22-5
- *CHEMICAL ABSTRACTS,* 1989604-53-6
- *CHEMICAL ABSTRACTS,* 1989605-17-5
- *CHEMICAL ABSTRACTS,* 1989605-69-7
- *CHEMICAL ABSTRACTS,* 1989606-23-6
- *CHEMICAL ABSTRACTS,* 1989604-54-7
- *CHEMICAL ABSTRACTS,* 1989605-18-6
- *CHEMICAL ABSTRACTS,* 1989605-70-0
- *CHEMICAL ABSTRACTS,* 1989606-24-7
- *CHEMICAL ABSTRACTS,* 1989604-55-8
- *CHEMICAL ABSTRACTS,* 1989605-19-7
- *CHEMICAL ABSTRACTS,* 1989605-71-1
- *CHEMICAL ABSTRACTS,* 1989606-26-9
- *CHEMICAL ABSTRACTS,* 1989604-56-9
- *CHEMICAL ABSTRACTS,* 1989605-20-0
- *CHEMICAL ABSTRACTS,* 1989605-72-2
- *CHEMICAL ABSTRACTS,* 1989606-27-0
- *CHEMICAL ABSTRACTS,* 1989604-57-0
- *CHEMICAL ABSTRACTS,* 1989605-21-1
- *CHEMICAL ABSTRACTS,* 1989605-73-3
- *CHEMICAL ABSTRACTS,* 1989606-28-1
- *CHEMICAL ABSTRACTS,* 1989604-58-1
- *CHEMICAL ABSTRACTS,* 1989605-22-2
- *CHEMICAL ABSTRACTS,* 1989605-74-4
- *CHEMICAL ABSTRACTS,* 1989606-29-2
- *CHEMICAL ABSTRACTS,* 1989604-59-2
- *CHEMICAL ABSTRACTS,* 1989605-23-3
- *CHEMICAL ABSTRACTS,* 1989605-75-5
- *CHEMICAL ABSTRACTS,* 1989606-30-5
- *CHEMICAL ABSTRACTS,* 1989604-60-5
- *CHEMICAL ABSTRACTS,* 1989605-24-4
- *CHEMICAL ABSTRACTS,* 1989605-76-6
- *CHEMICAL ABSTRACTS,* 1989606-31-6
- *CHEMICAL ABSTRACTS,* 1989604-61-6
- *CHEMICAL ABSTRACTS,* 1989605-25-5
- *CHEMICAL ABSTRACTS,* 1989605-77-7
- *CHEMICAL ABSTRACTS,* 1989606-32-7
- *CHEMICAL ABSTRACTS,* 1989604-62-7
- *CHEMICAL ABSTRACTS,* 1989605-26-6
- *CHEMICAL ABSTRACTS,* 1989605-78-8
- *CHEMICAL ABSTRACTS,* 1989606-33-8
- *CHEMICAL ABSTRACTS,* 1989604-63-8
- *CHEMICAL ABSTRACTS,* 1989605-27-7
- *CHEMICAL ABSTRACTS,* 1989605-79-9
- *CHEMICAL ABSTRACTS,* 1989606-34-9
- *CHEMICAL ABSTRACTS,* 1989604-64-9
- *CHEMICAL ABSTRACTS,* 1989605-28-8
- *CHEMICAL ABSTRACTS,* 1989605-81-3
- *CHEMICAL ABSTRACTS,* 1989606-35-0
- *CHEMICAL ABSTRACTS,* 1989604-65-0
- *CHEMICAL ABSTRACTS,* 1989605-29-9
- *CHEMICAL ABSTRACTS,* 1989605-82-4
- *CHEMICAL ABSTRACTS,* 1989606-36-1
- *CHEMICAL ABSTRACTS,* 1989604-66-1
- *CHEMICAL ABSTRACTS,* 1989605-30-2
- *CHEMICAL ABSTRACTS,* 1989605-83-5
- *CHEMICAL ABSTRACTS,* 1989606-37-2
- *CHEMICAL ABSTRACTS,* 1989604-67-2
- *CHEMICAL ABSTRACTS,* 1989605-31-3
- *CHEMICAL ABSTRACTS,* 1989605-84-6
- *CHEMICAL ABSTRACTS,* 1989606-38-3
- *CHEMICAL ABSTRACTS,* 1989604-68-3
- *CHEMICAL ABSTRACTS,* 1989605-32-4
- *CHEMICAL ABSTRACTS,* 1989605-85-7
- *CHEMICAL ABSTRACTS,* 1989606-39-4
- *CHEMICAL ABSTRACTS,* 1989604-69-4
- *CHEMICAL ABSTRACTS,* 1989605-33-5
- *CHEMICAL ABSTRACTS,* 1989605-86-8
- *CHEMICAL ABSTRACTS,* 1989606-40-7
- *CHEMICAL ABSTRACTS,* 1989604-70-7
- *CHEMICAL ABSTRACTS,* 1989605-34-6
- *CHEMICAL ABSTRACTS,* 1989605-87-9
- *CHEMICAL ABSTRACTS,* 1989606-41-8
- *CHEMICAL ABSTRACTS,* 1989604-71-8
- *CHEMICAL ABSTRACTS,* 1989605-35-7
- *CHEMICAL ABSTRACTS,* 1989605-88-0
- *CHEMICAL ABSTRACTS,* 1989606-42-9
- *CHEMICAL ABSTRACTS,* 1989604-72-9
- *CHEMICAL ABSTRACTS,* 1989605-36-8
- *CHEMICAL ABSTRACTS,* 1989605-89-1
- *CHEMICAL ABSTRACTS,* 1989606-43-0
- *CHEMICAL ABSTRACTS,* 1989604-73-0
- *CHEMICAL ABSTRACTS,* 1989605-37-9
- *CHEMICAL ABSTRACTS,* 1989605-90-4
- *CHEMICAL ABSTRACTS,* 1989606-44-1
- *CHEMICAL ABSTRACTS,* 1989604-74-1
- *CHEMICAL ABSTRACTS,* 1989605-38-0
- *CHEMICAL ABSTRACTS,* 1989605-91-5
- *CHEMICAL ABSTRACTS,* 1989606-45-2
- *CHEMICAL ABSTRACTS,* 1989604-75-2
- *CHEMICAL ABSTRACTS,* 1989605-39-1

- *CHEMICAL ABSTRACTS,* 1989605-92-6
- *CHEMICAL ABSTRACTS,* 1989606-46-3
- *CHEMICAL ABSTRACTS,* 1989604-76-3
- *CHEMICAL ABSTRACTS,* 1989605-40-4
- *CHEMICAL ABSTRACTS,* 1989605-93-7
- *CHEMICAL ABSTRACTS,* 1989606-48-5
- *CHEMICAL ABSTRACTS,* 1989604-77-4
- *CHEMICAL ABSTRACTS,* 1989605-41-5
- *CHEMICAL ABSTRACTS,* 1989605-94-8
- *CHEMICAL ABSTRACTS,* 1989606-49-6
- *CHEMICAL ABSTRACTS,* 1989604-78-5
- *CHEMICAL ABSTRACTS,* 1989605-42-6
- *CHEMICAL ABSTRACTS,* 1989605-95-9
- *CHEMICAL ABSTRACTS,* 1989606-53-2
- *CHEMICAL ABSTRACTS,* 1989604-79-6
- *CHEMICAL ABSTRACTS,* 1989605-43-7
- *CHEMICAL ABSTRACTS,* 1989605-96-0
- *CHEMICAL ABSTRACTS,* 1989606-55-4
- *CHEMICAL ABSTRACTS,* 1989604-80-9
- *CHEMICAL ABSTRACTS,* 1989605-44-8
- *CHEMICAL ABSTRACTS,* 1989605-97-1
- *CHEMICAL ABSTRACTS,* 1989606-56-5
- *CHEMICAL ABSTRACTS,* 1989604-81-0
- *CHEMICAL ABSTRACTS,* 1989605-45-9
- *CHEMICAL ABSTRACTS,* 1989605-98-2
- *CHEMICAL ABSTRACTS,* 1989606-61-2
- *CHEMICAL ABSTRACTS,* 1989604-82-1
- *CHEMICAL ABSTRACTS,* 1989605-46-0
- *CHEMICAL ABSTRACTS,* 1989605-99-3
- *CHEMICAL ABSTRACTS,* 1989606-62-3
- *CHEMICAL ABSTRACTS,* 1989604-83-2
- *CHEMICAL ABSTRACTS,* 1989605-47-1
- *CHEMICAL ABSTRACTS,* 1989606-00-9
- *CHEMICAL ABSTRACTS,* 1989606-63-4
- *CHEMICAL ABSTRACTS,* 1989604-84-3
- *CHEMICAL ABSTRACTS,* 1989605-48-2
- *CHEMICAL ABSTRACTS,* 1989606-01-0
- *CHEMICAL ABSTRACTS,* 1989606-67-8
- *CHEMICAL ABSTRACTS,* 1989604-85-4
- *CHEMICAL ABSTRACTS,* 1989605-49-3
- *CHEMICAL ABSTRACTS,* 1989606-04-3
- *CHEMICAL ABSTRACTS,* 1989606-69-0
- *CHEMICAL ABSTRACTS,* 1989604-86-5
- *CHEMICAL ABSTRACTS,* 1989605-50-6
- *CHEMICAL ABSTRACTS,* 1989606-05-4
- *CHEMICAL ABSTRACTS,* 1989606-70-3
- *CHEMICAL ABSTRACTS,* 1989604-87-6
- *CHEMICAL ABSTRACTS,* 1989605-51-7
- *CHEMICAL ABSTRACTS,* 1989606-06-5
- *CHEMICAL ABSTRACTS,* 1989606-74-7
- *CHEMICAL ABSTRACTS,* 1989658-39-0
- *CHEMICAL ABSTRACTS,* 2088184-56-7
- *CHEMICAL ABSTRACTS,* 2088185-07-1
- *CHEMICAL ABSTRACTS,* 2088185-66-2
- *CHEMICAL ABSTRACTS,* 1989658-41-4
- *CHEMICAL ABSTRACTS,* 2088184-57-8
- *CHEMICAL ABSTRACTS,* 2088185-08-2
- *CHEMICAL ABSTRACTS,* 2088185-67-3
- *CHEMICAL ABSTRACTS,* 1989658-43-6
- *CHEMICAL ABSTRACTS,* 2088184-58-9
- *CHEMICAL ABSTRACTS,* 2088185-09-3
- *CHEMICAL ABSTRACTS,* 2088185-68-4
- *CHEMICAL ABSTRACTS,* 1989658-47-0
- *CHEMICAL ABSTRACTS,* 2088184-59-0
- *CHEMICAL ABSTRACTS,* 2088185-10-6
- *CHEMICAL ABSTRACTS,* 2088185-69-5
- *CHEMICAL ABSTRACTS,* 1989658-49-2
- *CHEMICAL ABSTRACTS,* 2088184-60-3
- *CHEMICAL ABSTRACTS,* 2088185-11-7
- *CHEMICAL ABSTRACTS,* 2088185-70-8
- *CHEMICAL ABSTRACTS,* 2088184-07-8
- *CHEMICAL ABSTRACTS,* 2088184-61-4
- *CHEMICAL ABSTRACTS,* 2088185-12-8
- *CHEMICAL ABSTRACTS,* 2088185-71-9
- *CHEMICAL ABSTRACTS,* 2088184-08-9
- *CHEMICAL ABSTRACTS,* 2088184-62-5
- *CHEMICAL ABSTRACTS,* 2088185-13-9
- *CHEMICAL ABSTRACTS,* 2088185-72-0
- *CHEMICAL ABSTRACTS,* 2088184-09-0
- *CHEMICAL ABSTRACTS,* 2088184-63-6
- *CHEMICAL ABSTRACTS,* 2088185-14-0
- *CHEMICAL ABSTRACTS,* 2088185-73-1
- *CHEMICAL ABSTRACTS,* 2088184-10-3
- *CHEMICAL ABSTRACTS,* 2088184-64-7
- *CHEMICAL ABSTRACTS,* 2088185-15-1
- *CHEMICAL ABSTRACTS,* 2088185-74-2
- *CHEMICAL ABSTRACTS,* 2088184-11-4
- *CHEMICAL ABSTRACTS,* 2088184-65-8
- *CHEMICAL ABSTRACTS,* 2088185-16-2
- *CHEMICAL ABSTRACTS,* 2088185-75-3
- *CHEMICAL ABSTRACTS,* 2088184-13-6
- *CHEMICAL ABSTRACTS,* 2088184-66-9
- *CHEMICAL ABSTRACTS,* 2088185-17-3
- *CHEMICAL ABSTRACTS,* 2088185-76-4
- *CHEMICAL ABSTRACTS,* 2088184-14-7
- *CHEMICAL ABSTRACTS,* 2088184-67-0
- *CHEMICAL ABSTRACTS,* 2088185-18-4
- *CHEMICAL ABSTRACTS,* 2088185-77-5
- *CHEMICAL ABSTRACTS,* 2088184-15-8
- *CHEMICAL ABSTRACTS,* 2088184-68-1
- *CHEMICAL ABSTRACTS,* 2088185-19-5
- *CHEMICAL ABSTRACTS,* 2088185-78-6
- *CHEMICAL ABSTRACTS,* 2088184-16-9
- *CHEMICAL ABSTRACTS,* 2088184-69-2
- *CHEMICAL ABSTRACTS,* 2088185-20-8
- *CHEMICAL ABSTRACTS,* 2088185-79-7
- *CHEMICAL ABSTRACTS,* 2088184-17-0
- *CHEMICAL ABSTRACTS,* 2088184-70-5
- *CHEMICAL ABSTRACTS,* 2088185-21-9
- *CHEMICAL ABSTRACTS,* 2088185-80-0
- *CHEMICAL ABSTRACTS,* 2088184-18-1
- *CHEMICAL ABSTRACTS,* 2088184-71-6
- *CHEMICAL ABSTRACTS,* 2088185-22-0
- *CHEMICAL ABSTRACTS,* 2088185-81-1
- *CHEMICAL ABSTRACTS,* 2088184-19-2
- *CHEMICAL ABSTRACTS,* 2088184-72-7

- *CHEMICAL ABSTRACTS,* 2088185-23-1
- *CHEMICAL ABSTRACTS,* 2088185-82-2
- *CHEMICAL ABSTRACTS,* 2088184-20-5
- *CHEMICAL ABSTRACTS,* 2088184-73-8
- *CHEMICAL ABSTRACTS,* 2088185-32-2
- *CHEMICAL ABSTRACTS,* 2088185-83-3
- *CHEMICAL ABSTRACTS,* 2088184-21-6
- *CHEMICAL ABSTRACTS,* 2088184-74-9
- *CHEMICAL ABSTRACTS,* 2088185-33-3
- *CHEMICAL ABSTRACTS,* 2088185-84-4
- *CHEMICAL ABSTRACTS,* 2088184-22-7
- *CHEMICAL ABSTRACTS,* 2088184-75-0
- *CHEMICAL ABSTRACTS,* 2088185-34-4
- *CHEMICAL ABSTRACTS,* 2088185-85-5
- *CHEMICAL ABSTRACTS,* 2088184-23-8
- *CHEMICAL ABSTRACTS,* 2088184-76-1
- *CHEMICAL ABSTRACTS,* 2088185-35-5
- *CHEMICAL ABSTRACTS,* 2088185-86-6
- *CHEMICAL ABSTRACTS,* 2088184-24-9
- *CHEMICAL ABSTRACTS,* 2088184-77-2
- *CHEMICAL ABSTRACTS,* 2088185-36-6
- *CHEMICAL ABSTRACTS,* 2088185-87-7
- *CHEMICAL ABSTRACTS,* 2088184-25-0
- *CHEMICAL ABSTRACTS,* 2088184-78-3
- *CHEMICAL ABSTRACTS,* 2088185-37-7
- *CHEMICAL ABSTRACTS,* 2088185-88-8
- *CHEMICAL ABSTRACTS,* 2088184-26-1
- *CHEMICAL ABSTRACTS,* 2088184-79-4
- *CHEMICAL ABSTRACTS,* 2088185-38-8
- *CHEMICAL ABSTRACTS,* 2088185-89-9
- *CHEMICAL ABSTRACTS,* 2088184-27-2
- *CHEMICAL ABSTRACTS,* 2088184-80-7
- *CHEMICAL ABSTRACTS,* 2088185-39-9
- *CHEMICAL ABSTRACTS,* 2088185-90-2
- *CHEMICAL ABSTRACTS,* 2088184-28-3
- *CHEMICAL ABSTRACTS,* 2088184-81-8
- *CHEMICAL ABSTRACTS,* 2088185-40-2
- *CHEMICAL ABSTRACTS,* 2088185-91-3
- *CHEMICAL ABSTRACTS,* 2088184-29-4
- *CHEMICAL ABSTRACTS,* 2088184-82-9
- *CHEMICAL ABSTRACTS,* 2088185-41-3
- *CHEMICAL ABSTRACTS,* 2088185-92-4
- *CHEMICAL ABSTRACTS,* 2088184-30-7
- *CHEMICAL ABSTRACTS,* 2088184-83-0
- *CHEMICAL ABSTRACTS,* 2088185-42-4
- *CHEMICAL ABSTRACTS,* 2088185-93-5
- *CHEMICAL ABSTRACTS,* 2088184-32-9
- *CHEMICAL ABSTRACTS,* 2088184-84-1
- *CHEMICAL ABSTRACTS,* 2088185-43-5
- *CHEMICAL ABSTRACTS,* 2088185-94-6
- *CHEMICAL ABSTRACTS,* 2088184-34-1
- *CHEMICAL ABSTRACTS,* 2088184-85-2
- *CHEMICAL ABSTRACTS,* 2088185-44-6
- *CHEMICAL ABSTRACTS,* 2088185-95-7
- *CHEMICAL ABSTRACTS,* 2088184-35-2
- *CHEMICAL ABSTRACTS,* 2088184-86-3
- *CHEMICAL ABSTRACTS,* 2088185-45-7
- *CHEMICAL ABSTRACTS,* 2088185-96-8
- *CHEMICAL ABSTRACTS,* 2088184-36-3
- *CHEMICAL ABSTRACTS,* 2088184-87-4
- *CHEMICAL ABSTRACTS,* 2088185-46-8
- *CHEMICAL ABSTRACTS,* 2088185-97-9
- *CHEMICAL ABSTRACTS,* 2088184-37-4
- *CHEMICAL ABSTRACTS,* 2088184-88-5
- *CHEMICAL ABSTRACTS,* 2088185-47-9
- *CHEMICAL ABSTRACTS,* 2088185-98-0
- *CHEMICAL ABSTRACTS,* 2088184-38-5
- *CHEMICAL ABSTRACTS,* 2088184-89-6
- *CHEMICAL ABSTRACTS,* 2088185-48-0
- *CHEMICAL ABSTRACTS,* 2088185-99-1
- *CHEMICAL ABSTRACTS,* 2088184-39-6
- *CHEMICAL ABSTRACTS,* 2088184-90-9
- *CHEMICAL ABSTRACTS,* 2088185-49-1
- *CHEMICAL ABSTRACTS,* 2088186-00-7
- *CHEMICAL ABSTRACTS,* 2088184-40-9
- *CHEMICAL ABSTRACTS,* 2088184-91-0
- *CHEMICAL ABSTRACTS,* 2088185-50-4
- *CHEMICAL ABSTRACTS,* 2088186-01-8
- *CHEMICAL ABSTRACTS,* 2088184-41-0
- *CHEMICAL ABSTRACTS,* 2088184-92-1
- *CHEMICAL ABSTRACTS,* 2088185-51-5
- *CHEMICAL ABSTRACTS,* 2088186-02-9
- *CHEMICAL ABSTRACTS,* 2088184-42-1
- *CHEMICAL ABSTRACTS,* 2088184-93-2
- *CHEMICAL ABSTRACTS,* 2088185-52-6
- *CHEMICAL ABSTRACTS,* 2088195-88-2
- *CHEMICAL ABSTRACTS,* 2088184-43-2
- *CHEMICAL ABSTRACTS,* 2088184-94-3
- *CHEMICAL ABSTRACTS,* 2088185-53-7
- *CHEMICAL ABSTRACTS,* 2088195-89-3
- *CHEMICAL ABSTRACTS,* 2088184-44-3
- *CHEMICAL ABSTRACTS,* 2088184-95-4
- *CHEMICAL ABSTRACTS,* 2088185-54-8
- *CHEMICAL ABSTRACTS,* 2088195-90-6
- *CHEMICAL ABSTRACTS,* 2088184-45-4
- *CHEMICAL ABSTRACTS,* 2088184-96-5
- *CHEMICAL ABSTRACTS,* 2088185-55-9
- *CHEMICAL ABSTRACTS,* 2088195-91-7
- *CHEMICAL ABSTRACTS,* 2088184-46-5
- *CHEMICAL ABSTRACTS,* 2088184-97-6
- *CHEMICAL ABSTRACTS,* 2088185-56-0
- *CHEMICAL ABSTRACTS,* 861806-70-4
- *CHEMICAL ABSTRACTS,* 2088184-47-6
- *CHEMICAL ABSTRACTS,* 2088184-98-7
- *CHEMICAL ABSTRACTS,* 2088185-57-1
- *CHEMICAL ABSTRACTS,* 2088184-48-7
- *CHEMICAL ABSTRACTS,* 2088184-99-8
- *CHEMICAL ABSTRACTS,* 2088185-58-2
- *CHEMICAL ABSTRACTS,* 2088184-49-8
- *CHEMICAL ABSTRACTS,* 2088185-00-4
- *CHEMICAL ABSTRACTS,* 2088185-59-3
- *CHEMICAL ABSTRACTS,* 2088184-50-1
- *CHEMICAL ABSTRACTS,* 2088185-01-5
- *CHEMICAL ABSTRACTS,* 2088185-60-6
- *CHEMICAL ABSTRACTS,* 2088184-51-2
- *CHEMICAL ABSTRACTS,* 2088185-02-6

- *CHEMICAL ABSTRACTS,* 2088185-61-7
- *CHEMICAL ABSTRACTS,* 2088184-52-3
- *CHEMICAL ABSTRACTS,* 2088185-03-7
- *CHEMICAL ABSTRACTS,* 2088185-62-8
- *CHEMICAL ABSTRACTS,* 2088184-53-4
- *CHEMICAL ABSTRACTS,* 2088185-04-8
- *CHEMICAL ABSTRACTS,* 2088185-63-9
- *CHEMICAL ABSTRACTS,* 2088184-54-5
- *CHEMICAL ABSTRACTS,* 2088185-05-9
- *CHEMICAL ABSTRACTS,* 2088185-64-0
- *CHEMICAL ABSTRACTS,* 2088184-55-6
- *CHEMICAL ABSTRACTS,* 2088185-06-0
- *CHEMICAL ABSTRACTS,* 2088185-65-1
- **Y. SHIROTA et al.** *Chem. Rev.,* 2007, vol. 107 (4), 953-1010
- **M. S. ARNOLD et al.** *Appl. Phys. Lett.,* 2008, vol. 92, 053301
- *CHEMICAL ABSTRACTS,* 1224447-88-4
- *CHEMICAL ABSTRACTS,* 2225909-61-3
- *CHEMICAL ABSTRACTS,* 2239299-50-2
- *CHEMICAL ABSTRACTS,* 1028648-22-7
- *CHEMICAL ABSTRACTS,* 1240963-55-6
- *CHEMICAL ABSTRACTS,* 854952-60-6
- *CHEMICAL ABSTRACTS,* 1370555-65-9
- *CHEMICAL ABSTRACTS,* 1333002-41-7
- *CHEMICAL ABSTRACTS,* 2128690-41-3
- *CHEMICAL ABSTRACTS,* 2144800-21-3
- *CHEMICAL ABSTRACTS,* 854952-58-2
- *CHEMICAL ABSTRACTS,* 1373359-67-1
- *CHEMICAL ABSTRACTS,* 2055864-36-1
- *CHEMICAL ABSTRACTS,* 1416814-68-0
- *CHEMICAL ABSTRACTS,* 1361094-91-8
- *CHEMICAL ABSTRACTS,* 1174032-92-8
- *CHEMICAL ABSTRACTS,* 2142681-84-1
- *CHEMICAL ABSTRACTS,* 2226747-65-3
- *CHEMICAL ABSTRACTS,* 1883265-32-4
- *CHEMICAL ABSTRACTS,* 1618107-00-8
- *CHEMICAL ABSTRACTS,* 2074632-09-8
- *CHEMICAL ABSTRACTS,* 2172944-02-2
- *CHEMICAL ABSTRACTS,* 2142681-84
- *CHEMICAL ABSTRACTS,* 2260688-95-5
- *CHEMICAL ABSTRACTS,* 1472729-25-1
- *CHEMICAL ABSTRACTS,* 2251105-15-2
- *CHEMICAL ABSTRACTS,* 1421599-32-7
- *CHEMICAL ABSTRACTS,* 2299272-36-7
- *CHEMICAL ABSTRACTS,* 2286234-09-9
- *CHEMICAL ABSTRACTS,* 1428551-28-3
- *CHEMICAL ABSTRACTS,* 1454807-26-1
- *CHEMICAL ABSTRACTS,* 1153-85-1
- *CHEMICAL ABSTRACTS,* 109589-98-2
- *CHEMICAL ABSTRACTS,* 1028648-22
- *CHEMICAL ABSTRACTS,* 2108078-02-8
- *CHEMICAL ABSTRACTS,* 2108078-02
- *CHEMICAL ABSTRACTS,* 894791-46-9
- *CHEMICAL ABSTRACTS,* 1174032-85-9
- *CHEMICAL ABSTRACTS,* 1174032-81-5
- *CHEMICAL ABSTRACTS,* 94994-62-4
- *CHEMICAL ABSTRACTS,* 858507-84-3